# EUROPEAN PATENT APPLICATION

(11) **EP 4 378 931 A1**
(43) Date of publication of application: **05.06.2024**
(21) Application number: 22859785.2
(22) Date of filing: 17.01.2022
(51) Int. Cl.: C07D 249/12, A61K 31/4196, A61P 3/00

(54) **TRIAZOLONE COMPOUND AND MEDICINAL USE THEREOF**

(30) Priority: 23.08.2021 CN 202110970497; 04.01.2022 CN 202210004501
(71) Applicant: Harbin Medisan Pharmaceutical Co., LTD., Harbin, Heilongjiang 150025 (CN)
(72) Inventor: DAI, Liang, Nanjing, Jiangsu 211198 (CN); SUN, Hongbin, Nanjing, Jiangsu 211198 (CN); FENG, Zhiqi, Nanjing, Jiangsu 211198 (CN); XIANG, Jiehao, Nanjing, Jiangsu 211198 (CN); LIU, Hui, Nanjing, Jiangsu 211198 (CN); XU, Xiangrui, Nanjing, Jiangsu 211198 (CN); SUN, Gang, Nanjing, Jiangsu 211198 (CN); LI, Jiaxin, Nanjing, Jiangsu 211198 (CN); GU, Yuhao, Nanjing, Jiangsu 211198 (CN); ZHANG, Shangran, Nanjing, Jiangsu 211198 (CN); YUAN, Haoliang, Nanjing, Jiangsu 211198 (CN)
(74) Representative: Sun, Yiming
(86) International application number: PCT/CN2022/072221
(87) International publication number: WO 2023/024425

(57) **Abstract**

The present invention discloses a triazolone compound of formula (I) and pharmaceutical use thereof. Such compounds have a strong agonistic effect on PPARα and PPARδ, so the compounds or the pharmaceutically acceptable salts, the tautomers, the mesomers, the racemates, the stereoisomers, the metabolites, the metabolic precursors, the prodrugs or the solvates thereof can be applied to the preparation of a PPARα/δ dual agonist for preventing or treating diseases mediated by PPARα and/or PPARδ.

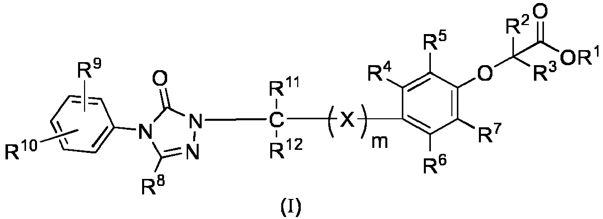

## Description

### TECHNICAL FIELD

The present invention belongs to the field of biomedicines, and particularly relates to a triazolone compound with dual agonistic activity for PPARα/δ and pharmaceutical use thereof as a PPARα/δ dual agonist.

### BACKGROUND

Peroxisome proliferator-activated receptors (PPARs) are a class of nuclear receptors that are critical in the regulation of homeostasis in the body. Activation of PPARs is dependent on the regulation of ligands. After PPARs are activated by the ligands, the ligand-activated transcription factors PPARs form heterodimers with a retinol X receptor (RXR) and bind to a specific DNA sequence PPRE to regulate the transcription of a target gene, thereby exerting biological effects (Nat. Rev. Immunol., 2006, 6, 44). PPARs have three subtypes, i.e., PPARα, PPARδ, and PPARγ, which have different tissue distributions. Activation of PPARs has a potentially positive effect on the improvement of metabolic diseases, cardiovascular and cerebrovascular diseases, inflammatory diseases, autoimmune diseases, organ fibrosis diseases, neurological injury diseases, secondary diseases caused by pathogen infections, mitochondrial dysfunction and disorders, or tumors (Nature, 2000, 405, 421; J. Neurochem., 2008, 107, 497; Mol. Cells., 2012, 33, 217; J. Biomed. Sci., 2017, 24, 5; Eur. J. Med. Chem., 2019, 166, 502). The development and application of PPAR agonists is a potential therapeutic strategy for intervention in the diseases described above. However, agonism of PPARγ has been shown to have a potential cardiac risk, leaving considerations on safety of its agonists. Therefore, the development of selective PPARα/δ dual agonists may offer new possibilities for the treatment of the diseases described above.

There is currently no PPARα/δ dual agonist on the market. The interim analysis results of an anti-nonalcoholic steatohepatitis (NASH) phase III clinical trial showed that the clinically investigational PPARα/δ dual agonist GFT505 (Elafibranor) was substantially ineffective (NCT02704403). The problems of weak agonistic activity and poor metabolic stability of GFT505 are found after the druggability analysis, which greatly limit the clinical application of GFT505 and may be an important reason that the anti-NASH phase III clinical trial on GFT505 did not achieve the expected effect. NASH, as a complex disease that seriously jeopardizes human health, has become a common cause of end-stage liver diseases and primary liver cancer, and has gradually replaced viral hepatitis as the leading cause of liver transplantation. Unfortunately, to date, there is no specific treatment method for NASH (Nat. Rev. Endocrinol., 2017, 13, 36). The potential effects of agonism of PPARα and PPARδ may counteract the development of NASH in a number of ways (Nat. Rev. Gastroenterol. Hepatol., 2021, 18, 24). Thus, PPARα/δ dual agonists with high activity and stable metabolism may be an important means of treating this disease.

In conclusion, there is an urgent need clinically to develop a novel PPARα/δ dual agonist with high activity and stable metabolism for the treatment of diseases mediated by PPARα and PPARδ, such as metabolic diseases, cardiovascular and cerebrovascular diseases, inflammatory diseases, autoimmune diseases, organ fibrosis diseases, neurological injury diseases, secondary diseases caused by pathogen infections, mitochondrial dysfunction and disorder diseases, or tumors; especially diseases with complex pathogenic causes, such as nonalcoholic fatty liver disease, alcoholic fatty liver disease, diabetes and its complications, dyslipidemia, obesity, atherosclerosis, cholestatic liver disease, neurodegenerative disease, and Duchenne muscular dystrophy.

### SUMMARY

Objective: in order to solve the problem of lack of effective PPARα/δ dual agonists clinically at present, the present invention provides a novel triazolone compound. The compound of the present invention has potent agonistic effects on PPARα and PPARδ, has good selectivity for PPARγ, and has good pharmacokinetic properties. Therefore, the compound and the pharmaceutically acceptable salt, the tautomer, the mesomer, the racemate, the stereoisomer, the metabolite, the metabolic precursor, the prodrug or the solvate of the present invention can be used for preparing a PPARα/δ dual agonist.

Another objective of the present invention is to provide pharmaceutical use of the triazolone compound as a PPARα/δ dual agonist. The compound and the pharmaceutically acceptable salt, the tautomer, the mesomer, the racemate, the stereoisomer, the metabolite, the metabolic precursor, the prodrug or the solvate thereof can be applied to the preparation of a PPARα/δ dual agonist, and can be used for preparing a medicament for preventing or treating diseases mediated by PPARα and/or PPARδ.

In order to achieve the above objectives, the present invention provides the following technical solutions:
The present invention provides a triazolone compound of formula (I) or a pharmaceutically acceptable salt or a solvate thereof: 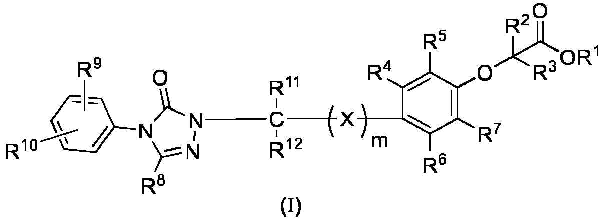
R¹ is selected from: H, linear or branched C1-C6 alkyl, C3-C6 cycloalkyl, (CH₂)ₚOR¹⁴, or (CH₂)_{q}NR¹⁵; wherein p = any integer from 2 to 6; q = any integer from 2 to 6; R¹⁴ and R¹⁵ are each independently selected from H, R¹⁶, and C(O)R¹⁷; wherein R¹⁶ and R¹⁷ are each independently selected from linear or branched C1-C6 alkyl or C3-C6 cycloalkyl;
R² and R³ are each independently selected from: H or linear or branched C1-C4 alkyl; or R² and R³, together with the carbon atoms to which they are bonded, form a 3- to 6-membered cycloalkyl ring;
R⁴, R⁵, R⁶, and R⁷ are each independently selected from: H, halogen, OR¹³, hydroxy, linear or branched C1-C4 alkyl, trifluoromethyl, methylthio, trifluoromethoxy, trifluoromethylthio, C3-C6 cycloalkyl, cycloalkenyl, heterocycloalkyl, heterocycloalkenyl, alkynyl, phenyl, substituted phenyl, heteroaryl, substituted heteroaryl, fused aryl, or substituted fused aryl; or at least two substituents of R⁴, R⁵, R⁶, and R⁷, together with the atoms to which they are attached, may form a substituted or unsubstituted benzene ring, a substituted or unsubstituted heteroaromatic ring, a substituted or unsubstituted cycloalkane ring, a substituted or unsubstituted heterocycloalkane ring, or a substituted or unsubstituted heterocycloalkene ring;
R¹³ is selected from: linear or branched C1-C4 alkyl, C3-C6 cycloalkyl, hydroxyalkyl, alkoxyalkyl, alkoxyalkoxyalkyl, cycloalkyl, or alkynylalkoxyalkyl;
X is selected from CH₂, O, or S;
m is selected from any integer from 0 to 4;
R⁸ is selected from H or C₁-C₄ alkyl;
R⁹ and R¹⁰ are independently selected from: H, hydroxy, halogen, cyano, linear or branched C1-C4 alkyl, trifluoromethyl, methylthio, trifluoromethoxy, trifluoromethylthio, alkylsulfonyl, alkoxy, cycloalkyl, cycloalkenyl, heterocycloalkyl, heterocycloalkenyl, alkynyl, phenyl, substituted phenyl, phenoxy, substituted phenyloxy, heteroaryl, substituted heteroaryl, fused aryl, or substituted fused aryl, wherein the substituted phenyl may independently be substituted with 1 to 2 of the following substituents: halogen, hydroxy, cyano, linear or branched C1-C4 alkyl, trifluoromethyl, methylthio, trifluoromethoxy, trifluoromethylthio, or alkylsulfonyl; or R⁹ and R¹⁰, together with the atoms to which they are attached, may form a substituted or unsubstituted benzene ring, a substituted or unsubstituted heteroaromatic ring, a substituted or unsubstituted cycloalkane ring, a substituted or unsubstituted heterocycloalkane ring, or a substituted or unsubstituted heterocycloalkene ring;
R¹¹ and R¹² are each independently selected from: H, C1-C4 linear or branched alkyl, or halogen; or R¹¹ and R¹², together with the carbon atoms to which they are bonded, form a 3- to 6-membered cycloalkyl ring.

In certain preferred embodiments, provided is the triazolone compound of formula (I) or the pharmaceutically acceptable salt or the solvate thereof:
R¹ is selected from: H, linear or branched C1-C4 alkyl, alkoxyalkyl, or acetamidoethyl;
R² and R³ are each independently selected from: H or linear or branched C1-C4 alkyl; or R² and R³, together with the carbon atoms to which they are bonded, form a 3- to 6-membered cycloalkyl ring;
R⁴, R⁵, R⁶, and R⁷ are each independently selected from: H, halogen, trifluoromethyl, trifluoromethoxy, trifluoromethylthio, OR¹³, or linear or branched C1-C4 alkyl;
R¹³ is selected from: linear or branched C1-C4 alkyl, hydroxyalkyl, alkoxyalkyl, alkoxyalkoxyalkyl, cycloalkyl, or alkynylalkoxyalkyl;
X is selected from CH₂, O, or S;
m is selected from any integer from 0 to 2;
R⁸ is selected from H or linear or branched C1-C4 alkyl;
R⁹ and R¹⁰ are each independently selected from: H, halogen, cyano, linear or branched C1-C4 alkyl, trifluoromethyl, methylthio, trifluoromethoxy, trifluoromethylthio, alkylsulfonyl, alkoxy, cycloalkyl, cycloalkenyl, heterocycloalkyl, heterocycloalkenyl, alkynyl, phenyl, substituted phenyl, phenoxy, substituted phenyloxy, heteroaryl, substituted heteroaryl, fused aryl, or substituted fused aryl, wherein the substituted phenyl may independently be substituted with 1 to 2 of the following substituents: halogen, cyano, linear or branched C1-C4 alkyl, trifluoromethyl, methylthio, trifluoromethoxy, trifluoromethylthio, or alkylsulfonyl;
R¹¹ and R¹² are each independently selected from: H, deuterium, linear or branched C1-C4 alkyl, or halogen; or R¹¹ and R¹², together with the carbon atoms to which they are bonded, form a 3- to 6-membered cycloalkyl ring.

In certain preferred embodiments, the triazolone compound further includes a pharmaceutically acceptable salt, a tautomer, a mesomer, a racemate, a stereoisomer, a metabolite, a metabolic precursor, a prodrug, or a solvate thereof. The present invention provides a triazolone compound of formula (I) or a pharmaceutically acceptable salt, a tautomer, a mesomer, a racemate, a stereoisomer, a metabolite, a metabolic precursor, a prodrug or a solvate thereof.

In certain more preferred embodiments, the triazolone compound or the pharmaceutically acceptable salt, the tautomer, the mesomer, the racemate, the stereoisomer, the metabolite, the metabolic precursor, the prodrug or the solvate thereof of the present invention is any one of the compounds shown in Table 1 below:

**Table 1. Structures and names of compounds**

| Compound No. | Compound structure | Name |
|---|---|---|
| 1 | 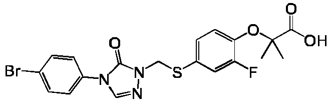 | 2-(4-(((4-(4-Bromophenyl)-5-oxo-4,5-dihydro-1*H*-1,2,4-triazol-1-yl)methyl)thio)-2-fluorophenoxy)-2-methylpropionic acid |
| 2 | 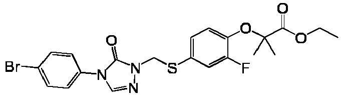 | Ethyl 2-(4-(((4-(4-bromophenyl)-5-oxo-4,5-dihydro-1*H*-1,2,4-triazol-1-yl)methyl)thio)-2-fluorophenoxy)ethyl-2-methylpropionate |
| 3 | 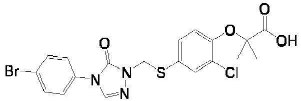 | 2-(4-(((4-(4-Bromophenyl)-5-oxo-4,5-dihydro-1*H*-1,2,4-triazol-1-yl)methyl)thio)-2-chlorophenoxy)-2-methylpropionic acid |
| 4 | 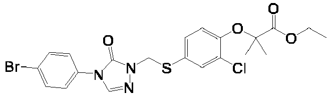 | Ethyl 2-(4-(((4-(4-bromophenyl)-5-oxo-4,5-dihydro-1*H*-1,2,4-triazol-1-yl)methyl)thio)-2-chlorophenoxy)ethyl-2-methylpropionate |
| 5 | 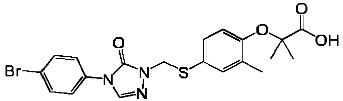 | 2-(4-(((4-(4-Bromophenyl)-5-oxo-4,5-dihydro-1*H*-1,2,4-triazol-1-yl)methyl)thio)-2-methylphenoxy)-2-methylpropionic acid |
| 6 | 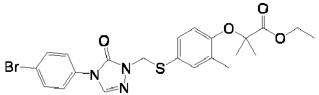 | Ethyl 2-(4-(((4-(4-bromophenyl)-5-oxo-4,5-dihydro-1*H*-1,2,4-triazol-1-yl)methyl)thio)-2-methylphenoxy)ethyl-2-methylpropionate |
| 7 | 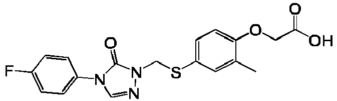 | 2-(4-(((4-(4-Fluorophenyl)-5-oxo-4,5-dihydro-1*H*-1,2,4-triazol-1-yl)methyl)thio)-2-methylphenoxy)acetic acid |
| 8 | 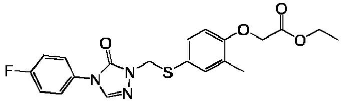 | Ethyl 2-(4-(((4-(4-fluorophenyl)-5-oxo-4,5-dihydro-1*H*-1,2,4-triazol-1-yl)methyl)thio)-2-methylphenoxy)acetate |
| 9 | 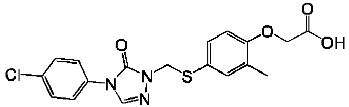 | 2-(4-(((4-(4-Chlorophenyl)-5-oxo-4,5-dihydro-1*H*-1,2,4-triazol-1-yl)methyl)thio)-2-methylphenoxy)acetic acid |
| 10 | 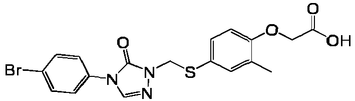 | 2-(4-(((4-(4-Bromophenyl)-5-oxo-4,5-dihydro-1*H*-1,2,4-triazol-1-yl)methyl)thio)-2-methylphenoxy)acetic acid |
| 11 | 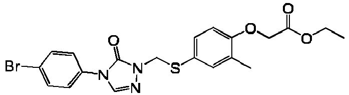 | Ethyl 2-(4-(((4-(4-bromophenyl)-5-oxo-4,5-dihydro-1*H*-1,2,4-triazol-1-yl)methyl)thio)-2-methylphenoxy)acetate |
| 12 | 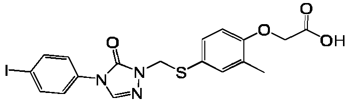 | 2-(4-(((4-(4-Iodophenyl)-5-oxo-4,5-dihydro-1*H*-1,2,4-triazol-1-yl)methyl)thio)-2-methylphenoxy)acetic acid |
| 13 | 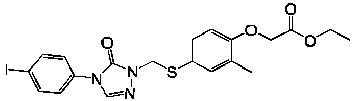 | Ethyl 2-(4-(((4-(4-iodophenyl)-5-oxo-4, 5-dihydro-1*H*-1,2,4-triazol-1-yl)methyl)thio)-2-methylphenoxy)acetate |
| 14 | 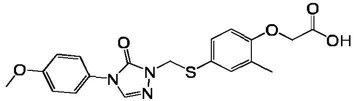 | 2-(4-(((4-(4-Methoxyphenyl)-5-oxo-4,5-dihydro-1*H*-1,2,4-triazol-1-yl)methyl)thio)-2-methylphenoxy)acetic acid |
| 15 | 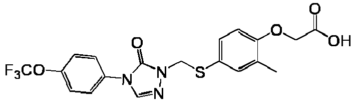 | 2-(2-Methyl-4-(((5-oxo-4-(4-(trifluoromethoxy)phenyl)-4,5-dihydro-1*H*-1,2,4-triazol-1-yl)methyl)thio)phenoxy)acetic acid |
| 16 | 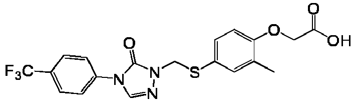 | 2-(2-Methyl-4-(((5-oxo-4-(4-(trifluoromethyl)phenyl)-4,5-dihydro-1*H*-1,2,4-triazol-1-yl)methyl)thio)phenoxy)acetic acid |
| 17 | 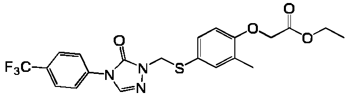 | Ethyl 2-(2-methyl-4-(((5-oxo-4-(4-(trifluoromethyl)phenyl)-4,5-dihydro-1*H*-1,2,4-triazol-1-yl)methyl)thio)phenoxy)acetate |
| 18 | 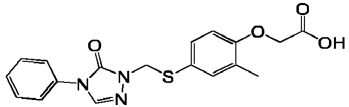 | 2-(2-Methyl-4-(((5-oxo-4-phenyl-4,5-dihydro-1*H*-1,2,4-triazol-1-yl)methyl)thio)phenoxy)acetic acid |
| 19 | 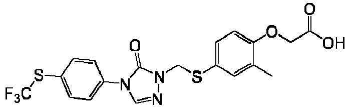 | 2-(2-Methyl-4-(((5-oxo-4-(4-((trifluoromethyl)thio)phenyl)-4,5-dihydro-1*H*-1,2,4-triazol-1-yl)4-methyl)thio)phenoxy)acetic acid |
| 20 | 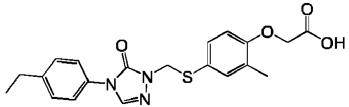 | 2-(4-(((4-(4-Ethylphenyl)-5-oxo-4,5-dihydro-1*H*-1,2,4-triazol-1-yl)methyl)thio)-2-methylphenoxy)acetic acid |
| 21 | 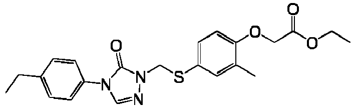 | Ethyl 2-(4-(((4-(4-ethylphenyl)-5-oxo-4, 5-dihydro-1*H*-1,2,4-triazol-1-yl)methyl)thio)-2-methylphenoxy)acetate |
| 22 | 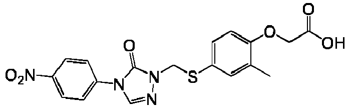 | 2-(2-Methyl-4-(((4-(4-nitrophenyl)-5-oxo-4,5-dihydro-1*H*-1,2,4-triazol-1-yl)methyl)thio]phenoxy)acetic acid |
| 23 | 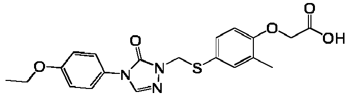 | 2-(4-(((4-(4-Ethoxyphenyl)-5-oxo-4,5-dihydro-1*H*-1,2,4-triazol-1-yl)methyl)thio)-2-methylphenoxy)acetic acid |
| 24 | 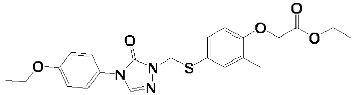 | Ethyl 2-(4-(((4-(4-ethoxyphenyl)-5-oxo-4,5-dihydro-1*H*-1,2,4-triazol-1-yl)methyl)thio)-2-methylphenoxy)acetate |
| 25 | 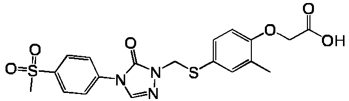 | 2-(2-Methyl-4-(((4-(4-(methylsulfonyl)phenyl)-5-oxo-4,5-dihydro-1*H*-1,2,4-triazol-1-yl)methyl]thio)phenoxy)acetate |
| 26 | 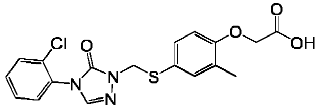 | 2-(4-(((4-(2-Chlorophenyl)-5-oxo-4,5-dihydro-1*H*-1,2,4-triazol-1-yl)methyl)thio)-2-methylphenoxy)acetic acid |
| 27 | 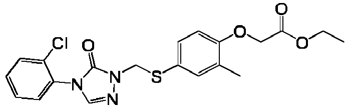 | Ethyl 2-(4-(((4-(2-chlorophenyl)-5-oxo-4,5-dihydro-1*H*-1,2,4-triazol-1-yl)methyl)thio)-2-methylphenoxy)acetate |
| 28 | 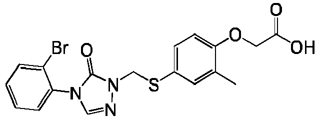 | 2-(4-(((4-(2-Bromophenyl)-5-oxo-4,5-dihydro-1*H*-1,2,4-triazol-1-yl)methyl)thio)-2-methylphenoxy)acetic acid |
| 29 | 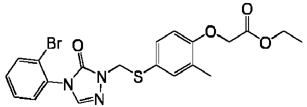 | Ethyl 2-(4-((4-(2-bromophenyl)-5-oxo-4,5-dihydro-1*H*-1,2,4-triazol-1-yl)methyl)thio)-2-methylphenoxy)acetate |
| 30 | 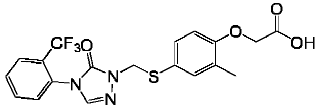 | 2-(2-Methyl-4-(((5-oxo-4-(2-(trifluoromethyl)phenyl)-4,5-dihydro-1*H*-1,2,4-triazol-1-yl)methyl)thio)phenoxy)acetic acid |
| 31 | 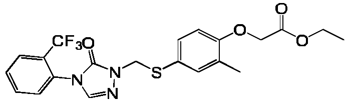 | Ethyl 2-(2-methyl-4-(((5-oxo-4-(2-(trifluoromethyl)phenyl)-4,5-dihydro-1*H*-1,2,4-triazol-1-yl)methyl)thio)phenoxy)acetate |
| 32 | 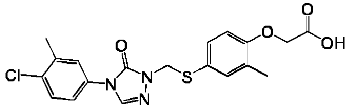 | 2-(4-(((4-(4-Chloro-3-methylphenyl)-5-oxo-4,5-dihydro-1*H*-1,2,4-triazol-1-yl)methyl)thio)-2-methylphenoxy)acetic acid |
| 33 | 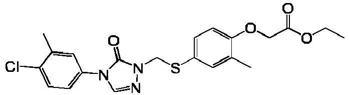 | Ethyl 2-(4-(((4-(4-chloro-3-methylphenyl)-5-oxo-4,5-dihydro-1*H*-1,2,4-triazol-1-yl)methyl)thio)-2-methylphenoxy)acetate |
| 34 | 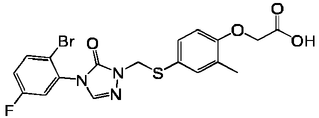 | 2-(4-(((4-(2-Bromo-5-fluorophenyl)-5-oxo-4,5-dihydro-1*H*-1,2,4-triazol-1-yl)methyl)thio)-2-methylphenoxy)acetic acid |
| 35 | 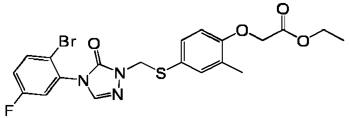 | Ethyl 2-(4-(((4-(2-bromo-5-fluorophenyl)-5-oxo-4,5-dihydro-1*H-*1,2,4-triazol-1-yl)methyl)thio)-2-methylphenoxy)acetate |
| 36 | 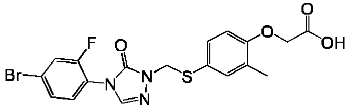 | 2-(4-(((4-(4-Bromo-2-fluorophenyl)-5-oxo-4,5-dihydro-1*H*-1,2,4-triazol-1-yl)methyl)thio)-2-methylphenoxy)acetic acid |
| 37 | 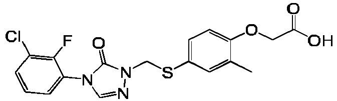 | 2-(4-(((4-(3-Chloro-2-fluorophenyl)-5-oxo-4,5-dihydro-1*H*-1,2,4-triazol-1-yl)methyl)thio)-2-methylphenoxy)acetic acid |
| 38 | 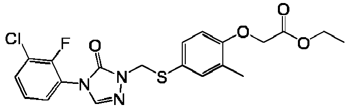 | Ethyl 2-(4-(((4-(3-chloro-2-fluorophenyl)-5-oxo-4,5-dihydro-1*H-*1,2,4-triazol-1-yl)methyl)thio)-2-methylphenoxy)acetate |
| 39 | 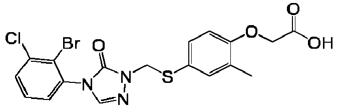 | 2-(4-(((4-(2-Bromo-3-chlorophenyl)-5-oxo-4,5-dihydro-1*H*-1,2,4-triazol-1-yl)methyl)thio)-2-methylphenoxy)acetic acid |
| 40 | 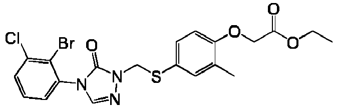 | Ethyl 2-(4-(((4-(2-bromo-3-chlorophenyl)-5-oxo-4,5-dihydro-1*H*-1,2,4-triazol-1-yl)methyl)thio)-2-methylphenoxy)acetate |
| 41 | 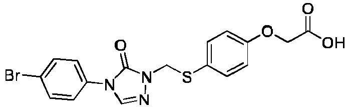 | 2-(4-(((4-(4-Bromophenyl)-5-oxo-4,5-dihydro-1*H*-1,2,4-triazol-1-yl)methyl)thio)phenoxy)acetic acid |
| 42 | 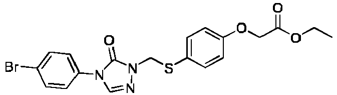 | Ethyl 2-(4-(((4-(4-bromophenyl)-5-oxo-4,5-dihydro-1*H*-1,2,4-triazol-1-yl)methyl)thio)phenoxy)acetate |
| 43 | 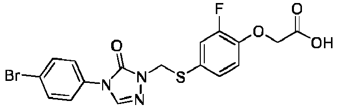 | 2-(4-(((4-(4-Bromophenyl)-5-oxo-4,5-dihydro-1*H*-1,2,4-triazol-1-yl)methyl)thio)-2-fluorophenoxy)acetic acid |
| 44 | 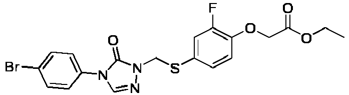 | Ethyl 2-(4-(((4-(4-bromophenyl)-5-oxo-4,5-dihydro-1*H*-1,2,4-triazol-1-yl)methyl)thio)-2-fluorophenoxy)acetate |
| 45 | 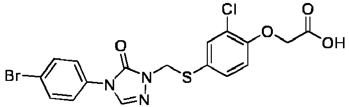 | 2-(4-(((4-(4-Bromophenyl)-5-oxo-4,5-dihydro-1*H*-1,2,4-triazol-1-yl)methyl)thio)-2-chlorophenoxy)acetic acid |
| 46 | 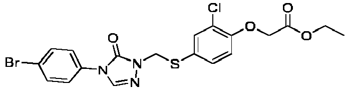 | Ethyl 2-(4-(((4-(4-bromophenyl)-5-oxo-4,5-dihydro-1*H-*1,2,4-triazol-1-yl)methyl)thio)-2-chlorophenoxy)acetate |
| 47 | 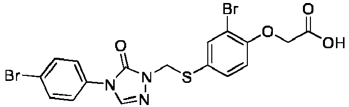 | 2-(2-Bromo-4-(((4-(4-bromophenyl)-5-oxo-4,5-dihydro-1*H*-1,2,4-triazol-1-yl)methyl)thio)phenoxy)acetic acid |
| 48 | 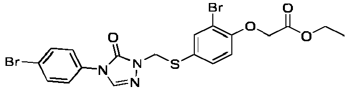 | Ethyl 2-(2-bromo-4-(((4-(4-bromophenyl)-5-oxo-4,5-dihydro-1*H*-1,2,4-triazol-1-yl)methyl)thio)phenoxy)acetate |
| 49 | 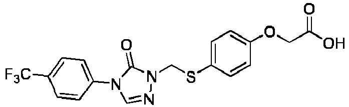 | 2-(4-(((5-Oxo-4-(4-(trifluoromethyl)phenyl)-4,5-dihydro-1*H*-1,2,4-triazol-1-yl)methyl)thio)phenoxy)acetic acid |
| 50 | 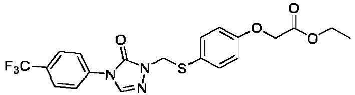 | Ethyl 2-(4-(((5-oxo-4-(4-(trifluoromethyl)phenyl)-4,5-dihydro-1*H*-1,2,4-triazol-1-yl)methyl)thio)phenoxy)acetate |
| 51 | 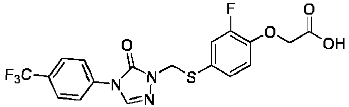 | 2-(2-Fluoro-4-(((5-oxo-4-(4-(trifluoromethyl)phenyl)-4,5-dihydro-1*H*-1,2,4-triazol-1-yl)methyl)thio)phenoxy)acetic acid |
| 52 | 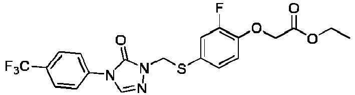 | Ethyl 2-(2-fluoro-4-(((5-oxo-4-(4-(trifluoromethyl)phenyl)-4,5-dihydro-1*H*-1,2,4-triazol-1-yl)methyl)thio)phenoxy)acetate |
| 53 | 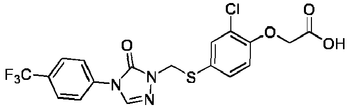 | 2-(2-Chloro-4-(((5-oxo-4-(4-(trifluoromethyl)phenyl)-4,5-dihydro-1*H*-1,2,4-triazol-1-yl)methyl)thio)phenoxy)acetic acid |
| 54 | 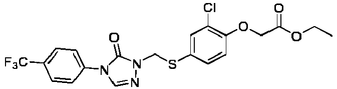 | Ethyl 2-(2-chloro-4-(((5-oxo-4-(4-(trifluoromethyl)phenyl)-4,5-dihydro-1*H*-1,2,4-triazol-1-yl)methyl)thio)phenoxy)acetate |
| 55 | 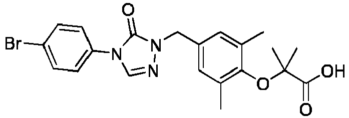 | 2-(4-((4-(4-Bromophenyl)-5-oxo-4,5-dihydro-1*H*-1,2,4-triazol-1-yl)methyl)-2,6-dimethylphenoxy)-2-methylpropionic acid |
| 56 | 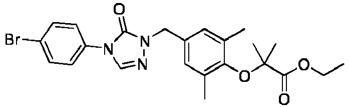 | Ethyl 2-(4-((4-(4-bromophenyl)-5-oxo-4,5-dihydro-1*H*-1,2,4-triazol-1-yl)methyl)-2,6-dimethylphenoxy)-2-methylpropionate |
| 57 | 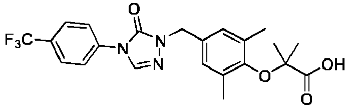 | 2-(2,6-Dimethyl-4-((5-oxo-4-(4-(trifluoromethyl)phenyl)-4,5-dihydro-1*H*-1,2,4-triazol-1-yl)methyl)phenoxy)-2-methylpropionic acid |
| 58 | 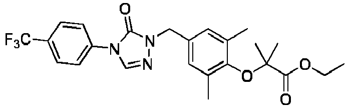 | Ethyl 2-(2,6-dimethyl-4-((5-oxo-4-(4-(trifluoromethyl)phenyl)-4,5-dihydro-1*H*-1,2,4-triazol-1-yl)methyl)phenoxy)-2-methylpropionate |
| 59 | 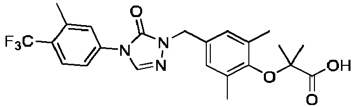 | 2-(2,6-Dimethyl-4-((4-(3-methyl-4-(trifluoromethyl)phenyl)-5-oxo-4,5-dihydro-1*H*-1,2,4-triazol-1-yl)methyl)phenoxy)-2-methylpropionic acid |
| 60 | 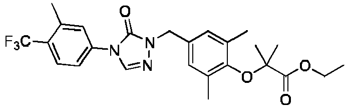 | Ethyl 2-(2,6-dimethyl-4-((4-(3-methyl-4-(trifluoromethyl)phenyl)-5-oxo-4,5-dihydro-1*H*-1,2,4-triazol-1-yl)methyl)phenoxy)-2-methylpropionate |
| 61 | 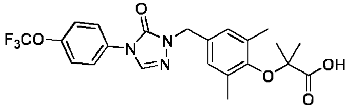 | 2-(2,6-Dimethyl-4-((5-oxo-4-(4-(trifluoromethoxy)phenyl)-4,5-dihydro-1*H*-1,2,4-triazol-1-yl)methyl)phenoxy)-2-methylpropionic acid |
| 62 | 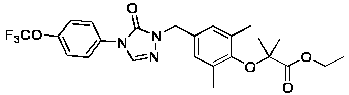 | Ethyl 2-(2,6-dimethyl-4-((5-oxo-4-(4-(trifluoromethoxy)phenyl)-4,5-dihydro-1*H*-1,2,4-triazol-1-yl)methyl)phenoxy)-2-methylpropionate |
| 63 | 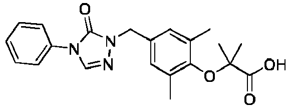 | 2-(2,6-Dimethyl-4-((5-oxo-4-phenyl-4,5-dihydro-1*H*-1,2,4-triazol-1-yl)methyl)phenoxy)-2-methylpropionic acid |
| 64 | 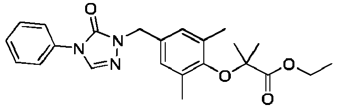 | Ethyl 2-(2,6-dimethyl-4-((5-oxo-4-phenyl-4,5-dihydro-1*H*-1,2,4-triazol-1-yl)methyl)phenoxy)-2-methylpropionate |
| 65 | 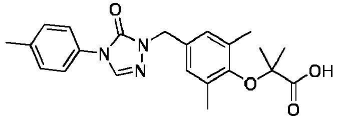 | 2-(2,6-Dimethyl-4-((5-oxo-4-(p-tolyl)-4,5-dihydro-1*H*-1,2,4-triazol-1-yl)methyl)phenoxy)-2-methylpropionic acid |
| 66 | 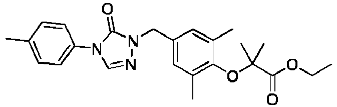 | Ethyl 2-(2,6-dimethyl-4-((5-oxo-4-(*p*-tolyl)-4,5-dihydro-1*H*-1,2,4-triazol-1-yl)methyl)phenoxy)-2-methylpropionate |
| 67 | 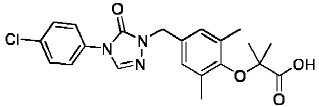 | 2-(4-((4-(4-Chlorophenyl)-5-oxo-4,5-dihydro-1*H*-1,2,4-triazol-1-yl)methyl)-2,6-dimethylphenoxy)-2-methylpropionic acid |
| 68 | 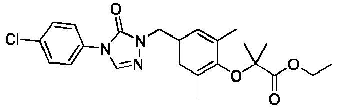 | Ethyl 2-(4-((4-(4-chlorophenyl)-5-oxo-4,5-dihydro-1*H*-1,2,4-triazol-1-yl)methyl)-2,6-dimethylphenoxy)-2-methylpropionate |
| 69 | 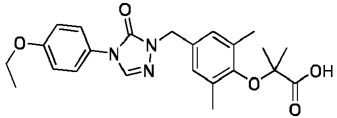 | 2-(4-((4-(4-Ethoxyphenyl)-5-oxo-4,5-dihydro-1*H*-1,2,4-triazol-1-yl)methyl)-2,6-dimethylphenoxy)-2-methylpropionic acid |
| 70 | 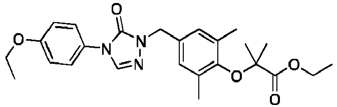 | Ethyl 2-(4-((4-(4-ethoxyphenyl)-5-oxo-4,5-dihydro-1*H*-1,2,4-triazol-1-yl)methyl)-2,6-dimethylphenoxy)-2-methylpropionate |
| 71 | 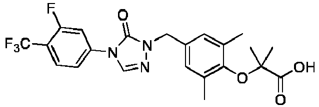 | 2-(4-((4-(3-Fluoro-4-(trifluoromethyl)phenyl)-5-oxo-4,5-dihydro-1*H*-1,2,4-triazol-1-yl)methyl)-2,6-dimethylphenoxy)-2-methylpropionic acid |
| 72 | 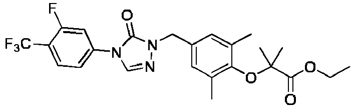 | Ethyl 2-(4-((4-(3-fluoro-4-(trifluoromethyl)phenyl)-5-oxo-4,5-dihydro-1*H*-1,2,4-triazol-1-yl)methyl)-2,6-dimethylphenoxy)-2-methylpropionate |
| 73 | 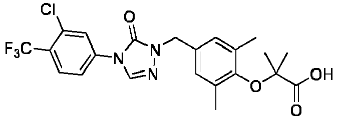 | 2-(4-((4-(3-Chloro-4-(trifluoromethyl)phenyl)-5-oxo-4,5-dihydro-1*H*-1,2,4-triazol-1-yl)methyl)-2,6-dimethylphenoxy)-2-methylpropionic acid |
| 74 | 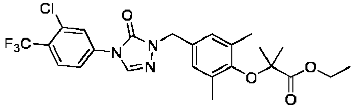 | Ethyl 2-(4-((4-(3-chloro-4-(trifluoromethyl)phenyl)-5-oxo-4,5-dihydro-1*H*-1,2,4-triazol-1-yl)methyl)-2,6-dimethylphenoxy)-2-methylpropionate |
| 75 | 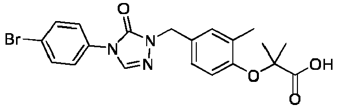 | 2-(4-((4-(4-Bromophenyl)-5-oxo-4,5-dihydro-1*H*-1,2,4-triazol-1-yl)methyl)-2-methylphenoxy)-2-methylpropionic acid |
| 76 | 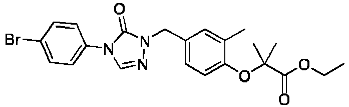 | Ethyl 2-(4-((4-(4-bromophenyl)-5-oxo-4,5-dihydro-1*H*-1,2,4-triazol-1-yl)methyl)-2-methylphenoxy)-2-methylpropionate |
| 77 | 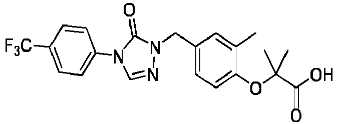 | 2-Methyl-2-(2-methyl-4-((5-oxo-4-(4-(trifluoromethyl)phenyl)-4,5-dihydro-1*H*-1,2,4-triazol-1-yl)methyl)phenoxy)propionic acid |
| 78 | 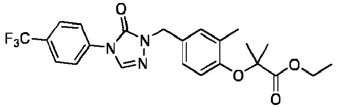 | Ethyl 2-methyl-2-(2-methyl-4-((5-oxo-4-(4-(trifluoromethyl)phenyl)-4,5-dihydro-1*H*-1,2,4-triazol-1-yl)methyl)phenoxy)propionate |
| 79 | 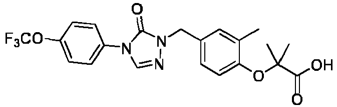 | 2-Methyl-2-(2-methyl-4-((5-oxo-4-(4-(trifluoromethoxy)phenyl)-4,5-dihydro-1*H*-1,2,4-triazol-1-yl)methyl)phenoxy)propionic acid |
| 80 | 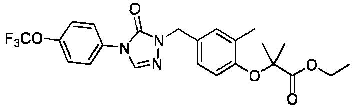 | Ethyl 2-methyl-2-(2-methyl-4-((5-oxo-4-(4-(trifluoromethoxy)phenyl)-4,5-dihydro-1*H*-1,2,4-triazol-1-yl)methyl)phenoxy)propionate |
| 81 | 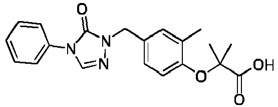 | 2-Methyl-2-(2-methyl-4-((5-oxo-4-phenyl-4,5-dihydro-1*H*-1,2,4-triazol-1-yl)methyl)phenoxy)propionic acid |
| 82 | 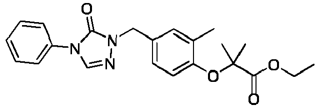 | Ethyl 2-methyl-2-(2-methyl-4-((5-oxo-4-phenyl-4,5-dihydro-1*H*-1,2,4-triazol-1-yl)methyl)phenoxy)propionate |
| 83 | 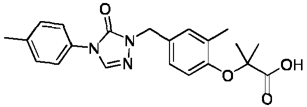 | 2-Methyl-2-(2-methyl-4-((5-oxo-4-(p-tolyl)-4,5-dihydro-1*H*-1,2,4-triazol-1-yl)methyl)phenoxy)propionate |
| 84 | 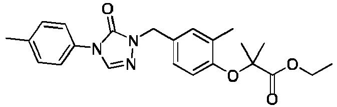 | Ethyl 2-methyl-2-(2-methyl-4-((5-oxo-4-(*p*-tolyl)-4,5-dihydro-1*H-*1,2,4-triazol-1-yl)methyl)phenoxy)propionate |
| 85 | 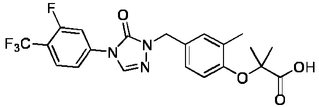 | 2-(4-((4-(3-Fluoro-4-(trifluoromethyl)phenyl)-5-oxo-4,5-dihydro-1*H*-1,2,4-triazol-1-yl)methyl)-2-methylphenoxy)-2-methylpropionic acid |
| 86 | 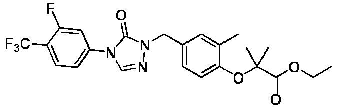 | Ethyl 2-(4-((4-(3-fluoro-4-(trifluoromethyl)phenyl)-5-oxo-4,5-dihydro-1*H*-1,2,4-triazol-1-yl)methyl)-2-methylphenoxy)-2-methylpropionate |
| 87 | 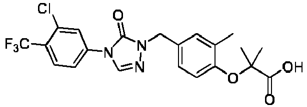 | 2-(4-((4-(3-Chloro-4-(trifluoromethyl)phenyl)-5-oxo-4,5-dihydro-1*H*-1,2,4-triazol-1-yl)methyl)-2-methylphenoxy)-2-methylpropionic acid |
| 88 | 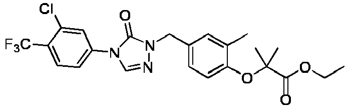 | Ethyl 2-(4-((4-(3-chloro-4-(trifluoromethyl)phenyl)-5-oxo-4,5-dihydro-1*H*-1,2,4-triazol-1-yl)methyl)-2-methylphenoxy)-2-methylpropionate |
| 89 | 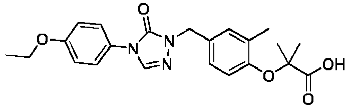 | 2-(4-((4-(4-Ethoxyphenyl)-5-oxo-4,5-dihydro-1*H*-1,2,4-triazol-1-yl)methyl)-2-methylphenoxy)-2-methylpropionic acid |
| 90 | 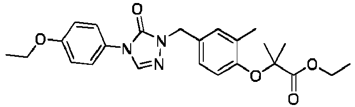 | Ethyl 2-(4-((4-(4-ethoxyphenyl)-5-oxo-4,5-dihydro-1*H*-1,2,4-triazol-1-yl)methyl)-2-methylphenoxy)-2-methylpropionate |
| 91 | 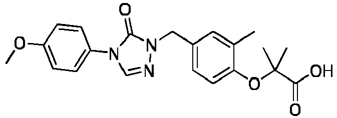 | 2-(4-((4-(4-Methoxyphenyl)-5-oxo-4,5-dihydro-1*H*-1,2,4-triazol-1-yl)methyl)-2-methylphenoxy)-2-methylpropionic acid |
| 92 | 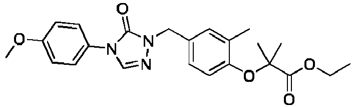 | Ethyl 2-(4-((4-(4-methoxyphenyl)-5-oxo-4,5-dihydro-1*H*-1,2,4-triazol-1-yl)methyl)-2-methylphenoxy)-2-methylpropionate |
| 93 | 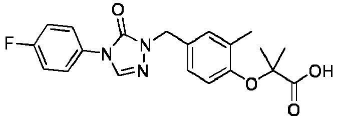 | 2-(4-((4-(4-Flourophenyl)-5-oxo-4,5-dihydro-1*H*-1,2,4-triazol-1-yl)methyl)-2-methylphenoxy)-2-methylpropionic acid |
| 94 | 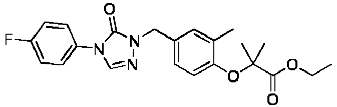 | Ethyl 2-(4-((4-(4-flourophenyl)-5-oxo-4,5-dihydro-1*H*-1,2,4-triazol-1-yl)methyl)-2-methylphenoxy)-2-methylpropionate |
| 95 | 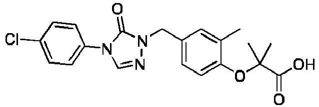 | 2-(4-((4-(4-Chlorophenyl)-5-oxo-4,5-dihydro-1*H*-1,2,4-triazol-1-yl)methyl)-2-methylphenoxy)-2-methylpropionic acid |
| 96 | 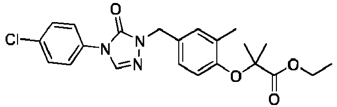 | Ethyl 2-(4-((4-(4-chlorophenyl)-5-oxo-4,5-dihydro-1*H*-1,2,4-triazol-1-yl)methyl)-2-methylphenoxy)-2-methylpropionate |
| 97 | 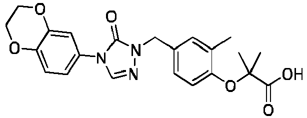 | 2-(4-((4-(2,3-Dihydrobenzo[*b*][1,4]dioxin-6-yl)-5-oxo-4,5-dihydro-1*H*-1,2,4-triazol-1-yl)methyl)-2-methylphenoxy)-2-methylpropionic acid |
| 98 | 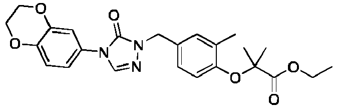 | Ethyl 2-(4-((4-(2,3-dihydrobenzo[*b*][1,4]dioxin-6-yl)-5-oxo-4,5-dihydro-1*H*-1,2,4-triazol-1-yl)methyl)-2-methylphenoxy)-2-methylpropionate |
| 99 | 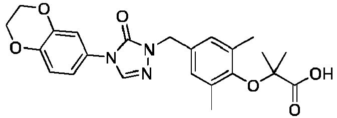 | 2-(4-((4-(2,3-Dihydrobenzo[*b*][1,4]dioxin-6-yl)-5-oxo-4,5-dihydro-1*H*-1,2,4-triazol-1-yl)methyl)-2,6-dimethylphenoxy)-2-methylpropionic acid |
| 100 | 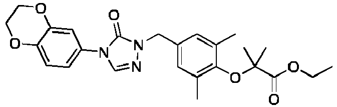 | Ethyl 2-(4-((4-(2,3-dihydrobenzo[*b*][1,4]dioxin-6-yl)-5-oxo-4,5-dihydro-1*H-*1,2,4-triazol-1-yl)methyl)-2,6-dimethylphenoxy)-2-methylpropionate |
| 101 | 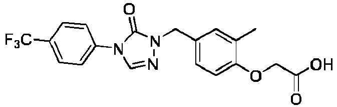 | 2-(2-Methyl-4-((5-oxo-4-(4-(trifluoromethyl)phenyl)-4,5-dihydro-1*H*-1,2,4-triazol-1-yl)methyl)phenoxyacetic acid |
| 102 | 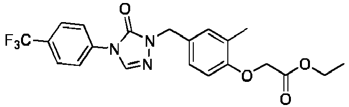 | Ethyl 2-(2-methyl-4-((5-oxo-4-(4-(trifluoromethyl)phenyl)-4,5-dihydro-1*H*-1,2,4-triazol-1-yl)methyl)phenoxyacetate |
| 103 | 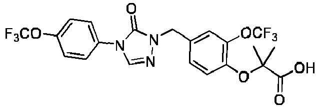 | 2-Methyl-2-(4-((5-oxo-4-(4-(trifluoromethoxy)phenyl)-4,5-dihydro-1*H*-1,2,4-triazol-1-yl)methyl)-2-(trifluoromethoxy)phenoxy)propionic acid |
| 104 | 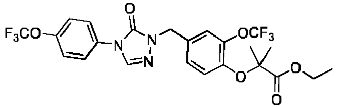 | Ethyl 2-methyl-2-(4-((5-oxo-4-(4-(trifluoromethoxy)phenyl)-4,5-dihydro-1*H*-1,2,4-triazol-1-yl)methyl)-2-(trifluoromethoxy)phenoxy)propionat e |
| 105 | 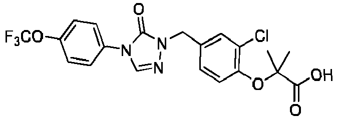 | 2-(2-Chloro-4-((5-oxo-4-(4-(trifluoromethoxy)phenyl)-4,5-dihydro-1*H*-1,2,4-triazol-1-yl)methyl)phenoxy)-2-methylpropionic acid |
| 106 | 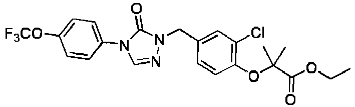 | Ethyl 2-(2-chloro-4-((5-oxo-4-(4-(trifluoromethoxy)phenyl)-4,5-dihydro-1*H*-1,2,4-triazol-1-yl)methyl)phenoxy)-2-methylpropionate |
| 107 | 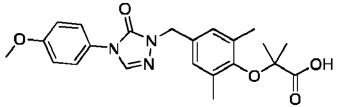 | 2-(4-((4-(4-Methoxyphenyl)-5-oxo-4,5-dihydro-1*H*-1,2,4-triazol-1- yl)methyl)-2,6-dimethylphenoxy)-2-methylpropionic acid |
| 108 | 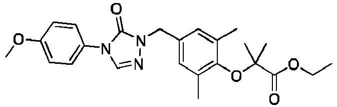 | Ethyl 2-(4-((4-(4-methoxyphenyl)-5-oxo-4,5-dihydro-1*H*-1,2,4-triazol-1-yl)methyl)-2,6-dimethylphenoxy)-2-methylpropionate |
| 109 | 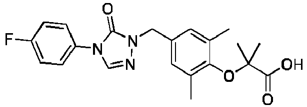 | 2-(4-((4-(4-Flourophenyl)-5-oxo-4,5-dihydro-1*H*-1,2,4-triazol-1-yl)methyl)-2,6-dimethylphenoxy)-2-methylpropionic acid |
| 110 | 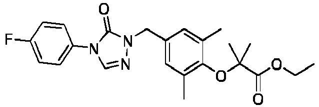 | Ethyl 2-(4-((4-(4-flourophenyl)-5-oxo-4,5-dihydro-1*H*-1,2,4-triazol-1-yl)methyl)-2,6-dimethylphenoxy)-2-methylpropionate |
| 111 | 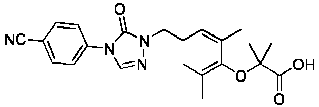 | 2-(4-((4-(4-Cyanophenyl)-5-oxo-4,5-dihydro-1*H*-1,2,4-triazol-1-yl)methyl)-2,6-dimethylphenoxy)-2-methylpropionic acid |
| 112 | 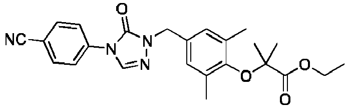 | Ethyl 2-(4-((4-(4-cyanophenyl)-5-oxo-4,5-dihydro-1*H*-1,2,4-triazol-1-yl)methyl)-2,6-dimethylphenoxy)-2-methylpropionate |
| 113 | 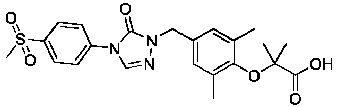 | 2-(2,6-Dimethyl-4-((4-(4-(methylsulfonyl)phenyl)-5-oxo-4,5-dihydro-1*H*-1,2,4-triazol-1-yl)methyl)phenoxy)-2-methylpropionic acid |
| 114 | 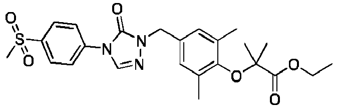 | Ethyl 2-(2,6-dimethyl-4-((4-(methylsulfonyl)phenyl)-5-oxo-4,5-dihydro-1*H*-1,2,4-triazol-1-yl)methyl)phenoxy)-2-methylpropionate |
| 115 | 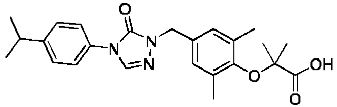 | 2-(4-((4-(4-Isopropylphenyl)-5-oxo-4,5-dihydro-1*H*-1,2,4-triazol-1-yl)methyl)-2,6-dimethylphenoxy)-2-methylpropionic acid |
| 116 | 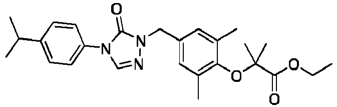 | Ethyl 2-(4-((4-(4-isopropylphenyl)-5-oxo-4,5-dihydro-1*H*-1,2,4-triazol-1-yl)methyl)-2,6-dimethylphenoxy)-2-methylpropionate |
| 117 | 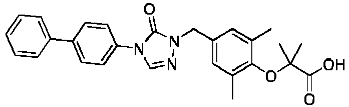 | 2-(4-((4-([1,1'-Biphenyl]-4-yl)-5-oxo-4, 5-dihydro-1*H*-1,2,4-triazol-1-yl)methyl)-2,6-dimethylphenoxy)-2-methylpropionic acid |
| 118 | 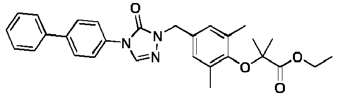 | Ethyl 2-(4-((4-([1,1'-biphenyl]-4-yl)-5-oxo-4,5-dihydro-1*H*-1,2,4-triazol-1-yl)methyl)-2,6-dimethylphenoxy)-2-methylpropionate |
| 119 | 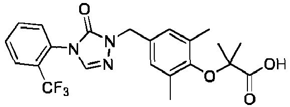 | 2-(2,6-Dimethyl-4-((5-oxo-4-(2-(trifluoromethyl)phenyl)-4,5-dihydro-1*H*-1,2,4-triazol-1-yl)methyl)phenoxy)-2-methylpropionic acid |
| 120 | 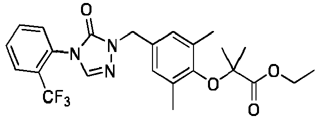 | Ethyl 2-(2,6-dimethyl-4-((5-oxo-4-(2-(trifluoromethyl)phenyl)-4,5-dihydro-1*H*-1,2,4-triazol-1-yl)methyl)phenoxy)-2-methylpropionate |
| 121 | 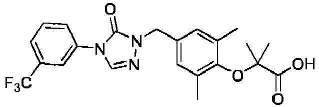 | 2-(2,6-Dimethyl-4-((5-oxo-4-(3-(trifluoromethyl)phenyl)-4,5-dihydro-1*H*-1,2,4-triazol-1-yl)methyl)phenoxy)-2-methylpropionic acid |
| 122 | 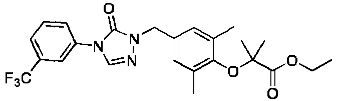 | Ethyl 2-(2,6-dimethyl-4-((5-oxo-4-(3-(trifluoromethyl)phenyl)-4,5- dihydro-1*H*-1,2,4-triazol-1-yl)methyl)phenoxy)-2-methylpropionate |
| 123 | 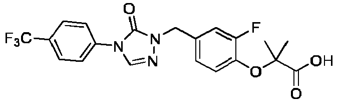 | 2-(2-Fluoro-4-((5-oxo-4-(4-(trifluoromethyl)phenyl)-4,5-dihydro-1*H*-1,2,4-triazol-1-yl)methyl)phenoxy)-2-methylpropionic acid |
| 124 | 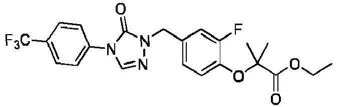 | Ethyl 2-(2-fluoro-4-((5-oxo-4-(4-(trifluoromethyl)phenyl)-4,5-dihydro-1*H*-1,2,4-triazol-1-yl)methyl)phenoxy)-2-methylpropionate |
| 125 | 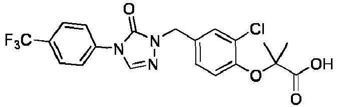 | 2-(2-Chloro-4-((5-oxo-4-(4-(trifluoromethyl)phenyl)-4,5-dihydro-1*H*-1,2,4-triazol-1-yl)methyl)phenoxy)-2-methylpropionic acid |
| 126 | 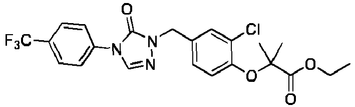 | Ethyl 2-(2-chloro-4-((5-oxo-4-(4-(trifluoromethyl)phenyl)-4,5-dihydro-1*H*-1,2,4-triazol-1-yl)methyl)phenoxy)-2-methylpropionate |
| 127 | 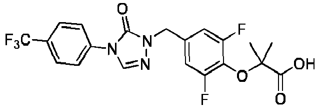 | 2-(2,6-Difluoro-4-((5-oxo-4-(4-(trifluoromethyl)phenyl)-4,5-dihydro-1*H*-1,2,4-triazol-1-yl)methyl)phenoxy)-2-methylpropionic acid |
| 128 | 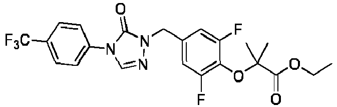 | Ethyl 2-(2,6-difluoro-4-((5-oxo-4-(4-(trifluoromethyl)phenyl)-4,5-dihydro-1*H*-1,2,4-triazol-1-yl)methyl)phenoxy)-2-methylpropionate |
| 129 | 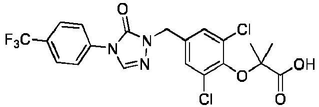 | 2-(2,6-Dichloro-4-((5-oxo-4-(4-(trifluoromethyl)phenyl)-4,5-dihydro-1*H*-1,2,4-triazol-1-yl)methyl)phenoxy)-2-methylpropionic acid |
| 130 | 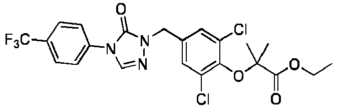 | Ethyl 2-(2,6-dichloro-4-((5-oxo-4-(4-(trifluoromethyl)phenyl)-4,5-dihydro-1*H*-1,2,4-triazol-1-yl)methyl)phenoxy)-2-methylpropionate |
| 131 | 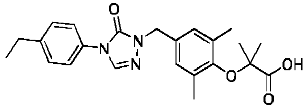 | 2-(4-((4-(4-Ethylphenyl)-5-oxo-4,5-dihydro-1*H*-1,2,4-triazol-1-yl)methyl)-2,6-dimethylphenoxy)-2-methylpropionic acid |
| 132 | 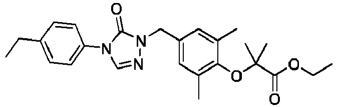 | Ethyl 2-(4-((4-(4-ethylphenyl)-5-oxo-4,5-dihydro-1*H*-1,2,4-triazol-1-yl)methyl)-2,6-dimethylphenoxy)-2-methylpropionate |
| 133 | 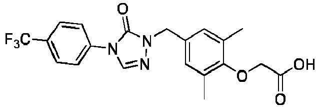 | 2-(2,6-Dimethyl-4-((5-oxo-4-(4-(trifluoromethyl)phenyl)-4,5-dihydro-1*H*-1,2,4-triazol-1-yl)methyl)phenoxy)acetic acid |
| 134 | 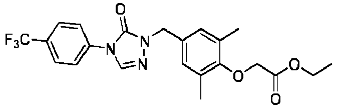 | Ethyl 2-(2,6-dimethyl-4-((5-oxo-4-(4-(trifluoromethyl)phenyl)-4,5-dihydro-1*H*-1,2,4-triazol-1-yl)methyl)phenoxy)acetate |
| 135 | 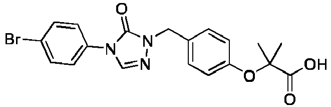 | 2-(4-((4-(4-Bromophenyl)-5-oxo-4,5-dihydro-1*H*-1,2,4-triazol-1-yl)methyl)phenoxy)-2-methylpropionic acid |
| 136 | 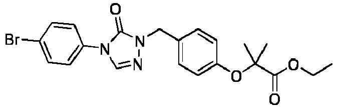 | Ethyl 2-(4-((4-(4-Bromophenyl)-5-oxo-4,5-dihydro-1*H*-1,2,4-triazol-1-yl)methyl)phenoxy)-2-methylpropionate |
| 137 | 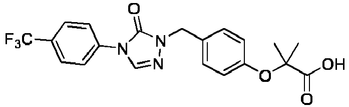 | 2-(4-((4-(4-Trifluoromethylphenyl)-5-oxo-4,5-dihydro-1*H*-1,2,4-triazol-1-yl)methyl)phenoxy)-2-methylpropionic acid |
| 138 | 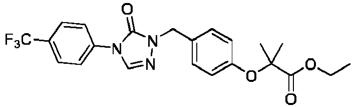 | Ethyl 2-(4-((4-(4-trifluoromethylphenyl)-5-oxo-4,5-dihydro-1*H*-1,2,4-triazol-1-yl)methyl)phenoxy)-2-methylpropionate |
| 139 | 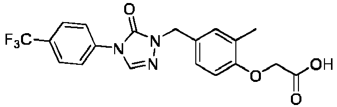 | 2-(2-Methyl-4-((5-oxo-4-(4-(trifluoromethyl)phenyl)-4,5-dihydro-1*H*-1,2,4-triazol-1-yl)methyl)phenoxy)acetic acid |
| 140 | 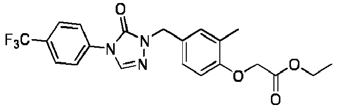 | Ethyl 2-(2-methyl-4-((5-oxo-4-(4-(trifluoromethyl)phenyl)-4,5-dihydro-1*H*-1,2,4-triazol-1-yl)methyl)phenoxy)acetate |
| 141 | 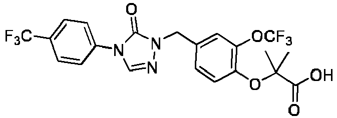 | 2-(2-Trifluoromethoxy-4-((5-oxo-4-(4-(trifluoromethyl)phenyl)-4,5-dihydro-1*H*-1,2,4-triazol-1-yl)methyl)phenoxy)acetic acid |
| 142 | 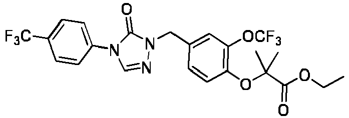 | Ethyl 2-(2-trifluoromethoxy-4-((5-oxo-4-(4-(trifluoromethyl)phenyl)-4,5-dihydro-1*H*-1,2,4-triazol-1-yl)methyl)phenoxy)acetate |
| 143 | 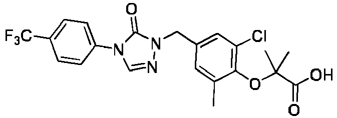 | 2-(2-Chloro-6-methyl-4-((5-oxo-4-(4-(trifluoromethyl)phenyl)-4,5-dihydro-1*H*-1,2,4-triazol-1-yl)methyl)phenoxy)-2-methylpropionic acid |
| 144 | 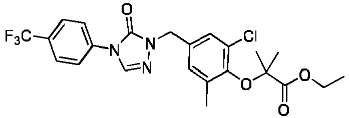 | Ethyl 2-(2-chloro-6-methyl-4-((5-oxo-4-(4-(trifluoromethyl)phenyl)-4,5-dihydro-1*H*-1,2,4-triazol-1-yl)methyl)phenoxy)-2-methylpropionate |
| 145 | 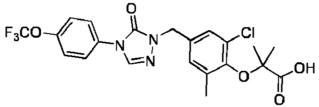 | 2-(2-Chloro-6-methyl-4-((5-oxo-4-(4-(trifluoromethoxy)phenyl)-4,5-dihydro-1*H*-1,2,4-triazol-1-yl)methyl)phenoxy)-2-methylpropionic acid |
| 146 | 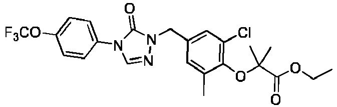 | Ethyl 2-(2-chloro-6-methyl-4-((5-oxo-4-(4-(trifluoromethoxy)phenyl)-4,5-dihydro-1*H*-1,2,4-triazol-1-yl)methyl)phenoxy)-2-methylpropionate |
| 147 | 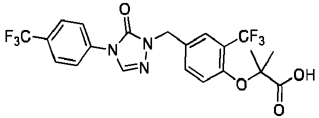 | 2-Methyl-2-(4-((5-oxo-4-(4-(trifluoromethyl)phenyl)-4,5-dihydro-1*H*-1,2,4-triazol-1-yl)methyl)-2-(trifluoromethyl)phenoxy)propionic acid |
| 148 | 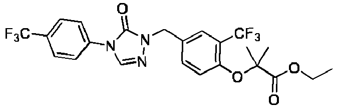 | Ethyl 2-methyl-2-(4-((5-oxo-4-(4-(trifluoromethyl)phenyl)-4,5-dihydro-1*H*-1,2,4-triazol-1-yl)methyl)-2-(trifluoromethyl)phenoxy)propionate |
| 149 | 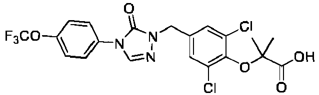 | 2-(2,6-Dichloro-4-((5-oxo-4-(4-(trifluoromethoxy)phenyl)-4,5-dihydro-1*H*-1,2,4-triazol-1-yl)methyl)phenoxy)-2-methylpropionic acid |
| 150 | 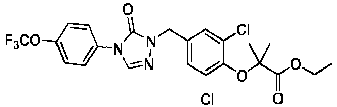 | Ethyl 2-(2,6-dichloro-4-((5-oxo-4-(4-(trifluoromethoxy)phenyl)-4,5-dihydro-1*H*-1,2,4-triazol-1-yl)methyl)phenoxy)-2-methylpropionate |
| 151 | 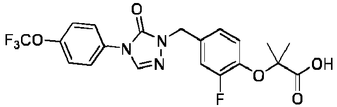 | 2-(2-Fluoro-4-((5-oxo-4-(4-(trifluoromethoxy)phenyl)-4,5-dihydro-1*H*-1,2,4-triazol-1-yl)methyl)phenoxy)-2-methylpropionic acid |
| 152 | 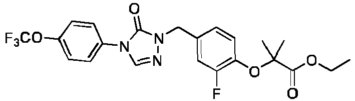 | Ethyl 2-(2-fluoro-4-((5-oxo-4-(4-(trifluoromethoxy)phenyl)-4,5-dihydro-1*H*-1,2,4-triazol-1-yl)methyl)phenoxy)-2-methylpropropioniate |
| 153 | 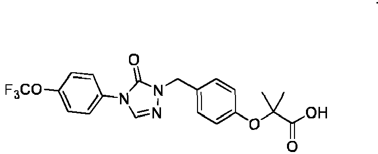 | 2-Methyl-2-(4-((5-oxo-4-(4-(trifluoromethoxy)phenyl)-4,5-dihydro-1*H*-1,2,4-triazol-1-yl)methyl)phenoxy)propionic acid |
| 154 | 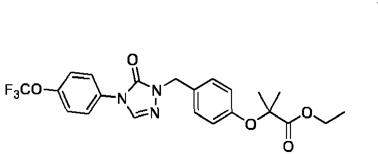 | Ethyl 2-methyl-2-(4-((5-oxo-4-(4-(trifluoromethoxy)phenyl)-4,5-dihydro-1*H*-1,2,4-triazol-1-yl)methyl)phenoxy)propionate |
| 155 | 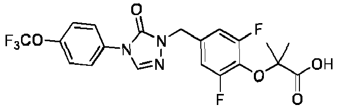 | 2-(2,6-Difluoro-4-((5-oxo-4-(4-(trifluoromethoxy)phenyl)-4,5-dihydro-1*H*-1,2,4-triazol-1-yl)methyl)phenoxy)-2-methylpropionic acid |
| 156 | 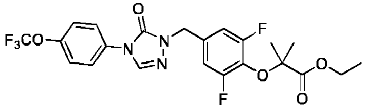 | Ethyl 2-(2,6-difluoro-4-((5-oxo-4-(4-(trifluoromethoxy)phenyl)-4,5-dihydro-1*H*-1,2,4-triazol-1-yl)methyl)phenoxy)-2-methylpropionate |
| 157 | 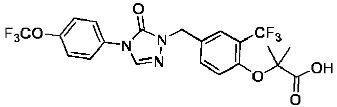 | 2-Methyl-2-(4-((5-oxo-4-(4-(trifluoromethoxy)phenyl)-4,5-dihydro-1*H*-1,2,4-triazol-1- yl)methyl)-2-(trifluoromethyl)phenoxy)propionic acid |
| 158 | 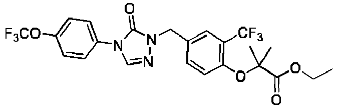 | Ethyl 2-methyl-2-(4-((5-oxo-4-(4-(trifluoromethoxy)phenyl)-4,5-dihydro-1*H*-1,2,4-triazol-1-yl)methyl)-2-(trifluoromethyl)phenoxy)propionate |
| 159 | 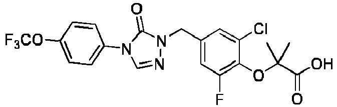 | 2-(2-Chloro-6-fluoro-4-((5-oxo-4-(4-(trifluoromethoxy)phenyl)-4,5-dihydro-1*H*-1,2,4-triazol-1-yl)methyl)phenoxy)-2-methylpropionic acid |
| 160 | 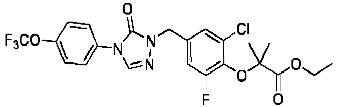 | Ethyl 2-(2-chloro-6-fluoro-4-((5-oxo-4-(4-(trifluoromethoxy)phenyl)-4,5-dihydro-1*H*-1,2,4-triazol-1-yl)methyl)phenoxy)-2-methylpropionate |
| 161 | 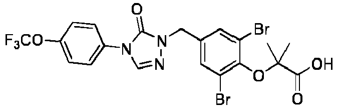 | 2-(2,6-Dibromo-4-((5-oxo-4-(4-(trifluoromethoxy)phenyl)-4,5-dihydro-1*H*-1,2,4-triazol-1-yl)methyl)phenoxy)-2-methylpropionic acid |
| 162 | 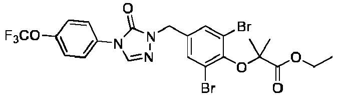 | Ethyl 2-(2,6-dibromo-4-((5-oxo-4-(4-(trifluoromethoxy)phenyl)-4,5-dihydro-1*H*-1,2,4-triazol-1-yl)methyl)phenoxy)-2-methylpropionate |
| 163 | 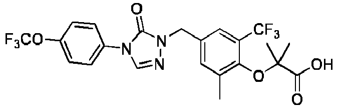 | 2-Methyl-2-(2-methyl-4-((5-oxo-4-(4-(trifluoromethoxy)phenyl)-4,5-dihydro-1*H*-1,2,4-triazol-1-yl)methyl)-6-(trifluoromethyl)phenoxy)propionic acid |
| 164 | 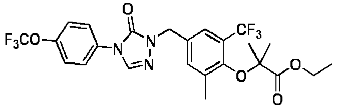 | Ethyl 2-methyl-2-(2-methyl-4-((5-oxo-4-(4-(trifluoromethoxy)phenyl)-4,5-dihydro-1*H*-1,2,4-triazol-1-yl)methyl)-6-(trifluoromethyl)phenoxy)propionate |
| 165 | 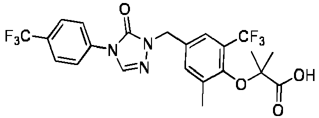 | 2-Methyl-2-(2-methyl-4-((5-oxo-4-(4-(trifluoromethyl)phenyl)-4,5-dihydro-1*H*-1,2,4-triazol-1-yl)methyl)-6-(trifluoromethyl)phenoxy)propionic acid |
| 166 | 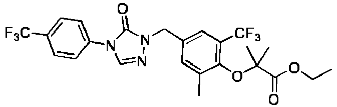 | Ethyl 2-methyl-2-(2-methyl-4-((5-oxo-4-(4-(trifluoromethyl)phenyl)-4,5-dihydro-1*H*-1,2,4-triazol-1-yl)methyl)-6-(trifluoromethyl)phenoxy)propionate |
| 167 | 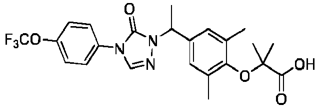 | 2-(2,6-Dimethyl-4-(1-(5-oxo-4-(4-(trifluoromethoxy)phenyl)-4,5-dihydro-1*H*-1,2,4-triazol-1-yl)ethyl)phenoxy)-2-methylpropionic acid |
| 168 | 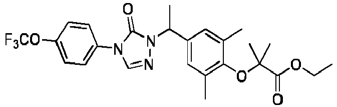 | Ethyl 2-(2,6-dimethyl-4-(1-(5-oxo-4-(4-(trifluoromethoxy)phenyl)-4,5-dihydro-1*H*-1,2,4-triazol-1-yl)ethyl)phenoxy)-2-methylpropionate |
| 169 | 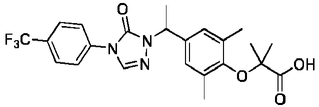 | 2-(2,6-Dimethyl-4-(1-(5-oxo-4-(4-(trifluoromethyl)phenyl)-4,5-dihydro-1*H*-1,2,4-triazol-1-yl)ethyl)phenoxy)-2-methylpropionic acid |
| 170 | 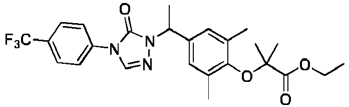 | Ethyl 2-(2,6-dimethyl-4-(1-(5-oxo-4-(4-(trifluoromethyl)phenyl)-4,5-dihydro-1*H*-1,2,4-triazol-1-yl)ethyl)phenoxy)-2-methylpropionate |
| 171 | 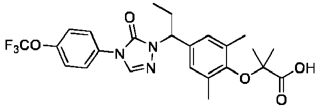 | 2-(2,6-Dimethyl-4-(1-(5-oxo-4-(4-(trifluoromethoxy)phenyl)-4,5-dihydro-1*H*-1,2,4-triazol-1-yl)propyl)phenoxy)-2-methylpropionic acid |
| 172 | 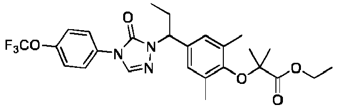 | Ethyl 2-(2,6-dimethyl-4-(1-(5-oxo-4-(4-(trifluoromethoxy)phenyl)-4,5-dihydro-1*H*-1,2,4-triazol-1-yl)propyl)phenoxy)-2-methylpropionate |
| 173 | 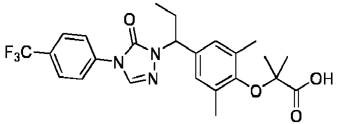 | 2-(2,6-Dimethyl-4-(1-(5-oxo-4-(4-(trifluoromethyl)phenyl)-4,5-dihydro-1*H*-1,2,4-triazol-1-yl)propyl)phenoxy)-2-methylpropionic acid |
| 174 | 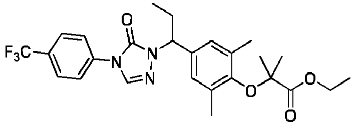 | Ethyl 2-(2,6-dimethyl-4-(1-(5-oxo-4-(4-(trifluoromethyl)phenyl)-4, 5-dihydro-1*H*-1,2,4-triazol-1-yl)propyl)phenoxy)-2-methylpropionate |
| 175 | 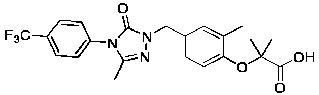 | 2-(2,6-Dimethyl-4-((3-methyl-5-oxo-4-(4-(trifluoromethyl)phenyl)-4,5-dihydro-1*H*-1,2,4-triazol-1-yl)methyl)phenoxy)-2-methylpropionic acid |
| 176 | 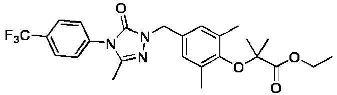 | Ethyl 2-(2,6-dimethyl-4-((3-methyl-5-oxo-4-(4-(trifluoromethyl)phenyl)-4,5-dihydro-1*H*-1,2,4-triazol-1-yl)methyl)phenoxy)-2-methylpropionate |
| 177 | 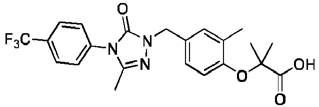 | 2-Methyl-2-(2-methyl-4-((3-methyl-5-oxo-4-(4-(trifluoromethyl)phenyl)-4,5-dihydro-1*H*-1,2,4-triazol-1-yl)methyl)phenoxy)propionic acid |
| 178 | 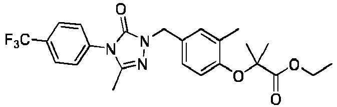 | Ethyl 2-methyl-2-(2-dimethyl-4-((3-methyl-5-oxo-4-(4-(trifluoromethyl)phenyl)-4,5-dihydro-1*H*-1,2,4-triazol-1-yl)methyl)phenoxy)propionate |
| 179 | 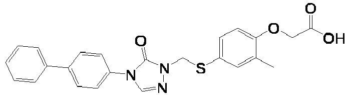 | 2-(4-(((4-([1,1'-Biphenyl]-4-yl)-5-oxo-4,5-dihydro-1*H*-1,2,4-triazol-1-yl)methyl)thio)-2-methylphenoxy)acetic acid |
| 180 | 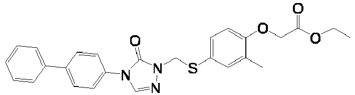 | Ethyl 2-(4-(((4-([1,1'-biphenyl]-4-yl]-5-oxo-4,5-dihydro-1*H*-1,2,4-triazol-1-yl)methyl)thio)-2-methylphenoxy)acetate |
| 181 | 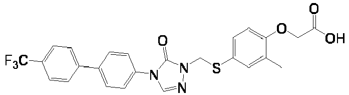 | 2-(2-Methyl-4-((((5-oxo-4-(4'-(trifluoromethyl)-[1,1'-biphenyl]-4-yl)-4,5-dihydro-1*H*-1,2,4-triazol-1-yl)methyl)thio)phenoxy)acetic acid |
| 182 | 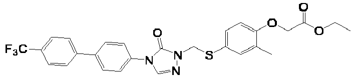 | Ethyl 2-(2-methyl-4-(((5-oxo-4-(4'-(trifluoromethyl)-[1,1'-biphenyl]-4-yl)-4,5-dihydro-1*H*-1,2,4-triazol-1-yl)methyl)thio)phenoxy)acetate |
| 183 | 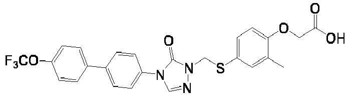 | 2-(2-Methyl-4-((((5-oxo-4-(4'-(trifluoromethoxy)-[1,1'-biphenyl]-4-yl)-4,5-dihydro-1*H*-1,2,4-triazol-1- yl)methyl)thio)phenoxy)acetic acid |
| 184 | 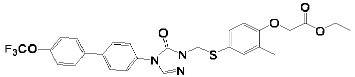 | Ethyl 2-(2-methyl-4-((((5-oxo-4-(4'-(trifluoromethoxy)-[1,1'-biphenyl]-4-yl)-4,5-dihydro-1*H*-1,2,4-triazol-1-yl)methyl)thio)phenoxy)acetate |
| 185 | 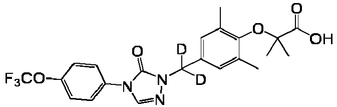 | 2-(2,6-Dimethyl-4-((5-oxo-4-(4-(trifluoromethoxy)phenyl)-4,5-dihydro-1*H*-1,2,4-triazol-1-yl)methyl-*d*₂)phenoxy)-2-methylpropionic acid |
| 186 | 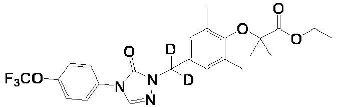 | Ethyl 2-(2,6-dimethyl-4-((5-oxo-4-(4-(trifluoromethoxy)phenyl)-4,5-dihydro-1*H*-1,2,4-triazol-1-yl)methyl-*d*₂)phenoxy)-2-methylpropionate |
| 187 | 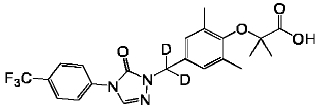 | 2-(2,6-Dimethyl-4-((5-oxo-4-(4-(trifluoromethyl)phenyl)-4,5-dihydro-1*H*-1,2,4-triazol-1-yl)methyl-*d*₂)phenoxy)-2-methylpropionic acid |
| 188 | 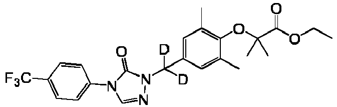 | Ethyl 2-(2,6-dimethyl-4-((5-oxo-4-(4-(trifluoromethyl)phenyl)-4,5-dihydro-1*H*-1,2,4-triazol-1-yl)methyl-*d*₂)phenoxy)-2-methylpropionate |
| 189 | 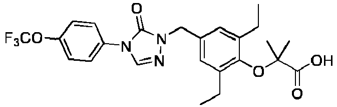 | 2-(2,6-Diethyl-4-((5-oxo-4-(4-(trifluoromethoxy)phenyl)-4,5-dihydro-1*H*-1,2,4-triazol-1-yl)methyl)phenoxy)-2-methylpropionic acid |
| 190 | 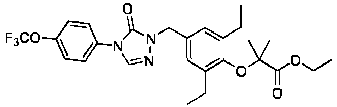 | Ethyl 2-(2,6-diethyl-4-((5-oxo-4-(4-(trifluoromethoxy)phenyl)-4,5-dihydro-1*H*-1,2,4-triazol-1-yl)methyl)phenoxy)-2-methylpropionate |
| 191 | 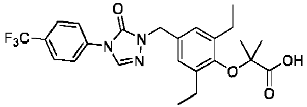 | 2-(2,6-Diethyl-4-((5-oxo-4-(4-(trifluoromethyl)phenyl)-4,5-dihydro-1*H*-1,2,4-triazol-1-yl)methyl)phenoxy)-2-methylpropionic acid |
| 192 | 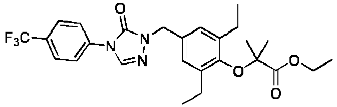 | Ethyl 2-(2,6-diethyl-4-((5-oxo-4-(4-(trifluoromethyl)phenyl)-4,5-dihydro-1*H*-1,2,4-triazol-1-yl)methyl)phenoxy)-2-methylpropionate |
| 193 | 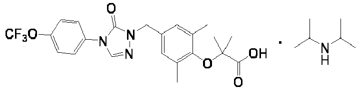 | 2-(2,6-Dimethyl-4-((5-oxo-4-(4-(trifluoromethoxy)phenyl)-4,5-dihydro-1*H*-1,2,4-triazol-1-yl)methyl)phenoxy)-2-methylpropionic acid diisopropylamine salt |
| 194 | 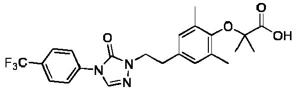 | 2-(2,6-Dimethyl-4-(2-(5-oxo-4-(4-(trifluoromethyl)phenyl)-4,5-dihydro-1*H*-1,2,4-triazol-1-yl)ethyl)phenoxy)-2-methylpropionic acid |
| 195 | 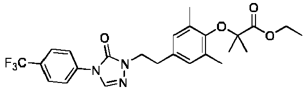 | Ethyl 2-(2,6-dimethyl-4-(2-(5-oxo-4-(4-(trifluoromethyl)phenyl)-4,5-dihydro-1*H*-1,2,4-triazol-1-yl)ethyl)phenoxy)-2-methylpropionate |
| 196 | 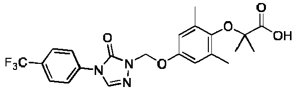 | 2-(2,6-Dimethyl-4-((5 -oxo-4-(4-(trifluoromethyl)phenyl)-4,5-dihydro-1*H*-1,2,4-triazol-1-yl)methoxy)phenoxy)-2-methylpropionic acid |
| 197 | 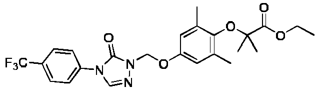 | Ethyl 2-(2,6-dimethyl-4-((5-oxo-4-(4-(trifluoromethyl)phenyl)-4,5-dihydro-1*H*-1,2,4-triazol-1-yl)methoxy)phenoxy)-2-methylpropionate |
| 198 | 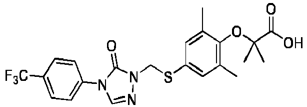 | 2-(2,6-Dimethyl-4-(((5-oxo-4-(4-(trifluoromethyl)phenyl)-4,5-dihydro-1*H*-1,2,4-triazol-1-yl)methyl)thio)phenoxy)-2-methylpropionic acid |
| 199 | 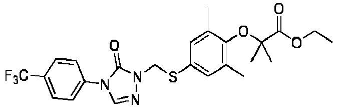 | Ethyl 2-(2,6-dimethyl-4-(((5-oxo-4-(4-(trifluoromethyl)phenyl)-4,5-dihydro-1*H*-1,2,4-triazol-1-yl)methyl)thio)phenoxy)-2-methylpropionate |
| 200 | 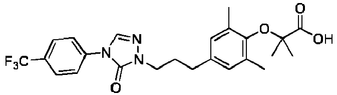 | 2-(2,6-Dimethyl-4-(3-(5-oxo-4-(4-(trifluoromethyl)phenyl)-4,5-dihydro-1*H*-1,2,4-triazol-1-yl)propyl)phenoxy)-2-methylpropionic acid |
| 201 | 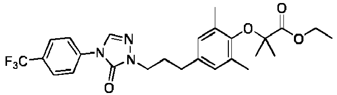 | Ethyl 2-(2,6-dimethyl-4-(3-(5-oxo-4-(4-(trifluoromethyl)phenyl)-4,5-dihydro-1*H*-1,2,4-triazol-1-yl)propyl)phenoxy)-2-methylpropionate |
| 202 | 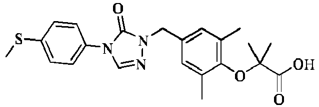 | 2-(2,6-Dimethyl-4-((4-(4-(methylthio)phenyl)-5-oxo-4,5-dihydro-1*H*-1,2,4-triazol-1-yl)methyl)phenoxy)-2-methylpropionic acid |
| 203 | 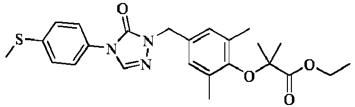 | Ethyl 2-(2,6-dimethyl-4-((4-(4-(methylthio)phenyl)-5-oxo-4,5-dihydro-1*H*-1,2,4-triazol-1-yl)methyl)phenoxy)-2-methylpropionate |
| 204 | 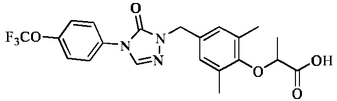 | 2-(2,6-Dimethyl-4-((5-oxo-4-(4-(trifluoromethoxy)phenyl)-4,5-dihydro-1*H*-1,2,4-triazol-1-yl)methyl)phenoxy)propionic acid |
| 205 | 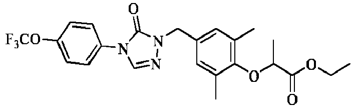 | Ethyl 2-(2,6-dimethyl-4-((5-oxo-4-(4-(trifluoromethoxy)phenyl)-4,5-dihydro-1*H*-1,2,4-triazol-1-yl)methyl)phenoxy)propionate |
| 206 | 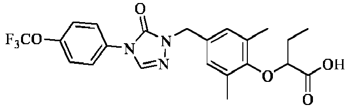 | 2-(2,6-Dimethyl-4-((5-oxo-4-(4-(trifluoromethoxy)phenyl)-4,5-dihydro-1*H*-1,2,4-triazol-1-yl)methyl)phenoxy)butyric acid |
| 207 | 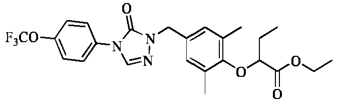 | Ethyl 2-(2,6-dimethyl-4-((5-oxo-4-(4-(trifluoromethoxy)phenyl)-4,5-dihydro-1*H*-1,2,4-triazol-1-yl)methyl)phenoxy)butyrate |
| 208 | 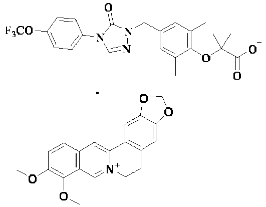 | 2-(2,6-Dimethyl-4-((5-oxo-4-(4-(trifluoromethoxy)phenyl)-4,5-dihydro-1*H*-1,2,4-triazol-1-yl)methyl)phenoxy)-2-methylpropionic acid berberine salt |

The triazolone compound of the present invention can be used as a pharmaceutical salt. The salt may be a salt formed by the compound of the present invention with metal (including sodium, potassium, calcium, etc.) ions or pharmaceutically acceptable amines (including ethylenediamine, tromethamine, diisopropylamine, meglumine, berberine, metformin, etc.) or ammonium ions.

Provided is use of the triazolone compound or the pharmaceutically acceptable salt, the tautomer, the mesomer, the racemate, the stereoisomer, the metabolite, the metabolic precursor, the prodrug or the solvate thereof described herein in the preparation of a PPARα/δ dual agonist.

The triazolone compound described herein is a novel PPARα/δ dual agonist, so the triazolone compound or the pharmaceutically acceptable salt, the tautomer, the mesomer, the racemate, the stereoisomer, the metabolite, the metabolic precursor, the prodrug or the solvate thereof of the present invention can be used for preparing a medicament for preventing or treating diseases mediated by PPARα and/or PPARδ.

Specifically, the compound of the present invention can be used for preparing a medicament for preventing and treating the following diseases mediated by PPARα and/or PPARδ.

The compound of the present invention can be used for preventing and treating metabolic diseases and cardiovascular and cerebrovascular diseases, including: insulin resistance, metabolic syndrome, type 1 or type 2 diabetes, hyperlipidemia, obesity, atherosclerosis, myocardial ischemia, myocardial infarction, arrhythmia, coronary heart disease, hypertension, heart failure, myocardial hypertrophy, myocarditis, diabetic complications (including diabetic cardiomyopathy, diabetic nephropathy, diabetic ulcer, retinopathy, neuropathy, etc.), nonalcoholic fatty liver disease, nonalcoholic steatohepatitis, alcoholic fatty liver disease, liver cirrhosis, hyperuricemia, gout, osteoporosis, polycystic ovary syndrome (PCOS), stroke, cerebral infarction, or the like.

The compound of the present invention can be used for preventing and treating inflammatory diseases, autoimmune diseases, organ fibrosis diseases, neurological injury diseases, or secondary diseases caused by pathogen infections, including: primary biliary cholangitis (PBC), primary sclerosing cholangitis (PSC), liver fibrosis, idiopathic pulmonary fibrosis, cystic fibrosis lung disease, interstitial pneumonia, tuberculosis, inflammatory bowel disease (such as Crohn's disease and ulcerative colitis), Behcet's disease, asthma, chronic obstructive pulmonary disease, chronic bronchitis, emphysema, bronchiolitis obliterans, allergic rhinitis, chronic rhinitis, sinusitis, systemic lupus erythematosus, rheumatoid arthritis, spondyloarthritis, osteoarthritis, synovitis, tendonitis, thromboangiitis obliterans, phlebitis, intermittent claudication, keloid, psoriasis, ichthyosis, bullous pemphigoid, dermatitis, contact dermatitis, pancreatitis, chronic nephritis, cystitis, meningitis, gastritis, septicemia, pyoderma gangrenosum, uveitis, Parkinson's disease, Alzheimer's disease, α-synucleinopathy, depression, multiple sclerosis, amyotrophic lateral sclerosis, fibromyalgia syndrome, neuralgia, Down's syndrome, Hallervorden-Spatz disease, Huntington's chorea, Wilson's disease, or the like.

The compound of the present invention can be used for treating and regulating mitochondrial dysfunction and disorder diseases, including: myasthenia, myoclonus, exercise intolerance, Kearns-Sayre syndrome, chronic fatigue syndrome, Leigh's syndrome, mitochondrial myopathy-encephalopathy-hyperlactacidemia, stroke syndrome or stroke-like episodes, Duchenne muscular dystrophy, Becker muscular dystrophy, Friedreich's ataxia, or the like.

The compound of the present invention can be used for treating tumors, including: bone cancer, acute myeloid leukemia, chronic myeloid leukemia, acute lymphocytic leukemia, chronic lymphocytic leukemia, myeloproliferative disease, multiple myeloma, myelodysplastic syndrome, Hodgkin's lymphoma, non-Hodgkin's lymphoma, hemangioma, granuloma, xanthoma, meningosarcoma, neuroglioma, astrocytoma, medulloblastoma, ependymoma, germ cell tumor (pinealoma), glioblastoma multiforme, oligodendroglioma, schwannoma, retinoblastoma, fibroneuroma, sarcoma, esophagus cancer, gastric cancer, pancreatic cancer, colorectal cancer, colon cancer, rectal cancer, renal cancer, prostate cancer, lymphatic cancer, testicular cancer, interstitial cell cancer, lung cancer, liver cancer, skin cancer, malignant melanoma, basal cell carcinoma, or the like.

The present invention also provides a pharmaceutical composition for preventing or treating diseases mediated by PPARα and/or PPAR6, which comprises a therapeutically effective amount of the triazolone compound of formula (I) or the pharmaceutically acceptable salt, the tautomer, the mesomer, the racemate, the stereoisomer, the metabolite, the metabolic precursor, the prodrug or the solvate thereof described herein as an active ingredient and a pharmaceutically acceptable carrier. The carrier that can be arbitrarily mixed may vary depending on the dosage form, the administration form, and the like. Examples of carriers include excipients, binders, disintegrants, lubricants, corrigents, flavoring agents, coloring agents, sweetening agents, and the like. The pharmaceutical composition can be in the form of a capsule, a powder, a tablet, a granule, a pill, an injection, a syrup, an oral liquid, an inhalant, an ointment, a suppository, a patch, or other pharmaceutically conventional preparations.

If desired, the compound of the present invention can be used in combination with one or more other types of agents for preventing or treating diseases mediated by PPARα and/or PPAR6, including but not limited to the following combinations.

Other types of prophylactic or therapeutic agents that may be selected for use in combination with the compound of the present invention may be one or more anti-diabetic agents, including metformin, sulfonylurea hypoglycemic agents (e.g., glibenclamide, glimepiride, etc.), glucosidase inhibitors (e.g., acarbose, miglitol, etc.), PPARγ agonists (e.g., pioglitazone and rosiglitazone), PPARα/γ dual agonists, dipeptidyl peptidase IV (DPP-IV) inhibitors (e.g., sitagliptin, saxagliptin, alogliptin, linagliptin, etc.), meglitinide hypoglycemic agents (e.g., repaglinide, nateglinide, etc.), SGLT2 inhibitors (e.g., canagliflozin, daragliflozin, empagliflozin, ipragliflozin, luseogliflozin, tofogliflozin, etc.), glucokinase agonists (e.g., HMS5552, etc.), glucose kinase agonists, insulin, glucagon-like peptide-1 (GLP-1) agents (e.g., exenatide, liraglutide, lixisenatide, dulaglutide, benaglutide, albiglutide, etc.), PTP1B inhibitors, glycogen phosphorylase inhibitors, glucose-6-phosphatase inhibitors, AMPK agonists (e.g., berberine), GPR40 agonists, or GPR120 agonists.

Other types of prophylactic or therapeutic agents that may be selected for use in combination with the compound of the present invention may be one or more weight-loss agents, including lorcaserin, orlistat, glucagon-like peptide-1 (GLP-1) agents (e.g., exenatide, liraglutide, lixisenatide, dulaglutide, benaglutide, albiglutide, etc.), and the like.

Other types of prophylactic or therapeutic agents that may be selected for use in combination with the compound of the present invention may be one or more anti-nonalcoholic fatty liver disease agents, including: AMPK agonists (e.g., metformin), Farnesoid X receptor (FXR) agonists (e.g., obeticholic acid, GS-9674, EDP-305, LJN452, etc.), acetyl-CoA carboxylase (ACC) inhibitors (e.g., GS-0976, etc.), apoptosis signal-regulating kinase-1 (ASK1) inhibitors (e.g., Selosertib, etc.), PPAR agonists (e.g., Elafibranor, Saroglitazar, IVA337, MSDC-0602K, etc.), caspase inhibitors (e.g., Emricasan, etc.), stearoyl-CoA desaturase 1 (SCD1) inhibitors (e.g., Aramchol, etc.), long-acting glucagon-like peptide-1 (GLP-1) receptor agonists (e.g., Semaglutide, etc.), apical sodium-dependent bile acid transporter (ASBT) inhibitors (e.g., Volixibat, etc.), vascular adhesion protein 1 (VAP-1) inhibitors (e.g., BI 1467335, etc.), CCR5R blockers (e.g., Cenicriviroc, etc.), thyroid hormone receptorβ (THR-β) agonists (e.g., MGL-3196, etc.), and the like.

Other types of prophylactic or therapeutic agents that may be selected for use in combination with the compound of the present invention may be one or more hypolipidemic agents, including nicotinic acid, statins (e.g., lovastatin, simvastatin, pravastatin, mevastatin, fluvastatin, atorvastatin, cerivastatin, rosuvastatin, and pitavastatin), cholesterol absorption inhibitors (e.g., ezetimibe, etc.), fibrates (e.g., clofibrate, bezafibrate, fenofibrate, etc.), PCSK9 inhibitors (e.g., Evolocumab, Alirocumab, etc.), CETP inhibitors (e.g., anacetrapib, etc.), AMPK agonists, ACC inhibitors (e.g., GS-0976, etc.), and the like.

The amount of the compound of formula (I) or the pharmaceutically acceptable salt, the tautomer, the mesomer, the racemate, the stereoisomer, the metabolite, the metabolic precursor, the prodrug or the solvate thereof of the present invention may be appropriately changed depending on the age, body weight, and symptoms of a patient, the administration route, and the like. For adults, the lower limit of a single dose is 0.1 mg (preferably 1 mg) and the upper limit is 1000 mg (preferably 500 mg) in oral administration; in the case of intravenous administration, the lower limit of a single dose is 0.01 mg (preferably 0.1 mg) and the upper limit is 500 mg (preferably 250 mg). The dose range may also vary depending on the degree of disease and the dosage form.

GFT505 is a PPARα/δ dual agonist in a phase III clinical research, but it has weak agonistic activity for PPARα and PPARδ and poor liver microsome stability. The inventor believes that this may have contributed to the poor interim results of the phase III clinical trial. Considering that the structure of "α,β-unsaturated ketone" in the GFT505 molecule may be the reason for its poor liver microsome stability, the inventor tries to replace the "α,β-unsaturated ketone" fragment in the molecular structure by means of molecular docking simulation, and then designs and synthesizes the triazolone compound of the present invention. By testing the agonistic activity of the triazolone compounds for PPAR, it is surprisingly found that: when the structure of "α,β-unsaturated ketone" is replaced by a "triazolone" fragment, a series of compounds with much stronger agonistic activity for PPARα and PPAR6 than GFT505 can be obtained, and the compounds of the present invention have much better liver microsomal stability than GFT505.

Advantages: Compared with the prior art, the present invention has the following advantages:
(1) The present invention provides a novel triazolone compound, which has a strong and activity-balanced agonistic effect on both PPARα and PPARδ. Under the same test system, the activity of the compound is significantly better than that of the PPARα/δ dual agonist reported in the literature, such as the phase III clinical trial drug GFT505 and compound 5c with the optimal activity reported in the literature at present (ACS Med. Chem. Lett., 2019, 10, 1068).
(2) By analyzing the eutectic structure of PPARδ and compound 61, the inventor unexpectedly found that in the present invention, in addition to the key hydrogen bonding interactions between the carboxylic acid group of compound 61 and the three key amino acid residues His287, His413 and Tyr437 of PPARδ, a special "water bridge" hydrogen bonding interaction exists between the triazolone structure of the compound and the amino acid Thr253 of PPARδ. No other types of PPARδ agonists and PPARδ have been reported in the literature to have the special "water bridge" hydrogen bonding interaction, which may be the main reason for the better efficacy and selectivity of the triazolone derivative PPAR agonist of the present invention.
(3) Compared with the phase III clinical trial drug GFT505, the compounds of the present invention have better metabolic stability and good pharmacokinetic properties *in vivo.* Therefore, the compounds and the pharmaceutically acceptable salts, the tautomers, the mesomers, the racemates, the stereoisomers, the metabolites, the metabolic precursors, the prodrugs or the solvates thereof of the present invention can be used for preparing a PPARα/δ dual agonist, and further can be used for preparing a medicament for preventing or treating diseases mediated by PPARα and/or PPARδ.
(4) The compounds of the present invention show very high selectivity for activating PPARα/PPARδ relative to activating PPARγ, and the selectivity is significantly better than that of GFT505 and compound 5c. However, it is well known that activation of PPARγ leads to the risk of weight gain, bone fracture and heart failure (Toxicol.sci., 2006, 90, 269). Therefore, the compounds of the present invention have potential advantages in terms of safety. In addition, the compounds of the present invention have no significant agonistic activity for other various nuclear receptors, showing high selectivity for PPARα/δ.
(5) In various experiments on NASH model mice, the compounds (such as compound 61) of the present invention have better anti-NASH efficacy than clinically investigational PPAR agonists GFT505 and IVA337 at the same dose.
(6) The triazolone compounds of the present invention are ingenious in design, simple in structure, cheap and easily available in starting materials, safe and environment-friendly in synthesis process, and easy for large-scale production.

### BRIEF DESCRIPTION OF THE DRAWINGS

FIG. 1 shows the effect of compound 57 on the lipid metabolism-associated gene expression of HepG2 cells (n = 3, *vs.* blank control group, *p < 0.05, **p < 0.01, *** p < 0.001);
FIG. 2 shows the effect of compound 61 on the lipid metabolism-associated gene expression of HepG2 cells (n = 3, *vs.* blank control group, *p < 0.05, **p < 0.01, *** p < 0.001);
FIG. 3 shows the effect of compound 57 on LPS-induced inflammation-associated gene expression of THP1 cells (n =3, *vs.* LPS group, *p < 0.05, **p < 0.01, *** p < 0.001);
FIG. 4 shows the effect of compound 61 on serum alanine transaminase (ALT) in NASH model mice (n = 9-10, *vs.* MCS group, ^{###}p < 0.001; vs. CDAA group, *p < 0.05, ***p < 0.001; vs. compound 61 medium dose group, ^{$$$}p < 0.001);
FIG. 5 shows the effect of compound 61 on serum aspartate transaminase (AST) in NASH model mice (n = 9-10, vs. MCS group, ^{###}p < 0.001; vs. CDAA group, *p < 0.05, ***p < 0.001; vs. compound 61 medium dose group, ^{$$$}p < 0.001);
FIG. 6 shows the effect of compound 61 on liver inflammation-associated genes of NASH model mice (n = 9-10, vs. MCS group, ^{#}p < 0.05, ^{##}p< 0.01, ^{###}p < 0.001; vs. CDAA group, *p < 0.05, **p < 0.01, ***p < 0.001; vs. compound 61 medium dose group, ^{$}p < 0.05, ^{$$}p < 0.01);
FIG. 7 shows the effect of compound 61 on liver fibrosis-associated genes of NASH model mice (n = 9-10, vs. MCS group, ^{#}p < 0.05, ^{###}p < 0.001; vs. CDAA group, *p < 0.05, **p < 0.01, ***p < 0.001; vs. compound 61 medium dose group, ^{$$$}p < 0.01);
FIG. 8 shows HE staining diagrams of liver sections for the therapeutic effect of compound 61 on NASH model mice;
FIG. 9 shows sirius red staining diagrams of liver sections for the therapeutic effect of compound 61 on NASH model mice;
FIG. 10 shows oil red staining diagrams of liver sections for the therapeutic effect of compound 61 on NASH model mice;
FIG. 11 shows the effect of compound 61 on hepatic hydroxyproline content in liver fibrosis model mice (n = 10, vs. Oil group, ^{###}p < 0.001; vs. CCl₄ group, ***p < 0.001; vs. compound 61 medium dose group, ^{$$$}p < 0.001);
FIG. 12 shows graphs of Western-Blot results and grayscale scan for the effect of compound 61 on the expression of αSMA and Col1a1 proteins in the liver of liver fibrosis model mice (n = 10, vs. Oil group, ^{##}p < 0.01, ^{###}p < 0.001; vs. CCl₄ group, *p < 0.05);
FIG. 13 shows HE staining diagrams of liver sections for the therapeutic effect of compound 61 on liver fibrosis model mice;
FIG. 14 shows sirius red staining diagrams of liver sections for the therapeutic effect of compound 61 on liver fibrosis model mice;
FIG. 15 shows heat maps for the effect of compound 61 on the target gene expression of PPARα/δ in mouse liver and skeletal muscle tissues;
FIG. 16 shows a heat map for the selectivity of compound 61 for various nuclear receptors;
FIG. 17 shows a diagram of the eutectic structure of compound 61 with a PPARδ protein.

### DETAILED DESCRIPTION

The content of the present invention will be specifically described below through examples. In the present invention, the following examples are given for better illustrating the present invention and are not intended to limit the scope of the present invention. Various changes and modifications can be made to the present invention without departing from the spirit and scope of the present invention.

### Example 1

### 2-(4-(((4-(4-Bromophenyl)-5-oxo-4,5-dihydro-1H-1,2,4-triazol-1-yl)methyl)thio)-2-fluorophenoxy)ethyl-2-methylpropionic acid (compound 1)

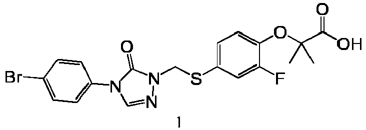

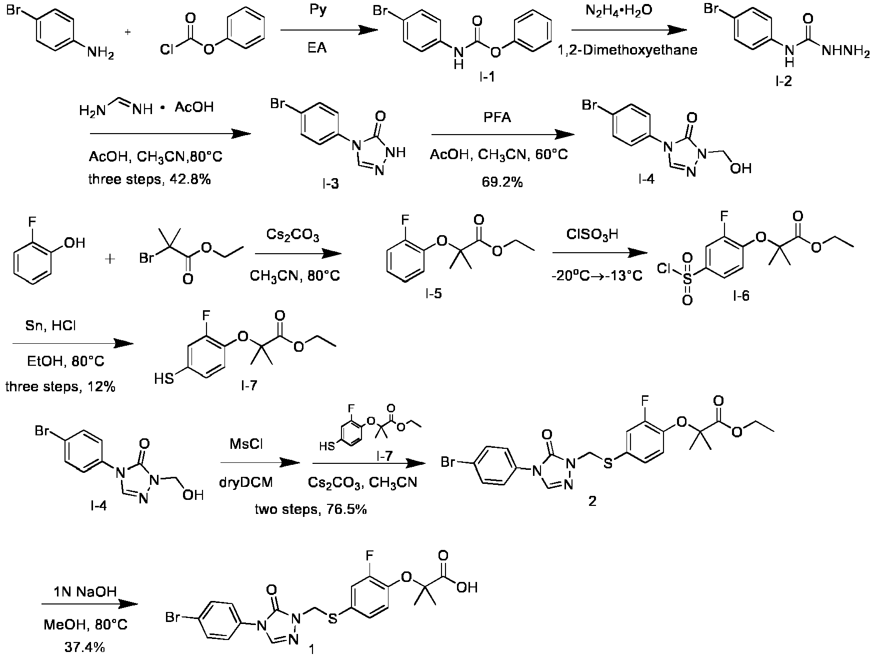

### Synthesis of intermediate I-1

p-Bromoaniline (3.44 g, 20 mmol) was dissolved in ethyl acetate (EA) (25 mL), pyridine (Py) (1.74 g, 22 mmol) was added, and phenyl chloroformate (3.44 g, 22 mmol) was slowly added under an ice bath. The mixture was stirred at room temperature for 4 h. After the reaction was completed, the reaction liquid was washed with water (50 mL × 3). The organic phase was washed with saturated brine (20 mL × 1), and concentrated by rotary evaporation under reduced pressure to remove the solvent to give a crude product of intermediate I-1, which was directly used in the next step without further purification.

### Synthesis of intermediate 1-2

All the residues obtained from the post-processing of the previous step containing intermediate I-1 (i.e., the crude product of I-1) were dissolved in glycol dimethyl ether (25 mL), and 98% hydrazine hydrate (2.67 mL) was added. The mixture was stirred at room temperature for 12 h. After the reaction was completed, the reaction liquid was concentrated by rotary evaporation under reduced pressure to remove the solvent, and EA (6 mL) was added to the residue. The mixture was stirred at room temperature for 12 h, and filtered under reduced pressure to give intermediate I-2 (white solid, 3.79 g).

### Synthesis of intermediate I-3

Intermediate I-2 (3.79 g, 16 mmol) was dissolved in acetonitrile (20 mL), and formamidine acetate (6.60 g, 64 mmol) was added. The mixture was stirred at room temperature for 30 min, and acetic acid (4.8 mL) was added. The system was transferred to an oil bath and reacted at 80 °C for 12 h. After the reaction was completed, the reaction liquid was concentrated by rotary evaporation under reduced pressure to remove the solvent. Water (50 mL) was added to the residue, and the resulting mixture was extracted with ethyl acetate (25 mL × 3). The organic phase was washed with saturated brine (20 mL × 1) and concentrated by rotary evaporation under reduced pressure to remove the solvent. A mixed solution of petroleum ether (5 mL) and ethyl acetate (1 mL) was added to the residue. The mixture was stirred at room temperature for 2 h and filtered under reduced pressure to give intermediate I-3 (orange solid, 2.05 g).

### Synthesis of intermediate I-4

Intermediate I-3 (2.05 g, 8.5 mmol) was dissolved in acetonitrile (15 mL), and paraformaldehyde (PFA) (1.275 g, 42.5 mmol) and acetic acid (60 mg, 1 mmol) were added. The system was transferred to an oil bath and reacted at 60 °C for 12 h. After the reaction was completed, the reaction liquid was concentrated by rotary evaporation under reduced pressure to remove the solvent. The residue was purified by column chromatography (petroleum ether/ethyl acetate = 2:1) to give intermediate I-4 (white solid, 1.59 g).

### Synthesis of intermediate I-5

o-Fluorophenol (6.72 g, 60 mmol) was dissolved in acetonitrile (150 mL), and ethyl 2-bromoisobutyrate (34.8 g, 180 mmol) and cesium carbonate (48.9 g, 150 mmol) were added. The system was transferred to an oil bath and reacted at 80 °C for 12 h. After the reaction was completed, the reaction liquid was filtered under reduced pressure through a Buchner funnel. The filtrate was diluted with water (500 mL), extracted with ethyl acetate (200 mL × 6), washed with 1 N sodium hydroxide (200 mL × 3), dried over anhydrous sodium sulfate, and concentrated by rotary evaporation under reduced pressure to remove the solvent to give a residue of intermediate I-5, which was directly used in the next step without further purification.

### Synthesis of intermediate I-6

Chlorosulfonic acid (18.0 mL, 270 mmol) was slowly added to all the residues obtained from the post-processing of the previous step containing intermediate I-5 at -20 °C. The reaction system was warmed to -13 °C and reacted for 1 h. After the reaction was completed, the reaction system was poured into 200 mL of ice water, stirred for 30 min, extracted with ethyl acetate (200 mL × 3), washed with saturated brine (150 mL × 1), and concentrated by rotary evaporation under reduced pressure to remove the solvent to give a residue of intermediate I-6, which was directly used in the next step without further purification.

### Synthesis of intermediate I-7

All the residues obtained from the post-processing of the previous step containing intermediate I-6 were dissolved in absolute ethanol (10 mL), and a saturated hydrogen chloride-ethanol (HCl-EtOH) solution (25 mL) and tin (Sn) powder (2.98 g, 25 mL) were added. The system was transferred to an oil bath and reacted at 80 °C for 24 h. After the reaction was completed, the reaction liquid was filtered under reduced pressure through a Buchner funnel, and the filtrate was concentrated by rotary evaporation under reduced pressure to remove the solvent. The residue was diluted with water (50 mL), extracted with ethyl acetate (25 mL × 3), washed with saturated brine (25 mL × 1), and concentrated by rotary evaporation under reduced pressure to remove the solvent. The residue was purified by column chromatography (petroleum ether/ethyl acetate = 80:1) to give intermediate I-7 (yellow liquid, 1.86 g).

### Synthesis of compound 2

Intermediate I-4 (358.6 mg, 1.3 mmol) was dissolved in 5 mL of anhydrous dichloromethane (DCM), and triethylamine (262.6 mg, 2.6 mmol) and methylsufonyl chloride (MsCl) (229.2 mg, 2 mmol) were added. The mixture was stirred at room temperature for 2 h. After the reaction was completed, the reaction liquid was concentrated by rotary evaporation under reduced pressure to remove the solvent. The residue was diluted with water (20 mL) and extracted with ethyl acetate (10 mL × 3). The organic phase was washed with saturated brine (20 mL × 1) and concentrated by rotary evaporation under reduced pressure to remove the solvent. The residue was dissolved in acetonitrile (10 mL), and intermediate I-7 (508 mg, 2 mmol) and cesium carbonate (1.07 g, 3.3 mmol) were added. The mixture was stirred at room temperature for 3 h. After the reaction was completed, the reaction liquid was concentrated by rotary evaporation under reduced pressure to remove the solvent. The residue was purified by column chromatography (petroleum ether/ethyl acetate = 5:1) to give compound 2 (colorless liquid, 519.8 mg).

### Synthesis of compound 1

Compound 2 (80 mg, 0.16 mmol) was dissolved in methanol (3 mL), and a 1 N NaOH solution (0.78 mL) was added. The reaction system was transferred to an oil bath and reacted at 80 °C for 4 h. After the reaction was completed, a 1 N HCl solution was added to adjust pH to 4, and the resulting mixture was concentrated by rotary evaporation under reduced pressure to remove the solvent. The residue was diluted with water (10 mL) and extracted with ethyl acetate (10 mL × 3). The organic phase was washed with saturated brine (10 mL × 1) and concentrated by rotary evaporation under reduced pressure to remove the solvent. The residue was purified by column chromatography (dichloromethane/methanol = 100:1) to give compound 1 (white solid, 28.8 mg): ¹H NMR (300 MHz, DMSO-*d₆*) δ 13.54 - 12.77 (s, 1H), 8.54 (s, 1H), 7.66 (dd, *J* = 30.8, 8.9 Hz, 4H), 7.45 (dd, *J* = 11.5, 2.1 Hz, 1H), 7.17 (d, *J* = 8.2 Hz, 1H), 6.93 (d, *J* = 8.7 Hz, 1H), 5.26 (s, 2H), 1.47 (s, 6H). HRMS (ESI) calcd. for C₁₉H₁₇BrFN₃O₄S [M+H]⁺ 482.0185, found 482.0185.

### Example 2

### Ethyl 2-(4-(((4-(4-bromophenyl)-5-oxo-4,5-dihydro-1H-1,2,4-triazol-1-yl)methyl)thio)-2-fluorophenoxy)ethyl-2-methylpropionate (compound 2)

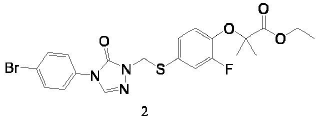

Compound 2 was prepared without hydrolysis according to the method of Example 1: ¹H NMR (300 MHz, CDCl₃) δ 7.71 (s, 1H), 7.62 (d, *J* = 8.7 Hz, 2H), 7.42 (d, *J* = 8.7 Hz, 2H), 7.32 (d, *J* = 2.2 Hz, 1H), 7.20 (d, *J* = 8.6 Hz, 1H), 6.92 (t, *J* = 8.5 Hz, 1H), 5.20 (s, 2H), 4.24 (d, *J* = 7.1 Hz, 2H), 1.58 (s, 6H), 1.28 (t, *J* = 7.1 Hz, 3H). MS (ESI): m/z 532.3 [M+Na]⁺.

### Example 3

### 2-(4-(((4-(4-Bromophenyl)-5-oxo-4,5-dihydro-1H-1,2,4-triazol-1-yl)methyl)thio)-2-chlorophenoxy)-2-methylpropionic acid (compound 3)

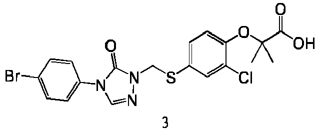

Compound 3 was prepared by replacing *o*-fluorophenol in Example 1 with *o*-chlorophenol according to the method of Example 1: ¹H NMR (300 MHz, DMSO-*d₆*) δ 13.20 (s, 1H), 8.54 (s, 1H), 7.70 (s, 2H), 7.61 (d, *J* = 9.1 Hz, 3H), 7.34 (dd, *J* = 8.6, 2.3 Hz, 1H), 6.86 (d, *J* = 8.6 Hz, 1H), 5.24 (s, 2H), 1.51 (s, 6H). HRMS (ESI) calcd. for C₁₉H₁₇BrClN₃O₄S [M+H]⁺ 497.9890, found 497.9881.

### Example 4

### Ethyl 2-(4-(((4-(4-bromophenyl)-5-oxo-4,5-dihydro-1H-1,2,4-triazol-1-yl)methyl)thio)-2-chlorophenoxy)ethyl-2-methylpropionate (compound 4)

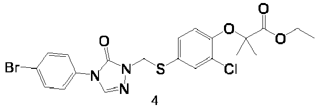

Compound 4 was prepared by replacing *o*-fluorophenol in Example 1 with *o*-chlorophenol without hydrolysis according to the method of Example 1: ¹H NMR (300 MHz, CDCl₃) δ 7.71 (s, 1H), 7.62 (d, *J* = 8.8 Hz, 2H), 7.54 (d, *J* = 2.2 Hz, 1H), 7.41 (d, *J* = 8.8 Hz, 2H), 7.33 (dd, *J* = 8.6, 2.3 Hz, 1H), 6.83 (d, *J* = 8.6 Hz, 1H), 5.17 (s, 2H), 4.24 (q, *J* = 7.1 Hz, 2H), 1.61 (s, 6H), 1.26 (t, *J* = 3.5 Hz, 3H). MS (ESI): m/z 548.2 [M+Na]⁺.

### Example 5

### 2-(4-(((4-(4-Bromophenyl)-5-oxo-4,5-dihydro-1H-1,2,4-triazol-1-yl)methyl)thio)-2-methylphenoxy)-2-methylpropionic acid (compound 5)

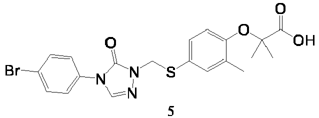

Compound 5 was prepared by replacing *o*-fluorophenol in Example 1 with *o*-cresol according to the method of Example 1: ¹H NMR (300 MHz, DMSO-*d₆*) δ 13.01 (s, 1H), 8.52 (s, 1H), 7.66 (dd, *J* = 31.7, 8.8 Hz, 4H), 7.21 (d, *J* = 23.9 Hz, 2H), 6.63 (d, *J* = 8.5 Hz, 1H), 5.14 (s, 2H), 2.09 (s, 3H), 1.48 (s, 6H). HRMS (ESI) calcd. for C₂₀H₂₀BrN₃O₄S [M+H]⁺ 478.0436, found 478.0432.

### Example 6

### Ethyl 2-(4-(((4-(4-bromophenyl)-5-oxo-4,5-dihydro-1H-1,2,4-triazol-1-yl)methyl)thio)-2-methylphenoxy)ethyl-2-methylpropionate (compound 6)

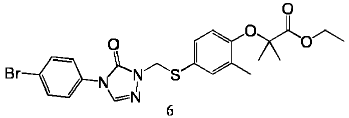

Compound 6 was prepared by replacing *o*-fluorophenol in Example 1 with *o*-cresol without hydrolysis according to the method of Example 1: ¹H NMR (300 MHz, CDCl₃) δ 7.70 (s, 1H), 7.61 (d, *J* = 8.8 Hz, 2H), 7.40 (d, *J* = 8.8 Hz, 2H), 7.31 (d, *J* = 1.8 Hz, 1H), 7.21 (dd, *J* = 8.5, 2.2 Hz, 1H), 6.59 (d, *J* = 8.5 Hz, 1H), 5.14 (s, 2H), 4.22 (q, *J* = 7.1 Hz, 2H), 2.19 (s, 3H), 1.59 (s, 6H), 1.24 (t, *J* = 7.1 Hz, 3H). MS (ESI): m/z 528.3 [M+Na]⁺.

### Example 7

### 2-(4-(((4-(4-Fluorophenyl)-5-oxo-4,5-dihydro-1H-1,2,4-triazol-1-yl)methyl)thio)-2-methylphenoxy)acetic acid (compound 7)

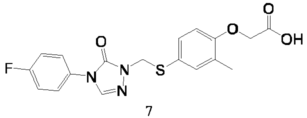

Compound 7 was prepared by replacing *p*-bromoaniline in Example 1 with *p*-fluoroaniline, *o*-fluorophenol with *o*-cresol, and ethyl bromoisobutyrate with ethyl 2-bromoacetate according to the method of Example 1: ¹H NMR (300 MHz, DMSO-*d₆*) *δ* 8.48 (s, 1H), 7.65 (dd, *J* = 8.9, 4.8 Hz, 2H), 7.37 (t, *J* = 8.8 Hz, 2H), 7.30 - 7.16 (m, 2H), 6.78 (d, *J* = 8.7 Hz, 1H), 5.15 (s, 2H), 4.68 (s, 2H), 2.13 (s, 3H). ESI-MS: m/z 388.1 [M-H]⁻.

### Example 8

### Ethyl 2-(4-(((4-(4-fluorophenyl)-5-oxo-4,5-dihydro-1H-1,2,4-triazol-1-yl)methyl)thio)-2-methylphenoxy)acetate (compound 8)

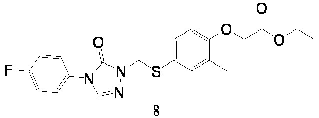

Compound 8 was prepared by replacing *p*-bromoaniline in Example 1 with *p*-fluoroaniline, *o*-fluorophenol with *o*-cresol, and ethyl bromoisobutyrate with ethyl 2-bromoacetate without hydrolysis according to the method of Example 1: ¹H NMR (300 MHz, CDCl₃) δ 7.67 (s, 1H), 7.51 - 7.42 (m, 2H), 7.33 (d, *J* = 7.3 Hz, 2H), 7.18 (t, *J* = 8.5 Hz, 2H), 6.65 (d, *J* = 8.8 Hz, 1H), 5.16 (s, 2H), 4.63 (s, 2H), 4.27 (q, *J* = 7.1 Hz, 2H), 2.25 (s, 3H), 1.31 (t, *J* = 7.1 Hz, 3H). MS (ESI): m/z 440.1 [M+Na]⁺.

### Example 9

### 2-(4-(((4-(4-Chlorophenyl)-5-oxo-4,5-dihydro-1H-1,2,4-triazol-1-yl)methyl)thio)-2-methylphenoxy)acetic acid (compound 9)

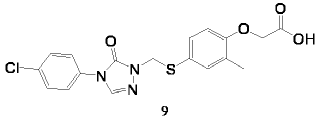

Compound 9 was prepared by replacing *p*-bromoaniline in Example 1 with *p*-chloroaniline, *o*-fluorophenol with *o*-cresol, and ethyl bromoisobutyrate with ethyl 2-bromoacetate according to the method of Example 1: ¹H NMR (300 MHz, DMSO-*d₆*) *δ* 12.99 (s, 1H), 8.52 (s, 1H), 7.67 (d, *J* = 8.5 Hz, 2H), 7.58 (d, *J* = 8.5 Hz, 2H), 7.34 - 7.07 (m, 2H), 6.79 (d, *J* = 8.6 Hz, 1H), 5.14 (s, 2H), 4.68 (s, 2H), 2.13 (s, 3H). ESI-MS: m/z 404.1 [M-H]⁻.

### Example 10

### 2-(4-(((4-(4-Bromophenyl)-5-oxo-4,5-dihydro-1H-1,2,4-triazol-1-yl)methyl)thio)-2-methylphenoxy)acetic acid (compound 10)

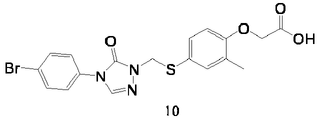

Compound 10 was prepared by replacing *o*-fluorophenol in Example 1 with *o*-cresol and ethyl 2-bromoisobutyrate with ethyl 2-bromoacetate according to the method of Example 1: ¹H NMR (300 MHz, DMSO-*d*₆) *δ* 8.53 (s, 1H), 7.72 (d, *J* = 8.8 Hz, 2H), 7.61 (d, *J* = 8.8 Hz, 2H), 7.26 - 7.14 (m, 2H), 6.72 (d, *J* = 9.2 Hz, 1H), 5.12 (s, 2H), 4.48 (s, 2H), 2.11 (s, 3H). ESI-MS: m/z 448.0 [M-H]⁻.

### Example 11

### Ethyl 2-(4-(((4-(4-bromophenyl)-5-oxo-4,5-dihydro-1H-1,2,4-triazol-1-yl)methyl)thio)-2-methylphenoxy)acetate (compound 11)

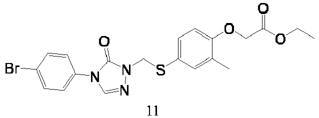

Compound 11 was prepared by replacing *o*-fluorophenol in Example 1 with *o*-cresol and ethyl 2-bromoisobutyrate with ethyl 2-bromoacetate without hydrolysis according to the method of Example 1: ¹H NMR (300 MHz, CDCl₃) δ 7.70 (s, 1H), 7.61 (d, *J* = 8.7 Hz, 2H), 7.40 (d, *J* = 8.7 Hz, 2H), 7.32 (d, *J* = 7.1 Hz, 2H), 6.65 (d, *J* = 8.9 Hz, 1H), 5.15 (s, 2H), 4.63 (s, 2H), 4.27 (q, *J* = 7.1 Hz, 2H), 2.25 (s, 3H), 1.31 (t, *J* = 7.2 Hz, 3H). MS (ESI): m/z 500.1 [M+Na]⁺.

### Example 12

### 2-(4-(((4-(4-Iodophenyl)-5-oxo-4,5-dihydro-1H-1,2,4-triazol-1 -yl)methyl)thio)-2-methylphenoxy)acetic acid (compound 12)

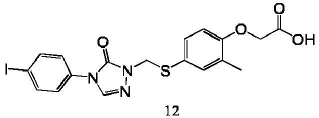

Compound 12 was prepared by replacing *p*-bromoaniline in Example 1 with *p*-iodoaniline, *o*-fluorophenol with *o*-cresol, and ethyl 2-bromoisobutyrate with ethyl 2-bromoacetate according to the method of Example 1: ¹H NMR (300 MHz, DMSO-*d*₆) *δ*12.97 (s, 1H), 8.50 (s, 1H), 7.85 (d, *J* = 8.6 Hz, 2H), 7.44 (d, *J* = 8.6 Hz, 2H), 7.29 - 7.18 (m, 2H), 6.77 (d, *J* = 9.1 Hz, 1H), 5.12 (s, 2H), 4.67 (s, 2H), 2.11 (s, 3H). HRMS (ESI) calcd. for C₁₈H₁₆IKN₃O₄S [M+K]⁺ 535.95433, found 535.95587.

### Example 13

### Ethyl 2-(4-(((4-(4-iodophenyl)-5-oxo-4,5-dihydro-1H-1,2,4-triazol-1-yl)methyl)thio)-2-methylphenoxy)acetate (compound 13)

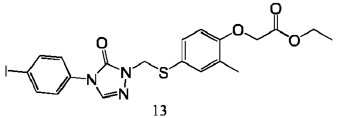

Compound 13 was prepared by replacing *p*-bromoaniline in Example 1 with *p*-iodoaniline, *o*-fluorophenol with *o*-cresol, and ethyl 2-bromoisobutyrate with ethyl 2-bromoacetate without hydrolysis according to the method of Example 1: ¹H NMR (300 MHz, CDCl₃) δ 7.81 (d, *J* = 8.7 Hz, 2H), 7.70 (s, 1H), 7.32 (d, *J* = 7.0 Hz, 2H), 7.27 (d, *J* = 4.6 Hz, 2H), 6.65 (d, *J* = 9.0 Hz, 1H), 5.15 (s, 2H), 4.63 (s, 2H), 4.27 (q, *J* = 7.1 Hz, 2H), 2.25 (s, 3H), 1.31 (t, *J* = 7.1 Hz, 3H). MS (ESI): m/z 548.1 [M+Na]⁺.

### Example 14

### 2-(4-(((4-(4-Methoxyphenyl)-5-oxo-4,5-dihydro-1H-1,2,4-triazol-1-yl)methyl)thio)-2-methylphenoxy)acetic acid (compound 14)

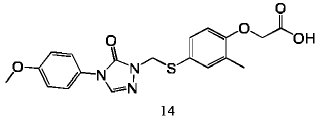

Compound 14 was prepared by replacing *p*-bromoaniline in Example 1 with *p-*methoxyaniline, *o*-fluorophenol with *o*-cresol, and ethyl 2-bromoisobutyrate with ethyl 2-bromoacetate according to the method of Example 1: ¹H NMR (300 MHz, DMSO-*d*₆) *δ* 13.00 (s, 1H), 8.38 (s, 1H), 7.48 (d, *J* = 9.0 Hz, 2H), 7.30 - 7.22 (m, 2H), 7.06 (d, *J* = 9.0 Hz, 2H), 6.79 (d, *J* = 9.2 Hz, 1H), 5.14 (s, 2H), 4.68 (s, 2H), 3.79 (s, 3H), 2.14 (s, 3H). ESI-MS: m/z 400.2 [M-H]⁻.

### Example 15

### 2-(2-Methyl-4-(((5-oxo-4-(4-(trifluoromethoxy)phenyl)-4,5-dihydro-1H-1,2,4-triazol-1-yl)methyl)thio)phenoxy)acetic acid (compound 15)

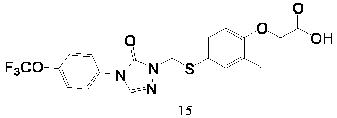

Compound 15 was prepared by replacing *p*-bromoaniline in Example 1 with *p-*trifluoromethoxyaniline, *o*-fluorophenol with *o*-cresol, and ethyl 2-bromoisobutyrate with ethyl 2-bromoacetate according to the method of Example 1: ¹H NMR (300 MHz, DMSO-*d*₆) *δ* 13.00 (s, 1H), 8.54 (s, 1H), 7.77 (d, *J* = 9.0 Hz, 2H), 7.54 (d, *J* = 8.6 Hz, 2H), 7.30 - 7.21 (m, 2H), 6.79 (d, *J* = 9.2 Hz, 1H), 5.15 (s, 2H), 4.69 (s, 2H), 2.13 (s, 3H). ESI-MS: m/z 454.2 [M-H]⁻.

### Example 16

### 2-(2-Methyl-4-(((5-oxo-4-(4-(trifluoromethyl)phenyl)-4,5-dihydro-1H-1,2,4-triazol-1-yl)methyl)thio)phenoxy)acetic acid (compound 16)

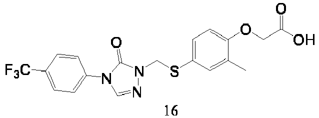

Compound 16 was prepared by replacing *p*-bromoaniline in Example 1 with *p-*trifluoromethylaniline, *o*-fluorophenol with *o*-cresol, and ethyl 2-bromoisobutyrate with ethyl 2-bromoacetate according to the method of Example 1: ¹H NMR (300 MHz, DMSO-*d*₆) *δ* 13.06 (s, 1H), 8.64 (s, 1H), 7.96 - 7.84 (m, 4H), 7.30 - 7.21 (m, 2H), 6.79 (d, *J* = 9.1 Hz, 1H), 5.16 (s, 2H), 4.68 (s, 2H), 2.12 (s, 3H). ESI-MS: m/z 438.2 [M-H]⁻.

### Example 17

### Ethyl 2-(2-methyl-4-(((5-oxo-4-(4-(trifluoromethyl)phenyl)-4,5-dihydro-1H-1,2,4-triazol-1-yl)methyl)thio)phenoxy)acetate (compound 17)

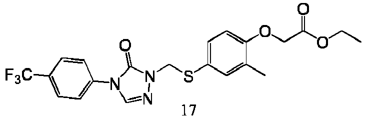

Compound 17 was prepared by replacing *p*-bromoaniline in Example 1 with *p-*trifluoromethylaniline, *o*-fluorophenol with *o*-cresol, and ethyl 2-bromoisobutyrate with ethyl 2-bromoacetate without hydrolysis according to the method of Example 1: ¹H NMR (300 MHz, CDCl₃) *δ* 7.77 (d, *J* = 10.1 Hz, 2H), 7.69 (d, *J* = 8.6 Hz, 2H), 7.33 (d, *J* = 6.1 Hz, 2H), 6.68 - 6.63 (m, 1H), 5.17 (s, 2H), 4.63 (s, 2H), 4.27 (q, *J* = 7.1 Hz, 2H), 2.25 (s, 3H), 1.31 (t, *J* = 7.1 Hz, 3H). ESI-MS: m/z 490.1 [M+Na]⁺.

### Example 18

### 2-(2-Methyl-4-(((5-oxo-4-phenyl-4,5-dihydro-1H-1,2,4-triazol-1-yl)methyl)thio)phenoxy)acetic acid (compound 18)

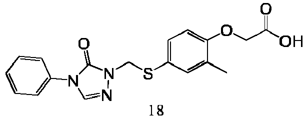

Compound 18 was prepared by replacing *p*-bromoaniline in Example 1 with aniline, *o-*fluorophenol with *o*-cresol, and ethyl 2-bromoisobutyrate with ethyl 2-bromoacetate according to the method of Example 1: ¹H NMR (300 MHz, DMSO-*d₆*) *δ*12.99 (s, 1H), 8.50 (s, 1H), 7.62 (d, *J* = 8.2 Hz, 2H), 7.52 (t, *J* = 7.8 Hz, 2H), 7.38 (t, *J* = 7.3 Hz, 1H), 7.29 - 7.21 (m, 2H), 6.80 (d, *J* = 9.2 Hz, 1H), 5.16 (s, 2H), 4.69 (s, 2H), 2.14 (s, 3H). HRMS (ESI) calcd. for C₁₈H₁₇N₃O₄S [M+Na]⁺ 394.08375, found 394.08348.

### Example 19

### 2-(2-Methyl-4-(((5-oxo-4-(4-((trifluoromethyl)thio)phenyl)-4, 5-dihydro-1H-1,2,4-triazol-1-yl)4-methyl)thio)phenoxy)acetic acid (compound 19)

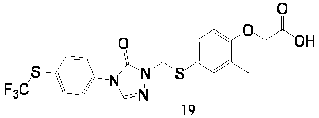

Compound 19 was prepared by replacing *p*-bromoaniline in Example 1 with *p-*trifluoromethioaniline, *o*-fluorophenol with *o*-cresol, and ethyl 2-bromoisobutyrate with ethyl 2-bromoacetate according to the method of Example 1: ¹H NMR (300 MHz, DMSO-*d₆*) δ 12.98 (s, 1H), 8.60 (s, 1H), 7.84 (q, *J* = 8.9 Hz, 4H), 7.23 (d, *J* = 5.1 Hz, 2H), 6.76 (d, *J* = 9.1 Hz, 1H), 5.14 (s, 2H), 4.67 (s, 2H), 2.10 (s, 3H). HRMS (ESI) calcd. for C₁₉H₁₆F₃N₃O₄S₂ [M+H]⁺ 472.0613, found 472.0613.

### Example 20

### 2-(4-(((4-(4-Ethylphenyl)-5-oxo-4,5-dihydro-1H-1,2,4-triazol-1-yl)methyl)thio)-2-methylphenoxy)acetic acid (compound 20)

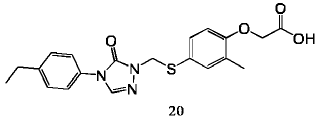

Compound 20 was prepared by replacing *p*-bromoaniline in Example 1 with *p*-ethylaniline, *o*-fluorophenol with *o*-cresol, and ethyl 2-bromoisobutyrate with ethyl 2-bromoacetate according to the method of Example 1: ¹H NMR (300 MHz, DMSO-*d*₆) *δ*12.98 (s, 1H), 8.45 (s, 1H), 7.50 (d, *J* = 8.3 Hz, 2H), 7.34 (d, *J* = 8.3 Hz, 2H), 7.29 - 7.22 (m, 2H), 6.79 (d, *J* = 9.1 Hz, 1H), 5.15 (s, 2H), 4.69 (s, 2H), 2.64 (q, *J* = 7.6 Hz, 2H), 2.14 (s, 3H), 1.19 (t, *J* = 7.6 Hz, 3H). HRMS (ESI) calcd. for C₂₀H₂₂N₃O₄S [M+H]⁺ 400.13310, found 400.13260.

### Example 21

### Ethyl 2-(4-(((4-(4-ethylphenyl)-5-oxo-4,5-dihydro-1H-1,2,4-triazol-1-yl)methyl)thio)-2-methylphenoxy)acetate (compound 21)

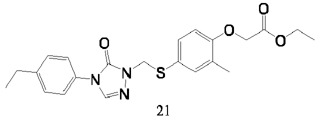

Compound 21 was prepared by replacing *p*-bromoaniline in Example 1 with *p*-ethylaniline, *o*-fluorophenol with *o*-cresol and ethyl 2-bromoisobutyrate with ethyl 2-bromoacetate without hydrolysis according to the method of Example 1: ¹H NMR (300 MHz, CDCl₃) δ 7.68 (s, 1H), 7.38 (d, *J* = 8.4 Hz, 2H), 7.34 (d, *J* = 7.3 Hz, 2H), 7.28 (s, 2H), 6.65 (d, *J =* 9.1 Hz, 1H), 5.16 (s, 2H), 4.63 (s, 2H), 4.27 (q, *J* = 7.2 Hz, 2H), 2.70 (q, *J* = 7.6 Hz, 2H), 2.26 (s, 3H), 1.31 (t, *J* = 5.8 Hz, 3H), 1.26 (t, *J* = 6.2 Hz, 3H). MS (ESI): m/z 450.2 [M+Na]⁺.

### Example 22

### 2-(2-Methyl-4-(((4-(4-nitrophenyl)-5-oxo-4,5-dihydro-1H-1,2,4-triazol-1-yl)methyl)thio]phenoxy)acetic acid (compound 22)

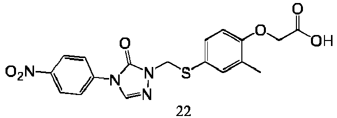

Compound 22 was prepared by replacing *p*-bromoaniline in Example 1 with *p*-nitroaniline, *o*-fluorophenol with *o*-cresol, and ethyl 2-bromoisobutyrate with ethyl 2-bromoacetate according to the method of Example 1: ¹H NMR (300 MHz, DMSO-*d*₆) *δ*12.99 (s, 1H), 8.73 (s, 1H), 8.39 (d, *J* = 9.0 Hz, 2H), 8.01 (d, *J* = 9.0 Hz, 2H), 7.29 - 7.21 (m, 2H), 6.79 (d, *J* = 9.0 Hz, 1H), 5.18 (s, 2H), 4.69 (s, 2H), 2.12 (s, 3H). HRMS (ESI) calcd. for C₁₈H₁₆N₄NaO₆S [M+Na]⁺ 439.06882, found 439.06834.

### Example 23

### 2-(4-(((4-(4-Ethoxyphenyl)-5-oxo-4,5-dihydro-1H-1,2,4-triazol-1-yl)methyl)thio)-2-methylphenoxy)acetic acid (compound 23)

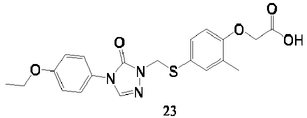

Compound 23 was prepared by replacing *p*-bromoaniline in Example 1 with *p-*ethoxyaniline, *o*-fluorophenol with *o*-cresol, and ethyl 2-bromoisobutyrate with ethyl 2-bromoacetate according to the method of Example 1: ¹H NMR (300 MHz, DMSO-*d*₆) *δ*12.99 (s, 1H), 8.38 (s, 1H), 7.46 (d, *J* = 8.9 Hz, 2H), 7.32 - 7.21 (m, 2H), 7.03 (d, *J* = 9.0 Hz, 2H), 6.79 (d, *J* = 9.2 Hz, 1H), 5.13 (s, 2H), 4.68 (s, 2H), 4.06 (q, *J* = 6.9 Hz, 2H), 2.14 (s, 3H), 1.33 (t, *J* = 7.0 Hz, 3H). HRMS (ESI) calcd. for C₂₀H₂₂N₃O₅S [M+H]⁺ 416.12802, found 416.12760.

### Example 24

### Ethyl 2-(4-(((4-(4-ethoxyphenyl)-5-oxo-4,5-dihydro-1H-1,2,4-triazol-1-yl)methyl)thio)-2-methylphenoxy)acetate (compound 24)

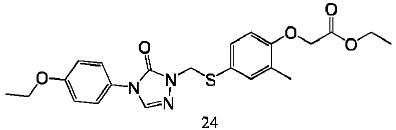

Compound 24 was prepared by replacing *p*-bromoaniline in Example 1 with *p-*ethoxyaniline, *o*-fluorophenol with *o*-cresol and ethyl 2-bromoisobutyrate with ethyl 2-bromoacetate without hydrolysis according to the method of Example 1: ¹H NMR (300 MHz, CDCl₃) δ 7.63 (s, 1H), 7.38 - 7.35 (m, 2H), 7.33 (d, *J* = 3.1 Hz, 2H), 6.97 (d, *J* = 8.9 Hz, 2H), 6.65 (d, *J* = 8.5 Hz, 1H), 5.16 (s, 2H), 4.63 (s, 2H), 4.27 (q, *J* = 7.1 Hz, 2H), 4.07 (q, *J* = 7.0 Hz, 2H), 2.26 (s, 3H), 1.45 (t, *J* = 7.0 Hz, 3H), 1.31 (t, *J* = 7.1 Hz, 3H). MS (ESI): m/z 466.2 [M+Na]⁺.

### Example 25

### 2-(2-Methyl-4-(((4-(4-(methylsulfonyl)phenyl)-5-oxo-4,5-dihydro-1H-1,2,4-triazol-1-yl)methyl]thio)phenoxy)acetate(compound 25)

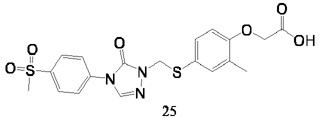

Compound 25 was prepared by replacing *p*-bromoaniline in Example 1 with *p-*methylsulfonylaniline, *o*-fluorophenol with *o*-cresol, and ethyl 2-bromoisobutyrate with ethyl 2-bromoacetate according to the method of Example 1: ¹H NMR (300 MHz, DMSO-*d*₆) δ13.00 (s, 1H), 8.67 (s, 1H), 8.07 (d, *J* = 8.6 Hz, 2H), 7.96 (d, *J* = 8.7 Hz, 2H), 7.29 - 7.22 (m, 2H), 6.79 (d, *J* = 9.1 Hz, 1H), 5.17 (s, 2H), 4.69 (s, 2H), 3.26 (s, 3H), 2.13 (s, 3H). HRMS (ESI) calcd. for C₁₉H₁₉N₃NaO₆S₂ [M+Na]⁺ 472.06130, found 472.06069.

### Example 26

### 2-(4-(((4-(2-Chlorophenyl)-5-oxo-4,5-dihydro-1H-1,2,4-triazol-1-yl)methyl)thio)-2-methylphenoxy)acetic acid (compound 26)

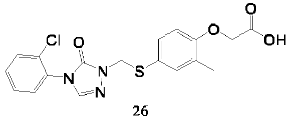

Compound 26 was prepared by replacing *p*-bromoaniline in Example 1 with *o-*chloroaniline, *o*-fluorophenol with *o*-cresol, and ethyl 2-bromoisobutyrate with ethyl 2-bromoacetate according to the method of Example 1: ¹H NMR (300 MHz, DMSO-*d*₆) *δ*12.97 (s, 1H), 8.25 (s, 1H), 7.73 - 7.65 (m, 1H), 7.58 - 7.49 (m, 3H), 7.31 - 7.23 (m, 2H), 6.80 (d, *J* = 8.3 Hz, 1H), 5.14 (s, 2H), 4.69 (s, 2H), 2.15 (s, 3H). HRMS (ESI) calcd. for C₁₈H₁₇ClN₃O₄S [M+H]⁺ 406.06283, found 406.06248.

### Example 27

### Ethyl 2-(4-(((4-(2-chlorophenyl)-5-oxo-4,5-dihydro-1H-1,2,4-triazol-1-yl)methyl)thio)-2-methylphenoxy)acetate (compound 27)

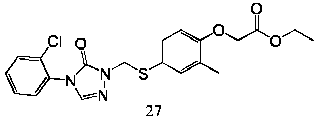

Compound 27 was prepared by replacing *p*-bromoaniline in Example 1 with *o-*chloroaniline, *o*-fluorophenol with *o*-cresol, and ethyl 2-bromoisobutyrate with ethyl 2-bromoacetate without hydrolysis according to the method of Example 1: ¹H NMR (300 MHz, CDCl₃) δ 7.61 (s, 1H), 7.58 - 7.52 (m, 1H), 7.42 (d, *J* = 5.7 Hz, 1H), 7.39 (d, *J* = 3.0 Hz, 2H), 7.34 (d, *J* = 6.4 Hz, 2H), 6.65 (d, *J* = 9.1 Hz, 1H), 5.16 (s, 2H), 4.63 (s, 2H), 4.27 (q, *J* = 7.1 Hz, 2H), 2.27 (s, 3H), 1.30 (t, *J* = 7.1 Hz, 3H). MS (ESI): m/z 456.1 [M+Na]⁺.

### Example 28

### 2-(4-(((4-(2-Bromophenyl)-5-oxo-4,5-dihydro-1H-1,2,4-triazol-1-yl)methyl)thio)-2-methylphenoxy)acetic acid (compound 28)

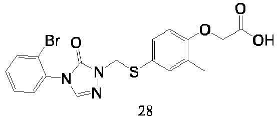

Compound 28 was prepared by replacing *p*-bromoaniline in Example 1 with *o-*bromoaniline, *o*-fluorophenol with *o*-cresol, and ethyl 2-bromoisobutyrate with ethyl 2-bromoacetate according to the method of Example 1: ¹H NMR (300 MHz, DMSO-*d*₆) *δ*8.24 (s, 1H), 7.89 - 7.80 (m, 1H), 7.59 - 7.43 (m, 3H), 7.30 (d, *J* = 1.6 Hz, 1H), 7.26 (dd, *J* = 8.5, 2.0 Hz, 1H), 6.81 (d, *J* = 8.5 Hz, 1H), 5.14 (s, 2H), 4.69 (s, 2H), 2.16 (s, 3H). HRMS (ESI) calcd. for C₁₈H₁₇BrN₃O₄S [M+H]⁺ 452.01027, found 452.01044.

### Example 29

### Ethyl 2-(4-((4-(2-bromophenyl)-5-oxo-4,5-dihydro-1H-1,2,4-triazol-1-yl)methyl)thio)-2-methylphenoxy)acetate (compound 29)

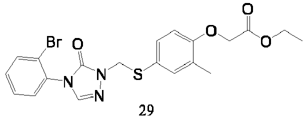

Compound 29 was prepared by replacing *p*-bromoaniline in Example 1 with *o-*bromoaniline, *o*-fluorophenol with *o*-cresol, and ethyl 2-bromoisobutyrate with ethyl 2-bromoacetate without hydrolysis according to the method of Example 1: ¹H NMR (300 MHz, CDCl₃) δ 7.72 (d, *J* = 7.7 Hz, 1H), 7.59 (s, 1H), 7.46 (t, *J* = 7.6 Hz, 1H), 7.37 (d, *J* = 2.7 Hz, 2H), 7.33 (d, *J* = 8.0 Hz, 2H), 6.65 (d, *J* = 9.0 Hz, 1H), 5.17 (s, 2H), 4.63 (s, 2H), 4.27 (q, *J* = 7.1 Hz, 2H), 2.27 (s, 3H), 1.30 (t, *J* = 7.2 Hz, 3H). MS (ESI): m/z 500.1 [M+Na]⁺.

### Example 30

### 2-(2-Methyl-4-(((5-oxo-4-(2-(trifluoromethyl)phenyl)-4,5-dihydro-1H-1,2,4-triazol-1-yl)methyl)thio)phenoxy)acetic acid (compound 30)

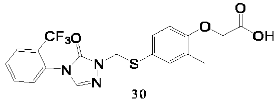

Compound 30 was prepared by replacing *p*-bromoaniline in Example 1 with *o-*trifluoromethylaniline, *o*-fluorophenol with *o*-cresol, and ethyl 2-bromoisobutyrate with ethyl 2-bromoacetate according to the method of Example 1: ¹H NMR (300 MHz, DMSO-*d*₆) *δ*8.20 (s, 1H), 7.95 (d, *J* = 7.9 Hz, 1H), 7.88 (t, *J* = 7.5 Hz, 1H), 7.77 (t, *J* = 7.7 Hz, 1H), 7.57 (d, *J* = 7.8 Hz, 1H), 7.30 - 7.22 (m, 2H), 6.81 (d, *J =* 8.3 Hz, 1H), 5.12 (s, 2H), 4.70 (s, 2H), 2.16 (s, 3H). HRMS (ESI) calcd. for C₁₉H₁₇F₃N₃O₄S [M+H]⁺ 440.08919, found 440.08896.

### Example 31

### Ethyl 2-(2-methyl-4-(((5-oxo-4-(2-(trifluoromethyl)phenyl)-4,5-dihydro-1H-1,2,4-triazol-1-yl)methyl)thio)phenoxy)acetate (compound 31)

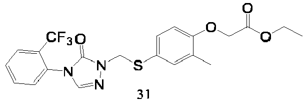

Compound 31 was prepared by replacing *p*-bromoaniline in Example 1 with *o-*trifluoromethylaniline, *o*-fluorophenol with *o*-cresol, and ethyl 2-bromoisobutyrate with ethyl 2-bromoacetate without hydrolysis according to the method of Example 1: ¹H NMR (300 MHz, CDCl₃) δ 7.83 (d, *J* = 7.9 Hz, 1H), 7.73 (t, 1H), 7.62 (t, 1H), 7.49 (s, 1H), 7.39 (d, *J* = 8.1 Hz, 1H), 7.34 (d, *J* = 8.5 Hz, 2H), 6.66 (d, *J* = 8.1 Hz, 1H), 5.14 (s, 2H), 4.63 (s, 2H), 4.27 (q, *J* = 14.2, 7.1 Hz, 2H), 2.27 (s, 3H), 1.30 (t, *J* = 7.1 Hz, 3H). MS (ESI): m/z 490.1 [M+Na]⁺.

### Example 32

### 2-(4-(((4-(4-Chloro-3-methylphenyl)-5-oxo-4,5-dihydro-1H-1,2,4-triazol-1-yl)methyl)thio)-2-methylphenoxy)acetic acid (compound 32)

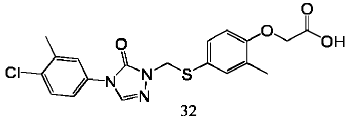

Compound 32 was prepared by replacing *p*-bromoaniline in Example 1 with 3-methyl-4-chloroaniline, *o*-fluorophenol with *o*-cresol, and ethyl 2-bromoisobutyrate with ethyl 2-bromoacetate according to the method of Example 1: ¹H NMR (300 MHz, DMSO-*d*₆) *δ*8.51 (s, 1H), 7.74 (d, *J* = 2.1 Hz, 1H), 7.55 - 7.43 (m, 2H), 7.30 - 7.16 (m, 2H), 6.77 (d, *J* = 9.1 Hz, 1H), 5.12 (s, 2H), 4.66 (s, 2H), 2.34 (s, 3H), 2.11 (s, 3H). HRMS (ESI) calcd. for C₁₉H₁₉ClN₃O₄S [M+H]⁺ 420.07848, found 420.07738.

### Example 33

### Ethyl 2-(4-(((4-(4-chloro-3-methylphenyl)-5-oxo-4,5-dihydro-1H-1,2,4-triazol-1-yl)methyl)thio)-2-methylphenoxy)acetate (compound 33)

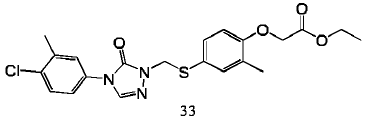

Compound 33 was prepared by replacing *p*-bromoaniline in Example 1 with 3-methyl-4-chloroaniline, *o*-fluorophenol with *o*-cresol and ethyl 2-bromoisobutyrate with ethyl 2-bromoacetate without hydrolysis according to the method of Example 1: ¹H NMR (300 MHz, CDCl₃) δ 7.68 (s, 1H), 7.52 (s, 1H), 7.32 (s, 4H), 6.65 (d, *J* = 9.0 Hz, 1H), 5.15 (s, 2H), 4.63 (s, 2H), 4.27 (q, *J* = 7.1 Hz, 2H), 2.42 (s, 3H), 2.26 (s, 3H), 1.31 (t, *J* = 7.1 Hz, 3H). MS (ESI): m/z 470.1 [M+Na]⁺.

### Example 34

### 2-(4-(((4-(2-Bromo-5-fluorophenyl)-5-oxo-4,5-dihydro-1H-1,2,4-triazol-1-yl)methyl)thio)-2-methylphenoxy)acetic acid (compound 34)

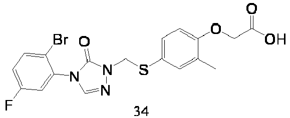

Compound 34 was prepared by replacing *p*-bromoaniline in Example 1 with 2-bromo-5-fluoroaniline, *o*-fluorophenol with *o*-cresol, and ethyl 2-bromoisobutyrate with ethyl 2-bromoacetate according to the method of Example 1: ¹H NMR (300 MHz, DMSO-*d*₆) *δ*8.24 (s, 1H), 7.89 (dd, *J* = 8.9, 5.6 Hz, 1H), 7.57 (dd, *J* = 9.0, 3.0 Hz, 1H), 7.44 - 7.36 (m, 1H), 7.32 - 7.24 (m, 2H), 6.80 (d, *J* = 8.4 Hz, 1H), 5.14 (s, 2H), 4.66 (s, 2H), 2.16 (s, 3H). HRMS (ESI) calcd. for C₁₈H₁₆BrFN₃O₄S [M+H]⁺ 470.00085, found 469.99949.

### Example 35

### Ethyl 2-(4-(((4-(2-bromo-5-fluorophenyl)-5-oxo-4,5-dihydro-1H-1,2,4-triazol-1-yl)methyl)thio)-2-methylphenoxy)acetate (compound 35)

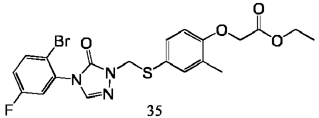

Compound 35 was prepared by replacing *p*-bromoaniline in Example 1 with 2-bromo-5-fluoroaniline, *o*-fluorophenol with *o*-cresol, and ethyl 2-bromoisobutyrate with ethyl 2-bromoacetate without hydrolysis according to the method of Example 1: ¹H NMR (300 MHz, CDCl₃) δ 7.68 (dd, *J* = 8.9, 5.5 Hz, 1H), 7.62 (s, 1H), 7.34 (d, *J* = 7.8 Hz, 2H), 7.19 - 7.02 (m, 2H), 6.66 (d, *J* = 8.3 Hz, 1H), 5.16 (s, 2H), 4.63 (s, 2H), 4.27 (q, *J* = 7.1 Hz, 2H), 2.27 (s, 3H), 1.30 (t, *J* = 7.1 Hz, 3H). MS (ESI): m/z 518.1 [M+Na]⁺.

### Example 36

### 2-(4-(((4-(4-Bromo-2-fluorophenyl)-5-oxo-4, 5-dihydro-1H-1,2,4-triazol-1-yl)methyl)thio)-2-methylphenoxy)acetic acid (compound 36)

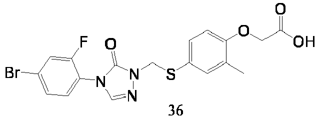

Compound 36 was prepared by replacing *p*-bromoaniline in Example 1 with 2-fluoro-4-bromoaniline, *o*-fluorophenol with *o*-cresol, and ethyl 2-bromoisobutyrate with ethyl 2-bromoacetate according to the method of Example 1: ¹H NMR (300 MHz, DMSO-*d*₆) *δ*12.98 (s, 1H), 8.29 (d, *J* = 1.2 Hz, 1H), 7.86 (dd, *J* = 10.0, 1.9 Hz, 1H), 7.60 (dd, *J* = 9.1, 1.5 Hz, 1H), 7.52 (t, *J* = 8.3 Hz, 1H), 7.29 - 7.20 (m, 2H), 6.79 (d, *J* = 9.0 Hz, 1H), 5.14 (s, 2H), 4.69 (s, 2H), 2.14 (s, 3H). HRMS (ESI) calcd. for C₁₈H₁₅BrFN₃NaO₄S [M+Na]⁺ 491.98279, found 491.98201.

### Example 37

### 2-(4-(((4-(3-Chloro-2-fluorophenyl)-5-oxo-4, 5-dihydro-1H-1,2,4-triazol-1-yl)methyl)thio)-2-methylphenoxy)acetic acid (compound 37)

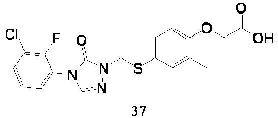

Compound 37 was prepared by replacing *p*-bromoaniline in Example 1 with 2-fluoro-3-chloroaniline, *o*-fluorophenol with *o*-cresol, and ethyl 2-bromoisobutyrate with ethyl 2-bromoacetate according to the method of Example 1: ¹H NMR (300 MHz, DMSO-*d*₆) *δ*12.97 (s, 1H), 8.33 (d, *J* = 1.5 Hz, 1H), 7.77 - 7.68 (m, 1H), 7.58 - 7.51 (m, 1H), 7.43 - 7.36 (m, 1H), 7.28 - 7.22 (m, 2H), 6.80 (d, *J* = 9.1 Hz, 1H), 5.14 (s, 2H), 4.69 (s, 2H), 2.15 (s, 3H). HRMS (ESI) calcd. for C₁₈H₁₅ClFN₃NaO₄S [M+Na]⁺ 446.03535, found 446.03464.

### Example 38

### Ethyl 2-(4-(((4-(3-chloro-2-fluorophenyl)-5-oxo-4,5-dihydro-1H-1,2,4-triazol-1-yl)methyl)thio)-2-methylphenoxy)acetate (compound 38)

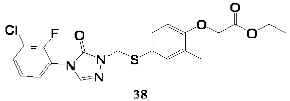

Compound 38 was prepared by replacing *p*-bromoaniline in Example 1 with 2-fluoro-3-chloroaniline, *o*-fluorophenol with *o*-cresol, and ethyl 2-bromoisobutyrate with ethyl 2-bromoacetate without hydrolysis according to the method of Example 1: ¹H NMR (300 MHz, CDCl₃) δ 7.69 (d, *J* = 2.8 Hz, 1H), 7.52 (t, *J* = 7.4 Hz, 1H), 7.46 (t, *J* = 7.5 Hz, 1H), 7.33 (d, *J* = 6.2 Hz, 2H), 7.23 (t, *J* = 8.1 Hz, 1H), 6.66 (d, *J* = 9.1 Hz, 1H), 5.15 (s, 2H), 4.63 (s, 2H), 4.27 (q, *J* = 7.1 Hz, 2H), 2.26 (s, 3H), 1.31 (t, *J* = 7.1 Hz, 3H). MS (ESI): m/z 474.1 [M+Na]⁺.

### Example 39

### 2-(4-(((4-(2-Bromo-3-chlorophenyl)-5-oxo-4,5-dihydro-1H-1,2,4-triazol-1-yl)methyl)thio)-2-methylphenoxy)acetic acid (compound 39)

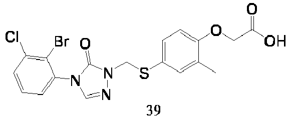

Compound 39 was prepared by replacing *p*-bromoaniline in Example 1 with 2-bromo-3-chloroaniline, *o*-fluorophenol with *o*-cresol, and ethyl 2-bromoisobutyrate with ethyl 2-bromoacetate according to the method of Example 1: ¹H NMR (300 MHz, DMSO-*d*₆) *δ*12.97 (s, 1H), 8.33 (d, *J* = 1.5 Hz, 1H), 7.77 - 7.68 (m, 1H), 7.58 - 7.51 (m, 1H), 7.43 - 7.36 (m, 1H), 7.28 - 7.22 (m, 2H), 6.80 (d, *J* = 9.1 Hz, 1H), 5.14 (s, 2H), 4.69 (s, 2H), 2.15 (s, 3H). HRMS (ESI) calcd. for C₁₈H₁₆BrClN₃O₄S [M+H]⁺ 485.97130, found 485.97167.

### Example 40

### Ethyl 2-(4-(((4-(2-bromo-3-chlorophenyl)-5-oxo-4,5-dihydro-1H-1,2,4-triazol-1-yl)methyl)thio)-2-methylphenoxy)acetate (compound 40)

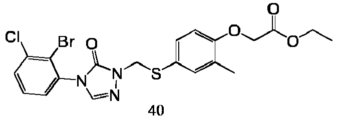

Compound 40 was prepared by replacing *p*-bromoaniline in Example 1 with 2-bromo-3-chloroaniline, *o*-fluorophenol with *o*-cresol, and ethyl 2-bromoisobutyrate with ethyl 2-bromoacetate without hydrolysis according to the method of Example 1: ¹H NMR (300 MHz, CDCl₃) δ 7.58 (d, *J* = 7.9 Hz, 2H), 7.42 (d, *J* = 8.0 Hz, 1H), 7.36 (t, *J* = 5.4 Hz, 2H), 7.28 (d, *J* = 2.7 Hz, 1H), 6.65 (d, *J* = 9.1 Hz, 1H), 5.16 (s, 2H), 4.64 (s, 2H), 4.27 (q, *J* = 7.1 Hz, 2H), 2.27 (s, 3H), 1.31 (t, *J* = 7.2 Hz, 3H). MS (ESI): m/z 534.0 [M+Na]⁺.

### Example 41

### 2-(4-(((4-(4-Bromophenyl)-5-oxo-4, 5-dihydro-1H-1,2,4-triazol-1-yl)methyl)thio)phenoxy)acetic acid (compound 41)

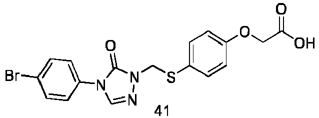

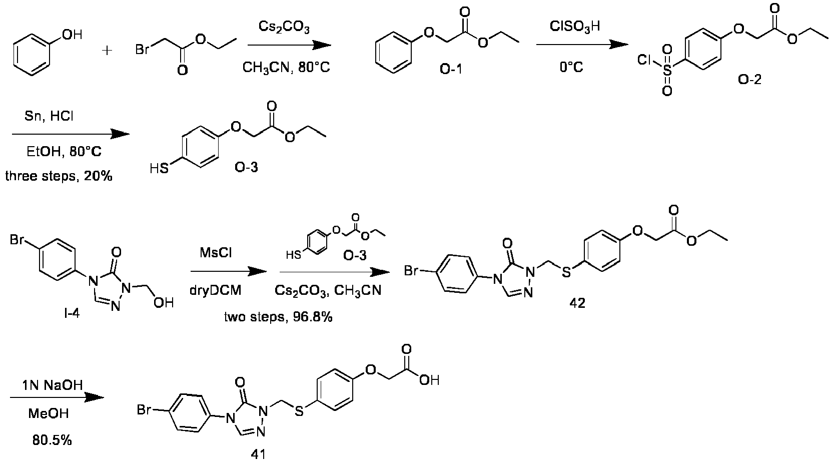

### Synthesis of intermediate O-1

Phenol (1.88 g, 20 mmol) was dissolved in acetonitrile (30 mL), and ethyl bromoacetate (2.66 mL, 24 mmol) and cesium carbonate (13.0 g, 40 mmol) were added. The system was transferred to an oil bath and reacted at 80 °C for 24 h. After the reaction was completed, the reaction liquid was filtered under reduced pressure through a Buchner funnel. The filtrate was diluted with water (200 mL), extracted with ethyl acetate (150 mL × 3), and concentrated by rotary evaporation under reduced pressure to remove the solvent to give a crude product of intermediate O-1, which was directly used in the next step without further purification.

### Synthesis of intermediate O-2

Chlorosulfonic acid (6.2 mL, 100 mmol) was slowly added to the crude product of intermediate O-1 obtained from the previous step under an ice bath. The mixture was stirred for 2 h. After the reaction was completed, the reaction system was poured into ice water (200 mL) and filtered under reduced pressure to give a crude product of intermediate O-2, which was directly used in the next step without further purification.

### Synthesis of intermediate O-3

The crude product of intermediate O-2 obtained from the previous step was dissolved in absolute ethanol (10 mL), and a saturated hydrogen chloride-ethanol solution (25 mL) and tin powder (3.3 g, 28 mmol) were added. The system was transferred to an oil bath and reacted at 80 °C for 24 h. After the reaction was completed, the reaction liquid was cooled to room temperature and filtered under reduced pressure. The filtrate was concentrated by rotary evaporation under reduced pressure to remove the solvent. The residue was diluted with water (50 mL) and extracted with ethyl acetate (25 mL × 3). The organic phase was washed with saturated brine (20 mL × 1), and concentrated by rotary evaporation under reduced pressure to remove the solvent. The residue was purified by column chromatography (petroleum ether/ethyl acetate = 80: 1) to give intermediate O-3 (yellow liquid, 634 mg).

### Synthesis of compound 42

Intermediate I-4 (162 mg, 0.6 mmol) was dissolved in anhydrous dichloromethane (5 mL), and triethylamine (0.17 mL, 1.2 mmol) and methylsufonyl chloride (0.07 mL, 0.9 mmol) were added. The mixture was stirred at room temperature for 2 h. After the reaction was completed, the reaction liquid was concentrated by rotary evaporation under reduced pressure to remove the solvent. The residue was diluted with water (10 mL) and extracted with ethyl acetate (15 mL × 3). The organic phase was washed with saturated brine (15 mL × 1), and concentrated by rotary evaporation under reduced pressure to remove the solvent. The residue was directly used in the next step without further purification.

The residue of the previous step was dissolved in acetonitrile (5 mL), and intermediate O-3 (489 mg, 1.5 mmol) and cesium carbonate (210 mg, 0.9 mmol) were added. The mixture was stirred at room temperature for 4 h. After the reaction was completed, the reaction liquid was concentrated by rotary evaporation under reduced pressure to remove the solvent. The residue was purified by column chromatography (petroleum ether/ethyl acetate = 5:1) to give compound 42 (white solid, 269.0 mg).

### Compound 41

Compound 42 (150 mg, 0.32 mmol) was dissolved in methanol (4 mL), and a 1 N NaOH solution (1.6 mL) was added. The mixture was stirred at room temperature for 24 h. After the reaction was completed, the reaction liquid was adjusted to pH 4 with a 1 N HCl solution, and concentrated by rotary evaporation under reduced pressure to remove the solvent. The residue was diluted with water (15 mL) and extracted with ethyl acetate (10 mL × 3). The organic phase was washed with saturated brine (15 mL × 1), and concentrated by rotary evaporation under reduced pressure to remove the solvent. The residue was purified by column chromatography (dichloromethane/methanol = 100:1) to give compound 41 (white solid, 112 mg): ¹H NMR (300 MHz, DMSO-*d₆*) δ 13.02 (s, 1H), 8.53 (s, 1H), 7.72 (d, *J* = 8.8 Hz, 2H), 7.61 (d, *J* = 8.8 Hz, 2H), 7.40 (d, *J* = 8.7 Hz, 2H), 6.88 (d, *J* = 8.7 Hz, 2H), 5.17 (s, 2H), 4.67 (s, 2H). HRMS (ESI) calcd. for C₁₇H₁₄BrN₃O₄S [M+H]⁺ 435.9967, found 435.9963.

### Example 42

### Ethyl 2-(4-(((4-(4-Bromophenyl)-5-oxo-4,5-dihydro-1H-1,2,4-triazol-1-yl)methyl)thio)phenoxy)acetate (compound 42)

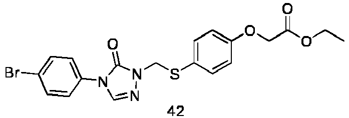

Compound 42 was prepared without hydrolysis according to the method of Example 41: ¹H NMR (300 MHz, DMSO-*d₆*) δ 8.53 (s, 1H), 7.67 (dd, *J* = 30.3, 8.8 Hz, 4H), 7.40 (d, *J* = 8.7 Hz, 2H), 6.90 (d, *J* = 8.7 Hz, 2H), 5.17 (s, 2H), 4.77 (s, 2H), 4.16 (q, *J* = 7.1 Hz, 2H), 1.20 (t, *J* = 7.1 Hz, 3H). MS (ESI): m/z 486.1 [M+Na]⁺.

### Example 43

### 2-(4-(((4-(4-Bromophenyl)-5-oxo-4,5-dihydro-1H-1,2,4-triazol-1-yl)methyl)thio)-2-fluorophenoxy)acetic acid (compound 43)

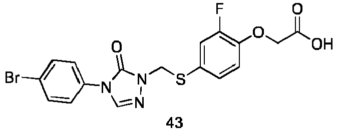

Compound 43 was prepared by replacing phenol in Example 41 with *o*-fluorophenol according to the method of Example 41: ¹H NMR (300 MHz, DMSO-*d₆*) δ 13.06 (s, 1H), 8.53 (s, 1H), 7.72 (d, *J* = 8.9 Hz, 2H), 7.62 (d, *J* = 8.9 Hz, 2H), 7.45 (dd, *J* = 11.7, 2.1 Hz, 1H), 7.21 (d, *J* = 8.6 Hz, 1H), 7.04 (t, *J* = 8.8 Hz, 1H), 5.25 (s, 2H), 4.77 (s, 2H). HRMS (ESI) calcd. for C₁₇H₁₃BrFN₃O₄S [M+H]⁺ 453.9872, found 453.9875.

### Example 44

### Ethyl 2-(4-(((4-(4-bromophenyl)-5-oxo-4,5-dihydro-1H-1,2,4-triazol-1-yl)methyl)thio)-2-fluorophenoxy)acetate (compound 44)

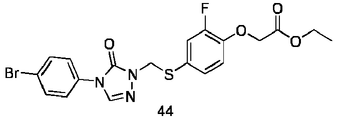

Compound 44 was prepared by replacing phenol in Example 41 with *o*-fluorophenol without hydrolysis according to the method of Example 41: ¹H NMR (300 MHz, DMSO-*d₆*) δ 8.54 (s, 1H), 7.72 (d, *J* = 8.8 Hz, 2H), 7.62 (d, *J* = 8.7 Hz, 2H), 7.46 (d, *J* = 11.7 Hz, 1H), 7.21 (d, *J* = 8.6 Hz,1H), 7.06 (t, 1H), 5.25 (s, 2H), 4.87 (s, 2H), 4.16 (q, *J* = 7.1 Hz, 2H), 1.20 (t, *J* = 7.1 Hz, 3H). MS (ESI): m/z 504.1 [M+Na]⁺.

### Example 45

### 2-(4-(((4-(4-Bromophenyl)-5-oxo-4,5-dihydro-1H-1,2,4-triazol-1-yl)methyl)thio)-2-chlorophenoxy)acetic acid (compound 45)

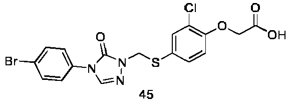

Compound 45 was prepared by replacing phenol in Example 41 with *o*-chlorophenol according to the method of Example 41: ¹H NMR (300 MHz, DMSO-*d₆*) δ 8.55 (s, 1H), 7.73 (d, *J* = 8.7 Hz, 2H), 7.62 (d, *J* = 8.7 Hz, 2H), 7.55 (d, *J* = 1.9 Hz, 1H), 7.35 (d, *J* = 8.7 Hz, 1H), 6.92 (d, *J* = 8.6 Hz, 1H), 5.21 (s, 2H), 4.60 (s, 2H). HRMS (ESI) calcd. for C₁₇H₁₃BrFN₃O₄S [M+H]⁺ 469.9577, found 469.9575.

### Example 46

### Ethyl 2-(4-(((4-(4-bromophenyl)-5-oxo-4,5-dihydro-1H-1,2,4-triazol-1-yl)methyl)thio)-2-chlorophenoxy)acetate (compound 46)

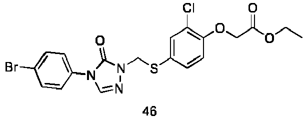

Compound 46 was prepared by replacing phenol in Example 41 with *o*-chlorophenol without hydrolysis according to the method of Example 41: ¹H NMR (300 MHz, DMSO-*d₆*) *δ* 8.55 (s, 1H), 7.72 (d, *J* = 8.9 Hz, 2H), 7.63 - 7.49 (m, 3H), 7.36 (dd, 1H), 7.02 (d, *J* = 8.7 Hz, 1H), 5.24 (s, 2H), 4.91 (s, 2H), 4.16 (q, *J* = 7.1 Hz, 2H), 1.20 (t, *J* = 7.1 Hz, 3H). MS (ESI): m/z 520.0 [M+Na]⁺.

### Example 47

### 2-(2-Bromo-4-(((4-(4-bromophenyl)-5-oxo-4,5-dihydro-1H-1,2,4-triazol-1-yl)methyl)thio)phenoxy)acetic acid (compound 47)

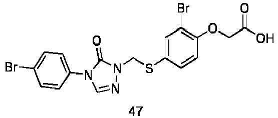

Compound 47 was prepared by replacing phenol in Example 41 with *o*-bromophenol according to the method of Example 41: ¹H NMR (300 MHz, DMSO-*d₆*) δ 13.08 (s, 1H), 8.55 (s, 1H), 7.73 (d, *J* = 8.9 Hz, 3H), 7.62 (d, *J* = 8.8 Hz, 2H), 7.42 (d, *J* = 8.6 Hz, 1H), 6.96 (d, *J* = 8.6 Hz, 1H), 5.22 (s, 2H), 4.80 (s, 2H). HRMS (ESI) calcd. for C₁₇H₁₃Br₂N₃O₄S [M+H]⁺ 513.9072, found 513.9072.

### Example 48

### Ethyl 2-(2-bromo-4-(((4-(4-bromophenyl)-5-oxo-4,5-dihydro-1H-1,2,4-triazol-1-yl)methyl)thio)phenoxy)acetate (compound 48)

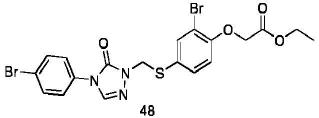

Compound 48 was prepared by replacing phenol in Example 41 with *o*-bromophenol without hydrolysis according to the method of Example 41: ¹H NMR (300 MHz, DMSO-*d₆*) δ 8.56 (s, 1H), 7.72 (d, *J* = 8.8 Hz, 3H), 7.62 (d, *J* = 8.9 Hz, 2H), 7.42 (dd, *J* = 8.6, 2.2 Hz, 1H), 6.99 (d, *J* = 8.7 Hz, 1H), 5.23 (s, 2H), 4.90 (s, 2H), 4.16 (q, *J* = 7.1 Hz, 2H), 1.20 (t, *J* = 7.2 Hz, 3H). MS (ESI): m/z 564.0 [M+Na]⁺.

### Example 49

### 2-(4-(((5-Oxo-4-(4-(trifluoromethyl)phenyl)-4,5-dihydro-1H-1,2,4-triazol-1-yl)methyl)thio)phenoxy)acetic acid (compound 49)

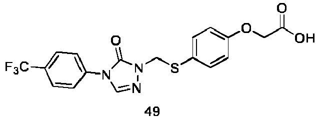

### Synthesis of intermediate A-1

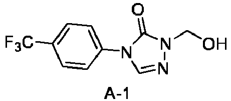

Intermediate A-1 was prepared by replacing *p*-bromoaniline with trifluoromethylaniline according to the method for intermediate I-4 of Example 1.

### Compound 49

Compound 49 was prepared by replacing intermediate I-4 in Example 41 with intermediate A-1 according to the method of Example 41: ¹H NMR (300 MHz, DMSO-*d₆*) δ 13.16 - 11.57 (s, 1H), 8.64 (s, 1H), 7.91 (s, 4H), 7.41 (d, *J* = 8.7 Hz, 2H), 6.88 (d, *J* = 8.7 Hz, 2H), 5.18 (s, 2H), 4.65 (s, 2H). HRMS (ESI) calcd. for C₁₈H₁₄F₃N₃O₄S [M+H]⁺ 426.0735, found 426.0739.

### Example 50

### Ethyl 2-(4-(((5-oxo-4-(4-(trifluoromethyl)phenyl)-4,5-dihydro-1H-1,2,4-triazol-1-yl)methyl)thio)phenoxy)acetate (compound 50)

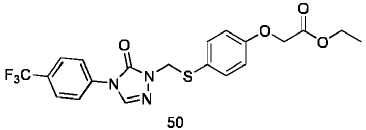

Compound 50 was prepared by replacing intermediate I-4 in Example 41 with intermediate A-1 without hydrolysis according to the method of Example 41: ¹H NMR (300 MHz, DMSO-*d₆*) δ 8.65 (s, 1H), 7.91 (s, 3H), 7.41 (d, *J* = 8.4 Hz, 2H), 6.90 (d, *J* = 8.4 Hz, 2H), 5.19 (s, 2H), 4.77 (s, 2H), 4.15 (q, *J* = 7.1 Hz, 2H), 1.20 (t, *J* = 7.1 Hz, 3H). MS (ESI): m/z 476.2 [M+Na]⁺.

### Example 51

### 2-(2-Fluoro-4-(((5-oxo-4-(4-(trifluoromethyl)phenyl)-4,5-dihydro-1H-1,2,4-triazol-1-yl)methyl)thio)phenoxy)acetic acid (compound 51)

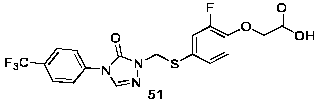

Compound 51 was prepared by replacing intermediate I-4 in Example 41 with intermediate A-1 and phenol with *o*-fluorophenol according to the method of Example 41: ¹H NMR (300 MHz, DMSO-*d₆*) δ 13.18 (s, 1H), 8.66 (s, 1H), 7.92 (s, 4H), 7.46 (dd, *J* = 11.7, 2.0 Hz, 1H), 7.21 (d, *J* = 8.4 Hz, 1H), 7.04 (t, *J* = 8.8 Hz, 1H), 5.27 (s, 2H), 4.75 (s, 2H). HRMS (ESI) calcd. for C₁₈H₁₃F₄N₃O₄S [M+H]⁺ 444.0641, found 444.0640.

### Example 52

### Ethyl 2-(2-fluoro-4-(((5-oxo-4-(4-(trifluoromethyl)phenyl)-4,5-dihydro-1H-1,2,4-triazol-1-yl)methyl)thio)phenoxy)acetate (compound 52)

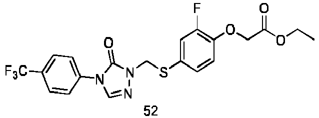

Compound 52 was prepared by replacing intermediate I-4 in Example 41 with intermediate A-1 and phenol with *o*-fluorophenol without hydrolysis according to the method of Example 41: ¹H NMR (300 MHz, DMSO-*d₆*) δ 8.66 (s, 1H), 7.92 (s, 4H), 7.47 (dd, *J* = 11.7, 2.1 Hz, 1H), 7.21 (d, *J* = 8.9 Hz, 1H), 7.06 (d, *J* = 8.8 Hz, 1H), 5.28 (s, 2H), 4.87 (s, 2H), 4.16 (q, *J* = 7.1 Hz, 2H), 1.20 (t, *J* = 7.1 Hz, 3H). MS (ESI): m/z 472.09 [M+Na]⁺.

### Example 53

### 2-(2-Chloro-4-(((5-oxo-4-(4-(trifluoromethyl)phenyl)-4,5-dihydro-1H-1,2,4-triazol-1-yl)methyl)thio)phenoxy)acetic acid (compound 53)

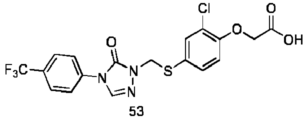

Compound 53 was prepared by replacing intermediate I-4 in Example 41 with intermediate A-1 and phenol with *o*-chlorophenol according to the method of Example 41: ¹H NMR (300 MHz, DMSO-*d₆*) δ 13.47 - 12.49 (s, 1H), 8.66 (s, 1H), 7.92 (s, 4H), 7.59 (d, *J* = 2.2 Hz, 1H), 7.38 (dd, *J* = 8.6, 2.2 Hz, 1H), 7.00 (d, *J* = 8.7 Hz, 1H), 5.25 (s, 2H), 4.79 (s, 2H). HRMS (ESI) calcd. for C₁₈H₁₃ClF₃N₃O₄S [M+H]⁺ 460.0346, found 460.0336.

### Example 54

### Ethyl 2-(2-chloro-4-(((5-oxo-4-(4-(trifluoromethyl)phenyl)-4,5-dihydro-1H-1,2,4-triazol-1-yl)methyl)thio)phenoxy)acetate (compound 54)

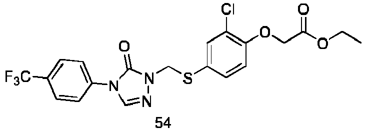

Compound 54 was prepared by replacing intermediate I-4 in Example 41 with intermediate A-1 and phenol with *o*-chlorophenol without hydrolysis according to the method of Example 41: ¹H NMR (300 MHz, DMSO-*d₆*) δ 8.66 (s, 1H), 7.91 (s, 4H), 7.60 (d, *J* = 2.2 Hz, 1H), 7.37 (dd, *J* = 8.6, 2.2 Hz, 1H), 7.02 (d, *J* = 8.7 Hz, 1H), 5.25 (s, 2H), 4.90 (s, 2H), 4.15 (q, *J* = 7.1 Hz, 2H), 1.19 (t, *J* = 7.1 Hz, 3H). MS (ESI): m/z 510.04 [M+Na]⁺.

### Example 55

### 2-(4-((4-(4-Bromophenyl)-5-oxo-4,5-dihydro-1H-1,2,4-triazol-1-yl)methyl)-2,6-dimethylphenoxy)-2-methylpropionic acid (compound 55)

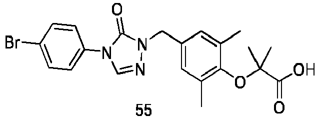

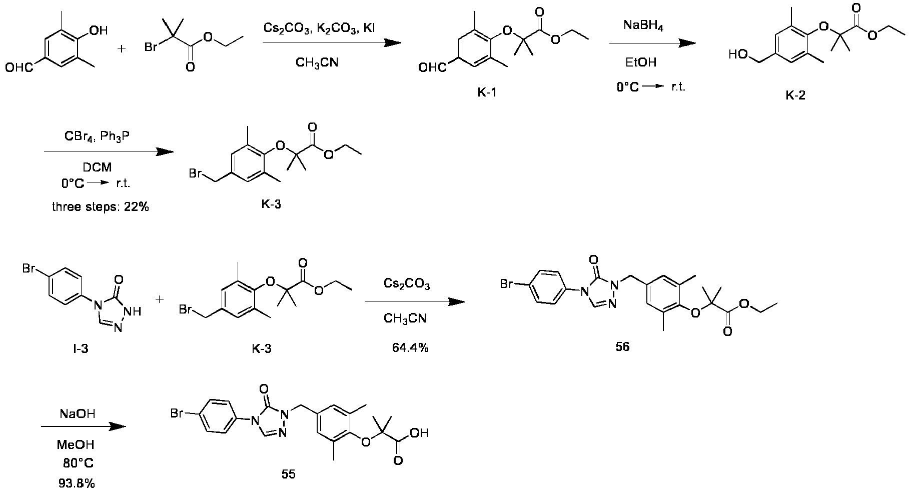

### Synthesis of intermediate K-1

3,5-Dimethyl-4-hydroxybenzaldehyde (21 g, 140 mmol) was dissolved in acetonitrile (200 mL), and ethyl 2-bromoisobutyrate (100.5 g, 520 mmol), cesium carbonate (45.6 g, 140 mmol), potassium carbonate (38.6 g, 280 mmol), and potassium iodide (1.66 g, 10 mmol) were added. The system was transferred to an oil bath and reacted at 80 °C for 36 h. After the reaction was completed, the reaction liquid was cooled to room temperature and filtered under reduced pressure. The filtrate was concentrated by rotary evaporation under reduced pressure to remove the solvent. The residue was diluted with water (200 mL) and extracted with ethyl acetate (200 mL × 3). The organic phases were combined, washed with 1 N sodium hydroxide (200 mL × 3) and saturated brine (200 mL × 1), dried over anhydrous sodium sulfate, and concentrated by rotary evaporation under reduced pressure to remove the solvent. The residue was purified by column chromatography (petroleum ether/ethyl acetate = 200:1) to give intermediate K-1 (yellow liquid, 16.3 g).

### Synthesis of intermediate K-2

Intermediate K-1 (3.66 g, 13.85 mmol) was dissolved in ethanol (20 mL), and sodium borohydride (280 mg, 7.5 mmol) was slowly added under an ice bath. After the addition, the reaction system was slowly warmed to room temperature and reacted for 4 h. After the reaction was completed, water was added to the reaction liquid to quench the reaction (20 mL). The reaction liquid was concentrated by rotary evaporation under reduced pressure to remove the solvent. The residue was diluted with 30 mL of water and extracted with ethyl acetate (20 mL × 3). The organic phases were combined, washed with saturated brine (30 mL × 1), dried over anhydrous sodium sulfate, and concentrated by rotary evaporation under reduced pressure to remove the solvent to give a crude product of intermediate K-2, which was directly used in the next step without further purification.

### Synthesis of intermediate K-3

The crude product of compound K-2 obtained from the previous step was dissolved in DCM (20 mL), carbon tetrabromide (13.6 g, 41 mmol) was added, and triphenylphosphine (9.9 g, 37.8 mmol) was slowly added under an ice bath. After the addition, the reaction system was slowly warmed to room temperature and reacted for 8 h. The reaction liquid was concentrated by rotary evaporation under reduced pressure to remove the solvent. The residue was purified by column chromatography (petroleum ether/ethyl acetate = 20: 1) to give intermediate K-3 (yellow liquid, 3.54 g).

### Synthesis of compound 56

Intermediate I-3 (95.6 mg, 0.4 mmol) was dissolved in acetonitrile (5 mL), and intermediate K-3 (180 mg, 0.6 mmol) and cesium carbonate (326 mg, 1 mmol) were added. The mixture was stirred at room temperature for 4 h. After the reaction was completed, the reaction liquid was concentrated by rotary evaporation under reduced pressure to remove the solvent. The residue was purified by column chromatography (petroleum ether/ethyl acetate = 10: 1) to give compound 56 (white solid, 125.4 mg).

### Synthesis of compound 55

Compound 56 (110 mg, 0.23 mmol) was dissolved in methanol (4 mL), and a 1 N NaOH solution (1.2 mL) was added. The mixture was stirred at room temperature for 24 h. After the reaction was completed, the reaction liquid was adjusted to pH 4 with a 1 N HCl solution, and concentrated by rotary evaporation under reduced pressure to remove the solvent. The residue was diluted with water (15 mL) and extracted with ethyl acetate (10 mL × 3). The organic phase was washed with saturated brine (15 mL × 1) and concentrated by rotary evaporation under reduced pressure to remove the solvent. The residue was purified by column chromatography (dichloromethane/methanol = 100:1) to give compound 55 (white solid, 99 mg): ¹H NMR (300 MHz, DMSO-*d₆*) δ 12.81 (s, 1H), 8.52 (s, 1H), 7.73 (s, 4H), 6.68 (s, 2H), 4.83 (s, 2H), 2.15 (s, 6H), 1.35 (s, 6H). HRMS (ESI) calcd. for C₂₁H₂₂BrN₃O₄ [M+H]⁺ 460.0872, found 460.0871.

### Example 56

### Ethyl 2-(4-((4-(4-bromophenyl)-5-oxo-4,5-dihydro-1H-1,2,4-triazol-1-yl)methyl)-2,6-dimethylphenoxy)-2-methylpropionate (compound 56)

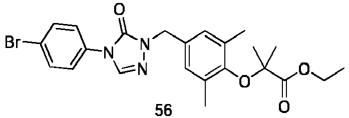

Compound 56 was prepared without hydrolysis according to the method of Example 55: ¹H NMR (300 MHz, DMSO-*d₆*) δ 8.52 (s, 1H), 7.72 (s, 4H), 6.96 (s, 2H), 4.83 (s, 2H), 4.17 (q, *J* = 7.1 Hz, 2H), 2.11 (s, 6H), 1.37 (s, 6H), 1.24 (t, *J* = 7.1 Hz, 3H). MS (ESI): m/z 510.2 [M+Na]⁺.

### Example 57

### 2-(2,6-Dimethyl-4-((5-oxo-4-(4-(trifluoromethyl)phenyl)-4,5-dihydro-1H-1,2,4-triazol-1-yl)methyl)phenoxy)-2-methylpropionic acid (compound 57)

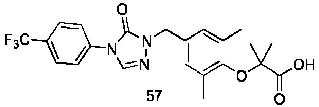

### Synthesis of intermediate D-1

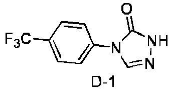

Intermediate D-1 was prepared by replacing p-bromoaniline in Example 1 with trifluoromethylaniline according to the method for intermediate I-3 of Example 1.

### Synthesis of compound 57

Compound 57 was prepared by replacing intermediate I-3 with intermediate D-1 according to the method of Example 55: ¹H NMR (300 MHz, DMSO-*d₆*) δ 12.84 (s, 1H), 8.64 (s, 1H), 8.04 (d, *J* = 8.5 Hz, 2H), 7.91 (d, *J* = 8.4 Hz, 2H), 6.96 (s, 2H), 4.85 (s, 2H), 2.16 (s, 6H), 1.35 (s, 6H). HRMS (ESI) calcd. for C₂₂H₂₂F₃N₃O₄ [M+H]⁺ 450.1641, found 450.1641.

### Example 58

### Ethyl 2-(2,6-dimethyl-4-((5-oxo-4-(4-(trifluoromethyl)phenyl)-4,5-dihydro-1H-1,2,4-triazol-1-yl)methyl)phenoxy)-2-methylpropionate (compound 58)

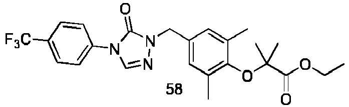

Compound 58 was prepared by replacing intermediate I-3 with intermediate D-1 without hydrolysis according to the method of Example 55: ¹H NMR (300 MHz, CDCl₃) δ 7.91 - 7.66 (m, 5H), 7.04 (s, 2H), 4.92 (s, 2H), 4.30 (q, *J* = 7.1 Hz, 2H), 2.21 (s, 6H), 1.47 (s, 6H), 1.36 (t, *J* = 7.1 Hz, 3H). MS (ESI): m/z 500.2 [M+Na]⁺.

### Example 59

### 2-(2,6-Dimethyl-4-((4-(3-methyl-4-(trifluoromethyl)phenyl)-5-oxo-4,5-dihydro-1H-1,2,4-triazol-1-yl)methyl)phenoxy)-2-methylpropionic acid (compound 59)

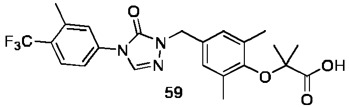

### Synthesis of intermediate D-2

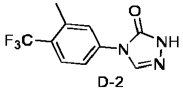

Intermediate D-2 was prepared by replacing *p*-bromoaniline in Example 1 with 3-methyl-4-trifluoromethylaniline according to the method for intermediate I-3 of Example 1.

### Synthesis of compound 59

Compound 59 was prepared by replacing intermediate I-3 with intermediate D-2 according to the method of Example 55: ¹H NMR (300 MHz, DMSO-*d₆*) δ 12.82 (s, 1H), 8.61 (s, 1H), 7.86 (d, *J* = 15.4 Hz, 3H), 6.96 (s, 2H), 4.84 (s, 2H), 2.50 (s, 3H), 2.16 (s, 6H), 1.35 (s, 6H). HRMS (ESI) calcd. for C₂₃H₂₄F₃N₃O₄ [M+H]⁺ 464.1797, found 464.1802.

### Example 60

### Ethyl 2-(2,6-dimethyl-4-((4-(3-methyl-4-(trifluoromethyl)phenyl)-5-oxo-4,5-dihydro-1H-1,2,4-triazol-1-yl)methyl)phenoxy)-2-methylpropionate (compound 60)

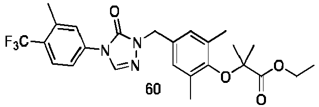

Compound 60 was prepared by replacing intermediate I-3 with intermediate D-2 without hydrolysis according to the method of Example 55: ¹H NMR (300 MHz, CDCl₃) δ 7.75 (d, *J* = 4.0 Hz, 1H), 7.71 (s, 1H), 7.63 (s, 1H), 7.52 (d, *J* = 8.3 Hz, 1H), 7.03 (s, 2H), 4.92 (s, 2H), 4.30 (q, *J* = 7.1 Hz, 2H), 2.56 (s, 3H), 2.21 (s, 6H), 1.59 (s, 3H), 1.47 (s, 6H), 1.36 (t, *J* = 7.1 Hz, 3H). MS (ESI): m/z 514.3 [M+Na]⁺.

### Example 61

### 2-(2,6-Dimethyl-4-((5-oxo-4-(4-(trifluoromethoxy)phenyl)-4,5-dihydro-1H-1,2,4-triazol-1-yl)methyl)phenoxy)-2-methylpropionic acid (compound 61)

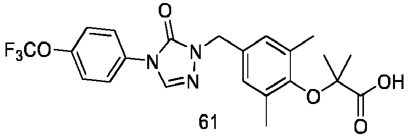

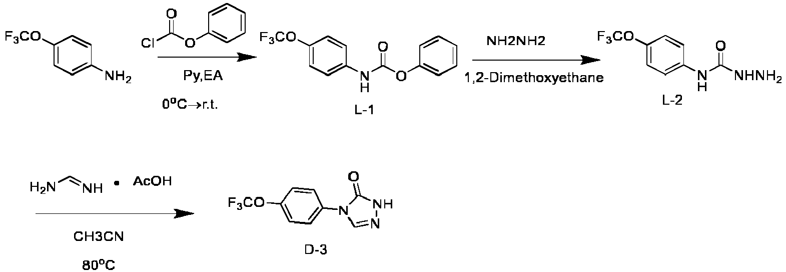

### Synthesis of intermediate L-1

*p*-Trifluoromethoxyaniline (3.54 g, 20 mmol) was dissolved in ethyl acetate (EA) (25 mL), pyridine (Py) (1.74 g, 22 mmol) was added, and phenyl chloroformate (3.44 g, 22 mmol) was slowly added under an ice bath. The mixture was stirred at room temperature for 4 h. After the reaction was completed, the reaction liquid was washed with water (50 mL × 3). The organic phase was washed with saturated brine (20 mL × 1), and concentrated by rotary evaporation under reduced pressure to remove the solvent to give a crude product of intermediate L-1, which was directly used in the next step without further purification.

### Synthesis of intermediate L-2

The crude product of intermediate L-1 obtained from the previous step was dissolved in glycol dimethyl ether (25 mL), and 98% hydrazine hydrate (2.67 mL) was added. The mixture was stirred at room temperature for 12 h. After the reaction was completed, the reaction liquid was concentrated by rotary evaporation under reduced pressure to remove the solvent, and EA (6 mL) was added to the residue. The mixture was stirred at room temperature for 12 h, and filtered under reduced pressure to give intermediate L-2 (white solid, 3.79 g).

### Synthesis of intermediate D-3

Intermediate L-2 (3.76 g, 16 mmol) was dissolved in acetonitrile (20 mL), and formamidine acetate (6.60 g, 64 mmol) was added. The mixture was stirred at room temperature for 30 min, and acetic acid (4.8 mL) was added. The system was transferred to an oil bath and reacted at 80 °C for 12 h. After the reaction was completed, the reaction liquid was cooled to room temperature and concentrated by rotary evaporation under reduced pressure to remove the solvent. Water (50 mL) was added to the residue, and the resulting mixture was extracted with ethyl acetate (25 mL × 3). The organic phase was washed with saturated brine (20 mL × 1) and concentrated by rotary evaporation under reduced pressure to remove the solvent. A mixed solution of petroleum ether (5 mL) and ethyl acetate (1 mL) was added to the residue. The mixture was stirred at room temperature for 2 h and filtered under reduced pressure to give intermediate D-3 (orange solid, 2.15 g).

### Synthesis of compound 61

Compound 61 was prepared by replacing intermediate I-3 with intermediate D-3 according to the method of Example 55: ¹H NMR (300 MHz, DMSO-*d₆*) δ 12.83 (s, 1H), 8.53 (s, 1H), 7.88 (d, *J* = 9.0 Hz, 2H), 7.55 (d, *J* = 8.7 Hz, 2H), 6.95 (s, 2H), 4.83 (s, 2H), 2.15 (s, 6H), 1.34 (s, 6H). HRMS (ESI) calcd. for C₂₂H₂₂F₃N₃O₅ [M+H]⁺ 466.1590, found 466.1590.

### Example 62

### Ethyl 2-(2,6-dimethyl-4-((5-oxo-4-(4-(trifluoromethoxy)phenyl)-4,5-dihydro-177-1,2,4-triazol-1-yl)methyl)phenoxy)-2-methylpropionate (compound 62)

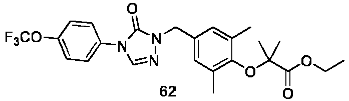

Compound 62 was prepared by replacing intermediate I-3 with intermediate D-3 without hydrolysis according to the method of Example 55: ¹H NMR (300 MHz, CDCl₃) δ 7.71 (s, 1H), 7.65 (d, *J* = 9.0 Hz, 2H), 7.35 (d, *J* = 8.6 Hz, 2H), 7.03 (s, 2H), 4.91 (s, 2H), 4.29 (q, *J* = 7.1 Hz, 2H), 2.21 (s, 6H), 1.47 (s, 6H), 1.36 (t, *J* = 7.1 Hz, 3H). MS (ESI): m/z 516.2 [M+Na]⁺.

### Example 63

### 2-(2,6-Dimethyl-4-((5-oxo-4-phenyl-4,5-dihydro-1H-1,2,4-triazol-1-yl)methyl)phenoxy)-2-methylpropionic acid (compound 63)

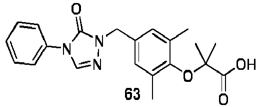

### Synthesis of intermediate D-4

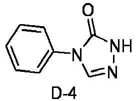

Intermediate D-4 was prepared by replacing *p*-bromoaniline in Example 1 with aniline according to the method for intermediate I-3 of Example 1.

### Synthesis of compound 63

Compound 63 was prepared by replacing intermediate I-3 with intermediate D-4 according to the method of Example 55: ¹H NMR (300 MHz, DMSO-*d₆*) δ 12.83 (s, 1H), 8.49 (s, 1H), 7.72 (d, *J* = 8.1 Hz, 2H), 7.52 (t, *J* = 7.7 Hz, 2H), 7.38 (t, *J* = 7.0 Hz, 1H), 6.95 (s, 1H), 4.83 (s, 2H), 2.15 (s, 6H), 1.35 (s, 6H). HRMS (ESI) calcd. for C₂₁H₂₃N₃O₄ [M+H]⁺ 382.1767, found 382.1763.

### Example 64

### Ethyl 2-(2,6-dimethyl-4-((5-oxo-4-phenyl-4,5-dihydro-1H-1,2,4-triazol-1-yl)methyl)phenoxy)-2-methylpropionate (compound 64)

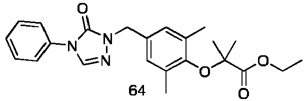

Compound 64 was prepared by replacing intermediate I-3 with intermediate D-4 without hydrolysis according to the method of Example 55: ¹H NMR (300 MHz, DMSO-*d₆*) δ 8.48 (s, 1H), 7.72 (d, 2H), 7.52 (t, *J* = 7.9 Hz, 2H), 7.38 (t, *J* = 7.4 Hz, 1H), 6.96 (s, 2H), 4.76 (s, 2H), 4.17 (q, *J* = 7.1 Hz, 2H), 2.11 (s, 6H), 1.37 (s, 6H), 1.24 (t, *J* = 7.1 Hz, 3H). MS (ESI): m/z 432.2 [M+Na]⁺.

### Example 65

### 2-(2,6-Dimethyl-4-((5-oxo-4-(p-tolyl)-4,5-dihydro-1H-1,2,4-triazol-1-yl)methyl)phenoxy)-2-methylpropionic acid (compound 65)

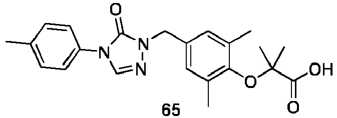

### Synthesis of intermediate D-5

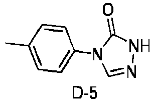

Intermediate D-5 was prepared by replacing *p*-bromoaniline in Example 1 with *p-*methylaniline according to the method for intermediate I-3 of Example 1.

### Synthesis of compound 65

Compound 65 was prepared by replacing intermediate I-3 with intermediate D-5 according to the method of Example 55: ¹H NMR (300 MHz, DMSO-*d₆*) δ 12.83 (s, 1H), 8.43 (s, 1H), 7.58 (d, *J* = 8.3 Hz, 2H), 7.30 (s, 2H), 6.94 (s, 2H), 4.82 (s, 2H), 2.34 (s, 3H), 2.15 (s, 6H), 1.34 (s, 6H). HRMS (ESI) calcd. for C₂₂H₂₅N₃O₄ [M+H]⁺ 396.1923, found 396.1920.

### Example 66

### Ethyl 2-(2,6-dimethyl-4-((5-oxo-4-(p-tolyl)-4,5-dihydro-1H-1,2,4-triazol-1-yl)methyl)phenoxy)-2-methylpropionate (compound 66)

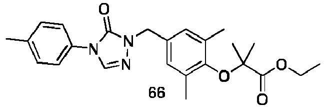

Compound 66 was prepared by replacing intermediate I-3 with intermediate D-5 without hydrolysis according to the method of Example 55: ¹H NMR (300 MHz, CDCl₃) δ 7.67 (s, 1H), 7.45 (d, *J* = 7.4 Hz, 3H), 7.29 (d, *J* = 2.8 Hz, 2H), 7.03 (s, 3H), 4.90 (s, 3H), 4.29 (q, *J* = 7.1 Hz, 3H), 2.39 (s, 5H), 2.20 (s, 10H), 1.47 (s, 9H), 1.35 (t, *J* = 7.6, 6.7 Hz, 6H). MS (ESI): m/z 446.3 [M+Na]⁺.

### Example 67

### 2-(4-((4-(4-Chlorophenyl)-5-oxo-4,5-dihydro-1H-1,2,4-triazol-1-yl)methyl)-2,6-dimethylphenoxy)-2-methylpropionic acid (compound 67)

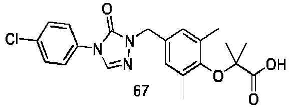

### Synthesis of intermediate D-6

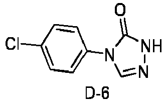

Intermediate D-6 was prepared by replacing *p*-bromoaniline in Example 1 with *p-*chloroaniline according to the method for intermediate I-3 of Example 1.

### Synthesis of compound 67

Compound 67 was prepared by replacing intermediate I-3 with intermediate D-6 according to the method of Example 55: ¹H NMR (300 MHz, DMSO-*d₆*) δ 12.84 (s, 1H), 8.51 (s, 1H), 7.78 (d, *J* = 8.8 Hz, 2H), 7.59 (d, *J* = 8.8 Hz, 2H), 6.95 (s, 2H), 4.82 (s, 2H), 2.15 (s, 6H), 1.34 (s, 6H). HRMS (ESI) calcd. for C₂₁H₂₂ClN₃O₄ [M+H]⁺ 416.1377, found 416.1374.

### Example 68

### Ethyl 2-(4-((4-(4-chlorophenyl)-5-oxo-4,5-dihydro-1H-1,2,4-triazol-1-yl)methyl)-2,6-dimethylphenoxy)-2-methylpropionate (compound 68)

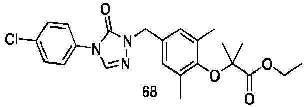

Compound 68 was prepared by replacing intermediate I-3 with intermediate D-6 without hydrolysis according to the method of Example 55: ¹H NMR (300 MHz, DMSO-*d₆*) δ 8.52 (s, 1H), 7.79 (d, *J* = 8.8 Hz, 2H), 7.60 (d, *J* = 8.8 Hz, 2H), 6.96 (s, 2H), 4.83 (s, 2H), 4.17 (q, *J* = 7.1 Hz, 2H), 2.11 (s, 6H), 1.37 (s, 6H), 1.24 (t, *J* = 7.1 Hz, 3H). MS (ESI): m/z 466.3 [M+Na]⁺.

### Example 69

### 2-(4-((4-(4-Ethoxyphenyl)-5-oxo-4,5-dihydro-1H-1,2,4-triazol-1-yl)methyl)-2, 6-dimethylphenoxy)-2-methylpropionic acid (compound 69)

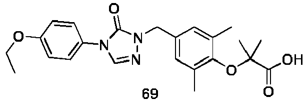

### Synthesis of intermediate D-7

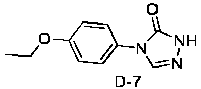

Intermediate D-7 was prepared by replacing *p*-bromoaniline in Example 1 with *p-*ethoxyaniline according to the method for intermediate I-3 of Example 1.

### Synthesis of compound 69

Compound 69 was prepared by replacing intermediate I-3 with intermediate D-7 according to the method of Example 55: ¹H NMR (300 MHz, DMSO-*d₆*) δ 12.80 (s, 1H), 8.36 (s, 1H), 7.57 (d, *J* = 8.9 Hz, 2H), 7.04 (d, *J* = 9.0 Hz, 2H), 6.94 (s, 2H), 4.81 (s, 2H), 4.06 (q, *J* = 6.9 Hz, 2H), 2.15 (s, 6H), 1.34 (s, 6H). HRMS (ESI) calcd. for C₂₃H₂₇N₃O₅ [M+H]⁺ 426.2029, found 426.2021.

### Example 70

### Ethyl 2-(4-((4-(4-ethoxyphenyl)-5-oxo-4,5-dihydro-1H-1,2,4-triazol-1-yl)methyl)-2,6-dimethylphenoxy)-2-methylpropionate (compound 70)

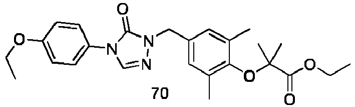

Compound 70 was prepared by replacing intermediate I-3 with intermediate D-7 without hydrolysis according to the method of Example 55: ¹H NMR (300 MHz, CDCl₃) δ 7.63 (s, 1H), 7.45 (d, *J* = 8.9 Hz, 2H), 7.28 (s, 1H), 6.98 (d, *J* = 8.9 Hz, 3H), 4.91 (s, 2H), 4.30 (q, *J* = 7.1 Hz, 2H), 4.07 (q, *J* = 7.0 Hz, 2H), 2.21 (s, 6H), 1.47 (s, 6H), 1.45 (t, 3H), 1.36 (t, *J* = 7.1 Hz, 3H). MS (ESI): m/z 476.3 [M+Na]⁺.

### Example 71

### 2-(4-((4-(3-Fluoro-4-(trifluoromethyl)phenyl)-5-oxo-4,5-dihydro-1H-1,2,4-triazol-1-yl)methyl)-2,6-dimethylphenoxy)-2-methylpropionic acid (compound 71)

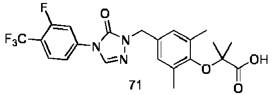

### Synthesis of intermediate D-8

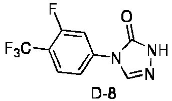

Intermediate D-8 was prepared by replacing *p*-bromoaniline in Example 1 with 3-fluoro-4-trifluoromethylaniline according to the method for intermediate I-3 of Example 1.

### Synthesis of compound 71

Compound 71 was prepared by replacing intermediate I-3 with intermediate D-8 according to the method of Example 55: ¹H NMR (300 MHz, DMSO-*d₆*) δ 12.82 (s, 1H), 8.68 (d, *J* = 5.0 Hz, 1H), 8.06 (d, *J* = 12.4 Hz, 1H), 7.96 (d, *J* = 6.6 Hz, 1H), 7.81 - 7.53 (m, 1H), 6.96 (s, 2H), 4.84 (s, 2H), 2.15 (s, 6H), 1.34 (s, 6H). HRMS (ESI) calcd. for C₂₂H₂₁F₄N₃O₄ [M+H]⁺ 468.1546, found 468.1545.

### Example 72

### Ethyl 2-(4-((4-(3-fluoro-4-(trifluoromethyl)phenyl)-5-oxo-4,5-dihydro-1H-1,2,4-triazol-1-yl)methyl)-2,6-dimethylphenoxy)-2-methylpropionate (compound 72)

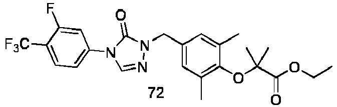

Compound 72 was prepared by replacing intermediate I-3 with intermediate D-8 without hydrolysis according to the method of Example 55: ¹H NMR (300 MHz, CDCl₃) δ 7.79 (s, 1H), 7.73 (dd, 2H), 7.53 (d, *J* = 8.5 Hz, 1H), 7.03 (s, 2H), 4.92 (s, 2H), 4.30 (q, *J* = 7.1 Hz, 2H), 2.21 (s, 6H), 1.47 (s, 6H), 1.36 (t, *J* = 7.1 Hz, 3H). MS (ESI): m/z 518.2 [M+Na]⁺.

### Example 73

### 2-(4-((4-(3-Chloro-4-(trifluoromethyl)phenyl)-5-oxo-4,5-dihydro-1H-1,2,4-triazol-1-yl)methyl)-2,6-dimethylphenoxy)-2-methylpropionic acid (compound 73)

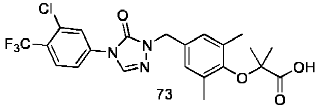

### Synthesis of intermediate D-9

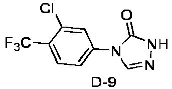

Intermediate D-9 was prepared by replacing *p*-bromoaniline in Example 1 with 3-chloro-4-trifluoromethylaniline according to the method for intermediate I-3 of Example 1.

### Compound 73

Compound 73 was prepared by replacing intermediate I-3 with intermediate D-9 according to the method of Example 55: ¹H NMR (300 MHz, DMSO-*d₆*) δ 12.84 (s, 1H), 8.71 (s, 1H), 8.25 (s, 1H), 8.11 - 7.92 (dd, 2H), 6.96 (s, 2H), 4.85 (s, 2H), 2.15 (s, 6H), 1.35 (s, 6H). HRMS (ESI) calcd. for C₂₂H₂₁ClF₃N₃O₄ [M+H]⁺ 484.1251, found 484.1246.

### Example 74

### Ethyl 2-(4-((4-(3-chloro-4-(trifluoromethyl)phenyl)-5-oxo-4,5-dihydro-1H-1,2,4-triazol-1-yl)methyl)-2,6-dimethylphenoxy)-2-methylpropionate (compound 74)

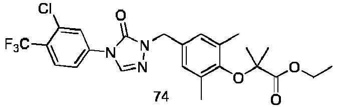

Compound 74 was prepared by replacing intermediate I-3 with intermediate D-9 without hydrolysis according to the method of Example 55: ¹H NMR (300 MHz, CDCl₃) δ 7.93 (s, 1H), 7.80 (d, 2H), 7.69 (d, *J* = 8.4 Hz, 11H), 7.03 (s, 2H), 4.91 (s, 2H), 4.30 (q, *J* = 7.1 Hz, 2H), 2.21 (s, 6H), 1.47 (s, 6H), 1.36 (t, *J* = 7.1 Hz, 3H). MS (ESI): m/z 534.3 [M+Na]⁺.

### Example 75

### 2-(4-((4-(4-Bromophenyl)-5-oxo-4,5-dihydro-1H-1,2,4-triazol-1-yl)methyl)-2-methylphenoxy)-2-methylpropionic acid (compound 75)

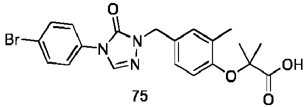

Compound 75 was prepared by replacing 4-hydroxy-3,5-dimethylbenzaldehyde with 4-hydroxy-3-methylbenzaldehyde according to the method of Example 55: ¹H NMR (300 MHz, DMSO-*d₆*) *δ* 13.03 (s, 1H), 8.50 (s, 1H), 7.72 (s, 4H), 7.12 (s, 1H), 7.04 (d, *J* = 9.5 Hz, 1H), 6.65 (d, *J* = 8.4 Hz, 1H), 4.83 (s, 2H), 2.14 (s, 3H), 1.50 (s, 6H). HRMS (ESI) calcd. for C₂₀H₂₀BrN₃O₄ [M+H]⁺ 446.0715, found 446.0708.

### Example 76

### Ethyl 2-(4-((4-(4-bromophenyl)-5-oxo-4,5-dihydro-1H-1,2,4-triazol-1-yl)methyl)-2-methylphenoxy)-2-methylpropionate (compound 76)

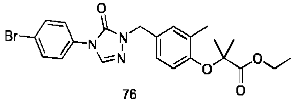

Compound 76 was prepared by replacing 4-hydroxy-3,5-dimethylbenzaldehyde with 4-hydroxy-3-methylbenzaldehyde without hydrolysis according to the method of Example 55: ¹H NMR (300 MHz, CDCl₃) *δ* 7.67 (s, 1H), 7.62 (d, *J* = 8.9 Hz, 2H), 7.49 (d, *J* = 8.9 Hz, 2H), 7.28 (s, 1H), 7.12 (d, *J* = 8.4 Hz, 1H), 6.63 (d, *J* = 8.4 Hz, 1H), 4.92 (s, 2H), 4.25 (q, *J* = 7.1 Hz, 2H), 2.24 (s, 3H), 1.60 (s, 6H), 1.28 (t, 3H). MS (ESI): m/z 496.1 [M+Na]⁺.

### Example 77

### 2-Methyl-2-(2-methyl-4-((5-oxo-4-(4-(trifluoromethyl)phenyl)-4,5-dihydro-1H-1,2,4-triazol-1-yl)methyl)phenoxy)propionic acid (compound 77)

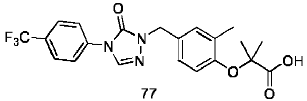

Compound 77 was prepared by replacing intermediate I-3 with intermediate D-1 and 4-hydroxy-3,5-dimethylbenzaldehyde with 4-hydroxy-3-methylbenzaldehyde according to the method of Example 55: ¹H NMR (300 MHz, DMSO-*d₆*) *δ* 13.03 (s, 1H), 8.62 (s, 1H), 8.02 (d, *J* = 8.5 Hz, 2H), 7.91 (d, *J* = 8.6 Hz, 2H), 7.14 (s, 1H), 7.06 (d, *J* = 8.4 Hz, 1H), 6.66 (d, *J* = 8.3 Hz, 1H), 4.85 (s, 2H), 2.14 (s, 3H), 1.50 (s, 6H). HRMS (ESI) calcd. for C₂₁H₂₀F₃N₃O₄ [M+H]⁺ 436.1484, found 436.1475.

### Example 78

### Ethyl 2-methyl-2-(2-methyl-4-((5-oxo-4-(4-(trifluoromethyl)phenyl)-4,5-dihydro-1H-1,2,4-triazol-1-yl)methyl)phenoxy)propionate (compound 78)

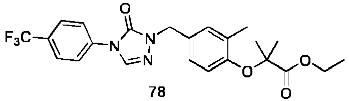

Compound 78 was prepared by replacing intermediate I-3 with intermediate D-1 and 4-hydroxy-3,5-dimethylbenzaldehyde with 4-hydroxy-3-methylbenzaldehyde without hydrolysis according to the method of Example 55: ¹H NMR (300 MHz, CDCl₃) *δ* 7.77 (s, 4H), 7.76 (s, 1H), 7.23 (s, 1H), 7.12 (d, *J* = 8.3 Hz, 1H), 6.64 (d, *J* = 8.3 Hz, 1H), 4.94 (s, 2H), 4.25 (q, *J* = 7.1 Hz, 2H), 2.24 (s, 3H), 1.60 (s, 6H), 1.26 (t, *J* = 7.1 Hz, 3H). MS (ESI): m/z 486.2 [M+Na]⁺.

### Example 79

### 2-Methyl-2-(2-methyl-4-((5-oxo-4-(4-(trifluoromethoxy)phenyl)-4,5-dihydro-1H-1,2,4-triazol-1-yl)methyl)phenoxy)propionic acid (compound 79)

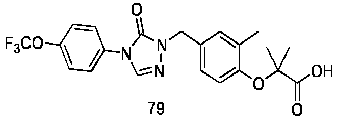

Compound 79 was prepared by replacing intermediate I-3 with intermediate D-3 and 4-hydroxy-3,5-dimethylbenzaldehyde with 4-hydroxy-3-methylbenzaldehyde according to the method of Example 55: ¹H NMR (300 MHz, DMSO-*d₆*) *δ* 13.04 (s, 1H), 8.51 (s, 1H), 7.86 (d, *J* = 8.9 Hz, 2H), 7.54 (d, *J* = 8.7 Hz, 2H), 7.13 (s, 1H), 7.05 (d, *J* = 8.4 Hz, 1H), 6.66 (d, *J* = 8.3 Hz, 1H), 4.84 (s, 2H), 2.14 (s, 3H), 1.50 (s, 6H). HRMS (ESI) calcd. for C₂₁H₂₀F₃N₃O₅ [M+H]⁺ 452.1433, found 452.1428.

### Example 80

### Ethyl 2-methyl-2-(2-methyl-4-((5-oxo-4-(4-(trifluoromethoxy)phenyl)-4,5-dihydro-1H-1,2,4-triazol-1-yl)methyl)phenoxy)propionate (compound 80)

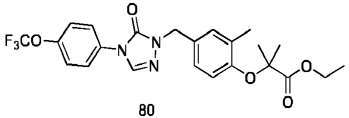

Compound 80 was prepared by replacing intermediate I-3 with intermediate D-3 and 4-hydroxy-3,5-dimethylbenzaldehyde with 4-hydroxy-3-methylbenzaldehyde without hydrolysis according to the method of Example 55: ¹H NMR (300 MHz, CDCl₃) *δ* 7.69 (s, 1H), 7.64 (d, *J* = 9.0 Hz, 2H), 7.35 (d, *J* = 8.6 Hz, 2H), 7.22 (s, 1H), 7.12 (d, *J* = 8.3 Hz, 1H), 6.63 (d, *J* = 8.3 Hz, 1H), 4.93 (s, 2H), 4.25 (q, *J* = 7.1 Hz, 2H), 2.24 (s, 3H), 1.60 (s, 6H), 1.27 (t, 3H). MS (ESI): m/z 502.2 [M+Na]⁺.

### Example 81

### 2-Methyl-2-(2-methyl-4-((5-oxo-4-phenyl-4,5-dihydro-1H-1,2,4-triazol-1-yl)methyl)phenoxy)propionic acid (compound 81)

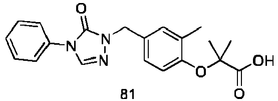

Compound 81 was prepared by replacing intermediate I-3 with intermediate D-4 and 4-hydroxy-3,5-dimethylbenzaldehyde with 4-hydroxy-3-methylbenzaldehyde according to the method of Example 55: ¹H NMR (300 MHz, DMSO-*d₆*) *δ* 13.03 (s, 1H), 8.47 (s, 1H), 7.71 (d, *J* = 7.9 Hz, 2H), 7.52 (t, *J* = 7.8 Hz, 2H), 7.38 (t, *J* = 7.4 Hz, 1H), 7.13 (s, 1H), 7.05 (d, *J* = 8.4 Hz, 1H), 6.66 (d, *J* = 8.3 Hz, 1H), 4.83 (s, 2H), 2.14 (s, 3H), 1.51 (s, 6H). HRMS (ESI) calcd. for C₂₀H₂₁N₃O₄ [M+H]⁺ 368.1610, found 368.1610.

### Example 82

### Ethyl 2-methyl-2-(2-methyl-4-((5-oxo-4-phenyl-4,5-dihydro-1H-1,2,4-triazol-1-yl)methyl)phenoxy)propionate (compound 82)

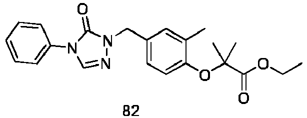

Compound 82 was prepared by replacing intermediate I-3 with intermediate D-4 and 4-hydroxy-3,5-dimethylbenzaldehyde with 4-hydroxy-3-methylbenzaldehyde without hydrolysis according to the method of Example 55: ¹H NMR (300 MHz, CDCl₃) *δ* 7.69 (s, 1H), 7.58 (d, *J* = 8.1 Hz, 2H), 7.49 (t, *J* = 7.8 Hz, 2H), 7.38 (d, *J* = 7.4 Hz, 1H), 7.23 (s, 1H), 7.12 (d, *J* = 6.5 Hz, 1H), 6.64 (d, *J* = 8.3 Hz, 1H), 4.93 (s, 2H), 4.25 (q, *J* = 7.1 Hz, 2H), 2.24 (s, 3H), 1.59 (s, 6H), 1.28 (t, *J* = 3.5 Hz, 3H). MS (ESI): m/z 418.2 [M+Na]⁺.

### Example 83

### 2-Methyl-2-(2-methyl-4-((5-oxo-4-(p-tolyl)-4,5-dihydro-1H-1,2,4-triazol-1-yl)methyl)phenoxypropionic acid (compound 83)

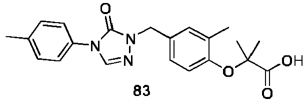

Compound 83 was prepared by replacing intermediate I-3 with intermediate D-5 and 4-hydroxy-3,5-dimethylbenzaldehyde with 4-hydroxy-3-methylbenzaldehyde according to the method of Example 55: ¹H NMR (300 MHz, DMSO-*d₆*) *δ* 13.02 (s, 1H), 8.41 (s, 1H), 7.57 (d, *J* = 8.2 Hz, 2H), 7.31 (d, *J* = 8.1 Hz, 2H), 7.12 (s, 1H), 7.04 (d, *J* = 8.1 Hz, 1H), 6.66 (d, *J* = 8.3 Hz, 1H), 4.82 (s, 2H), 2.34 (s, 3H), 2.14 (s, 3H), 1.50 (s, 6H). HRMS (ESI) calcd. for C₂₁H₂₃N₃O₄ [M+H]⁺ 382.1767, found 382.1760.

### Example 84

### Ethyl 2-methyl-2-(2-methyl-4-((5-oxo-4-(p-tolyl)-4,5-dihydro-1H-1,2,4-triazol-1-yl)methyl)phenoxy)propionate (compound 84)

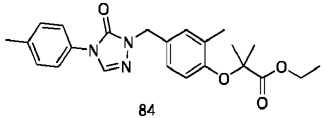

Compound 84 was prepared by replacing intermediate I-3 with intermediate D-5 and 4-hydroxy-3,5-dimethylbenzaldehyde with 4-hydroxy-3-methylbenzaldehyde without hydrolysis according to the method of Example 55: ¹H NMR (300 MHz, CDCl₃) *δ* 7.65 (s, 1H), 7.44 (d, *J* = 8.3 Hz, 2H), 7.29 (d, *J* = 4.0 Hz, 2H), 7.22 (s, 1H), 7.12 (d, *J* = 8.4 Hz, 1H), 6.63 (d, *J* = 8.4 Hz, 1H), 4.92 (s, 2H), 4.25 (q, *J* = 7.1 Hz, 2H), 2.40 (s, 3H), 2.24 (s, 3H), 1.60 (s, 6H), 1.26 (t, *J* = 5.5 Hz, 3H). MS (ESI): m/z 432.2 [M+Na]⁺.

### Example 85

### 2-(4-((4-(3-Fluoro-4-(trifluoromethyl)phenyl)-5-oxo-4,5-dihydro-1H-1,2,4-triazol-1-yl)methyl)-2-methylphenoxy)-2-methylpropionic acid (compound 85)

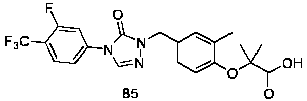

Compound 85 was prepared by replacing intermediate I-3 with intermediate D-8 and 4-hydroxy-3,5-dimethylbenzaldehyde with 4-hydroxy-3-methylbenzaldehyde according to the method of Example 55: ¹H NMR (300 MHz, DMSO-*d₆*) *δ* 13.03 (s, 1H), 8.67 (d, *J* = 5.7 Hz, 1H), 8.05 (d, *J* = 12.3 Hz, 1H), 7.90 (dd, *J* = 34.7, 24.7 Hz, 1H), 7.62 (dd, *J* = 48.4, 16.1 Hz, 1H), 7.13 (s, 1H), 7.05 (d, *J* = 8.7 Hz, 1H), 6.66 (d, *J* = 8.4 Hz, 1H), 4.85 (s, 2H), 2.14 (s, 3H), 1.50 (s, 6H). HRMS (ESI) calcd. for C₂₁H₁₉F₄N₃O₄ [M+H]⁺ 454.1390, found 454.1392.

### Example 86

### Ethyl 2-(4-((4-(3-fluoro-4-(trifluoromethyl)phenyl)-5-oxo-4,5-dihydro-1H-1,2,4-triazol-1-yl)methyl)-2-methylphenoxy)-2-methylpropionate (compound 86)

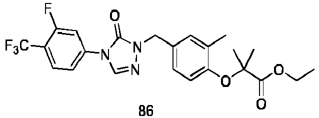

Compound 86 was prepared by replacing intermediate I-3 with intermediate D-8 and 4-hydroxy-3,5-dimethylbenzaldehyde with 4-hydroxy-3-methylbenzaldehyde without hydrolysis according to the method of Example 55: ¹H NMR (300 MHz, CDCl₃) *δ* 7.77 (s, 1H), 7.74 (d, *J* = 7.9 Hz, 1H), 7.70 (s, 1H), 7.50 (d, *J* = 8.2 Hz, 1H), 7.21 (s, 1H), 7.11 (dd, *J* = 8.3, 2.0 Hz, 1H), 6.63 (d, *J* = 8.3 Hz, 1H), 4.92 (s, 2H), 4.25 (q, *J* = 7.1 Hz, 2H), 2.24 (s, 3H), 1.60 (s, 6H), 1.26 (t, *J* = 5.6 Hz, 6H). MS (ESI): m/z 504.3 [M+Na]⁺.

### Example 87

### 2-(4-((4-(3-Chloro-4-(trifluoromethyl)phenyl)-5-oxo-4, 5-dihydro-1H-1,2,4-triazol-1-yl)methyl)-2-methylphenoxy) 2-methylpropionic acid (compound 87)

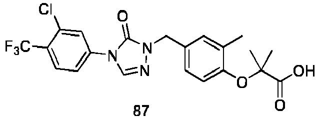

Compound 87 was prepared by replacing intermediate I-3 with intermediate D-9 and 4-hydroxy-3,5-dimethylbenzaldehyde with 4-hydroxy-3-methylbenzaldehyde according to the method of Example 55: ¹H NMR (300 MHz, DMSO-*d₆*) *δ* 13.03 (s, 1H), 8.69 (s, 1H), 8.24 (s, 1H), 8.03 (s, 2H), 7.13 (s, 1H), 7.05 (d, *J* = 8.1 Hz, 1H), 6.65 (d, *J* = 8.4 Hz, 1H), 4.84 (s, 2H), 2.14 (s, 3H), 1.50 (s, 6H). HRMS (ESI) calcd. for C₂₁H₁₉ClF₃N₃O₄ [M+H]⁺ 470.1094, found 470.1096.

### Example 88

### Ethyl 2-(4-((4-(3-chloro-4-(trifluoromethyl)phenyl)-5-oxo-4,5-dihydro-1H-1,2,4-triazol-1-yl)methyl)-2-methylphenoxy)-2-methylpropionate (compound 88)

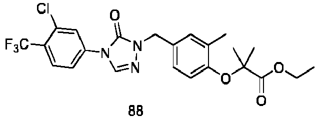

Compound 88 was prepared by replacing intermediate I-3 with intermediate D-9 and 4-hydroxy-3,5-dimethylbenzaldehyde with 4-hydroxy-3-methylbenzaldehyde without hydrolysis according to the method of Example 55: ¹H NMR (300 MHz, DMSO-*d₆*) δ 8.69 (s, 1H), 8.24 (s, 1H), 8.03 (s, 2H), 7.14 (s, 1H), 7.05 (dd, *J* = 21.8, 11.7 Hz, 1H), 6.58 (d, *J* = 8.3 Hz, 1H), 4.85 (s, 2H), 4.17 (q, *J* = 7.1 Hz, 2H), 2.15 (s, 3H), 1.51 (d, *J* = 3.6 Hz, 6H), 1.16 (t, *J* = 7.1 Hz, 3H). MS (ESI): m/z 520.2 [M+Na]⁺.

### Example 89

### 2-(4-((4-(4-Ethoxyphenyl)-5-oxo-4,5-dihydro-1H-1,2,4-triazol-1-yl)methyl)-2-methylphenoxy)-2-methylpropionic acid (compound 89)

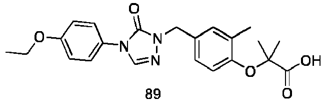

Compound 89 was prepared by replacing intermediate I-3 with intermediate D-7 and 4-hydroxy-3,5-dimethylbenzaldehyde with 4-hydroxy-3-methylbenzaldehyde according to the method of Example 55: ¹H NMR (300 MHz, DMSO) *δ* 13.00 (s, 1H), 8.33 (s, 1H), 7.54 (d, *J* = 8.9 Hz, 2H), 7.10 (s, 1H), 7.02 (d, *J* = 8.9 Hz, 3H), 6.64 (d, *J* = 8.4 Hz, 1H), 4.80 (s, 2H), 4.04 (q, *J* = 7.0 Hz, 2H), 2.12 (s, 3H), 1.49 (s, 6H), 1.32 (t, *J* = 7.0 Hz, 3H). HRMS (ESI) calcd. for C₂₂H₂₅N₃O₅ [M+H]⁺ 412.1872, found 412.1870.

### Example 90

### Ethyl 2-(4-((4-(4-ethoxyphenyl)-5-oxo-4,5-dihydro-1H-1,2,4-triazol-1-yl)methyl)-2-methylphenoxy)-2-methylpropionate (compound 90)

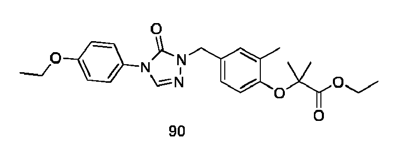

Compound 90 was prepared by replacing intermediate I-3 with intermediate D-7 and 4-hydroxy-3,5-dimethylbenzaldehyde with 4-hydroxy-3-methylbenzaldehyde without hydrolysis according to the method of Example 55: ¹H NMR (300 MHz, DMSO-*d₆*) δ 8.35 (s, 1H), 7.56 (d, *J* = 8.9 Hz, 2H), 7.13 (s, 1H), 7.04 (d, *J* = 9.0 Hz, 3H), 6.58 (d, *J* = 8.4 Hz, 1H), 4.82 (s, 2H), 4.17 (q, *J* = 7.1 Hz, 2H), 4.06 (q, *J* = 6.9 Hz, 2H), 2.15 (s, 3H), 1.52 (s, 6H), 1.34 (t, *J* = 6.9 Hz, 3H), 1.16 (t, *J* = 7.1 Hz, 3H). MS (ESI): m/z 462.3 [M+Na]⁺.

### Example 91

### 2-(4-((4-(4-Methoxyphenyl)-5-oxo-4,5-dihydro-1H-1,2,4-triazol-1-yl)methyl)-2-methylphenoxy)-2-methylpropionic acid (compound 91)

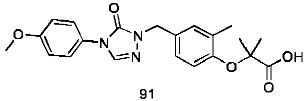

### Synthesis of intermediate D-10

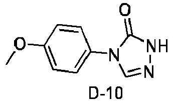

Intermediate D-10 was prepared by replacing *p*-bromoaniline in Example 1 with *p-*methoxyaniline according to the method for intermediate I-3 of Example 1.

### Synthesis of compound 91

Compound 91 was prepared by replacing intermediate I-3 with intermediate D-10 and 4-hydroxy-3,5-dimethylbenzaldehyde with 4-hydroxy-3-methylbenzaldehyde according to the method of Example 55: ¹H NMR (300 MHz, DMSO-*d₆*) δ 13.01 (s, 1H), 8.35 (s, 1H), 7.57 (d, *J* = 8.9 Hz, 2H), 7.12 (s, 1H), 7.05 (t, *J* = 7.2 Hz, 3H), 6.66 (d, *J* = 8.4 Hz, 1H), 4.81 (s, 2H), 3.79 (s, 3H), 2.14 (s, 3H), 1.51 (s, 6H). HRMS (ESI) calcd. for C₂₁H₂₃N₃O₅ [M+H]⁺ 398.1710, found 398.1717.

### Example 92

### Ethyl 2-(4-((4-(4-methoxyphenyl)-5-oxo-4,5-dihydro-1H-1,2,4-triazol-1-yl)methyl)-2-methylphenoxy)-2-methylpropionate (compound 92)

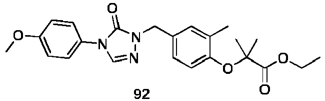

Compound 92 was prepared by replacing intermediate I-3 with intermediate D-10 and 4-hydroxy-3,5-dimethylbenzaldehyde with 4-hydroxy-3-methylbenzaldehyde without hydrolysis according to the method of Example 55: ¹H NMR (300 MHz, DMSO-*d₆*) δ 8.36 (s, 1H), 7.57 (d, *J* = 8.8 Hz, 2H), 7.13 (s, 1H), 7.04 (t, *J* = 8.9 Hz, 3H), 6.58 (d, *J* = 8.3 Hz, 1H), 4.82 (s, 2H), 4.17 (q, *J* = 7.0 Hz, 2H), 3.79 (s, 3H), 2.15 (s, 3H), 1.51 (s, 6H), 1.16 (t, *J* = 7.1 Hz, 3H). MS (ESI): m/z 448.2 [M+Na]⁺.

### Example 93

### 2-(4-((4-(4-Fluorophenyl)-5-oxo-4,5-dihydro-1H-1,2,4-triazol-1-yl)methyl)-2-methylphenoxy)-2-methylpropionic acid (compound 93)

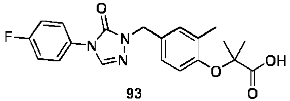

### Synthesis of intermediate D-11

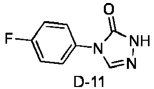

Intermediate D-11 was prepared by replacing *p*-bromoaniline in Example 1 with *p-*fluoroaniline according to the method for intermediate I-3 of Example 1.

### Synthesis of compound 93

Compound 93 was prepared by replacing intermediate I-3 with intermediate D-11 and 4-hydroxy-3,5-dimethylbenzaldehyde with 4-hydroxy-3-methylbenzaldehyde according to the method of Example 55: ¹H NMR (300 MHz, DMSO) δ 13.00 (s, 1H), 8.42 (s, 1H), 7.73 (dd, *J* = 9.0, 4.8 Hz, 2H), 7.35 (t, *J* = 8.8 Hz, 2H), 7.11 (s, 1H), 7.03 (d, *J* = 8.4 Hz, 1H), 6.64 (d, *J* = 8.4 Hz, 1H), 4.81 (s, 2H), 2.12 (s, 3H), 1.49 (s, 6H). HRMS (ESI) calcd. for C₂₀H₂₀FN₃O₄ [M+H]⁺ 386.1511, found 386.1512.

### Example 94

### Ethyl 2-(4-((4-(4-fluorophenyl)-5-oxo-4,5-dihydro-1H-1,2,4-triazol-1-yl)methyl)-2-methylphenoxy)-2-methylpropionate (compound 94)

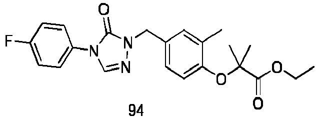

Compound 94 was prepared by replacing intermediate I-3 with intermediate D-11 and 4-hydroxy-3,5-dimethylbenzaldehyde with 4-hydroxy-3-methylbenzaldehyde without hydrolysis according to the method of Example 55: ¹H NMR (300 MHz, DMSO-*d₆*) δ 8.45 (s, 1H), 7.85 - 7.69 (m, 2H), 7.38 (t, *J* = 8.8 Hz, 2H), 7.14 (s, 1H), 7.04 (d, *J* = 8.2 Hz, 1H), 6.59 (d, *J* = 8.3 Hz, 1H), 4.84 (s, 2H), 4.17 (q, *J* = 7.1 Hz, 2H), 2.15 (s, 3H), 1.52 (s, 6H), 1.17 (t, *J* = 7.1 Hz, 3H). MS (ESI): m/z 436.2 [M+Na]⁺.

### Example 95

### 2-(4-((4-(4-Chlorophenyl)-5-oxo-4,5-dihydro-1H-1,2,4-triazol-1-yl)methyl)-2-methylphenoxy)-2-methylpropionic acid (compound 95)

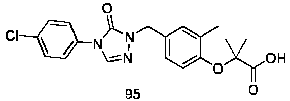

Compound 95 was prepared by replacing intermediate I-3 with intermediate D-6 and 4-hydroxy-3,5-dimethylbenzaldehyde with 4-hydroxy-3-methylbenzaldehyde according to the method of Example 55: ¹H NMR (300 MHz, DMSO-*d₆*) δ 13.02 (s, 1H), 8.50 (s, 1H), 7.77 (d, *J* = 8.8 Hz, 2H), 7.59 (d, *J* = 8.8 Hz, 2H), 7.12 (s, 1H), 7.04 (d, *J* = 8.7 Hz, 1H), 6.65 (d, *J* = 8.4 Hz, 1H), 4.83 (s, 2H), 2.14 (s, 3H), 1.50 (s, 3H). HRMS (ESI) calcd. for C₂₀H₂₀ClN₃O₄ [M+H]⁺402.1215, found 402.1220.

### Example 96

### Ethyl 2-(4-((4-(4-chlorophenyl)-5-oxo-4,5-dihydro-1H-1,2,4-triazol-1-yl)methyl)-2-methylphenoxy)-2-methylpropionate (compound 96)

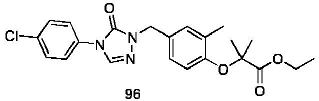

Compound 96 was prepared by replacing intermediate I-3 with intermediate D-6 and 4-hydroxy-3,5-dimethylbenzaldehyde with 4-hydroxy-3-methylbenzaldehyde without hydrolysis according to the method of Example 55: ¹H NMR (300 MHz, DMSO-*d₆*) δ 8.51 (s, 1H), 7.78 (d, *J* = 8.6 Hz, 2H), 7.60 (d, *J* = 8.7 Hz, 2H), 7.14 (s, 1H), 7.03 (d, *J* = 8.4 Hz, 1H), 6.58 (d, *J* = 8.2 Hz, 1H), 4.84 (s, 2H), 4.17 (q, *J* = 7.0 Hz, 2H), 2.15 (s, 3H), 1.52 (s, 6H), 1.17 (t, *J* = 7.0 Hz, 3H). MS (ESI): m/z 452.2 [M+Na]⁺.

### Example 97

### 2-(4-((4-(2,3-Dihydrobenzo[b][1,4]dioxin-6-yl)-5-oxo-4,5-dihydro-1H-1,2,4-triazol-1-yl)methyl)-2-methylphenoxy)-2-methylpropionic acid (compound 97)

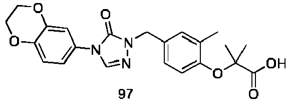

### Synthesis of intermediate D-12

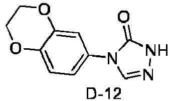

Intermediate D-12 was prepared by replacing p-bromoaniline in Example 1 with 6-amino-1,4-benzodioxane according to the method for intermediate I-3 of Example 1.

### Synthesis of compound 97

Compound 97 was prepared by replacing intermediate I-3 with intermediate D-12 and 4-hydroxy-3,5-dimethylbenzaldehyde with 4-hydroxy-3-methylbenzaldehyde according to the method of Example 55: ¹H NMR (300 MHz, DMSO) δ 13.00 (s, 1H), 8.33 (s, 1H), 7.22 (d, *J* = 2.4 Hz, 1H), 7.15 - 7.08 (m, 2H), 7.01 (dd, *J* = 8.3, 1.4 Hz, 1H), 6.95 (d, *J* = 8.7 Hz, 1H), 6.64 (d, *J* = 8.4 Hz, 1H), 4.79 (s, 2H), 4.26 (s, 4H), 2.12 (s, 3H), 1.49 (s, 6H). HRMS (ESI) calcd. for C₂₂H₂₃N₃O₆ [M+H]⁺ 426.1660, found 426.1661.

### Example 98

### Ethyl 2-(4-((4-(2,3-dihydrobenzo[b][1,4]dioxin-6-yl])-5-oxo-4,5-dihydro-1H-1,2,4-triazol-1-yl)methyl)-2-methylphenoxy)-2-methylpropionate (compound 98)

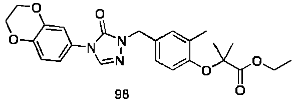

Compound 98 was prepared by replacing intermediate I-3 with intermediate D-12 and 4-hydroxy-3,5-dimethylbenzaldehyde with 4-hydroxy-3-methylbenzaldehyde without hydrolysis according to the method of Example 55: ¹H NMR (300 MHz, DMSO-*d₆*) δ 8.36 (s, 1H), 7.24 (d, *J* = 2.4 Hz, 1H), 7.18 - 7.10 (m, 2H), 7.02 (dd, *J* = 8.4, 1.8 Hz, 1H), 6.97 (d, *J* = 8.7 Hz, 1H), 6.58 (d, *J* = 8.3 Hz, 1H), 4.81 (s, 2H), 4.28 (s, 4H), 4.17 (q, *J* = 7.1 Hz, 2H), 2.15 (s, 3H), 1.52 (s, 6H), 1.16 (t, *J* = 7.1 Hz, 3H). MS (ESI): m/z 476.2 [M+Na]⁺.

### Example 99

### 2-(4-((4-(2,3-Dihydrobenzo[b][1,4]dioxin-6-yl])-5-oxo-4,5-dihydro-1H-1,2,4-triazol-1-yl)methyl)-2,6-dimethylphenoxy)-2-methylpropionic acid (compound 99)

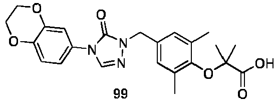

Compound 99 was prepared by replacing intermediate I-3 with intermediate D-12 according to the method of Example 55: ¹H NMR (300 MHz, DMSO-*d₆*) δ 12.82 (s, 1H), 8.35 (s, 1H), 7.23 (d, *J* = 2.4 Hz, 1H), 7.13 (dd, *J* = 8.7, 2.4 Hz, 1H), 6.97 (s, 1H), 6.93 (d, *J* = 7.5 Hz, 2H), 4.78 (s, 2H), 4.25 (s, 4H), 2.13 (s, 6H), 1.32 (s, 6H). HRMS (ESI) calcd. for C₂₃H₂₅N₃O₆ [M+H]⁺ 440.1816, found 440.1812.

### Example 100

### Ethyl 2-(4-((4-(2,3-dihydrobenzo[b][1,4]dioxin-6-yl])-5-oxo-4,5-dihydro-1H-1,2,4-triazol-1-yl)methyl)-2,6-dimethylphenoxy)-2-methylpropionate (compound 100)

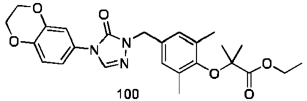

Compound 100 was prepared by replacing intermediate I-3 with intermediate D-12 without hydrolysis according to the method of Example 55: ¹H NMR (300 MHz, CDCl₃) δ 7.60 (s, 1H), 7.10 (d, *J* = 2.3 Hz, 1H), 7.02 (s, 2H), 6.98 (d, *J* = 2.4 Hz, 1H), 6.93 (d, *J* = 8.7 Hz, 1H), 4.89 (s, 2H), 4.49 - 4.22 (m, 6H), 2.20 (s, 6H), 1.47 (s, 6H), 1.35 (t, *J* = 7.1 Hz, 3H). m/z 490.3 [M+Na]⁺.

### Example 101

### 2-(2-Methyl-4-((5-oxo-4-(4-(trifluoromethyl)phenyl)-4,5-dihydro-1H-1,2,4-triazol-1-yl)methyl)phenoxyacetic acid (compound 101)

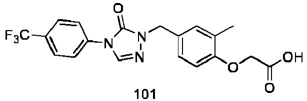

Compound 101 was prepared by replacing intermediate I-3 with intermediate D-1, 4-hydroxy-3,5-dimethylbenzaldehyde with 4-hydroxy-3-methylbenzaldehyde, and ethyl bromoisobutyrate with ethyl bromoacetate according to the method of Example 55: ¹H NMR (300 MHz, DMSO-*d₆*)) δ 13.02 (s, 1H), 8.62 (s, 1H), 8.02 (d, *J* = 8.3 Hz, 2H), 7.91 (d, *J* = 8.2 Hz, 2H), 7.14 (s, 1H), 7.10 (d, *J* = 8.6 Hz, 1H), 6.79 (d, *J* = 8.1 Hz, 1H), 4.86 (s, 2H), 4.68 (s, 2H), 2.18 (s, 3H). MS (ESI): m/z 430.2 [M+Na]⁺.

### Example 102

### Ethyl 2-(2-methyl-4-((5-oxo-4-(4-(trifluoromethyl)phenyl)-4,5-dihydro-1H-1,2,4-triazol-1-yl)methyl)phenoxyacetate (compound 102)

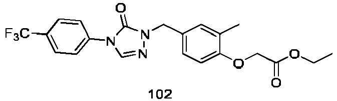

Compound 102 was obtained by replacing intermediate I-3 with intermediate D-1, 4-hydroxy-3,5-dimethylbenzaldehyde with 4-hydroxy-3-methylbenzaldehyde, and ethyl bromoisobutyrate with ethyl bromoacetate without hydrolysis according to the method of Example 55: ¹H NMR (300 MHz, CDCl₃) δ 7.76 (d, *J* = 4.4 Hz, 4H), 7.28 (s, 2H), 7.23 (d, *J* = 8.5 Hz, 1H), 6.69 (d, *J* = 8.2 Hz, 1H), 4.95 (s, 2H), 4.64 (s, 2H), 4.28 (q, *J* = 7.1 Hz, 2H), 2.31 (s, 3H), 1.32 (t, 3H). MS (ESI): m/z 458.2 [M+Na]⁺.

### Example 103

### 2-Methyl-2-(4-((5-oxo-4-(4-(trifluoromethoxy)phenyl)-4,5-dihydro-1H-1,2,4-triazol-1-yl)methyl)-2-(trifluoromethoxy)phenoxy)propionic acid (compound 103)

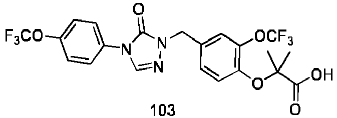

Compound 103 was prepared by replacing intermediate I-3 with intermediate D-3 and 4-hydroxy-3,5-dimethylbenzaldehyde with 4-hydroxy-3-fluoromethoxybenzaldehyde according to the method of Example 55: ¹H NMR (300 MHz, DMSO-*d₆*) δ 13.23 (s, 1H), 8.55 (s, 1H), 7.86 (d, *J* = 9.0 Hz, 2H), 7.55 (d, *J* = 8.6 Hz, 2H), 7.35 (s, 1H), 7.26 (d, *J* = 8.6 Hz, 1H), 6.93 (d, *J* = 8.5 Hz, 1H), 4.94 (s, 2H), 1.52 (s, 6H). HRMS (ESI) calcd. for C₂₁H₁₇F₆N₃O₆ [M+H]⁺ 522.1100, found 522.1097.

### Example 104

### Ethyl 2-methyl-2-(4-((5-oxo-4-(4-(trifluoromethoxy)phenyl)-4,5-dihydro-1H-1,2,4-triazol-1 -yl)methyl)-2-(trifluoromethoxy)phenoxy)propionate (compound 104)

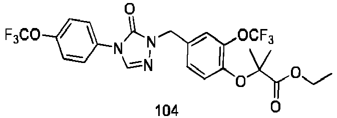

Compound 104 was prepared by replacing intermediate I-3 with intermediate D-3 and 4-hydroxy-3,5-dimethylbenzaldehyde with 4-hydroxy-3-trifluoromethoxybenzaldehyde without hydrolysis according to the method of Example 55: ¹H NMR (300 MHz, CDCl₃) δ 7.71 (s, 1H), 7.64 (d, *J* = 8.9 Hz, 2H), 7.37 (s, 1H), 7.33 (d, *J* = 6.1 Hz, 2H), 7.23 (dd, *J* = 8.5, 1.9 Hz, 1H), 6.88 (d, *J* = 8.5 Hz, 1H), 4.97 (s, 2H), 4.24 (q, *J* = 7.1 Hz, 2H), 1.61 (s, 6H), 1.26 (t, *J* = 7.1 Hz, 3H). MS (ESI): m/z 572.1 [M+Na]⁺.

### Example 105

### 2-Methyl-2-(2-methyl-4-((5-oxo-4-(4-(trifluoromethoxy)phenyl)-4,5-dihydro-1H-1,2,4-triazol-1-yl)methyl)-6-(trifluoromethyl)phenoxy)propionic acid (compound 105)

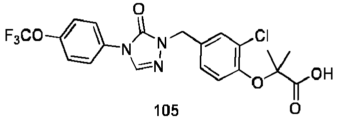

Compound 105 was prepared by replacing intermediate I-3 with intermediate D-3 and 4-hydroxy-3,5-dimethylbenzaldehyde with 4-hydroxy-3-chlorobenzaldehyde according to the method of Example 55: ¹H NMR (300 MHz, DMSO-*d₆*) δ 13.22 (s, 1H), 8.54 (s, 1H), 7.85 (s, 1H), 7.55 (d, *J* = 8.6 Hz, 2H), 7.42 (d, *J* = 1.8 Hz, 2H), 7.21 (d, *J* = 8.6 Hz, 1H), 6.90 (d, *J* = 8.5 Hz, 1H), 4.90 (s, 2H), 1.53 (s, 6H). HRMS (ESI) calcd. for C₂₀H₁₇ClF₃N₃O₅ [M+H]⁺ 472.0087, found 472.0891.

### Example 106

### Ethyl 2-methyl-2-(2-methyl-4-((5-oxo-4-(4-(trifluoromethoxy)phenyl)-4,5-dihydro-1H-1,2,4-triazol-1-yl)methyl)-6-(trifluoromethyl)phenoxy)propionate (compound 106)

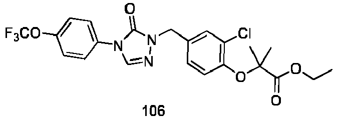

Compound 106 was prepared by replacing intermediate I-3 with intermediate D-3 and 4-hydroxy-3,5-dimethylbenzaldehyde with 4-hydroxy-3-chlorobenzaldehyde without hydrolysis according to the method of Example 55: ¹H NMR (300 MHz, CDCl₃) δ 7.71 (s, 1H), 7.64 (d, *J* = 8.9 Hz, 2H), 7.46 (d, *J* = 1.9 Hz, 1H), 7.36 (d, *J* = 8.7 Hz, 2H), 7.20 (dd, *J* = 8.5, 1.8 Hz, 1H), 6.87 (d, *J* = 8.4 Hz, 1H), 4.95 (s, 2H), 4.26 (q, *J* = 7.1 Hz, 2H), 1.63 (s, 6H), 1.28 (t, *J* = 7.1 Hz, 3H). MS (ESI): m/z 522.1 [M+Na]⁺.

### Example 107

### 2-(4-((4-(4-Methoxyphenyl)-5-oxo-4,5-dihydro-1H-1,2,4-triazol-1-yl)methyl)-2,6-dimethylphenoxy)-2-methylpropionic acid (compound 107)

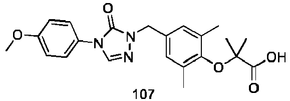

Compound 107 was prepared by replacing intermediate I-3 with intermediate D-10 according to the method of Example 55: ¹H NMR (300 MHz, DMSO-*d₆*) δ 12.81 (s, 1H), 8.35 (s, 1H), 7.57 (d, *J* = 9.0 Hz, 2H), 7.04 (d, *J* = 9.0 Hz, 2H), 6.92 (s, 2H), 4.79 (s, 2H), 3.77 (s, 3H), 2.13 (s, 6H), 1.32 (s, 6H). HRMS (ESI) calcd. for C₂₂H₂₅N₃O₅ [M+H]⁺ 412.1867, found 412.1868.

### Example 108

### Ethyl 2-(4-((4-(4-methoxyphenyl)-5-oxo-4,5-dihydro-1H-1,2,4-triazol-1-yl)methyl)-2,6-dimethylphenoxy)-2-methylpropionate (compound 108)

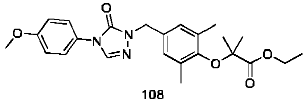

Compound 108 was prepared by replacing intermediate I-3 with intermediate D-10 without hydrolysis according to the method of Example 55: ¹H NMR (300 MHz, CDCl₃) δ 7.63 (s, 1H), 7.46 (d, *J* = 8.9 Hz, 2H), 7.12 - 6.94 (m, 4H), 4.90 (s, 2H), 4.29 (q, *J* = 7.1 Hz, 2H), 3.85 (s, 3H), 2.20 (s, 6H), 1.47 (s, 6H), 1.36 (t, *J* = 7.1 Hz, 3H). MS (ESI): m/z 462.3 [M+Na]⁺.

### Example 109

### 2-(4-((4-(4-Fluorophenyl)-5-oxo-4,5-dihydro-1H-1,2,4-triazol-1-yl)methyl)-2,6-dimethylphenoxy)-2-methylpropionic acid (compound 109)

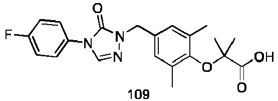

Compound 109 was prepared by replacing intermediate I-3 with intermediate D-11 according to the method of Example 55: ¹H NMR (300 MHz, DMSO-*d₆*) δ 12.80 (s, 1H), 8.44 (s, 1H), 7.74 (dd, *J* = 8.9, 4.8 Hz, 2H), 7.35 (t, *J* = 8.8 Hz, 2H), 6.93 (s, 2H), 4.81 (s, 2H), 2.13 (s, 6H), 1.33 (s, 6H). HRMS (ESI) calcd. for C₂₁H₂₂FN₃O₄ [M+H]⁺ 400.1667, found 400.1675.

### Example 110

### Ethyl 2-(4-((4-(4-fluorophenyl)-5-oxo-4,5-dihydro-1H-1,2,4-triazol-1-yl)methyl)-2,6-dimethylphenoxy)-2-methylpropionate (compound 110)

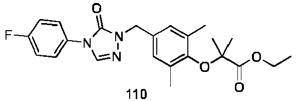

Compound 110 was prepared by replacing intermediate I-3 with intermediate D-11 without hydrolysis according to the method of Example 55: ¹H NMR (300 MHz, DMSO-*d₆*) δ 8.46 (s, 1H), 7.76 (dd, *J* = 9.0, 4.8 Hz, 2H), 7.38 (t, *J* = 8.8 Hz, 2H), 6.96 (s, 2H), 4.83 (s, 2H), 4.17 (q, *J* = 7.1 Hz, 2H), 2.11 (s, 6H), 1.37 (s, 6H), 1.24 (t, *J* = 7.1 Hz, 3H). MS (ESI): m/z 450.2 [M+Na]⁺.

### Example 111

### 2-(4-((4-(4-Cyanophenyl)-5-oxo-4,5-dihydro-1H-1,2,4-triazol-1-yl)methyl)-2, 6-dimethylphenoxy)-2-methylpropionic acid (compound 111)

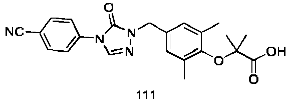

### Synthesis of intermediate D-13

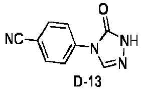

Intermediate D-13 was prepared by replacing *p*-bromoaniline in Example 1 with *p-*cyanoaniline according to the method for intermediate I-3 of Example 1.

### Compound 111

Compound 111 was prepared by replacing intermediate I-3 with intermediate D-13 according to the method of Example 55: ¹H NMR (300 MHz, DMSO-*d₆*) δ 12.99 (s, 1H), 8.62 (s, 1H), 8.07 (d, *J* = 8.5 Hz, 2H), 7.92 (d, *J* = 8.5 Hz, 2H), 6.96 (s, 2H), 4.85 (s, 2H), 2.16 (s, 6H), 1.35 (s, 6H). HRMS (ESI) calcd. for C₂₂H₂₂N₄O₄ [M+H]⁺ 407.1719, found 407.1719.

### Example 112

### Ethyl 2-(4-((4-(4-cyanophenyl)-5-oxo-4,5-dihydro-1H-1,2,4-triazol-1-yl)methyl)-2,6-dimethylphenoxy)-2-methylpropionate (compound 112)

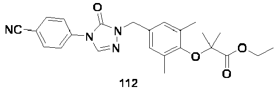

Compound 112 was prepared by replacing intermediate I-3 with intermediate D-13 without hydrolysis according to the method of Example 55: ¹H NMR (300 MHz, CDCl₃) δ 7.84 (d, *J* = 8.8 Hz, 2H), 7.81 (d, *J* = 3.8 Hz, 2H), 7.29 (s, 1H), 7.03 (s, 2H), 4.92 (s, 2H), 4.30 (q, *J* = 7.1 Hz, 2H), 2.21 (s, 6H), 1.48 (s, 6H), 1.37 (t, *J* = 7.1 Hz, 3H). MS (ESI): m/z 457.2 [M+Na]⁺.

### Example 113

### 2-(2,6-Dimethyl-4-((4-(4-(methylsulfonyl)phenyl)-5-oxo-4,5-dihydro-1H-1,2,4-triazol-1-yl)methyl)phenoxy)-2-methylpropionic acid (compound 113)

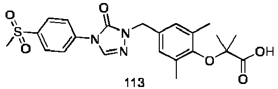

### Synthesis of intermediate D-14

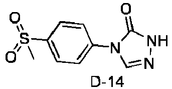

Intermediate D-14 was prepared by replacing *p*-bromoaniline in Example 1 with *p-*methylsulfonylaniline according to the method for intermediate I-3 of Example 1.

### Compound 113

Compound 113 was prepared by replacing intermediate I-3 with intermediate D-14 according to the method of Example 55: ¹H NMR (300 MHz, DMSO-*d₆*) δ 12.87 (s, 1H), 8.67 (s, 1H), 8.08 (d, *J* = 1.2 Hz, 4H), 6.96 (s, 2H), 4.85 (s, 2H), 3.27 (s, 3H), 2.16 (s, 6H), 1.35 (s, 6H). HRMS (ESI) calcd. for C₂₂H₂₅N₃O₆S [M+H]⁺ 460.1542, found 460.1538.

### Example 114

### Ethyl 2-(2,6-dimethyl-4-((4-(methylsulfonyl)phenyl)-5-oxo-4,5-dihydro-1H-1,2,4-triazol-1-yl)methyl)phenoxy)-2-methylpropionate (compound 114)

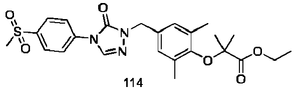

Compound 114 was prepared by replacing intermediate I-3 with intermediate D-14 without hydrolysis according to the method of Example 55: ¹H NMR (300 MHz, CDCl₃) δ 8.07 (d, *J* = 8.6 Hz, 2H), 7.88 (d, *J* = 8.6 Hz, 2H), 7.80 (s, 1H), 7.01 (s, 2H), 4.90 (s, 2H), 4.28 (q, *J* = 7.1 Hz, 2H), 3.07 (s, 3H), 2.19 (s, 6H), 1.45 (s, 6H), 1.34 (t, *J* = 7.1 Hz, 3H). MS (ESI): m/z 510.2 [M+Na]⁺.

### Example 115

### 2-(4-((4-(4-Isopropylphenyl)-5-oxo-4,5-dihydro-1H-1,2,4-triazol-1-yl)methyl)-2,6-dimethylphenoxy)-2-methylpropionic acid (compound 115)

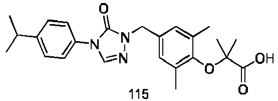

### Synthesis of intermediate D-15

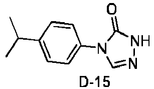

Intermediate D-15 was prepared by replacing *p*-bromoaniline in Example 1 with *p-*isopropylaniline according to the method for intermediate I-3 of Example 1.

### Synthesis of compound 115

Compound 115 was prepared by replacing intermediate I-3 with intermediate D-15 according to the method of Example 55: ¹H NMR (300 MHz, DMSO-*d₆*) δ 12.86 (s, 1H), 8.43 (s, 1H), 7.60 (d, *J* = 8.2 Hz, 2H), 7.39 (d, *J* = 8.3 Hz, 2H), 6.95 (s, 2H), 4.83 (s, 2H), 3.09 - 2.84 (m, 1H), 2.15 (s, 6H), 1.35 (s, 6H), 1.22 (d, *J* = 6.9 Hz, 6H). HRMS (ESI) calcd. for C₂₄H₂₉N₃O₄ [M+H]⁺ 424.2236, found 424.2241.

### Example 116

### Ethyl 2-(4-((4-(4-Isopropylphenyl)-5-oxo-4,5-dihydro-1H-1,2,4-triazol-1-yl)methyl)-2,6-dimethylphenoxy)-2-methylpropionate (compound 116)

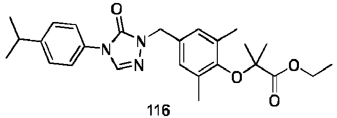

Compound 116 was prepared by replacing intermediate I-3 with intermediate D-15 without hydrolysis according to the method of Example 55: ¹H NMR (300 MHz, CDCl₃) δ 7.65 (s, 1H), 7.46 (d, *J* = 8.5 Hz, 2H), 7.32 (d, *J* = 8.4 Hz, 2H), 7.01 (s, 2H), 4.89 (s, 2H), 4.28 (q, *J* = 7.1 Hz, 2H), 2.94 (dt, *J* = 13.9, 6.9 Hz, 1H), 2.18 (s, 6H), 1.45 (s, 6H), 1.34 (t, *J* = 7.1 Hz, 3H), 1.25 (d, *J* = 6.9 Hz, 6H). MS (ESI): m/z 474.2 [M+Na]⁺.

### Example 117

### 2-(4-((4-([1,1'-Biphenyl]-4-yl)-5-oxo-4,5-dihydro-1H-1,2,4-triazol-1-yl)methyl)-2,6-dimethylphenoxy)-2-methylpropionic acid (compound 117)

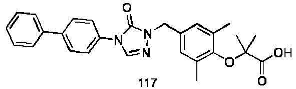

### Synthesis of intermediate D-16

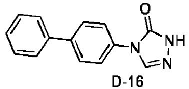

Intermediate D-16 was prepared by replacing *p*-bromoaniline in Example 1 with 4-phenylaniline according to the method for intermediate I-3 of Example 1.

### Synthesis of compound 117

Compound 117 was prepared by replacing intermediate I-3 with intermediate D-16 according to the method of Example 55: ¹H NMR (300 MHz, DMSO-*d₆*) δ 12.84 (s, 1H), 8.56 (s, 1H), 7.83 (s, 4H), 7.66 (t, *J* = 32.4 Hz, 2H), 7.50 (t, *J* = 7.4 Hz, 2H), 7.41 (d, *J =* 7.2 Hz, 1H), 6.97 (s, 2H), 4.85 (s, 2H), 2.16 (s, 6H), 1.35 (s, 6H). HRMS (ESI) calcd. for C₂₇H₂₇N₃O₄ [M+H]⁺ 458.2080, found 458.2080.

### Example 118

### Ethyl 2-(4-((4-([1,1'-biphenyl]-4-yl)-5-oxo-4,5-dihydro-1H-1,2,4-triazol-1-yl)methyl)-2,6-dimethylphenoxy)-2-methylpropionate (compound 118)

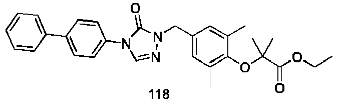

Compound 118 was prepared by replacing intermediate I-3 with intermediate D-16 without hydrolysis according to the method of Example 55: ¹H NMR (300 MHz, CDCl₃) δ 7.76 (s, 1H), 7.72 (d, *J* = 8.9 Hz, 4H), 7.68 (d, *J* = 8.1 Hz, 2H), 7.62 (d, *J* = 7.0 Hz, 2H), 7.49 (t, *J* = 7.3 Hz,2H), 7.42 (d, *J* = 6.7 Hz, 1H), 7.06 (s, 2H), 4.94 (s, 2H), 4.31 (q, *J* = 13.4, 6.6 Hz, 2H), 2.22 (s, 6H), 1.48 (s, 6H), 1.37 (t, *J* = 6.8 Hz, 3H). MS (ESI): m/z 508.4 [M+Na]⁺.

### Example 119

### 2-(2,6-Dimethyl-4-((5-oxo-4-(2-(trifluoromethyl)phenyl)-4,5-dihydro-1H-1,2,4-triazol-1-yl)methyl)phenoxy)-2-methylpropionic acid (compound 119)

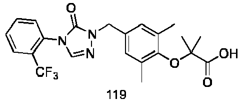

### Synthesis of intermediate D-17

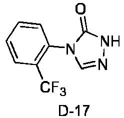

Intermediate D-17 was prepared by replacing p-bromoaniline in Example 1 with *o-*trifluoromethylaniline according to the method for intermediate I-3 of Example 1.

### Synthesis of compound 119

Compound 119 was prepared by replacing intermediate I-3 with intermediate D-17 according to the method of Example 55: ¹H NMR (300 MHz, DMSO-*d₆*) δ 12.85 (s, 1H), 8.21 (s, 1H), 7.97 (d, *J* = 7.5 Hz, 1H), 7.90 (t, *J* = 7.4 Hz, 1H), 7.80 (d, *J* = 7.7 Hz, 1H), 7.74 (t, *J* = 7.3 Hz, 1H), 6.89 (s, 2H), 4.84 (s, 2H), 2.16 (s, 6H), 1.36 (s, 6H). HRMS (ESI) calcd. for C₂₂H₂₂F₃N₃O₄ [M+H]⁺ 458.2080, found 458.2080.

### Example 120

### Ethyl 2-(2,6-dimethyl-4-((5-oxo-4-(2-(trifluoromethyl)phenyl)-4,5-dihydro-1H-1,2,4-triazol-1-yl)methyl)phenoxy)-2-methylpropionate (compound 120)

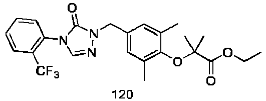

Compound 120 was prepared by replacing intermediate I-3 with intermediate D-17 without hydrolysis according to the method of Example 55: ¹H NMR (300 MHz, CDCl₃) δ 7.86 (d, *J* = 7.7 Hz, 1H), 7.74 (t, *J* = 7.7 Hz, 1H), 7.64 (t, *J* = 7.5 Hz, 1H), 7.53 (d, *J* = 7.6 Hz, 1H), 7.49 (s, 1H), 6.99 (s, 2H), 4.94 (s, 2H), 4.31 (q, *J* = 7.1 Hz, 2H), 2.22 (s, 6H), 1.49 (s, 6H), 1.37 (t, *J* = 7.1 Hz, 3H). MS (ESI): m/z 500.4 [M+Na]⁺.

### Example 121

### 2-(2,6-Dimethyl-4-((5-oxo-4-(3-(trifluoromethyl)phenyl)-4,5-dihydro-1H-1,2,4-triazol-1-yl)methyl)phenoxy)-2-methylpropionic acid (compound 121)

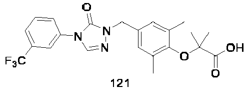

### Synthesis of intermediate D-18

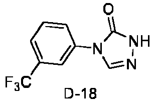

Intermediate D-18 was prepared by replacing p-bromoaniline in Example 1 with *m-*trifluoromethylaniline according to the method for intermediate I-3 of Example 1.

### Synthesis of compound 121

Compound 121 was prepared by replacing intermediate I-3 with intermediate D-18 according to the method of Example 55: ¹H NMR (300 MHz, DMSO-*d₆*) δ 12.85 (s, 1H), 8.64 (s, 1H), 8.20 (s, 1H), 8.08 (d, *J* = 7.1 Hz, 1H), 7.83 - 7.65 (m, 2H), 6.96 (s, 2H), 4.85 (s, 2H), 2.16 (s, 6H), 1.35 (s, 6H). HRMS (ESI) calcd. for C₂₂H₂₂F₃N₃O₄ [M+H]⁺ 458.2080, found 458.2080.

### Example 122

### Ethyl 2-(2,6-dimethyl-4-((5-oxo-4-(3-(trifluoromethyl)phenyl)-4,5-dihydro-1H-1,2,4-triazol-1-yl)methyl)phenoxy)-2-methylpropionate (compound 122)

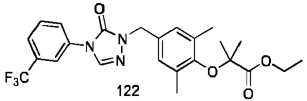

Compound 122 was prepared by replacing intermediate I-3 with intermediate D-18 without hydrolysis according to the method of Example 55: ¹H NMR (300 MHz, CDCl₃) δ 7.91 (s, 1H), 7.85 (s, 1H), 7.77 (s, 1H), 7.64 (d, *J* = 4.8 Hz, 2H), 7.04 (s, 2H), 4.92 (s, 2H), 4.30 (q, *J* = 7.1 Hz, 2H), 2.21 (s, 6H), 1.48 (s, 6H), 1.36 (t, *J* = 7.1 Hz, 3H). MS (ESI): m/z 500.2 [M+Na]⁺.

### Example 123

### 2-(2-Fluoro-4-((5-oxo-4-(4-(trifluoromethyl)phenyl)-4, 5-dihydro-1H-1,2,4-triazol-1-yl)methyl)phenoxy)-2-methylpropionic acid (compound 123)

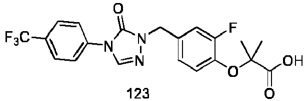

Compound 123 was prepared by replacing intermediate I-3 with intermediate D-1 and 4-hydroxy-3,5-dimethylbenzaldehyde with 4-hydroxy-3-fluorobenzaldehyde according to the method of Example 55: ¹H NMR (300 MHz, DMSO-*d₆*) δ 13.12 (s, 1H), 8.65 (s, 1H), 8.02 (d, *J* = 8.5 Hz, 2H), 7.91 (d, *J* = 8.6 Hz, 2H), 7.20 (d, *J* = 12.0 Hz, 1H), 7.06 (d, *J* = 7.9 Hz, 1H), 6.97 (t, *J* = 8.3 Hz, 1H), 4.93 (s, 2H), 1.50 (s, 6H). HRMS (ESI) calcd. for C₂₀H₁₇F₄N₃O₄ [M+H]⁺ 440.1233, found 440.1299.

### Example 124

### Ethyl 2-(2-fluoro-4-((5-oxo-4-(4-(trifluoromethyl)phenyl)-4,5-dihydro-1H-1,2,4-triazol-1-yl)methyl)phenoxy)-2-methylpropionate (compound 124)

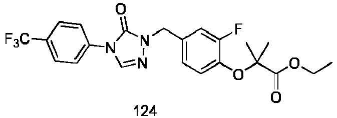

Compound 124 was prepared by replacing intermediate I-3 with intermediate D-1 and 4-hydroxy-3,5-dimethylbenzaldehyde with 4-hydroxy-3-fluorobenzaldehyde without hydrolysis according to the method of Example 55: ¹H NMR (300 MHz, CDCl₃) δ 7.76 (d, *J* = 2.5 Hz, 4H), 7.26 (s, 1H), 7.15 (dd, *J* = 11.3, 1.9 Hz, 1H), 7.06 (d, *J* = 8.8 Hz, 1H), 6.95 (t, *J* = 8.2 Hz, 1H), 4.95 (s, 2H), 4.24 (q, *J* = 7.1 Hz, 2H), 1.58 (s, 6H), 1.28 (t, *J* = 7.1 Hz, 3H). MS (ESI): m/z 490.2 [M+Na]⁺.

### Example 125

### 2-(2-Chloro-4-((5-oxo-4-(4-(trifluoromethyl)phenyl)-4,5-dihydro-1H-1,2,4-triazol-1-yl)methyl)phenoxy)-2-methylpropionic acid (compound 125)

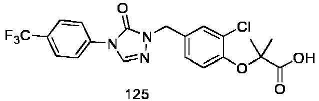

Compound 125 was prepared by replacing intermediate I-3 with intermediate D-1 and 4-hydroxy-3,5-dimethylbenzaldehyde with 4-hydroxy-3-chlorobenzaldehyde according to the method of Example 55: ¹H NMR (300 MHz, DMSO-*d₆*) δ 13.22 (s, 1H), 8.65 (s, 1H), 8.02 (d, *J* = 8.6 Hz, 2H), 7.91 (d, *J* = 8.7 Hz, 2H), 7.43 (d, *J* = 1.7 Hz, 1H), 7.22 (d, *J* = 8.4 Hz, 1H), 6.90 (d, *J* = 8.5 Hz, 1H), 4.92 (s, 2H), 1.53 (s, 6H). HRMS (ESI) calcd. for C₂₀H₁₇ClF₃N₃O₄ [M+H]⁺ 456.0938, found 456.0938.

### Example 126

### Ethyl 2-(2-chloro-4-((5-oxo-4-(4-(trifluoromethyl)phenyl)-4,5-dihydro-1H-1,2,4-triazol-1-yl)methyl)phenoxy)-2-methylpropionate (compound 126)

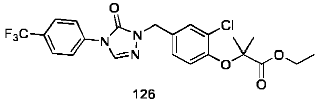

Compound 126 was prepared by replacing intermediate I-3 with intermediate D-1 and 4-hydroxy-3,5-dimethylbenzaldehyde with 4-hydroxy-3-chlorobenzaldehyde without hydrolysis according to the method of Example 55: ¹H NMR (300 MHz, CDCl₃) δ 7.76 (s, 5H), 7.44 (d, *J* = 2.0 Hz, 1H), 7.18 (dd, *J* = 8.4, 2.0 Hz, 1H), 6.86 (d, *J* = 8.4 Hz, 1H), 4.94 (s, 2H), 4.24 (q, *J* = 7.1 Hz, 2H), 1.56 (s, 6H), 1.26 (t, *J* = 7.1 Hz, 3H). MS (ESI): m/z 506.3 [M+Na]⁺.

### Example 127

### 2-(2,6-Difluoro-4-((5-oxo-4-(4-(trifluoromethyl)phenyl)-4,5-dihydro-1H-1,2,4-triazol-1-yl)methyl)phenoxy)-2-methylpropionic acid (compound 127)

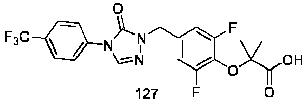

Compound 127 was prepared by replacing intermediate I-3 with intermediate D-1 and 4-hydroxy-3,5-dimethylbenzaldehyde with 4-hydroxy-3,5-difluorobenzaldehyde according to the method of Example 55: ¹H NMR (300 MHz, DMSO-*d₆*) δ 12.97 (s, 1H), 8.68 (s, 1H), 8.03 (d, *J* = 8.5 Hz, 2H), 7.92 (d, *J* = 8.6 Hz, 2H), 7.11 (d, *J* = 8.8 Hz, 2H), 4.98 (s, 2H), 1.44 (s, 6H). HRMS (ESI) calcd. for C₂₀H₁₆F₅N₃O₄ [M+H]⁺ 458.1139, found 458.1140.

### Example 128

### Ethyl 2-(2,6-difluoro-4-((5-oxo-4-(4-(trifluoromethyl)phenyl)-4,5-dihydro-1H-1,2,4-triazol-1-yl)methyl)phenoxy)-2-methylpropionate (compound 128)

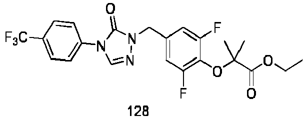

Compound 128 was prepared by replacing intermediate I-3 with intermediate D-1 and 4-hydroxy-3,5-dimethylbenzaldehyde with 4-hydroxy-3,5-difluorobenzaldehyde without hydrolysis according to the method of Example 55: ¹H NMR (300 MHz, CDCl₃) δ 7.79 (d, *J* = 7.5 Hz, 1H), 7.28 (s, 1H), 6.97 (d, *J* = 7.9 Hz, 1H), 4.96 (s, 1H), 4.26 (q, *J* = 7.1 Hz, 1H), 1.57 (s, 2H), 1.32 (t, *J* = 7.1 Hz, 1H). MS (ESI): m/z 508.1 [M+Na]⁺.

### Example 129

### 2-(2,6-Dichloro-4-((5-oxo-4-(4-(trifluoromethyl)phenyl)-4,5-dihydro-1H-1,2,4-triazol-1-yl)methyl)phenoxy)-2-methylpropionic acid (compound 129)

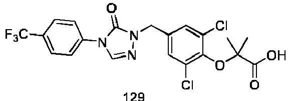

Compound 129 was prepared by replacing intermediate I-3 with intermediate D-1 and 4-hydroxy-3,5-dimethylbenzaldehyde with 4-hydroxy-3,5-dichlorobenzaldehyde according to the method of Example 55: ¹H NMR (300 MHz, DMSO-*d₆*) δ 12.91 (s, 1H), 8.68 (s, 1H), 8.03 (d, *J* = 8.5 Hz, 2H), 7.92 (d, *J* = 8.7 Hz, 2H), 7.45 (s, 2H), 4.98 (s, 2H), 1.46 (s, 3H). HRMS (ESI) calcd. for C₂₀H₁₆Cl₂F₃N₃O₄ [M+H]⁺ 490.0548, found 490.0545.

### Example 130

### Ethyl 2-(2, 6-dichloro-4-((5-oxo-4-(4-(trifluoromethyl)phenyl)-4, 5-dihydro-1H-1,2,4-triazol-1-yl)methyl)phenoxy)-2-methylpropionate (compound 130)

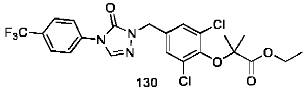

Compound 130 was prepared by replacing intermediate I-3 with intermediate D-1 and 4-hydroxy-3,5-dimethylbenzaldehyde with 4-hydroxy-3,5-dichlorobenzaldehyde without hydrolysis according to the method of Example 55: ¹H NMR (300 MHz, CDCl₃) δ 7.80 (d, *J* = 7.5 Hz, 4H), 7.35 (s, 1H), 7.28 (s, 2H), 4.95 (s, 2H), 4.30 (q, *J* = 7.1 Hz, 2H), 1.60 (s, 6H), 1.36 (t, *J* = 7.1 Hz, 3H). MS (ESI): m/z 540.1 [M+Na]⁺.

### Example 131

### 2-(4-((4-(4-Ethylphenyl)-5-oxo-4,5-dihydro-1H-1,2,4-triazol-1-yl)methyl)-2,6-dimethylphenoxy)-2-methylpropionic acid (compound 131)

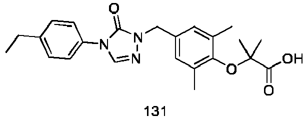

### Synthesis of intermediate D-19

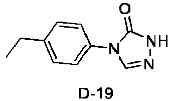

Intermediate D-19 was prepared by replacing p-bromoaniline in Example 1 with *p-*ethylaniline according to the method for intermediate I-3 of Example 1.

### Synthesis of compound 131

Compound 131 was prepared by replacing intermediate I-3 with intermediate D-19 according to the method of Example 55: ¹H NMR (300 MHz, DMSO-*d₆*) δ 12.87 (s, 1H), 8.44 (s, 1H), 7.61 (d, *J* = 8.3 Hz, 2H), 7.35 (d, *J* = 8.3 Hz, 2H), 6.95 (s, 2H), 4.83 (s, 2H), 2.65 (q, *J* = 7.6 Hz, 2H), 2.15 (s, 6H), 1.35 (s, 6H), 1.20 (t, *J* = 7.6 Hz, 3H). HRMS (ESI) calcd. for C₂₃H₂₇N₃O₄ [M+H]⁺ 410.2080, found 410.2080.

### Example 132

### Ethyl 2-(4-((4-(4-Ethylphenyl)-5-oxo-4,5-dihydro-1H-1,2,4-triazol-1-yl)methyl)-2,6-dimethylphenoxy)-2-methylpropionate (compound 132)

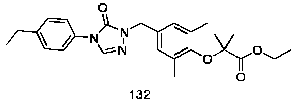

Compound 132 was prepared by replacing intermediate I-3 in Example 55 with intermediate D-19 without hydrolysis according to the method of Example 55: ¹H NMR (300 MHz, CDCl₃) δ 7.68 (s, 1H), 7.48 (d, *J* = 8.4 Hz, 2H), 7.31 (d, *J* = 8.4 Hz, 2H), 7.04 (s, 2H), 4.92 (s, 2H), 4.30 (q, *J* = 7.1 Hz, 2H), 2.70 (q, *J* = 7.6 Hz, 2H), 2.21 (s, 6H), 1.47 (s, 6H), 1.36 (t, *J* = 7.1 Hz, 3H), 1.28 (t, 3H). MS(ESI): m/z 460.2 [M+Na]⁺.

### Example 133

### 2-(2,6-Dimethyl-4-((5-oxo-4-(4-(trifluoromethyl)phenyl)-4,5-dihydro-1H-1,2,4-triazol-1-yl)methyl)phenoxy)acetic acid (compound 133)

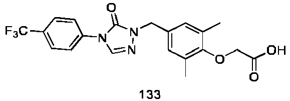

Compound 133 was prepared by replacing intermediate I-3 with intermediate D-1 and ethyl bromoisobutyrate with ethyl 2-bromoacetate according to the method of Example 55: ¹H NMR (300 MHz, DMSO-*d₆*) δ 12.87 (s, 1H), 8.61 (s, 1H), 8.01 (d, *J* = 8.5 Hz, 2H), 7.89 (d, *J* = 8.7 Hz, 2H), 6.97 (s, 2H), 4.83 (s, 2H), 4.33 (s, 2H), 2.20 (s, 6H). HRMS (ESI) calcd. for C₂₀H₁₈F₃N₃O₄ [M+H]⁺ 422.1322, found 422.1327.

### Example 134

### Ethyl 2-(2, 6-dimethyl-4-((5-oxo-4-(4-(trifluoromethyl)phenyl)-4, 5-dihydro-1H-1,2,4-triazol-1-yl)methyl)phenoxy)acetate (compound 134)

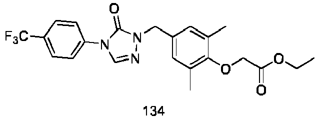

Compound 134 was prepared by replacing intermediate I-3 with intermediate D-1 and ethyl bromoisobutyrate with ethyl 2-bromoacetate without hydrolysis according to the method of Example 55: ¹H NMR (300 MHz, CDCl₃) *δ*7.78 (s, 4H), 7.08 (s, 2H), 4.93 (s, 2H), 4.39 (s, 2H), 4.31 (q, *J* = 7.0 Hz, 2H), 2.31 (s, 6H), 1.34 (t, *J* = 7.1 Hz, 3H). HRMS (ESI) calcd. for C₂₂H₂₂F₃N₃O₄ [M+H]⁺ 450.1635, found 450.1644.

### Example 135

### 2-(4-((4-(4-Bromophenyl)-5-oxo-4,5-dihydro-1H-1,2,4-triazol-1-yl)methyl)phenoxy)-2-methylpropionic acid (compound 135)

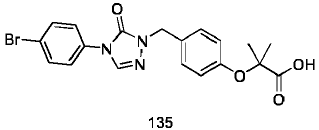

Compound 135 was prepared by replacing 4-hydroxy-3,5-dimethylbenzaldehyde in Example 55 with 4-hydroxybenzaldehyde according to the method of Example 55: ¹H NMR (300 MHz, DMSO-*d₆*) δ 13.07 (s, 1H), 8.51 (s, 1H), 7.72 (s, 4H), 7.22 (d, *J* = 8.5 Hz, 2H), 6.80 (d, *J* = 8.5 Hz, 2H), 4.88 (s, 2H), 1.50 (s, 6H). MS (ESI): m/z 454.1 [M+Na]⁺.

### Example 136

### Ethyl 2-(4-((4-(4-bromophenyl)-5-oxo-4,5-dihydro-1H-1,2,4-triazol-1-yl)methyl)phenoxy)-2-methylpropionate (compound 136)

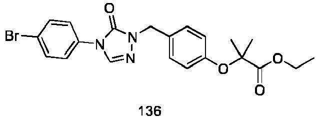

Compound 136 was prepared by replacing 4-hydroxy-3,5-dimethylbenzaldehyde in Example 55 with 4-hydroxybenzaldehyde without hydrolysis according to the method of Example 55: ¹H NMR (300 MHz, CDCl₃) δ 7.68 (s, 1H), 7.62 (d, *J* = 8.8 Hz, 2H), 7.48 (d, *J* = 8.8 Hz, 2H), 7.31 (d, *J* = 8.6 Hz, 2H), 6.83 (d, *J* = 8.6 Hz, 2H), 4.96 (s, 2H), 4.24 (q, *J* = 7.1 Hz, 2H), 1.27 (s, 6H), 0.87 (t, 3H). MS (ESI): m/z 482.1 [M+Na]⁺.

### Example 137

### 2-(4-((4-(4-Trifluoromethylphenyl)-5-oxo-4, 5-dihydro-1H-1,2,4-triazol-1-yl)methyl)phenoxy)-2-methylpropionic acid (compound 137)

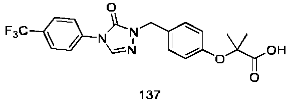

Compound 137 was prepared by replacing intermediate I-3 with intermediate D-1 and 4-hydroxy-3,5-dimethylbenzaldehyde with 4-hydroxybenzaldehyde according to the method of Example 55: ¹H NMR (300 MHz, DMSO-*d₆*) δ 13.06 (s, 1H), 8.64 (s, 1H), 8.02 (d, *J* = 8.5 Hz, 2H), 7.91 (d, *J* = 8.6 Hz, 2H), 7.24 (d, *J* = 8.6 Hz, 2H), 6.80 (d, *J* = 8.6 Hz, 2H), 4.90 (s, 2H), 1.50 (s, 6H). MS (ESI): m/z 444.2 [M+Na]⁺.

### Example 138

### Ethyl 2-(4-((4-(4-trifluoromethylphenyl)-5-oxo-4,5-dihydro-1H-1,2,4-triazol-1-yl)methyl)phenoxy)-2-methylpropionate (compound 138)

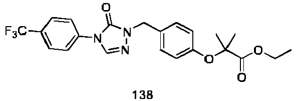

Compound 138 was prepared by replacing intermediate I-3 with intermediate D-1 and 4-hydroxy-3,5-dimethylbenzaldehyde with 4-hydroxybenzaldehyde without hydrolysis according to the method of Example 55: ¹H NMR (300 MHz, CDCl₃) δ 7.77 (s, 4H), 7.32 (d, *J* = 8.5 Hz, 2H), 6.84 (d, *J* = 8.5 Hz, 2H), 4.97 (s, 2H), 4.24 (q, *J* = 7.1 Hz, 2H), 1.60 (s, 6H), 1.26 (t, *J* = 7.1 Hz, 3H). MS (ESI): m/z 472.2 [M+Na]⁺.

### Example 139

### 2-(2-Methyl-4-((5-oxo-4-(4-(trifluoromethyl)phenyl)-4,5-dihydro-1H-1 ,2,4-triazol-1-yl)methyl)phenoxy)acetic acid (compound 139)

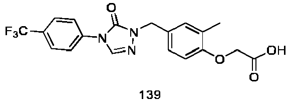

Compound 139 was prepared by replacing intermediate I-3 with intermediate D-1, 4-hydroxy-3,5-dimethylbenzaldehyde with 3-methyl-4-hydroxybenzaldehyde, and ethyl bromoisobutyrate with ethyl 2-bromoacetate according to the method of Example 55: ¹H NMR (300 MHz, DMSO-*d₆*) δ 13.02 (s, 1H), 8.62 (s, 1H), 8.02 (d, *J* = 8.3 Hz, 2H), 7.91 (d, *J* = 8.2 Hz, 2H), 7.14 (s, 1H), 7.10 (d, *J* = 8.6 Hz, 1H), 6.79 (d, *J* = 8.1 Hz, 1H), 4.86 (s, 2H), 4.68 (s, 2H), 2.18 (s, 3H). MS (ESI): m/z 430.2 [M+Na]⁺.

### Example 140

### Ethyl 2-(2-methyl-4-((5-oxo-4-(4-(trifluoromethyl)phenyl)-4,5-dihydro-1H-1,2,4-triazol-1-yl)methyl)phenoxy)acetate (compound 140)

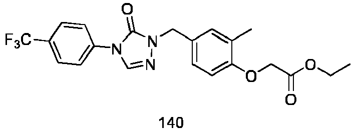

Compound 140 was prepared by replacing intermediate I-3 with intermediate D-1, 4-hydroxy-3,5-dimethylbenzaldehyde with 3-methyl-4-hydroxybenzaldehyde, and ethyl bromoisobutyrate with ethyl 2-bromoacetate without hydrolysis according to the method of Example 55: ¹H NMR (300 MHz, CDCl₃) δ 7.76 (d, *J* = 4.4 Hz, 4H), 7.28 (s, 2H), 7.23 (d, *J* = 8.5 Hz, 1H), 6.69 (d, *J* = 8.2 Hz, 1H), 4.95 (s, 2H), 4.64 (s, 2H), 4.28 (q, *J* = 7.1 Hz, 2H), 2.31 (s, 3H), 1.32 (t, 3H). MS (ESI): m/z 458.2 [M+Na]⁺.

### Example 141

### 2-(2-Trifluoromethoxy-4-((5-oxo-4-(4-(trifluoromethyl)phenyl)-4,5-dihydro-1H-1,2,4-triazol-1-yl)methyl)phenoxy)acetic acid (compound 141)

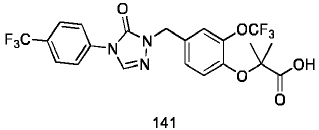

Compound 141 was prepared by replacing intermediate I-3 with intermediate D-1 and 4-hydroxy-3,5-dimethylbenzaldehyde with 4-hydroxy-3-trifluoromethoxybenzaldehyde according to the method of Example 55: ¹H NMR (300 MHz, DMSO-*d₆*) δ 13.23 (s, 1H), 8.66 (s, 1H), 8.02 (d, *J* = 8.5 Hz, 2H), 7.91 (d, *J* = 8.6 Hz, 2H), 7.36 (s, 1H), 7.27 (d, *J* = 8.5 Hz, 1H), 6.94 (d, *J* = 8.5 Hz, 1H), 4.95 (s, 2H), 1.52 (s, 6H). HRMS (ESI) calcd. for C₂₁H₁₇F₆N₃O₅ [M+H]⁺ 506.1151, found 506.1152.

### Example 142

### Ethyl 2-(2-trifluoromethoxy-4-((5-oxo-4-(4-(trifluoromethyl)phenyl)-4,5-dihydro-1H-1,2,4-triazol-1-yl)methyl)phenoxy)acetate (compound 142)

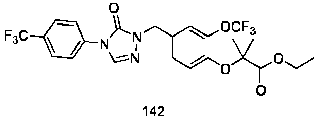

Compound 142 was prepared by replacing intermediate I-3 with intermediate D-1 and 4-hydroxy-3,5-dimethylbenzaldehyde with 4-hydroxy-3-trifluoromethoxybenzaldehyde without hydrolysis according to the method of Example 55: ¹H NMR (300 MHz, CDCl₃) δ 7.77 (s, 4H), 7.33 (s, 1H), 7.28 (s, 1H), 7.24 (d, *J* = 8.7 Hz, 1H), 6.88 (d, *J* = 8.4 Hz, 1H), 4.98 (s, 2H), 4.24 (q, *J* = 7.0 Hz, 2H), 1.61 (s, 6H), 1.26 (t, *J* = 7.0 Hz, 3H). MS (ESI): m/z 556.1 [M+Na]⁺.

### Example 143

### 2-(2-Chloro-6-methyl-4-((5-oxo-4-(4-(trifluoromethyl)phenyl)-4, 5-dihydro-1H-1,2,4-triazol-1-yl)methyl)phenoxy)-2-methylpropionic acid (compound 143)

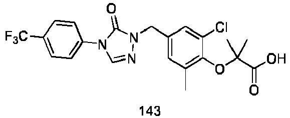

Compound 143 was prepared by replacing intermediate I-3 with intermediate D-1 and 4-hydroxy-3,5-dimethylbenzaldehyde with 3-chloro-4-hydroxy-5-methylbenzaldehyde according to the method of Example 55: ¹H NMR (300 MHz, DMSO-*d₆*) δ 12.82 (s, 1H), 8.67 (s, 1H), 8.03 (d, *J* = 8.5 Hz, 2H), 7.92 (d, *J* = 8.5 Hz, 2H), 7.25 (s, 1H), 7.13 (s, 1H), 4.91 (s, 2H), 2.21 (s, 3H), 1.41 (s, 6H). HRMS (ESI) calcd. for C₂₁H₁₉ClF₃N₃O₄ [M+H]⁺ 470.1094, found 470.1091.

### Example 144

### Ethyl 2-(2-chloro-6-methyl-4-((5-oxo-4-(4-(trifluoromethyl)phenyl)-4,5-dihydro-1H-1,2,4-triazol-1-yl)methyl)phenoxy)-2-methylpropionate (compound 144)

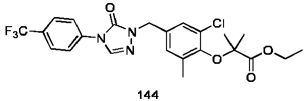

Compound 144 was prepared by replacing intermediate I-3 with intermediate D-1 and 4-hydroxy-3,5-dimethylbenzaldehyde with 3-chloro-4-hydroxy-5-methylbenzaldehyde without hydrolysis according to the method of Example 55: ¹H NMR (300 MHz, CDCl₃) δ 7.73 (s, 1H), 7.65 (d, *J* = 8.9 Hz, 2H), 7.36 (d, *J* = 8.6 Hz, 2H), 7.26 (s, 1H), 7.11 (s, 1H), 4.93 (s, 2H), 4.30 (q, *J* = 7.1 Hz, 2H), 2.25 (s, 3H), 1.54 (s, 6H), 1.36 (t, *J* = 7.1 Hz, 3H). MS (ESI): m/z 520.1 [M+Na]⁺.

### Example 145

### 2-(2-Chloro-6-methyl-4-((5-oxo-4-(4-(trifluoromethoxy)phenyl)-4, 5-dihydro-1H-1,2,4-triazol-1-yl)methyl)phenoxy)-2-methylpropionic acid (compound 145)

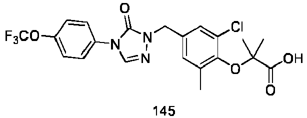

Compound 145 was prepared by replacing intermediate I-3 with intermediate D-3 and 4-hydroxy-3,5-dimethylbenzaldehyde with 3-chloro-4-hydroxy-5-methylbenzaldehyde according to the method of Example 55: ¹H NMR (300 MHz, DMSO-*d₆*) δ 12.89 (s, 1H), 8.56 (s, 1H), 7.88 (d, *J* = 9.0 Hz, 2H), 7.56 (d, *J* = 8.4 Hz, 2H), 7.24 (s, 1H), 7.12 (s, 1H), 4.90 (s, 2H), 2.21 (s, 3H), 1.41 (s, 6H). HRMS (ESI) calcd. for C₂₁H₁₉ClF₃N₃O₅ [M+H]⁺ 486.1044, found 486.1047.

### Example 146

### Ethyl 2-(2-chloro-6-methyl-4-((5-oxo-4-(4-(trifluoromethoxy)phenyl)-4,5-dihydro-1H-1,2,4-triazol-1-yl)methyl)phenoxy)-2-methylpropionate (compound 146)

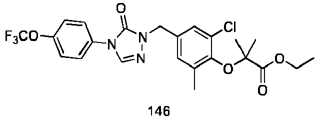

Compound 146 was prepared by replacing intermediate I-3 with intermediate D-3 and 4-hydroxy-3,5-dimethylbenzaldehyde with 3-chloro-4-hydroxy-5-methylbenzaldehyde without hydrolysis according to the method of Example 55: ¹H NMR (300 MHz, CDCl₃) δ 7.73 (s, 1H), 7.65 (d, *J* = 8.9 Hz, 2H), 7.36 (d, *J* = 8.6 Hz, 2H), 7.26 (s, 1H), 7.11 (s, 1H), 4.93 (s, 2H), 4.30 (q, *J* = 7.1 Hz, 2H), 2.25 (s, 3H), 1.54 (s, 6H), 1.36 (t, *J* = 7.1 Hz, 3H). MS (ESI): m/z 536.1 [M+Na]⁺.

### Example 147

### 2-(2-Trifluoromethyl-4-((5-oxo-4-(4-(trifluoromethyl)phenyl)-4,5-dihydro-1H-1,2,4-triazol-1-yl)methyl)phenoxy)acetic acid (compound 147)

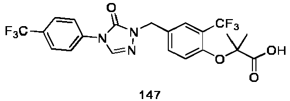

Compound 147 was prepared by replacing intermediate I-3 with intermediate D-1 and 4-hydroxy-3,5-dimethylbenzaldehyde with 4-hydroxy-3-trifluoromethylbenzaldehyde according to the method of Example 55: ¹H NMR (300 MHz, DMSO-*d₆*) δ 13.34 (s, 1H), 8.65 (s, 1H), 8.01 (d, *J* = 8.6 Hz, 2H), 7.91 (d, *J* = 8.6 Hz, 2H), 7.63 (s, 1H), 7.53 (d, *J* = 8.7 Hz, 1H), 6.91 (d, *J* = 8.6 Hz, 1H), 4.98 (s, 2H), 1.54 (s, 6H). HRMS (ESI) calcd. for C₂₁H₁₇F₆N₃O₄ [M+H]⁺ 490.1202, found 490.1204.

### Example 148

### Ethyl 2-(2-trifluoromethyl-4-((5-oxo-4-(4-(trifluoromethyl)phenyl)-4,5-dihydro-1H-1,2,4-triazol-1-yl)methyl)phenoxy)acetate (compound 148)

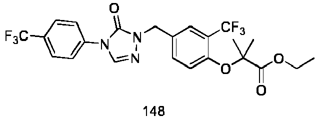

Compound 148 was prepared by replacing intermediate I-3 with intermediate D-1 and 4-hydroxy-3,5-dimethylbenzaldehyde with 4-hydroxy-3-trifluoromethylbenzaldehyde without hydrolysis according to the method of Example 55: ¹H NMR (300 MHz, CDCl₃) δ 7.78 (d, *J* = 3.0 Hz, 5H), 7.65 (s, 1H), 7.53 - 7.42 (m, 1H), 6.81 (d, *J* = 8.6 Hz, 1H), 5.00 (s, 2H), 4.26 (q, *J* = 7.1 Hz, 2H), 1.63 (s, 6H), 1.26 (t, *J* = 7.1 Hz, 3H). MS (ESI): m/z 540.1 [M+Na]⁺.

### Example 149

### 2-(2,6-Dichloro-4-((5-oxo-4-(4-(trifluoromethoxy)phenyl)-4,5-dihydro-1H-1,2,4-triazol-1-yl)methyl)phenoxy)-2-methylpropionic acid (compound 149)

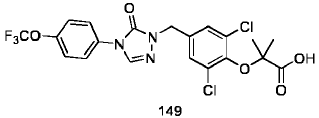

Compound 149 was prepared by replacing intermediate I-3 with intermediate D-3 and 4-hydroxy-3,5-dimethylbenzaldehyde with 3,5-dichloro-4-hydroxybenzaldehyde according to the method of Example 55: ¹H NMR (300 MHz, DMSO-*d₆*) δ 12.92 (s, 1H), 8.58 (s, 1H), 7.88 (d, *J* = 8.9 Hz, 2H), 7.56 (d, *J* = 8.7 Hz, 2H), 7.44 (s, 2H), 4.98 (s, 2H), 1.47 (s, 6H). HRMS (ESI) calcd. for C₂₀H₁₆Cl₂F₃N₃O₅ [M+H]⁺ 506.0497, found 506.0503.

### Example 150

### Ethyl 2-(2,6-dichloro-4-((5-oxo-4-(4-(trifluoromethoxy)phenyl)-4,5-dihydro-1H-1,2,4-triazol-1-yl)methyl)phenoxy)-2-methylpropionate (compound 150)

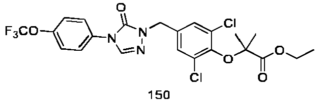

Compound 150 was prepared by replacing intermediate I-3 with intermediate D-3 and 4-hydroxy-3,5-dimethylbenzaldehyde with 3,5-dichloro-4-hydroxybenzaldehyde without hydrolysis according to the method of Example 55: ¹H NMR (300 MHz, CDCl₃) δ 7.74 (s, 1H), 7.65 (d, *J* = 8.9 Hz, 2H), 7.38 (s, 1H), 7.35 (s, 2H), 7.28 (s, 1H), 4.95 (s, 2H), 4.30 (q, *J* = 7.1 Hz, 2H), 1.60 (s, 6H), 1.36 (t, *J* = 7.1 Hz, 3H). MS (ESI): m/z 556.1 [M+Na]⁺.

### Example 151

### 2-(2-Fluoro-4-((5-oxo-4-(4-(trifluoromethoxy)phenyl)-4, 5-dihydro-1H-1,2,4-triazol-1-yl)methyl)phenoxy)-2-methylpropionic acid (compound 151)

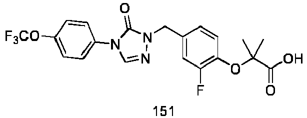

Compound 151 was prepared by replacing intermediate I-3 with intermediate D-3 and 4-hydroxy-3,5-dimethylbenzaldehyde with 3-fluoro-4-hydroxybenzaldehyde according to the method of Example 55: ¹H NMR (300 MHz, DMSO-*d₆*) δ 13.16 (s, 1H), 8.55 (s, 1H), 7.87 (d, *J* = 9.0 Hz, 2H), 7.56 (d, *J* = 8.6 Hz, 2H), 7.20 (d, *J* = 10.7 Hz, 1H), 7.06 (d, *J* = 8.5 Hz, 1H), 6.97 (t, *J* = 8.4 Hz, 1H), 4.92 (s, 2H), 1.50 (s, 6H). HRMS (ESI) calcd. for C₂₀H₁₇F₄N₃O₅ [M+H]⁺ 456.1183, found 456.1182.

### Example 152

### Ethyl 2-(2-fluoro-4-((5-oxo-4-(4-(trifluoromethoxy)phenyl)-4,5-dihydro-1H-1,2,4-triazol-1-yl)methyl)phenoxy)-2-methylpropionate (compound 152)

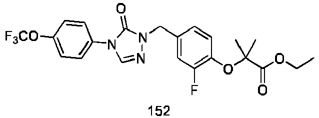

Compound 152 was prepared by replacing intermediate I-3 with intermediate D-3 and 4-hydroxy-3,5-dimethylbenzaldehyde with 3-fluoro-4-hydroxybenzaldehyde without hydrolysis according to the method of Example 55: ¹H NMR (300 MHz, CDCl₃) δ 7.71 (s, 1H), 7.64 (d, *J* = 8.9 Hz, 2H), 7.36 (d, *J* = 8.7 Hz, 2H), 7.16 (d, *J =* 11.2 Hz, 1H), 7.07 (d, *J* = 8.7 Hz, 1H), 6.97 (t, *J* = 8.3 Hz, 1H), 4.97 (s, 2H), 4.26 (q, *J* = 7.1 Hz, 2H), 1.59 (s, 6H), 1.30 (t, *J* = 7.0 Hz, 3H). MS (ESI): m/z 506.1 [M+Na]⁺.

### Example 153

### 2-Methyl-2-(4-((5-oxo-4-(4-(trifluoromethoxy)phenyl)-4, 5-dihydro-1H-1,2,4-triazol-1-yl)methyl)phenoxy)propionic acid (compound 153)

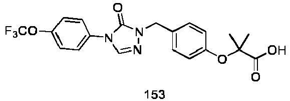

Compound 153 was prepared by replacing intermediate I-3 with intermediate D-3 and 4-hydroxy-3,5-dimethylbenzaldehyde with *p*-hydroxybenzaldehyde according to the method of Example 55: ¹H NMR (300 MHz, DMSO-*d₆*) δ 13.09 (s, 1H), 8.52 (s, 1H), 7.87 (d, *J* = 8.9 Hz, 2H), 7.55 (d, *J* = 8.4 Hz, 2H), 7.23 (d, *J* = 8.4 Hz, 2H), 6.80 (d, *J* = 8.5 Hz, 2H), 4.88 (s, 2H), 1.50 (s, 6H). HRMS (ESI) calcd. for C₂₀H₁₈F₃N₃O₅ [M+H]⁺ 438.1277, found 438.1267.

### Example 154

### Ethyl 2-methyl-2-(4-((5-oxo-4-(4-(trifluoromethoxy)phenyl)-4,5-dihydro-1H-1,2,4-triazol-1-yl)methyl)phenoxy)propionate (compound 154)

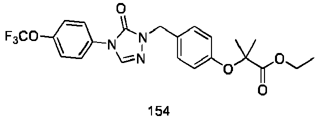

Compound 154 was prepared by replacing intermediate I-3 with intermediate D-3 and 4-hydroxy-3,5-dimethylbenzaldehyde with *p*-hydroxybenzaldehyde without hydrolysis according to the method of Example 55: ¹H NMR (300 MHz, CDCl₃) δ 7.69 (s, 1H), 7.64 (d, *J* = 9.0 Hz, 2H), 7.37 (s, 1H), 7.33 (d, *J* = 3.0 Hz, 2H), 7.30 (s, 1H), 6.83 (d, *J* = 8.6 Hz, 2H), 4.97 (s, 2H), 4.25 (q, *J* = 7.1 Hz, 2H), 1.60 (s, 6H), 1.28 (t, 3H). MS (ESI): m/z 488.1 [M+Na]⁺.

### Example 155

### 2-(2,6-Difluoro-4-((5-oxo-4-(4-(trifluoromethoxy)phenyl)-4,5-dihydro-1H-1,2,4-triazol-1-yl)methyl)phenoxy)-2-methylpropionic acid (compound 155)

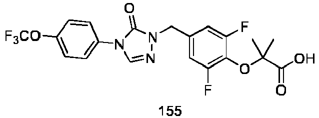

Compound 155 was prepared by replacing intermediate I-3 with intermediate D-3 and 4-hydroxy-3,5-dimethylbenzaldehyde with 3,5-fluoro-4-hydroxybenzaldehyde according to the method of Example 55: ¹H NMR (300 MHz, DMSO-*d₆*) δ 12.97 (s, 1H), 8.58 (s, 1H), 7.88 (d, *J* = 9.0 Hz, 2H), 7.56 (d, *J* = 8.5 Hz, 2H), 7.10 (d, *J* = 8.8 Hz, 2H), 4.97 (s, 2H), 1.44 (s, 6H). HRMS (ESI) calcd. for C₂₀H₁₆F₅N₃O₅ [M+H]⁺ 474.1088, found 474.1087.

### Example 156

### Ethyl 2-(2,6-difluoro-4-((5-oxo-4-(4-(trifluoromethoxy)phenyl)-4,5-dihydro-1H-1,2,4-triazol-1-yl)methyl)phenoxy)-2-methylpropionate (compound 156)

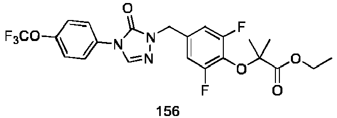

Compound 156 was prepared by replacing intermediate I-3 with intermediate D-3 and 4-hydroxy-3,5-dimethylbenzaldehyde with 3,5-fluoro-4-hydroxybenzaldehyde without hydrolysis according to the method of Example 55: ¹H NMR (300 MHz, CDCl₃) δ 7.74 (s, 1H), 7.65 (d, *J* = 8.9 Hz, 2H), 7.37 (d, *J* = 8.7 Hz, 2H), 6.96 (d, *J* = 8.1 Hz, 2H), 4.96 (s, 2H), 4.26 (q, *J* = 7.1 Hz, 2H), 1.57 (s, 6H), 1.33 (t, *J* = 7.1 Hz, 3H). MS (ESI): m/z 524.1 [M+Na]⁺.

### Example 157

### 2-Methyl-2-(4-((5-oxo-4-(4-(trifluoromethoxy)phenyl)-4, 5-dihydro-1H-1,2,4-triazol-1-yl)methyl)-2-(trifluoromethyl)phenoxy)propionic acid (compound 157)

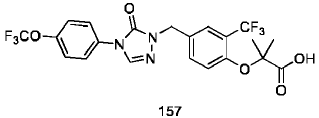

Compound 157 was prepared by replacing intermediate I-3 with intermediate D-3 and 4-hydroxy-3,5-dimethylbenzaldehyde with 4-hydroxy-3-trifluoromethylbenzaldehyde according to the method of Example 55: ¹H NMR (300 MHz, DMSO-*d₆*) δ 13.35 (s, 1H), 8.53 (s, 1H), 7.86 (d, *J* = 9.0 Hz, 2H), 7.62 (s, 1H), 7.55 (d, *J* = 8.7 Hz, 2H), 7.50 (s, 1H), 6.92 (d, *J* = 8.6 Hz, 1H), 4.97 (s, 2H), 1.54 (s, 6H). HRMS (ESI) calcd. for C₂₁H₁₇F₆N₃O₅ [M+H]⁺ 506.1151, found 506.1154.

### Example 158

### Ethyl 2-methyl-2-(4-((5-oxo-4-(4-(trifluoromethoxy)phenyl)-4,5-dihydro-1H-1,2,4-triazol-1-yl)methyl)-2-(trifluoromethyl)phenoxy)propionate (compound 158)

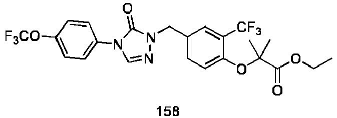

Compound 158 was prepared by replacing intermediate I-3 with intermediate D-3 and 4-hydroxy-3,5-dimethylbenzaldehyde with 4-hydroxy-3-trifluoromethylbenzaldehyde without hydrolysis according to the method of Example 55: ¹H NMR (300 MHz, CDCl₃) δ 7.71 (s, 1H), 7.64 (d, *J* = 8.7 Hz, 3H), 7.47 (d, *J* = 8.5 Hz, 1H), 7.36 (d, *J* = 8.5 Hz, 2H), 6.81 (d, *J* = 8.6 Hz, 1H), 4.99 (s, 2H), 4.26 (q, *J* = 7.1 Hz, 2H), 1.64 (s, 6H), 1.26 (t, 3H). MS (ESI): m/z 556.1 [M+Na]⁺.

### Example 159

### 2-(2-Chloro-6-fluoro-4-((5-oxo-4-(4-(trifluoromethoxy)phenyl)-4,5-dihydro-1H-1,2,4-triazol-1-yl)methyl)phenoxy)-2-methylpropionic acid (compound 159)

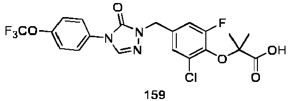

Compound 159 was prepared by replacing intermediate I-3 with intermediate D-3 and 4-hydroxy-3,5-dimethylbenzaldehyde with 3-chloro-5-fluoro-4-hydroxybenzaldehyde according to the method of Example 55: ¹H NMR (300 MHz, DMSO-*d₆*) δ 12.94 (s, 1H), 8.58 (s, 1H), 7.88 (d, *J* = 9.0 Hz, 2H), 7.57 (d, *J* = 8.6 Hz, 2H), 7.31 (s, 1H), 7.23 (d, *J* = 11.5 Hz, 1H), 4.97 (s, 2H), 1.46 (s, 6H). HRMS (ESI) calcd. for C₂₀H1₆ClF₄N₃O₅ [M+H]⁺ 490.0793, found 490.0795.

### Example 160

### Ethyl 2-(2-chloro-6-fluoro-4-((5-oxo-4-(4-(trifluoromethoxy)phenyl)-4,5-dihydro-1H-1,2,4-triazol-1-yl)methyl)phenoxy)-2-methylpropionate (compound 160)

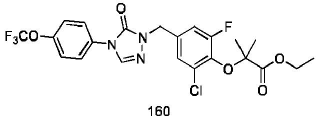

Compound 160 was prepared by replacing intermediate I-3 with intermediate D-3 and 4-hydroxy-3,5-dimethylbenzaldehyde with 3-chloro-5-fluoro-4-hydroxybenzaldehyde without hydrolysis according to the method of Example 55: ¹H NMR (300 MHz, CDCl₃) δ 7.74 (s, 1H), 7.64 (d, *J* = 9.0 Hz, 2H), 7.37 (d, *J* = 8.6 Hz, 2H), 7.24 (s, 1H), 7.06 (dd, *J* = 10.7, 1.9 Hz, 1H), 4.95 (s, 2H), 4.28 (q, *J* = 7.1 Hz, 2H), 1.58 (s, 6H), 1.33 (t, *J* = 7.1 Hz, 3H). MS (ESI): m/z 540.1 [M+Na]⁺.

### Example 161

### 2-(2,6-Dibromo-4-((5-oxo-4-(4-(trifluoromethoxy)phenyl)-4,5-dihydro-1H-1,2,4-triazol-1-yl)methyl)phenoxy)-2-methylpropionic acid (compound 161)

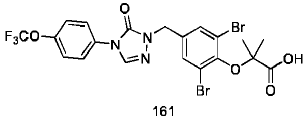

Compound 161 was prepared by replacing intermediate I-3 with intermediate D-3 and 4-hydroxy-3,5-dimethylbenzaldehyde with 3,5-dibromo-4-hydroxybenzaldehyde according to the method of Example 55: ¹H NMR (300 MHz, DMSO-*d₆*) δ 12.89 (s, 1H), 8.58 (s, 1H), 7.88 (d, *J* = 7.7 Hz, 2H), 7.63 (s, 2H), 7.57 (d, *J* = 8.3 Hz, 2H), 4.98 (s, 2H), 1.51 (s, 6H). HRMS (ESI) calcd. for C₂₀H₁₆Br₂F₃N₃O₅ [M+H]⁺ 593.9487, found 593.9483.

### Example 162

### Ethyl 2-(2,6-dibromo-4-((5-oxo-4-(4-(trifluoromethoxy)phenyl)-4,5-dihydro-1H-1,2,4-triazol-1-yl)methyl)phenoxy)-2-methylpropionate (compound 162)

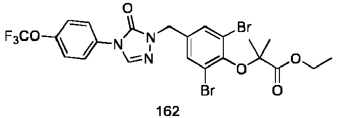

Compound 162 was prepared by replacing intermediate I-3 with intermediate D-3 and 4-hydroxy-3,5-dimethylbenzaldehyde with 3,5-dibromo-4-hydroxybenzaldehyde without hydrolysis according to the method of Example 55: ¹H NMR (300 MHz, CDCl₃) δ 7.74 (s, 1H), 7.65 (d, *J* = 8.8 Hz, 2H), 7.57 (s, 2H), 7.37 (d, *J* = 8.5 Hz, 2H), 4.94 (s, 2H), 4.31 (q, *J* = 7.0 Hz, 2H), 1.64 (s, 6), 1.38 (t, *J* = 7.1 Hz, 3H). MS (ESI): m/z 644.0 [M+Na]⁺.

### Example 163

### 2-Methyl-2-(2-methyl-4-((5-oxo-4-(4-(trifluoromethoxy)phenyl)-4, 5-dihydro-1H-1,2,4-triazol-1-yl)methyl)-6-(trifluoromethyl)phenoxy)propionic acid (compound 163)

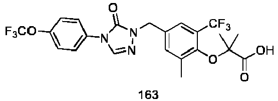

Compound 163 was prepared by replacing intermediate I-3 with intermediate D-3 and 4-hydroxy-3,5-dimethylbenzaldehyde with 4-hydroxy-3-methyl-5-(trifluoromethyl)benzaldehyde according to the method of Example 55: ¹H NMR (300 MHz, DMSO-*d₆*) δ 13.04 (s, 1H), 8.56 (s, 1H), 7.88 (d, *J* = 9.0 Hz, 2H), 7.56 (d, *J* = 8.5 Hz, 2H), 7.47 (d, *J* = 14.8 Hz, 2H), 4.98 (s, 2H), 2.22 (s, 3H), 1.37 (s, 6H). HRMS (ESI) calcd. for C₂₂H₁₉F₆N₃O₅ [M+H]⁺ 520.1307, found 520.1305.

### Example 164

### Ethyl 2-methyl-2-(2-methyl-4-((5-oxo-4-(4-(trifluoromethoxy)phenyl)-4,5-dihydro-1H-1,2,4-triazol-1-yl)methyl)-6-(trifluoromethyl)phenoxy)propionate (compound 164)

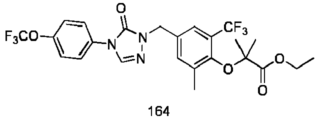

Compound 164 was prepared by replacing intermediate I-3 with intermediate D-3 and 4-hydroxy-3,5-dimethylbenzaldehyde with 4-hydroxy-3-methyl-5-(trifluoromethyl)benzaldehyde without hydrolysis according to the method of Example 55: ¹H NMR (300 MHz, CDCl₃) δ 7.73 (s, 1H), 7.65 (d, *J* = 9.0 Hz, 2H), 7.50 (s, 1H), 7.36 (d, *J* = 8.6 Hz, 3H), 4.99 (s, 2H), 4.31 (q, *J* = 7.1 Hz, 2H), 2.24 (s, 3H), 1.50 (s, 6H), 1.37 (t, *J* = 7.1 Hz, 3H). MS (ESI): m/z 570.1 [M+Na]⁺.

### Example 165

### 2-Methyl-2-(2-methyl-4-((5-oxo-4-(4-(trifluoromethyl)phenyl)-4,5-dihydro-1H-1,2,4-triazol-1-yl)methyl)-6-(trifluoromethyl)phenoxy)propionic acid (compound 165)

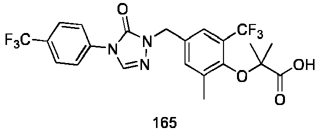

Compound 165 was prepared by replacing intermediate I-3 with intermediate D-1 and 4-hydroxy-3,5-dimethylbenzaldehyde with 4-hydroxy-3-methyl-5-(trifluoromethyl)benzaldehyde according to the method of Example 55: ¹H NMR (300 MHz, DMSO-*d₆*) δ 13.03 (s, 1H), 8.67 (s, 1H), 8.03 (d, *J* = 8.6 Hz, 2H), 7.92 (d, *J* = 8.7 Hz, 2H), 7.48 (d, *J* = 14.2 Hz, 2H), 5.00 (s, 2H), 2.22 (s, 3H), 1.37 (s, 6H). HRMS (ESI) calcd. for C₂₂H₁₉F₆N₃O₄ [M+H]⁺ 504.1358, found 504.1359.

### Example 166

### Ethyl 2-methyl-2-(2-methyl-4-((5-oxo-4-(4-(trifluoromethyl)phenyl)-4,5-dihydro-1H-1,2,4-triazol-1-yl)methyl)-6-(trifluoromethyl)phenoxy)propionate (compound 166)

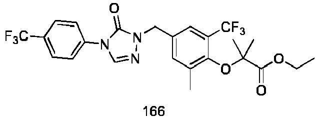

Compound 166 was prepared by replacing intermediate I-3 with intermediate D-1 and 4-hydroxy-3,5-dimethylbenzaldehyde with 4-hydroxy-3-methyl-5-(trifluoromethyl)benzaldehyde without hydrolysis according to the method of Example 55: ¹H NMR (300 MHz, CDCl₃) δ 7.80 (s, 1H), 7.78 (s, 3H), 7.50 (s, 1H), 7.40 (s, 1H), 7.28 (s, 1H), 5.00 (s, 2H), 4.31 (q, *J* = 7.1 Hz, 2H), 2.24 (s, 3H), 1.50 (s, 6H), 1.37 (t, *J* = 7.1 Hz, 3H). MS (ESI): m/z 554.2 [M+Na]⁺.

### Example 167

### 2-(2,6-Dimethyl-4-(1-(5-oxo-4-(4-(trifluoromethoxy)phenyl)-4,5-dihydro-1H-1,2,4-triazol-1-yl)ethyl)phenoxy)-2-methylpropionic acid (compound 167)

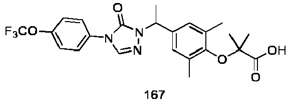

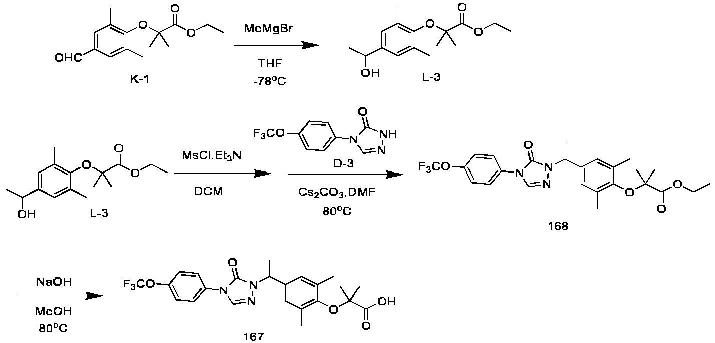

### Synthesis of intermediate L-3

Intermediate K-1 (13.2 g, 50 mmol) was dissolved in 100 mL of THF, and a solution of methylmagnesium bromide (11.4 mL, 100 mmol) in THF was slowly added at -78 °C under argon atmosphere. The mixture was reacted at -78 °C for 3 h. After the reaction was completed, 50 mL of a saturated ammonium chloride solution was added. The mixture was stirred at room temperature for 30 min. The reaction liquid was concentrated by rotary evaporation under reduced pressure to remove the solvent. The residue was diluted with water (100 mL) and extracted with ethyl acetate (100 mL × 3). The organic phases were combined, washed with saturated brine (200 mL × 1), dried over anhydrous sodium sulfate, and concentrated by rotary evaporation under reduced pressure to remove the solvent. The residue was purified by column chromatography (petroleum ether/ethyl acetate = 10: 1) to give intermediate L-3 (colorless liquid, 7.3 g).

### Synthesis of compound 168

Intermediate L-3 (361.4 mg, 1.3 mmol) was dissolved in 5 mL of anhydrous dichloromethane (DCM), and triethylamine (262.6 mg, 2.6 mmol) and methylsufonyl chloride (MsCl) (229.2 mg, 2 mmol) were added. The mixture was stirred at room temperature for 2 h. After the reaction was completed, the reaction liquid was concentrated by rotary evaporation under reduced pressure to remove the solvent. The residue was diluted with water (20 mL) and extracted with ethyl acetate (10 mL × 3). The organic phase was washed with saturated brine (20 mL × 1) and concentrated by rotary evaporation under reduced pressure to remove the solvent. The residue was dissolved in acetonitrile (10 mL), and intermediate D-3 (318.5 mg, 1.3 mmol) and cesium carbonate (1.07 g, 3.3 mmol) were added. The mixture was stirred at room temperature for 3 h. After the reaction was completed, the reaction liquid was concentrated by rotary evaporation under reduced pressure to remove the solvent. The residue was purified by column chromatography (petroleum ether/ethyl acetate = 10:1) to give compound 168 (colorless liquid, 519.8 mg).

### Synthesis of compound 167

Compound 168 (80 mg, 0.16 mmol) was dissolved in methanol (3 mL), and a 1 N NaOH solution (0.78 mL) was added. The reaction system was transferred to an oil bath and reacted at 80 °C for 4 h. After the reaction was completed, the reaction liquid was cooled to room temperature. A 1 N HCl solution was added to adjust pH to 4, and the resulting mixture was concentrated by rotary evaporation under reduced pressure to remove the solvent. The residue was diluted with water (10 mL) and extracted with ethyl acetate (10 mL × 3). The organic phase was washed with saturated brine (10 mL × 1) and concentrated by rotary evaporation under reduced pressure to remove the solvent. The residue was purified by column chromatography (dichloromethane/methanol = 100:1) to give compound 167 (white solid, 33.7 mg): ¹H NMR (300 MHz, DMSO-*d₆*) δ 12.83 (s, 1H), 8.54 (s, 1H), 7.87 (d, *J* = 8.9 Hz, 2H), 7.54 (d, *J* = 8.8 Hz, 2H), 7.01 (s, 2H), 5.33 (q, *J* = 6.9 Hz, 1H), 2.16 (s, 6H), 1.66 (d, *J* = 7.0 Hz, 3H), 1.34 (s, 6H). HRMS (ESI) calcd. for C₂₃H₂₄F₃N₃O₅ [M+H]⁺ 480.1746, found 480.1746.

### Example 168

### Ethyl 2-(2, 6-dimethyl-4-(1-(5-oxo-4-(4-(trifluoromethoxy)phenyl)-4, 5-dihydro-1H-1,2,4-triazol-1-yl)ethyl)phenoxy)-2-methylpropionate (compound 168)

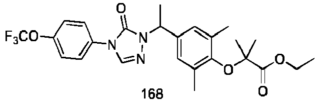

Compound 168 was prepared without hydrolysis according to the method of Example 167: ¹H NMR (300 MHz, CDCl₃) δ 7.71 (s, 1H), 7.64 (d, *J* = 9.0 Hz, 2H), 7.34 (d, *J* = 8.4 Hz, 2H), 7.06 (s, 2H), 5.47 (q, *J* = 7.1 Hz, 1H), 4.29 (q, *J* = 7.1 Hz, 2H), 2.21 (s, 6H), 1.78 (d, *J* = 7.1 Hz, 3H), 1.47 (s, 6H), 1.36 (t, *J* = 7.1 Hz, 3H). MS (ESI): m/z 530.2 [M+Na]⁺.

### Example 169

### 2-(2,6-Dimethyl-4-(1-(5-oxo-4-(4-(trifluoromethyl)phenyl)-4,5-dihydro-1H-1,2,4-triazol-1-yl)ethyl)phenoxy)-2-methylpropionic acid (compound 169)

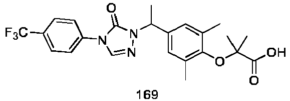

Compound 169 was prepared by replacing intermediate D-3 in Example 167 with D-1 according to the method of Example 167: ¹H NMR (300 MHz, DMSO-*d₆*) δ 12.83 (s, 1H), 8.65 (s, 1H), 8.03 (d, *J* = 8.4 Hz, 2H), 7.91 (d, *J* = 8.5 Hz, 2H), 7.02 (s, 2H), 5.35 (q, *J* = 6.9 Hz, 1H), 2.16 (s, 6H), 1.67 (d, *J* = 7.0 Hz, 3H), 1.35 (s, 6H). HRMS (ESI) calcd. for C₂₃H₂₄F₃N₃O₄ [M+H]⁺ 464.1797, found 464.1797.

### Example 170

### Ethyl 2-(2,6-dimethyl-4-(1-(5-oxo-4-(4-(trifluoromethyl)phenyl)-4, 5-dihydro-1H-1,2,4-triazol-1-yl)ethyl)phenoxy)-2-methylpropionate (compound 170)

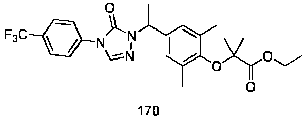

Compound 170 was prepared by replacing D-3 in Example 167 with D-1 without hydrolysis according to the method of Example 167: ¹H NMR (300 MHz, CDCl₃) δ 7.79 (d, *J* = 8.8 Hz, 2H), 7.75 (d, *J* = 9.2 Hz, 2H), 7.28 (s, 1H), 7.07 (s, 2H), 5.47 (q, *J* = 7.1 Hz, 1H), 4.29 (q, *J* = 7.1 Hz, 2H), 2.21 (s, 6H), 1.79 (d, *J* = 7.1 Hz, 3H), 1.48 (d, *J* = 3.3 Hz, 6H), 1.36 (t, *J* = 7.1 Hz, 3H). MS (ESI): m/z 514.2 [M+Na]⁺.

### Example 171

### 2-(2,6-Dimethyl-4-(1-(5-oxo-4-(4-(trifluoromethoxy)phenyl)-4,5-dihydro-1H-1,2,4-triazol-1-yl)propyl)phenoxy)-2-methylpropionic acid (compound 171)

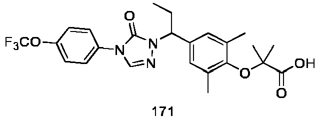

Compound 171 was prepared by replacing methylmagnesium bromide in Example 167 with ethylmagnesium bromide according to the method of Example 167: ¹H NMR (300 MHz, DMSO-*d₆*) δ 12.83 (s, 1H), 8.56 (s, 1H), 7.88 (d, *J* = 9.0 Hz, 2H), 7.54 (d, *J* = 8.7 Hz, 2H), 7.05 (s, 2H), 5.03 (q, *J* = 9.7, 5.8 Hz, 1H), 2.16 (s, 6H), 2.12 - 1.91 (m, 2H), 1.35 (s, 6H), 0.83 (t, *J* = 7.2 Hz, 3H). HRMS (ESI) calcd. for C₂₄H₂₆F₃N₃O₅ [M+H]⁺ 494.1903, found 494.1902.

### Example 172

### Ethyl 2-(2, 6-dimethyl-4-(1-(5-oxo-4-(4-(trifluoromethoxy)phenyl)-4, 5-dihydro-1H-1,2,4-triazol-1-yl)propyl)phenoxy)-2-methylpropionate (compound 172)

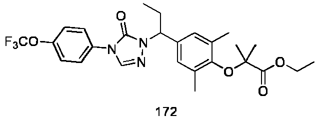

Compound 172 was prepared by replacing methylmagnesium bromide in Example 167 with ethylmagnesium bromide without hydrolysis according to the method of Example 167: ¹H NMR (300 MHz, CDCl₃) δ 7.72 (s, 1H), 7.65 (d, *J* = 9.0 Hz, 2H), 7.34 (d, *J* = 8.4 Hz, 2H), 7.08 (s, 2H), 5.13 (t, 1H), 4.29 (q, *J* = 7.1 Hz, 2H), 2.21 (s, 6H), 2.17 - 2.02 (m, 2H), 1.47 (s, 6H), 1.36 (t, *J* = 7.1 Hz, 3H), 0.94 (t, *J* = 7.3 Hz, 3H). MS (ESI): m/z 544.2 [M+Na]⁺.

### Example 173

### 2-(2,6-Dimethyl-4-(1-(5-oxo-4-(4-(trifluoromethyl)phenyl)-4,5-dihydro-1H-1,2,4-triazol-1-yl)propyl)phenoxy)-2-methylpropionic acid (compound 173)

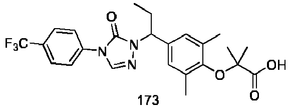

Compound 173 was prepared by replacing D-3 in Example 167 with D-1 and methylmagnesium bromide with ethylmagnesium bromide according to the method of Example 167: ¹H NMR (300 MHz, DMSO-*d₆*) δ 12.83 (s, 1H), 8.66 (s, 1H), 8.03 (d, *J* = 8.5 Hz, 2H), 7.90 (d, *J* = 8.7 Hz, 2H), 7.05 (s, 2H), 5.04 (q, *J* = 9.5, 5.8 Hz, 1H), 2.16 (s, 6H), 2.14 - 1.88 (m, 2H), 1.35 (s, 6H), 0.84 (t, *J* = 7.2 Hz, 3H). HRMS (ESI) calcd. for C₂₄H₂₆F₃N₃O₄ [M+H]⁺ 478.1954, found 478.1957.

### Example 174

### Ethyl 2-(2,6-dimethyl-4-(1-(5-oxo-4-(4-(trifluoromethyl)phenyl)-4,5-dihydro-1H-1,2,4-triazol-1-yl)propyl)phenoxy)-2-methylpropionate (compound 174)

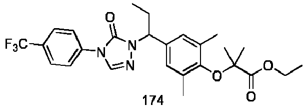

Compound 174 was prepared by replacing D-3 in Example 167 with D-1 and methylmagnesium bromide with ethylmagnesium bromide without hydrolysis according to the method of Example 167: ¹H NMR (300 MHz, CDCl₃) δ 7.80 (d, *J* = 5.2 Hz, 2H), 7.78 (s, 1H), 7.75 (d, *J* = 9.0 Hz, 2H), 7.08 (s, 2H), 5.13 (t, 1H), 4.29 (q, *J* = 7.1 Hz, 2H), 2.21 (s, 6H), 2.18 - 1.92 (m, 2H), 1.47 (s, 6H), 1.36 (t, *J* = 7.1 Hz, 3H), 0.94 (t, *J* = 7.3 Hz, 3H). MS (ESI): m/z 528.2 [M+Na]⁺.

### Example 175

### 2-(2,6-Dimethyl-4-((3-methyl-5-oxo-4-(4-(trifluoromethyl)phenyl)-4,5-dihydro-1H-1,2,4-triazol-1-yl)methyl)phenoxy)-2-methylpropionic acid (compound 175)

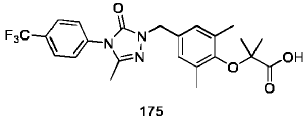

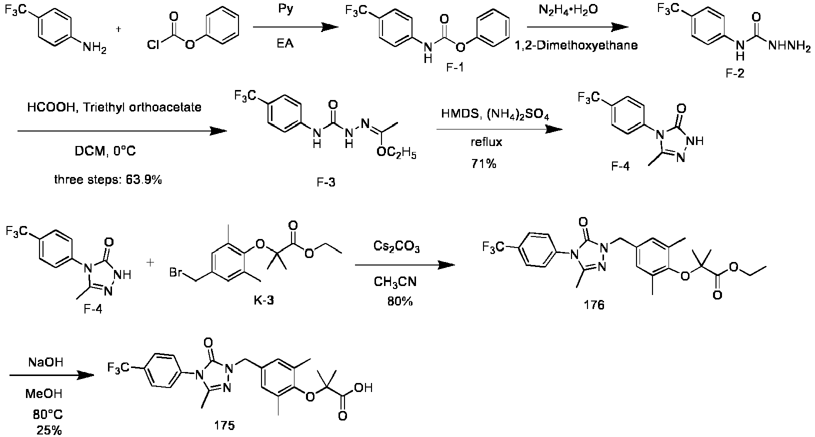

### Synthesis of intermediate F-1

p-Trifluoromethylaniline (1.24 mL, 10 mmol) was dissolved in ethyl acetate (EA) (25 mL), and pyridine (Py) (0.885 mL, 11 mmol) was added. Phenyl chloroformate (1.38 mL, 11 mmol) was slowly added under an ice bath. The mixture was stirred at room temperature and reacted for 4 h. After the reaction was completed, the reaction liquid was washed with water (50 mL × 3). The organic phase was washed with saturated brine (20 mL × 1), and concentrated by rotary evaporation under reduced pressure to remove the solvent to give a crude product of intermediate F-1 (brown solid, 3.116 g).

### Synthesis of intermediate F-2

Intermediate F-1 (1.687 g, 6 mmol) was dissolved in glycol dimethyl ether (20 mL), and 98% hydrazine hydrate (1 mL) was added. The mixture was stirred at room temperature for 12 h. After the reaction was completed, the reaction liquid was concentrated by rotary evaporation under reduced pressure to remove the solvent to give a crude product of intermediate F-2 (yellow solid, 1.12 g).

### Synthesis of intermediate F-3

Intermediate F-2 (438 mg, 2 mmol) was dissolved in DCM (20 mL), and triethyl orthoacetate (5 mL, 27 mmol) and formic acid (20 µL, 0.52 mmol) were added under an ice bath. The mixture was slowly warmed to room temperature and reacted for 12 h. After the reaction was completed, the reaction liquid was concentrated by rotary evaporation under reduced pressure to remove the solvent to 5 mL. Petroleum ether (20 mL) was added to the residue, and the mixture was filtered under reduced pressure to give intermediate F-3 (white solid, 370 mg).

### Synthesis of intermediate F-4

Intermediate F-3 (289 mg, 1 mmol) was added to hexamethyldisilazane (12 mL), and ammonium sulfate (6 mg, 0.045 mmol) was added. The mixture was purged with argon three times. The system was transferred to an oil bath and reacted at 130 °C for 16 h. After the reaction was completed, the reaction liquid was cooled to room temperature and concentrated by rotary evaporation under reduced pressure to remove the solvent, and toluene (8 mL × 2) was added. The mixture was concentrated by rotary evaporation under reduced pressure to remove the solvent. The residue was diluted with water (20 mL) and extracted with ethyl acetate (10 mL × 3). The organic phase was washed with saturated brine (20 mL × 1) and concentrated by rotary evaporation under reduced pressure to remove the solvent to give intermediate F-4 (white solid, 172 mg).

### Synthesis of compound 176

Intermediate F-4 (97 mg, 0.4 mmol) was dissolved in acetonitrile (5 mL), and compound K-3 (170 mg, 0.52 mmol) and cesium carbonate (326 mg, 1 mmol) were added. The mixture was stirred at room temperature for 4 h. After the reaction was completed, the reaction liquid was concentrated by rotary evaporation under reduced pressure to remove the solvent. The residue was purified by column chromatography (petroleum ether/ethyl acetate = 2:1) to give compound 176 (colorless liquid, 160 mg).

### Synthesis of compound 175

Compound 176 (150 mg, 0.305 mmol) was dissolved in methanol (3 mL), and a 1 NNaOH solution (1.5 mL) was added. The reaction system was transferred to an oil bath and reacted at 80 °C for 4 h. After the reaction was completed, the reaction liquid was cooled to room temperature. A 1 N HCl solution was added to adjust pH to 4, and the resulting mixture was concentrated by rotary evaporation under reduced pressure to remove the solvent. The residue was diluted with water (10 mL) and extracted with ethyl acetate (10 mL × 3). The organic phase was washed with saturated brine (10 mL × 1) and concentrated by rotary evaporation under reduced pressure to remove the solvent. The residue was purified by column chromatography (dichloromethane/methanol = 80:1) to give compound 131 (white solid, 37 mg): ¹H NMR (300 MHz, DMSO-*d₆*) δ 12.84 (s, 1H), 7.93 (d, *J* = 8.4 Hz, 2H), 7.75 (d, *J* = 8.3 Hz, 2H), 6.98 (s, 2H), 4.79 (s, 2H), 2.17 (s, 6H), 2.15 (s, 3H), 1.36 (s, 6H). HRMS (ESI) calcd. for C₁₉H₁₉N₃O₄S [M+H]⁺ 464.1792, found 464.1799.

### Example 176

### Ethyl 2-(2,6-dimethyl-4-((3-methyl-5-oxo-4-(4-(trifluoromethyl)phenyl)-4,5-dihydro-1H-1,2,4-triazol-1-yl)methyl)phenoxy)-2-methylpropionate (compound 176)

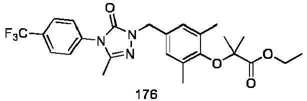

Compound 176 was prepared without hydrolysis according to the method of Example 175: ¹H NMR (300 MHz, DMSO-*d₆*) δ 7.93 (d, *J* = 8.5 Hz, 2H), 7.75 (d, *J* = 8.3 Hz, 2H), 6.98 (s, 2H), 4.79 (s, 2H), 4.18 (q, *J=* 7.1 Hz, 2H), 2.15 (s, 3H), 2.13 (s, 6H), 1.38 (s, 6H), 1.25 (t, *J* = 7.1 Hz, 3H). MS (ESI): m/z 514.3 [M+Na]⁺.

### Example 177

### 2-Methyl-2-(2-methyl-4-((3-methyl-5-oxo-4-(4-(trifluoromethyl)phenyl)-4,5-dihydro-1H-1,2,4-triazol-1-yl)methyl)phenoxy)propionic acid (compound 177)

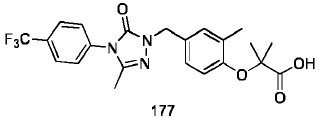

### Synthesis of intermediate G-1

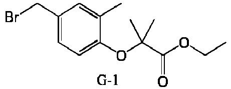

Intermediate G-1 was prepared by replacing 4-hydroxy-3,5-dimethylbenzaldehyde with 4-hydroxy-3-methylbenzaldehyde according to the method for intermediate K-3 of Example 55.

### Synthesis of compound 177

Compound 177 was prepared by replacing K-3 in Example 175 with G-1 according to the method of Example 175: ¹H NMR (300 MHz, DMSO-*d₆*) δ 13.04 (s, 1H), 7.93 (d, *J* = 8.4 Hz, 2H), 7.74 (d, *J* = 8.3 Hz, 2H), 7.14 (s, 1H), 7.06 (d, *J* = 8.3 Hz, 1H), 6.66 (d, *J* = 8.3 Hz, 1H), 4.79 (s, 2H), 2.15 (s, 3H), 2.13 (s, 3H), 1.51 (s, 6H). HRMS (ESI) calcd. for C₂₂H₂₂F₃N₃O₄ [M+H]⁺ 450.1635, found 450.1641.

### Example 178

### Ethyl 2-methyl-2-(2-methyl-4-((3-methyl-5-oxo-4-(4-(trifluoromethyl)phenyl)-4,5-dihydro-1H-1,2,4-triazol-1-yl)methyl)phenoxy)propionate (compound 178)

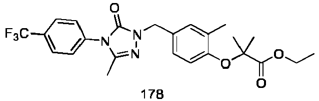

Compound 178 was prepared by replacing K-3 in Example 175 with G-5 without hydrolysis according to the method of Example 175: ¹H NMR (300 MHz, DMSO-*d₆*) δ 7.93 (d, *J* = 8.4 Hz, 2H), 7.74 (d, *J* = 8.3 Hz, 2H), 7.16 (s, 1H), 7.06 (d, *J* = 8.2 Hz, 1H), 6.59 (d, *J* = 8.3 Hz, 1H), 4.80 (s, 2H), 4.18 (q, *J* = 7.1 Hz, 2H), 2.16 (s, 3H), 2.14 (s, 3H), 1.53 (s, 6H), 1.20 (dd, *J* = 16.1, 9.1 Hz, 3H). MS (ESI): m/z 500.3 [M+Na]⁺

### Example 179

### 2-(4-(((4-([1,1'-Biphenyl]-4-yl)-5-oxo-4,5-dihydro-1H-1,2,4-triazol-1-yl)methyl)thio)-2-methylphenoxy)acetic acid (compound 179)

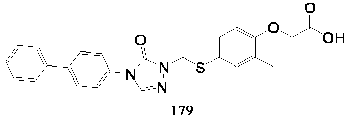

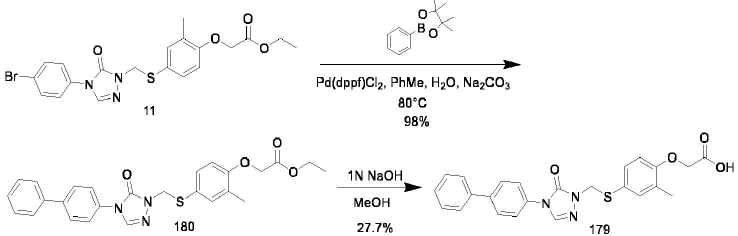

### Synthesis of compound 180

Compound 11 (102 mg, 0.2 mmol) prepared in Example 11, pinacol phenylboronate (70.5 mg, 0.3 mmol), [1,1'-bis(diphenylphosphino)ferrocene]dichloropalladium(II) (Pd(dppf)Cl₂) (43.9 mg, 0.06 mmol), and sodium carbonate (Na₂CO₃) (32 mg, 0.3 mmol) were added to a reaction flask and purged with argon three times, and toluene (PhMe) (4 mL) and water (0.4 mL) were added. The reaction system was transferred to an oil bath and reacted at 80 °C for 12 h. After the reaction was completed, the reaction liquid was cooled to room temperature and concentrated by rotary evaporation under reduced pressure to remove the solvent. The residue was purified by column chromatography (petroleum ether/ethyl acetate = 1:1) to give compound 180 (white solid, 93.1 mg).

### Synthesis of compound 179

Compound 180 (93.1 mg, 0.2 mmol) was dissolved in methanol (4 mL), and a 1 N NaOH solution (98 mg, 0.2 mmol) was added. The mixture was stirred at room temperature for 24 h. After the reaction was completed, the reaction liquid was adjusted to pH 4 with a 1 N HCl solution, and concentrated by rotary evaporation under reduced pressure to remove the solvent. The residue was diluted with water (15 mL) and extracted with ethyl acetate (10 mL × 3). The organic phase was washed with saturated brine (15 mL × 1) and concentrated by rotary evaporation under reduced pressure to remove the solvent. The residue was purified by column chromatography (dichloromethane/methanol = 100:1) to give compound 179 (white solid, 24.8 mg): ¹H NMR (300 MHz, DMSO-*d₆*) δ 7.68 - 7.56 (m, 4H), 7.45 (t, *J* = 7.5 Hz, 2H), 7.40 - 7.31 (m, 3H), 6.90 (s, 2H), 3.50 (s, 2H), 3.34 (s, 2H), 2.47 - 2.27 (m, 8H), 2.14 (s, 6H), 1.33 (s, 6H). MS(ESI): m/z 473.3 [M+H]⁺.

### Example 180

### Ethyl 2-(4-(((4-([[1,1'-biphenyl]-4-yl]-5-oxo-4,5-dihydro-1H-1,2,4-triazol-1-yl)methyl)thio)-2-methylphenoxy)acetate (compound 180)

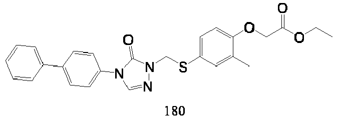

Compound 180 was prepared without hydrolysis according to the method of Example 179: ¹H NMR (300 MHz, CDCl₃) δ 7.76 (s, 1H), 7.71 (d, *J* = 8.5 Hz, 2H), 7.59 (t, *J* = 8.2 Hz, 4H), 7.48 (t, *J* = 7.4 Hz, 2H), 7.41 (d, *J* = 7.2 Hz, 1H), 7.35 (d, *J* = 6.5 Hz, 2H), 6.66 (d, *J* = 9.1 Hz, 1H), 5.19 (s, 2H), 4.63 (s, 2H), 4.27 (q, *J* = 7.2 Hz, 2H), 2.27 (s, 3H), 1.30 (t, *J* = 7.1 Hz, 3H). MS (ESI): m/z 498.1 [M+Na]⁺.

### Example 181

### 2-(2-Methyl-4-((((5-oxo-4-(4'-(trifluoromethyl)-[1,1'-biphenyl]-4-yl]-4,5-dihydro-1H-1,2,4-triazol-1-yl)methyl)thio)phenoxy)acetic acid (compound 181)

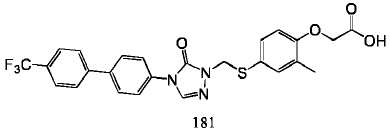

Compound 181 was prepared by replacing pinacol phenylboronate with pinacol *p-*trifluoromethylphenylboronate according to the method of Example 179: ¹H NMR (300 MHz, DMSO-*d₆*) δ 12.97 (s, 1H), 8.59 (s, 1H), 7.93 (dd, *J* = 13.9, 8.4 Hz, 4H), 7.81 (dd, *J* = 15.5, 8.5 Hz, 4H), 7.27 (d, *J* = 6.4 Hz, 2H), 6.80 (d, *J* = 9.2 Hz, 1H), 5.18 (s, 2H), 4.69 (s, 2H), 2.14 (s, 3H). MS (ESI): m/z 538.1 [M+Na]⁺.

### Example 182

### Ethyl 2-(2-methyl-4-(((5-oxo-4-(4'-(trifluoromethyl)-[1,1'-biphenyl]-4-yl)-4,5-dihydro-1H-1,2,4-triazol-1-yl)methyl)thio)phenoxy)acetate (compound 182)

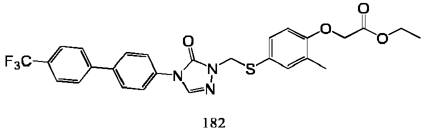

Compound 182 was prepared by replacing pinacol phenylboronate with pinacol *p-*trifluoromethylphenylboronate without hydrolysis according to the method of Example 179: ¹H NMR (300 MHz, CDCl₃) δ 7.77 (s, 1H), 7.74 (d, *J* = 8.6 Hz, 3H), 7.72 - 7.68 (m, 3H), 7.63 (d, *J* = 8.7 Hz, 2H), 7.35 (d, *J* = 6.8 Hz, 2H), 6.66 (d, *J* = 9.1 Hz, 1H), 5.18 (s, 2H), 4.63 (s, 2H), 4.27 (q, *J* = 7.1 Hz, 2H), 2.27 (s, 3H), 1.30 (t, *J* = 7.1 Hz, 3H). MS (ESI): m/z 566.1 [M+Na]⁺.

### Example 183

### 2-(2-Methyl-4-((((5-oxo-4-(4'-(trifluoromethoxy)-[1,1'-biphenyl]-4-yl]-4,5-dihydro-1H-1,2,4-triazol-1-yl)methyl)thio)phenoxy)acetic acid (compound 183)

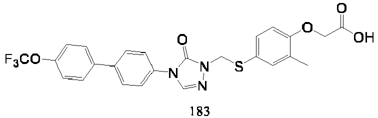

Compound 183 was prepared by replacing pinacol phenylboronate with pinacol *p-*trifluoromethoxyphenylboronate according to the method of Example 179: ¹H NMR (300 MHz, DMSO-*d₆*) δ 13.07 (s, 1H), 8.57 (s, 1H), 7.84 (d, *J* = 8.8 Hz, 4H), 7.75 (d, *J* = 8.7 Hz, 2H), 7.48 (d, *J* = 8.1 Hz, 2H), 7.27 (d, *J* = 6.5 Hz, 2H), 6.80 (d, *J* = 9.2 Hz, 1H), 5.17 (s, 2H), 4.69 (s, 2H), 2.14 (s, 3H). MS (ESI): m/z 554.1 [M+Na]⁺.

### Example 184

### Ethyl 2-(2-methyl-4-((((5-oxo-4-(4'-(trifluoromethoxy)-[1,1'-biphenyl]-4-yl]-4,5-dihydro-1H-1,2,4-triazol-1-yl)methyl)thio)phenoxy)acetate (compound 184)

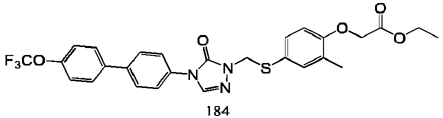

Compound 184 was prepared by replacing pinacol phenylboronate with pinacol *p-*trifluoromethoxyphenylboronate without hydrolysis according to the method of Example 179: ¹H NMR (300 MHz, CDCl₃) δ 7.76 (s, 1H), 7.67 (d, *J* = 8.6 Hz, 2H), 7.60 (m, 4H), 7.34 (t, *J* = 7.2 Hz, 4H), 6.66 (d, *J* = 9.1 Hz, 1H), 5.18 (s, 2H), 4.63 (s, 2H), 4.26 (q, *J* = 7.1 Hz, 2H), 2.27 (s, 3H), 1.30 (t, *J* = 7.1 Hz, 3H). MS (ESI): m/z 582.1 [M+Na]⁺.

### Example 185

### 2-(2,6-Dimethyl-4-((5-oxo-4-(4-(trifluoromethoxy)phenyl)-4,5-dihydro-1H-1,2,4-triazol-1-yl)methyl-d₂)phenoxy)-2-methylpropionic acid (compound 185)

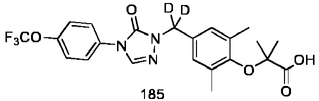

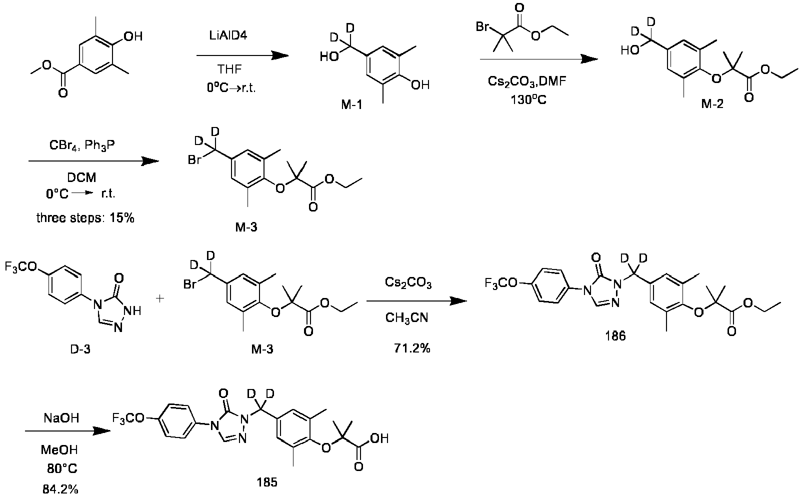

### Synthesis of intermediate M-1

Methyl 4-hydroxy-3,5-dimethylbenzoate (180 mg, 1 mmol) was dissolved in anhydrous tetrahydrofuran (5 mL), and deuterated lithium aluminum hydride (76 mg, 2 mmol) was slowly added under an ice bath. After the addition, the reaction system was slowly warmed to room temperature and stirred overnight. The reaction liquid was concentrated by rotary evaporation under reduced pressure to remove the solvent. The residue was diluted with water (20 mL) and extracted with ethyl acetate (20 mL × 3). The organic phases were combined, washed with saturated brine (20 mL × 1), dried over anhydrous sodium sulfate, and concentrated by rotary evaporation under reduced pressure to remove the solvent to give a crude product of intermediate M-1, which was directly used in the next step without further purification.

### Synthesis of intermediate M-2

The crude product of M-1 obtained from the previous step was dissolved in acetonitrile (10 mL), and ethyl bromoisobutyrate (433 µL, 3 mmol), cesium carbonate (326 mg, 1 mmol), potassium carbonate (276 mg, 2 mmol), and potassium iodide (12 mg, 0.07 mmol) were added. The system was transferred to an oil bath and reacted at 80 °C for 36 h. After the reaction was completed, the reaction liquid was cooled to room temperature and filtered under reduced pressure. The filtrate was concentrated by rotary evaporation under reduced pressure to remove the solvent. The residue was diluted with water (20 mL) and extracted with ethyl acetate (20 mL × 3). The organic phases were combined, washed with 1 N sodium hydroxide (20 mL × 3) and saturated brine (20 mL × 1), dried over anhydrous sodium sulfate, and concentrated by rotary evaporation under reduced pressure to remove the solvent. The residue was purified by column chromatography (petroleum ether/ethyl acetate = 200:1) to give intermediate M-2 (yellow liquid, 150 mg).

### Synthesis of intermediate M-3

M-2 (150 mg, 0.56 mmol) was dissolved in DCM (5 mL), carbon tetrabromide (278 mg, 0.84 mmol) was added, and triphenylphosphine (205 mg, 0.78 mmol) was slowly added under an ice bath. After the addition, the reaction system was slowly warmed to room temperature and reacted for 8 h. The reaction liquid was concentrated by rotary evaporation under reduced pressure to remove the solvent. The residue was purified by column chromatography (petroleum ether/ethyl acetate = 20: 1) to give compound M-3 (yellow liquid, 148 mg).

### Synthesis of compound 186

Intermediate D-3 (73.5 mg, 0.3 mmol) was dissolved in acetonitrile (3 mL), and M-3 (148.5 mg, 0.45 mmol) and cesium carbonate (244.5 mg, 0.75 mmol) were added. The mixture was stirred at room temperature for 4 h. After the reaction was completed, the reaction liquid was concentrated by rotary evaporation under reduced pressure to remove the solvent. The residue was purified by column chromatography (petroleum ether/ethyl acetate = 10: 1) to give compound 186 (white solid, 105.7 mg).

### Synthesis of compound 185

Compound 186 (15 mg, 0.03 mmol) was dissolved in methanol (2 mL), and a 1 N NaOH solution (0.15 mL) was added. The mixture was stirred at room temperature for 24 h. After the reaction was completed, the reaction liquid was adjusted to pH 4 with a 1 N HCl solution, and concentrated by rotary evaporation under reduced pressure to remove the solvent. The residue was diluted with water (5 mL) and extracted with ethyl acetate (10 mL × 3). The organic phase was washed with saturated brine (10 mL × 1), and concentrated by rotary evaporation under reduced pressure to remove the solvent. The residue was purified by column chromatography (dichloromethane/methanol = 100:1) to give compound 185 (white solid, 11.8 mg): ¹H NMR (300 MHz, DMSO-*d₆*) δ 12.86 (s, 1H), 8.43 (s, 1H), 7.60 (d, *J =* 8.2 Hz, 2H), 7.39 (d, *J* = 8.3 Hz, 2H), 6.95 (s, 2H), 4.83 (s, 2H), 3.09 - 2.84 (m, 1H), 2.15 (s, 6H), 1.35 (s, 6H), 1.22 (d, *J* = 6.9 Hz, 6H). HRMS (ESI) calcd. for C₂₂H₂₀D₂F₃N₃O₅ [M+H]⁺ 468.1715, found 468.1715.

### Example 186

### Ethyl 2-(2,6-dimethyl-4-((5-oxo-4-(4-(trifluoromethoxy)phenyl)-4,5-dihydro-177-1,2,4-triazol-1-yl)methyl-d₂)phenoxy)-2-methylpropionate (compound 186)

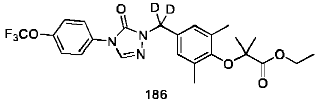

Compound 186 was prepared without hydrolysis according to the method of Example 185: ¹H NMR (300 MHz, CDCl₃) δ 7.71 (s, 1H), 7.65 (d, *J* = 8.9 Hz, 2H), 7.35 (d, *J* = 8.7 Hz, 2H), 7.03 (s, 2H), 4.30 (q, *J* = 7.1 Hz, 2H), 2.21 (s, 6H), 1.47 (s, 6H), 1.36 (t, *J* = 7.1 Hz, 3H). MS (ESI): m/z 518.0 [M+Na]⁺.

### Example 187

### 2-(2,6-Dimethyl-4-((5-oxo-4-(4-(trifluoromethyl)phenyl)-4,5-dihydro-1H-1,2,4-triazol-1-yl)methyl-d₂)phenoxy)-2-methylpropionic acid (compound 187)

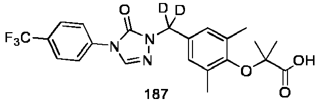

Compound 187 was prepared by replacing intermediate D-3 with intermediate D-1 according to the method of Example 185: ¹H NMR (300 MHz, DMSO) δ 12.85 (s, 1H), 8.64 (s, 1H), 8.04 (d, *J* = 8.5 Hz, 2H), 7.92 (d, *J* = 8.5 Hz, 2H), 6.97 (s, 2H), 2.16 (s, 6H), 1.35 (s, 6H). HRMS (ESI) calcd. for C₂₂H₂₀D₂F₃N₃O₄ [M+H]⁺ 451.1688, found 451.1688.

### Example 188

### Ethyl 2-(2, 6-dimethyl-4-((5-oxo-4-(4-(trifluoromethyl)phenyl)-4, 5-dihydro-1H-1,2,4-triazol-1-yl)methyl-d₂)phenoxy)-2-methylpropionate (compound 188)

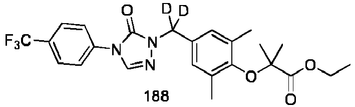

Compound 188 was prepared without hydrolysis according to the method of Example 187: ¹H NMR (300 MHz, CDCl₃) δ 7.87 - 7.68 (m, 5H), 7.04 (s, 2H), 4.30 (q, *J* = 7.1 Hz, 2H), 2.21 (s, 6H), 1.47 (s, 6H), 1.36 (t, *J* = 7.1 Hz, 3H). MS (ESI): m/z 502.2 [M+Na]⁺.

### Example 189

### 2-(2,6-Diethyl-4-((5-oxo-4-(4-(trifluoromethoxy)phenyl)-4,5-dihydro-1H-1,2,4-triazol-1-yl)methyl)phenoxy)-2-methylpropionic acid (compound 189)

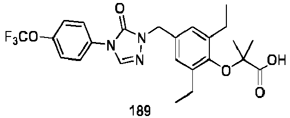

Compound 189 was prepared by replacing intermediate I-3 with intermediate D-3 and 4-hydroxy-3,5-dimethylbenzaldehyde with 4-hydroxy-3,5-diethylbenzaldehyde according to the method of Example 55: ¹H NMR (300 MHz, DMSO-*d₆*) δ 12.85 (s, 1H), 8.54 (s, 1H), 7.88 (d, *J* = 8.9 Hz, 2H), 7.56 (d, *J* = 8.6 Hz, 2H), 7.00 (s, 2H), 4.88 (s, 2H), 2.57 (q, 4H), 1.35 (s, 6H), 1.12 (t, *J* = 7.4 Hz, 6H). HRMS (ESI) calcd. for C₂₄H₂₆F₃N₃O₅ [M+H]⁺ 494.1903, found 494.1903.

### Example 190

### Ethyl 2-(2,6-diethyl-4-((5-oxo-4-(4-(trifluoromethoxy)phenyl)-4,5-dihydro-1H-1,2,4-triazol-1-yl)methyl)phenoxy)-2-methylpropropionate (compound 190)

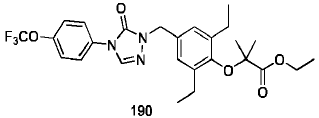

Compound 190 was prepared without hydrolysis according to the method of Example 189: ¹H NMR (300 MHz, CDCl₃) δ 7.71 (s, 1H), 7.66 (d, *J* = 9.0 Hz, 2H), 7.36 (d, *J* = 8.5 Hz, 2H), 7.08 (s, 2H), 4.96 (s, 2H), 4.30 (q, *J* = 7.1 Hz, 2H), 2.59 (q, *J* = 7.5 Hz, 4H), 1.47 (s, 6H), 1.37 (t, *J* = 7.1 Hz, 3H), 1.20 (t, *J* = 7.5 Hz, 6H). MS (ESI): m/z 544.2 [M+Na]⁺.

### Example 191

### 2-(2,6-Diethyl-4-((5-oxo-4-(4-(trifluoromethyl)phenyl)-4,5-dihydro-1H-1,2,4-triazol-1-yl)methyl)phenoxy)-2-methylpropionic acid (compound 191)

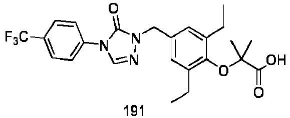

Compound 191 was prepared by replacing intermediate I-3 with intermediate D-1 and 4-hydroxy-3,5-dimethylbenzaldehyde with 4-hydroxy-3,5-diethylbenzaldehyde according to the method of Example 55: ¹H NMR (300 MHz, DMSO-*d₆*) δ 12.85 (s, 1H), 8.65 (s, 1H), 8.04 (d, *J* = 7.9 Hz, 2H), 7.92 (d, *J* = 8.2 Hz, 2H), 7.01 (s, 2H), 4.90 (s, 2H), 2.58 (q, *J =* 6.9 Hz, 4H), 1.35 (s, 6H), 1.12 (t, *J* = 6.7 Hz, 6H). HRMS (ESI) calcd. for C₂₄H₂₆F₃N₃O₄ [M+H]⁺ 478.1954, found 478.1954.

### Example 192

### Ethyl 2-(2,6-di ethyl-4-((5-oxo-4-(4-(trifluoromethoxy)phenyl)-4,5-dihydro-1/7-1,2,4-triazol-1-yl)methyl)phenoxy)-2-methylpropropionate (compound 192)

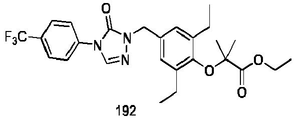

Compound 192 was prepared without hydrolysis according to the method of Example 191: ¹H NMR (300 MHz, CDCl₃) δ 8.04 - 7.60 (m, 5H), 7.08 (s, 2H), 4.97 (s, 2H), 4.30 (q, *J =* 7.1 Hz, 2H), 2.59 (q, *J* = 7.5 Hz, 4H), 1.47 (s, 6H), 1.37 (t, *J* = 7.1 Hz, 3H), 1.20 (t, *J* = 7.5 Hz, 6H). MS (ESI): m/z 528.2 [M+Na]⁺.

### Example 193

### 2-(2,6-Dimethyl-4-((5-oxo-4-(4-(trifluoromethoxy)phenyl)-4,5-dihydro-1H-1,2,4-triazol-1-yl)methyl)phenoxy)-2-methylpropionic acid diisopropylamine salt (compound 193)

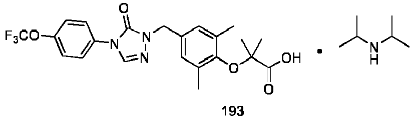

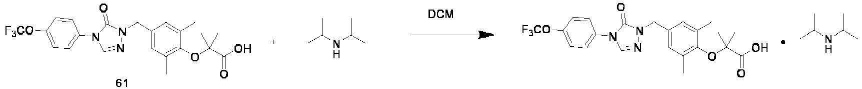

Compound 61 (80 mg, 0.17 mmol) prepared in Example 61 was dissolved in DCM (2 mL), and diisopropylamine (29 mL, 0.20 mmol) was added. The mixture was stirred at room temperature overnight. After the reaction was completed, the reaction liquid was concentrated by rotary evaporation under reduced pressure to remove the solvent. The residue was dispersed in n-hexane (2 mL), and DCM (5 drops) was added. The mixture was stirred at room temperature for half an hour. The reaction liquid was filtered under reduced pressure to give compound 193 (white solid, 83 mg): ¹H NMR (300 MHz, DMSO-*d₆*) δ 8.52 (s, 1H), 7.88 (d, *J* = 8.9 Hz, 2H), 7.55 (d, *J* = 8.6 Hz, 2H), 6.90 (s, 2H), 4.82 (s, 2H), 3.12 (dt, *J* = 12.6, 6.3 Hz, 2H), 2.17 (s, 6H), 1.29 (s, 6H), 1.12 (d, *J* = 6.3 Hz, 12H).

### Example 194

### 2-(2,6-Dimethyl-4-(2-(5-oxo-4-(4-(trifluoromethyl)phenyl)-4,5-dihydro-1H-1,2,4-triazol-1-yl)ethyl)phenoxy)-2-methylpropionic acid (compound 194)

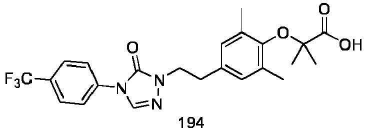

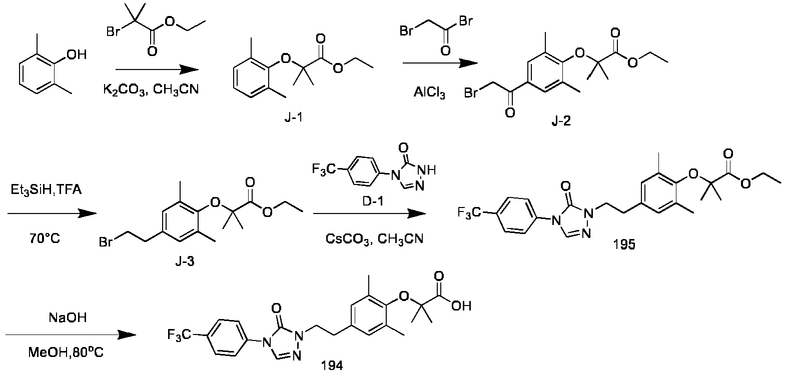

### Synthesis of intermediate J-1

2,6-Dimethylphenol (1.2 g, 10 mmol) was dissolved in acetonitrile (15 mL), and ethyl 2-bromoisobutyrate (3.4 mL, 30 mmol) and cesium carbonate (8.1 g, 25 mmol) were added. The reaction system was warmed to 80 °C and stirred for 12 h. After the reaction was completed, the reaction liquid was concentrated by rotary evaporation under reduced pressure to remove the solvent. Water (20 mL) was added to the residue. The mixture was extracted with EA (50 mL × 3). The organic phase was washed with 1 N NaOH (20 mL × 3) and saturated brine (20 mL), dried over anhydrous MgSO₄, and concentrated by rotary evaporation under reduced pressure to remove the solvent. The residue, i.e., the crude product of J-1, was directly used in the next step.

### Synthesis of intermediate J-2

DCM (20 mL) and bromoacetyl bromide (2.1 mL, 24 mmol) were added to AlCl₃ (3.2 g, 24 mmol) under argon atmosphere and an ice bath. The mixture was stirred at room temperature for 1 h. J-1 (1.9 g, 8 mmol) was added to the mixture under an ice bath. The mixture was stirred at room temperature overnight. After the reaction was completed, the reaction liquid was concentrated by rotary evaporation under reduced pressure to remove the solvent. Water (20 mL) was added to the residue, and the resulting mixture was extracted with EA (20 mL × 3). The organic phase was washed with saturated brine (20 mL), and concentrated by rotary evaporation under reduced pressure to remove the solvent. The residue, i.e., the crude product of J-2, was directly used in the next step.

### Synthesis of intermediate J-3

Intermediate J-2 (1.8 g, 5 mmol) was dissolved in trifluoroacetic acid (15 mL), and triethylsilane (1.0 mL, 7.5 mmol) was added. The reaction system was warmed to 70 °C and stirred for 12 h. After the reaction was completed, the reaction liquid was cooled to room temperature, and diluted with water (20 mL) under an ice bath. The mixture was stirred at room temperature for 10 min, and concentrated by rotary evaporation under reduced pressure to remove the solvent. The residue was extracted with EA (20 mL × 3). The organic phase was washed with saturated sodium bicarbonate (20 mL) and saturated brine (20 mL), and concentrated by rotary evaporation under reduced pressure to remove the solvent. The residue, i.e., the crude product of J-3, was directly used in the next step.

### Synthesis of compound 195

Intermediate D-1 (91.2 mg, 0.4 mmol) was dissolved in acetonitrile (3 mL), and J-3 (180 mg, 0.6 mmol) and cesium carbonate (130 mg, 1 mmol) were added. The reaction mixture was stirred at room temperature overnight. After the reaction was completed, the reaction liquid was concentrated by rotary evaporation under reduced pressure to remove the solvent. The residue was purified by silica gel column chromatography (EA/PE = 1:5) to give compound 195 (white solid, 125 mg).

### Synthesis of compound 194

Compound 195 (125 mg, 0.26 mmol) was dissolved in MeOH (3 mL), and 1 N NaOH (1.3 mL, 1.3 mmol) was added. The reaction system was warmed to 80 °C and stirred for 12 h. After the reaction was completed, the reaction liquid was cooled to room temperature. 1 N HCl (1.3 mL) was added, and the resulting mixture was concentrated by rotary evaporation under reduced pressure to remove the solvent. Water (10 mL) was added to the residue, and the resulting mixture was extracted with EA (20 mL × 3). The organic phase was washed with saturated brine (20 mL × 1), and concentrated by rotary evaporation under reduced pressure to remove the solvent. The residue was purified by silica gel column chromatography (DCM/MeOH = 100:1) to give compound 194 (white solid, 78.9 mg): ¹H NMR (300 MHz, DMSO-*d₆*) δ 12.81 (s, 1H), 8.63 (s, 1H), 7.95 (d, *J* = 8.9 Hz, 2H), 7.90 (d, *J* = 8.8 Hz, 2H), 6.82 (s, 2H), 3.94 (t, *J* = 7.0 Hz, 2H), 2.87 (t, *J* = 7.2 Hz, 2H), 2.10 (s, 6H), 1.29 (s, 6H). HRMS (ESI): exact mass calculated for C₂₃H₂₄F₃N₃O₄ [M+H]⁺ 464.1797, found 464.1793.

### Example 195

### Ethyl 2-(2,6-dimethyl-4-(2-(5-oxo-4-(4-(trifluoromethyl)phenyl)-4,5-dihydro-1H-1,2,4-triazol-1-yl)ethyl)phenoxy)-2-methylpropionate (compound 195)

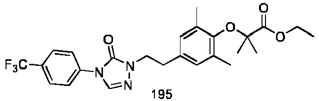

Compound 195 was prepared without hydrolysis according to the method of Example 194: ¹H NMR (300 MHz, CDCl₃) δ 7.84 - 7.69 (m, 4H), 7.28 (s, 1H), 6.86 (s, 2H), 4.30 (q, *J =* 7.1 Hz, 2H), 4.07 (t, 2H), 3.00 (t, 2H), 2.18 (s, 6H), 1.46 (s, 6H), 1.37 (t, *J* = 7.1 Hz, 3H). MS (ESI): m/z 514.2 [M+Na]⁺.

### Example 196

### 2-(2,6-Dimethyl-4-((5-oxo-4-(4-(trifluoromethyl)phenyl)-4,5-dihydro-1H-1,2,4-triazol-1-yl)methoxy)phenoxy)-2-methylpropionic acid (compound 196)

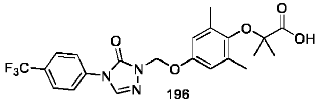

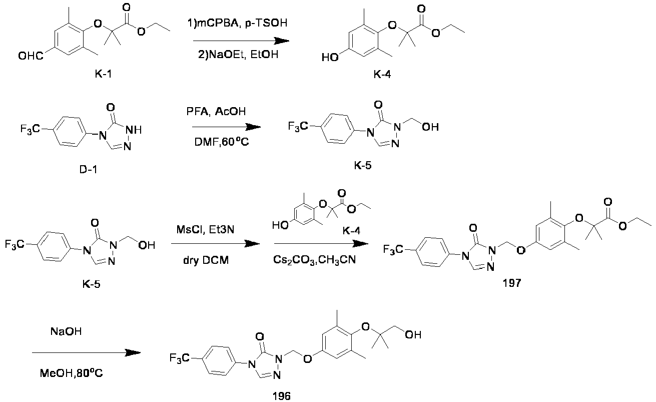

### Synthesis of intermediate K-4

Intermediate K-1 (1.4 g, 10 mmol) was dissolved in DCM (15 mL), and 3-chloroperbenzoic acid (3.5 g, 20 mmol) and p-toluenesulfonic acid (172 mg, 1 mmol) were added. The reaction mixture was stirred at room temperature overnight. After the reaction was completed, the reaction liquid was filtered under reduced pressure. Water (50 mL) was added to the filtrate, and the resulting mixture was extracted with EA (50 mL × 3). The organic phase was washed with saturated sodium bicarbonate (50 mL × 3) and saturated brine (30 mL), dried over anhydrous MgSO₄, and concentrated by rotary evaporation under reduced pressure to remove the solvent. EtOH (20 mL) and a solution of sodium ethoxide (0.8 g, 11 mmol) in EtOH (10 mL) were added to the residue under nitrogen atmosphere. The reaction mixture was stirred at room temperature overnight. After the reaction was completed, the mixture was adjusted to pH = 3 with 1 N HCl (11 mL), and the resulting mixture was concentrated by rotary evaporation under reduced pressure to remove the solvent. Water (10 mL) was added to the residue, and the resulting mixture was extracted with EA (20 mL × 3). The organic phase was washed with saturated brine (20 mL), and concentrated by rotary evaporation under reduced pressure to remove the solvent. The residue was purified by silica gel column chromatography (EA/PE = 1:3) to give intermediate K-4.

### Synthesis of intermediate K-5

Intermediate D-1 (2.1 g, 8.5 mmol) was dissolved in acetonitrile (15 mL), and paraformaldehyde (1.3 g, 42.5 mmol) and acetic acid (60 mg, 1 mmol) were added. The reaction system was warmed to 60 °C and stirred for 12 h. After the reaction was completed, the reaction liquid was concentrated by rotary evaporation under reduced pressure to remove the solvent. The residue was purified by silica gel column chromatography (EA/PE = 1:2) to give intermediate K-5.

### Synthesis of compound 197

K-5 (77 mg, 0.3 mmol) was dissolved in DCM (3 mL), and triethylamine (60.6 mg, 0.6 mmol) and methylsufonyl chloride (51.5 mg, 0.45 mmol) were added dropwise. The mixture was stirred at room temperature overnight. After the reaction was completed, the reaction liquid was concentrated by rotary evaporation under reduced pressure to remove the solvent. The above residue was dissolved in acetonitrile (3 mL) at room temperature, and cesium carbonate (244.5 mg, 0.75 mmol) and K-4 (113.5 mg, 0.45 mmol) were added. The mixture was stirred at room temperature overnight. After the reaction was completed, the reaction liquid was concentrated by rotary evaporation under reduced pressure to remove the solvent. The residue was purified by silica gel column chromatography (EA/PE = 1:5) to give compound 197 (white solid, 59.1 mg).

### Synthesis of compound 196

Compound 197 (59.1 mg, 0.12 mmol) was dissolved in MeOH (3 mL), and 1 NNaOH (0.3 mL, 0.6 mmol) was added. The reaction system was warmed to 80 °C and stirred for 12 h. After the reaction was completed, the reaction liquid was cooled to room temperature. 1 N HCl (0.5 mL) was added, and the resulting mixture was concentrated by rotary evaporation under reduced pressure to remove the solvent. Water (10 mL) was added to the residue, and the resulting mixture was extracted with EA (20 mL × 3). The organic phase was washed with saturated brine (20 mL), and concentrated by rotary evaporation under reduced pressure to remove the solvent. The residue was purified by silica gel column chromatography (DCM/MeOH = 100:1) to give compound 196 (white solid, 42.7 mg): ¹H NMR (300 MHz, DMSO-*d₆*) δ 12.75 (s, 1H), 8.72 (s, 1H), 7.99 (d, *J* = 8.7 Hz, 2H), 7.93 (d, *J=* 8.8 Hz, 2H), 6.79 (s, 2H), 5.67 (s, 2H), 2.14 (s, 6H), 1.33 (s, 6H). HRMS (ESI): exact mass calculated for C₂₂H₂₂F₃N₃O₅ [M+H]⁺ 466.1590, found 466.1590.

### Example 197

### Ethyl 2-(2, 6-dimethyl-4-((5-oxo-4-(4-(trifluoromethyl)phenyl)-4, 5-dihydro-1H-1,2,4-triazol-1-yl)methoxy)phenoxy)-2-methylpropionate (compound 197)

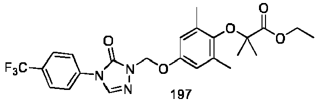

Compound 195 was prepared without hydrolysis according to the method of Example 196: ¹H NMR (300 MHz, CDCl₃) δ 7.84 (s, 1H), 7.77 (q, *J* = 8.9 Hz, 4H), 6.79 (s, 2H), 5.73 (s, 2H), 4.30 (q, *J* = 6.8 Hz, 2H), 2.20 (s, 67H), 1.47 (s, 6H), 1.37 (t, *J* = 7.2 Hz, 3H). MS (ESI): m/z 516.2 [M+Na]⁺.

### Example 198

### 2-(2,6-Dimethyl-4-(((5-oxo-4-(4-(trifluoromethyl)phenyl)-4,5-dihydro-1H-1,2,4-triazol-1-yl)methyl)thio)phenoxy)-2-methylpropionic acid (compound 198)

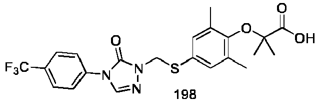

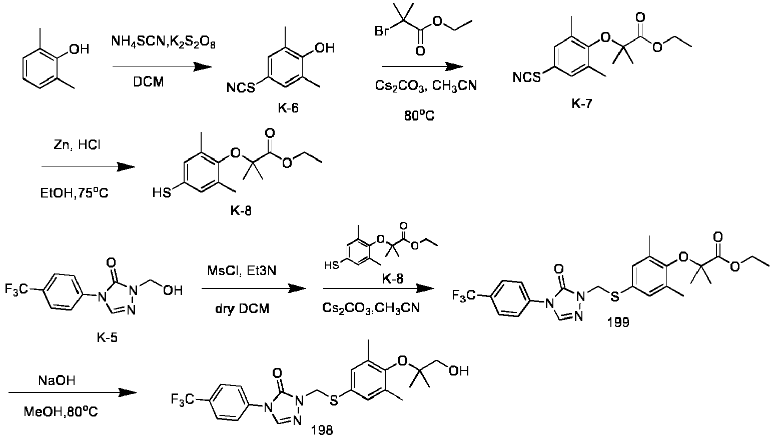

### Synthesis of intermediate K-6

2,6-Dimethylphenol (1.2 g, 10 mmol) was dissolved in DCM (25 mL), and ammonium thiocyanate (1.1 g, 15 mmol) and potassium persulfate (5.4 g, 20 mmol) were added. The mixture was stirred at room temperature overnight. After the reaction was completed, the reaction mixture was concentrated by rotary evaporation under reduced pressure to remove the solvent, and the residue, i.e., the crude product of intermediate K-6, was directly used in the next step.

### Synthesis of intermediate K-7

Compound K-6 (1.4 g, 8 mmol) was dissolved in acetonitrile (15 mL), and ethyl 2-bromoisobutyrate (2.7 mL, 24 mmol) and cesium carbonate (6.5 g, 20 mmol) were added. The reaction system was warmed to 80 °C and stirred for 12 h. After the reaction was completed, the reaction liquid was concentrated by rotary evaporation under reduced pressure to remove the solvent. Water (20 mL) was added to the residue, and the resulting mixture was extracted with EA (25 mL × 3). The organic phase was washed with 1 N NaOH (20 mL × 3) and saturated brine (25 mL), dried over anhydrous MgSO₄, and concentrated by rotary evaporation under reduced pressure to remove the solvent. The residue, i.e., the crude product of intermediate K-7, was directly used in the next step.

### Synthesis of intermediate K-8

Compound K-7 (1.4 g, 5 mmol) was dissolved in absolute ethanol (15 mL), and 6 N HCl (1.25 mL, 7.5 mmol) and zinc powder (1.6 g, 25 mmol) were added. The reaction system was warmed to 75 °C and stirred for 8 h. After the reaction was completed, the reaction liquid was concentrated by rotary evaporation under reduced pressure to remove the solvent. The residue was purified by silica gel column chromatography (EA/PE = 1:20) to give K-8.

### Synthesis of compound 199

K-5 (77 mg, 0.3 mmol) was dissolved in DCM (3 mL), and triethylamine (60.6 mg, 0.6 mmol) and methylsufonyl chloride (51.5 mg, 0.45 mmol) were added dropwise. The reaction mixture was stirred at room temperature overnight. After the reaction was completed, the reaction liquid was concentrated by rotary evaporation under reduced pressure to remove the solvent. The above residue was dissolved in an acetonitrile solution (3 mL) at room temperature, and cesium carbonate (244.5 mg, 0.75 mmol) and K-8 (113.5 mg, 0.45 mmol) were added. The mixture was stirred at room temperature overnight. After the reaction was completed, the reaction liquid was concentrated by rotary evaporation under reduced pressure to remove the solvent. The residue was purified by silica gel column chromatography (EA/PE = 1:5) to give compound 199 (59.1 mg).

### Synthesis of compound 198

Compound 199 (59.1 mg, 0.12 mmol) was dissolved in MeOH (3 mL), and 1 NNaOH (0.3 mL, 0.6 mmol) was added. The reaction system was warmed to 80 °C and stirred for 12 h. After the reaction was completed, the reaction liquid was cooled to room temperature. 1 N HCl (0.5 mL) was added, and the resulting mixture was concentrated by rotary evaporation under reduced pressure to remove the solvent. Water (10 mL) was added to the residue, and the resulting mixture was extracted with EA (20 mL × 3). The organic phase was washed with saturated brine (20 mL), and concentrated by rotary evaporation under reduced pressure to remove the solvent. The residue was purified by silica gel column chromatography (DCM/MeOH = 100:1) to give compound 198 (white solid, 67.2 mg): ¹H NMR (300 MHz, DMSO-*d₆*) δ 12.85 (s, 1H), 8.69 (s, 1H), 7.92 (s, 4H), 7.11 (s, 2H), 5.21 (s, 2H), 2.09 (s, 6H), 1.29 (s, 6H). HRMS (ESI): exact mass calculated for C₂₂H₂₂F₃N₃O₄S [M+H]⁺ 482.1361, found 482.1362.

### Example 199

### Ethyl 2-(2,6-dimethyl-4-(((5-oxo-4-(4-(trifluoromethyl)phenyl)-4,5-dihydro-1H-1,2,4-triazol-1-yl)methyl)thio)phenoxy)-2-methylpropionate (compound 199)

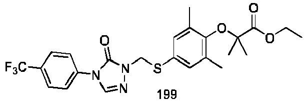

Compound 199 was prepared without hydrolysis according to the method of Example 198: ¹H NMR (300 MHz, CDCl₃) δ 7.78 (s, 1H), 7.77 (d, *J* = 9.2 Hz, 2H), 7.70 (d, *J* = 8.6 Hz, 2H), 7.16 (s, 2H), 5.20 (s, 2H), 4.29 (q, *J* = 7.1 Hz, 2H), 2.17 (s, 6H), 1.45 (s, 6H), 1.36 (t, *J* = 7.1 Hz, 3H).

### Example 200

### 2-(2,6-Dimethyl-4-(3-(5-oxo-4-(4-(trifluoromethyl)phenyl)-4,5-dihydro-1H-1,2,4-triazol-1-yl)propyl)phenoxy)-2-methylpropionic acid (compound 200)

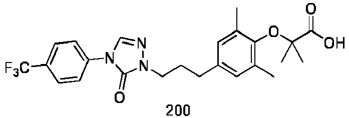

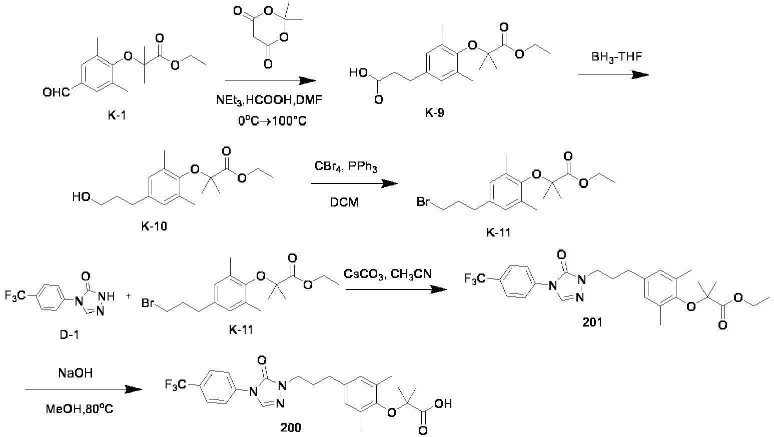

### Synthesis of intermediate K-9

Intermediate K-1 (0.5 g, 2.25 mmol) was dissolved in DMF (5 mL), and 2,2-dimethyl-1,3-dioxane-4,6-dione (0.5 g, 3.38 mmol) and triethylamine (0.4 mL, 2.7 mmol) were added. Formic acid (189 µL, 5 mmol) was added to the mixture under an ice bath, and the resulting mixture was stirred for 5 min. The reaction system was warmed to 100 °C, and stirred overnight. After the reaction was completed, the reaction liquid was cooled to room temperature. Water (20 mL) was added, and the resulting mixture was extracted with EA (50 mL × 3). The organic phase was washed with saturated brine (20 mL), dried over anhydrous MgSO₄, and concentrated by rotary evaporation under reduced pressure to remove the solvent to give a residue, i.e., the crude product of intermediate K-9.

### Synthesis of intermediate K-10

Intermediate K-9 (0.5 g, 1.5 mmol) was dissolved in tetrahydrofuran (5 mL). Boranetetrahydrofuran complex (1 mL, 1 mmol) was added under an ice bath. The mixture was stirred overnight. After the reaction was completed, water (10 mL) was added, and the resulting mixture was stirred for 30 min. The mixture was extracted with EA (20 mL × 3). The organic phase was washed with saturated brine (20 mL), and concentrated by rotary evaporation under reduced pressure to remove the solvent to give a residue, i.e., the crude product of intermediate K-10.

### Synthesis of intermediate K-11

Intermediate K-10 (0.2 g, 1 mmol) was dissolved in DCM (5 mL). Carbon tetrabromide (0.5 g, 1.5 mmol) and triphenylphosphine (0.5 g, 1.4 mmol) were added under an ice bath. The mixture was stirred at room temperature overnight. After the reaction was completed, the reaction liquid was concentrated by rotary evaporation under reduced pressure to remove the solvent. The residue was purified by silica gel column chromatography (EA/PE = 1:20) to give intermediate K-11.

### Synthesis of compound 201

Intermediate D-1 (91.2 mg, 0.4 mmol) was dissolved in acetonitrile (3 mL), and intermediate K-11 (180 mg, 0.6 mmol) and cesium carbonate (130 mg, 1 mmol) were added. The mixture was stirred at room temperature overnight. After the reaction was completed, the reaction liquid was concentrated by rotary evaporation under reduced pressure to remove the solvent. The residue was purified by silica gel column chromatography (EA/PE = 1:5) to give compound 201 (125 mg).

### Synthesis of compound 200

Compound 201 (125 mg, 0.26 mmol) was dissolved in MeOH (3 mL), and 1 N NaOH (1.3 mL, 1.3 mmol) was added. The reaction system was warmed to 80 °C and stirred for 12 h. After the reaction was completed, the reaction liquid was cooled to room temperature. 1 N HCl (1.3 mL) was added, and the resulting mixture was concentrated by rotary evaporation under reduced pressure to remove the solvent. Water (10 mL) was added to the residue, and the resulting mixture was extracted with EA (20 mL × 3). The organic phase was washed with saturated brine (20 mL), and concentrated by rotary evaporation under reduced pressure to remove the solvent. The residue was purified by silica gel column chromatography (DCM/MeOH = 100:1) to give compound 200 (white solid, 78.9 mg): ¹H NMR (300 MHz, DMSO-*d₆*) δ 12.75 (s, 1H), 8.62 (s, 1H), 8.01 (d, *J* = 8.4 Hz, 2H), 7.91 (d, *J* = 8.4 Hz, 2H), 6.84 (s, 2H), 3.76 (t, *J* = 6.4 Hz, 2H), 3.32 (t, 2H), 2.12 (s, 6H), 2.05 - 1.75 (m, 2H), 1.32 (s, 6H). HRMS (ESI): exact mass calculated for C₂₄H₂₆F₃N₃O₄ [M+H]⁺ 478.1954, found 478.1950.

### Example 201

### 2-(2,6-Dimethyl-4-(3-(5-oxo-4-(4-(trifluoromethyl)phenyl)-4,5-dihydro-1H-1,2,4-triazol-1-yl)propyl)phenoxy)-2-methylpropionic acid (compound 201)

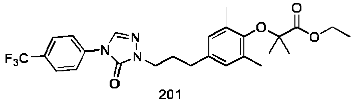

Compound 201 was prepared without hydrolysis according to the method of Example 200: ¹H NMR (300 MHz, CDCl₃) δ 7.77 (s, 4H), 7.28 (s, 1H), 6.82 (s, 2H), 4.30 (q, *J* = 7.1 Hz, 2H), 3.91 (t, *J* = 7.0 Hz, 2H), 2.61 (t, *J* = 7.7 Hz, 2H), 2.17 (s, 6H), 2.16 - 2.03 (m, 2H), 1.47 (s, 6H), 1.36 (t, *J* = 7.1 Hz, 3H). MS (ESI): m/z 528.2 [M+Na]⁺.

### Example 202

### 2-(2,6-Dimethyl-4-((4-(4-(methylthio)phenyl)-5-oxo-4,5-dihydro-1H-1,2,4-triazol-1-yl)methyl)phenoxy)-2-methylpropionic acid (compound 202)

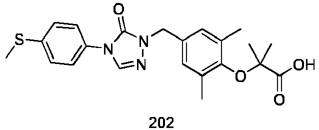

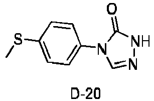

### Synthesis of intermediate D-20

Intermediate D-20 was prepared by replacing p-bromoaniline in Example 1 with p-methylthioaniline according to the method for intermediate I-3 of Example 1.

### Synthesis of compound 202

Compound 202 was prepared by replacing intermediate I-3 with intermediate D-20 according to the method of Example 55: ¹H NMR (300 MHz, DMSO-*d₆*) δ 12.83 (s, 1H), 8.45 (s, 1H), 7.66 (d, *J* = 8.4 Hz, 2H), 7.39 (d, *J* = 8.5 Hz, 2H), 6.94 (s, 2H), 4.82 (s, 2H), 2.50 (s, 3H), 2.15 (s, 6H), 1.34 (s, 6H). MS (ESI): m/z 450.1 [M+Na]⁺.

### Example 203

### Ethyl 2-(2,6-dimethyl-4-((4-(4-(methylthio)phenyl)-5-oxo-4,5-dihydro-1H-1,2,4-triazol-1-yl)methyl)phenoxy)-2-methylpropionate (compound 203)

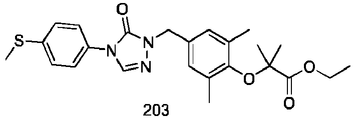

Compound 203 was prepared by replacing intermediate I-3 with intermediate D-20 without hydrolysis according to the method of Example 55: ¹H NMR (300 MHz, CDCl₃) δ 7.67 (s, 1H), 7.51 (d, *J* = 8.4 Hz, 2H), 7.35 (d, *J* = 8.3 Hz, 2H), 7.03 (s, 2H), 4.91 (s, 2H), 4.29 (q, *J* = 7.1 Hz, 2H), 2.52 (s, 3H), 2.20 (s, 6H), 1.47 (s, 6H), 1.36 (t, *J=* 7.1 Hz, 3H). MS (ESI): m/z 478.2 [M+Na]⁺.

### Example 204

### 2-(2,6-Dimethyl-4-((5-oxo-4-(4-(trifluoromethoxy)phenyl)-4,5-dihydro-1H-1,2,4-triazol-1-yl)methyl)phenoxy)propionic acid (compound 204)

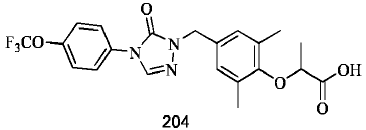

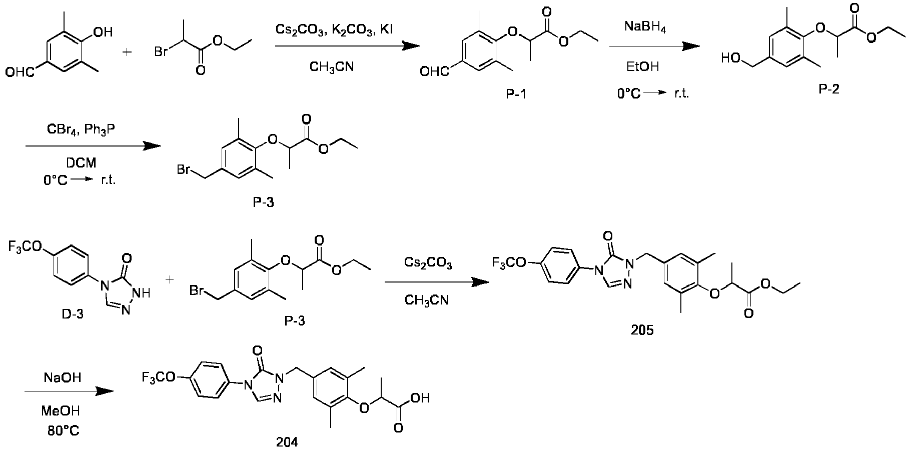

### Synthesis of intermediate P-1

3,5-Dimethyl-4-hydroxybenzaldehyde (21.0 g, 140 mmol) was dissolved in acetonitrile (200 mL), and ethyl 2-bromopropionate (94.1 g, 520 mmol), cesium carbonate (45.6 g, 140 mmol), potassium carbonate (38.6 g, 280 mmol), and potassium iodide (1.66 g, 10 mmol) were added. The system was transferred to an oil bath and reacted at 80 °C for 36 h. After the reaction was completed, the reaction liquid was cooled to room temperature and filtered under reduced pressure. The filtrate was concentrated by rotary evaporation under reduced pressure to remove the solvent. The residue was diluted with water (200 mL) and extracted with ethyl acetate (200 mL × 3). The organic phases were combined, washed with 1 N sodium hydroxide (200 mL × 3) and saturated brine (200 mL × 1), dried over anhydrous sodium sulfate, and concentrated by rotary evaporation under reduced pressure to remove the solvent. The residue was purified by column chromatography (petroleum ether/ethyl acetate = 200:1) to give intermediate P-1 (yellow liquid, 16.3 g).

### Synthesis of intermediate P-2

Intermediate P-1 (3.46 g, 13.85 mmol) was dissolved in ethanol (20 mL), and sodium borohydride (280 mg, 7.5 mmol) was slowly added under an ice bath. After the addition, the reaction system was slowly warmed to room temperature and reacted for 4 h. After the reaction was completed, water was added to the reaction liquid to quench the reaction (20 mL). The reaction liquid was concentrated by rotary evaporation under reduced pressure to remove the solvent. The residue was diluted with 30 mL of water and extracted with ethyl acetate (20 mL × 3). The organic phases were combined, washed with saturated brine (30 mL × 1), dried over anhydrous sodium sulfate, and concentrated by rotary evaporation under reduced pressure to remove the solvent to give a crude product of intermediate P-2, which was directly used in the next step without further purification.

### Synthesis of intermediate P-3

The crude product of compound P-2 obtained from the previous step was dissolved in DCM (20 mL), carbon tetrabromide (13.6 g, 41 mmol) was added, and triphenylphosphine (9.9 g, 37.8 mmol) was slowly added under an ice bath. After the addition, the reaction system was slowly warmed to room temperature and reacted for 8 h. The reaction liquid was concentrated by rotary evaporation under reduced pressure to remove the solvent. The residue was purified by column chromatography (petroleum ether/ethyl acetate = 20:1) to give intermediate P-3 (yellow liquid, 3.6 g).

### Synthesis of compound 205

Intermediate D-3 (229.1 mg, 0.5 mmol) was dissolved in acetonitrile (5 mL), and intermediate P-3 (235.5 mg, 0.75 mmol) and cesium carbonate (326 mg, 1 mmol) were added. The mixture was stirred at room temperature for 4 h. After the reaction was completed, the reaction liquid was concentrated by rotary evaporation under reduced pressure to remove the solvent. The residue was purified by column chromatography (petroleum ether/ethyl acetate = 10:1) to give compound 205 (white solid, 302 mg).

### Synthesis of compound 204

Compound 205 (302 mg, 0.63 mmol) was dissolved in methanol (5 mL), and a 1 N NaOH solution (3.5 mL) was added. The mixture was stirred at room temperature for 24 h. After the reaction was completed, the reaction liquid was adjusted to pH 4 with a 1 N HCl solution, and concentrated by rotary evaporation under reduced pressure to remove the solvent. The residue was diluted with water (15 mL) and extracted with ethyl acetate (10 mL × 3). The organic phase was washed with saturated brine (15 mL × 1), and concentrated by rotary evaporation under reduced pressure to remove the solvent. The residue was purified by column chromatography (dichloromethane/methanol = 100:1) to give compound 204 (white solid, 150 mg): ¹H NMR (300 MHz, DMSO-*d₆*) δ 12.87 (s, 1H), 8.53 (s, 1H), 7.88 (d, *J =* 9.0 Hz, 2H), 7.56 (d, *J* = 8.5 Hz, 2H), 6.98 (s, 2H), 4.84 (s, 2H), 4.40 (q, *J* = 6.5 Hz, 1H), 2.21 (s, 6H), 1.41 (d, *J* = 6.6 Hz, 3H). MS (ESI): m/z 474.2 [M+Na]⁺.

### Example 205

### Ethyl 2-(2,6-dimethyl-4-((5-oxo-4-(4-(trifluoromethoxy)phenyl)-4,5-dihydro-1H-1,2,4-triazol-1-yl)methyl)phenoxy)propionate (compound 205)

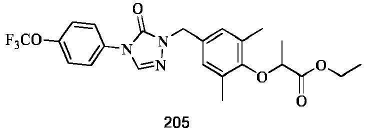

Compound 205 was prepared without hydrolysis according to the method of Example 204: ¹H NMR (300 MHz, CDCl₃) δ 7.71 (s, 1H), 7.65 (d, *J* = 8.9 Hz, 2H), 7.36 (d, *J* = 8.6 Hz, 2H), 7.06 (s, 2H), 4.92 (s, 2H), 4.48 (q, *J* = 6.7 Hz, 1H), 4.24 (q, *J* = 7.1 Hz, 2H), 2.29 (s, 6H), 1.55 (t, *J=* 6.7 H, 3H), 1.29 (t, *J=* 7.0 Hz, 3H). MS (ESI): m/z 502.2 [M+Na]⁺.

### Example 206

### 2-(2,6-Dimethyl-4-((5-oxo-4-(4-(trifluoromethoxy)phenyl)-4,5-dihydro-1H-1,2,4-triazol-1-yl)methyl)phenoxy)butyric acid (compound 206)

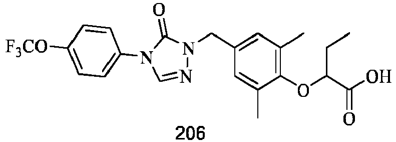

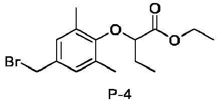

### Synthesis of intermediate P-4

Intermediate P-4 was prepared by replacing ethyl 2-bromopropionate in Example 204 with ethyl 2-bromobutyrate according to the method for intermediate P-3 of Example 204.

### Synthesis of compound 206

Compound 206 was prepared by replacing intermediate P-3 with intermediate P-4 according to the method of Example 204: ¹H NMR (300 MHz, DMSO-*d₆*) δ 12.85 (s, 1H), 8.53 (s, 1H), 7.88 (d, *J=* 9.0 Hz, 2H), 7.56 (d, *J=* 8.4 Hz, 2H), 6.97 (s, 2H), 4.83 (s, 2H), 4.31 (t, *J* = 5.9 Hz, 1H), 2.22 (s, 6H), 1.86 (dt, 2H), 0.96 (t, *J* = 7.3 Hz, 3H). MS (ESI): m/z 488.2 [M+Na]⁺.

### Example 207

### Ethyl 2-(2,6-dimethyl-4-((5-oxo-4-(4-(trifluoromethoxy)phenyl)-4,5-dihydro-1H-1,2,4-triazol-1-yl)methyl)phenoxy)butyrate (compound 207)

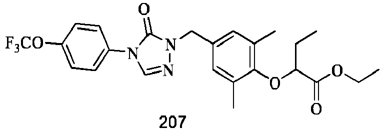

Compound 207 was prepared without hydrolysis according to the method of Example 206: ¹H NMR (300 MHz, CDCl₃) δ 7.70 (s, 1H), 7.64 (d, *J* = 8.9 Hz, 2H), 7.35 (d, *J* = 8.7 Hz, 2H), 7.05 (s, 2H), 4.91 (s, 2H), 4.37 (t, *J=* 6.1 Hz, 1H), 4.21 (q, *J=* 14.2, 7.1 Hz, 2H), 2.29 (s, 6H), 1.98 (dt, 2H), 1.26 (t, *J* = 7.1 Hz, 3H), 1.03 (t, *J* = 7.4 Hz, 3H). MS (ESI): m/z 516.2 [M+Na]⁺.

### Example 208

### 2-(2,6-Dimethyl-4-((5-oxo-4-(4-(trifluoromethoxy)phenyl)-4,5-dihydro-1H-1,2,4-triazol-1-yl)methyl)phenoxy)-2-methylpropionic acid berberine salt (compound 208)

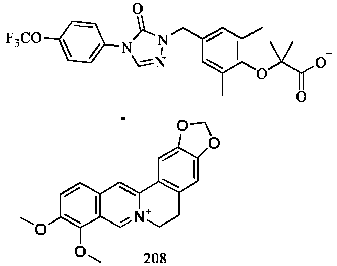

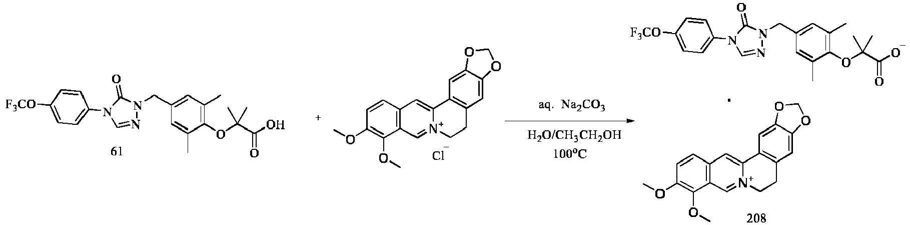

Berberine chloride (185.9 mg, 0.5 mmol) was dissolved in hot water (7.5 mL). The mixture was adjusted to pH 8-9 with a saturated sodium carbonate solution, and stirred and reacted for 1 h. An ethanol solution (4 mL) of compound 61 (232.6 mg, 0.5 mmol) was added. The reaction system was heated to 100 °C and stirred for 2 h. The reaction liquid was cooled for crystallization, filtered under reduced pressure, washed with cold water, and dried under infrared ray irradiation to give compound 208 (yellow solid, 40 mg): ¹H NMR (300 MHz, DMSO-*d₆*) δ 9.90 (s, 1H), 8.95 (s, 1H), 8.53 (s, 1H), 8.21 (d, *J* = 9.1 Hz, 1H), 8.00 (d, *J* = 8.9 Hz, 1H), 7.88 (d, *J* = 8.9 Hz, 2H), 7.80 (s, 2H), 7.55 (d, *J* = 8.8 Hz, 2H), 7.09 (s, 1H), 6.92 (s, 1H), 6.18 (s, 2H), 4.94 (t, 2H), 4.82 (s, 2H), 4.10 (s, 3H), 4.07 (s, 3H), 3.21 (t, 2H), 2.15 (s, 6H), 1.30 (s, 6H).

### Example 209

### Assay on Agonistic Activity of Compounds for PPARα/PPARδ/PPARγ

Cos-7 cells (African green monkey kidney fibroblasts, commonly used tool cells) were cultured in a 10 cm cell culture dish in a DMEM complete medium containing 10% fetal bovine serum. When the cells grew to a density of about 70%, the medium was replaced with a fresh medium for later transfection. The procedures for preparing the plasmid working fluid were as follows: 15 µg of pBIND-Gal4-PPARα (LBD) plasmid or pBIND-Gal4-PPARδ (LBD) plasmid or pBIND-Gal4-PPARy (LBD) plasmid (J. Chem. Inf. Model., 2020, 60, 1717), 15 µg of pGL4.35-9×Gal4 UAS plasmid (purchased from Promega (Beijing) Biotech Co., Ltd.), and 60 µL of transfection reagent (HighGene, purchased from Wuhan ABclonal Technology Co., Ltd.) were added to 2 mL of Opti-MEM, and the mixture was left to stand at room temperature for 15 min to obtain a working solution. The plasmid working solution was then added to the cell culture dish for cell transfection. After 4 h of transfection, the cells were washed with PBS, digested with trypsin, seeded into a 96-well plate with 20,000-30,000 cells in each well, and subjected to adherent culture for 24 h. Test compounds were prepared into appropriate test concentrations with a complete medium and then added to the 96-well plate. Meanwhile, the PPARα agonistic activity was defined as 100% for GW7647 (purchased from MCE) at a final concentration of 10 nM, the PPARδ agonistic activity was defined as 100% for GW501516 (purchased from MCE) at a final concentration of 10 nM, and the PPARy agonistic activity was defined as 100% for Rosiglitazone (purchased from Adamas) at a final concentration of 1 µM. After 16 h of drug action, the medium was discarded, and 100 µL of reporter gene lysis buffer (purchased from Shanghai Beyotime Biological Tech. Co., Ltd.) was added to lyse the cells for 15 min. 10 µL of lysate was pipetted into a white opaque 384-well plate, and then 10 µL of reporter gene assay buffer (purchased from Shanghai Beyotime Biological Tech. Co., Ltd.) was added. The bioluminescence was detected by using a multimode microplate reader, and the corresponding half maximal effective concentration (EC₅₀) value was calculated according to the detection value. In addition to assaying the compounds of the present invention, the PPARα/δ agonist GFT505 in the clinical trial and the most potent PPARα/δ agonist 5c reported in the literature at present (ACS Med. Chem. Lett., 2019, 10, 1068) were also assayed. The experimental results are shown in Table 2.

**Table 2. Agonistic activity of compounds for PPARα/PPARδ/PPARγ**

| Compound No. | PPARα | PPARδ | PPARγ |
|---|---|---|---|
| | EC₅₀ (nM) | EC₅₀ (nM) | EC₅₀ (nM) |
| 1 | 2236 | 1001 | >10 µM |
| 3 | 4588 | 343 | >10 µM |
| 5 | 4782 | 286 | >10 µM |
| 55 | 62 | 22 | 1779 |
| 57 | 26 | 2 | 1429 |
| 59 | 31 | 6 | 454 |
| 61 | 7 | 8 | 1316 |
| 63 | 1027 | 647 | 8650 |
| 65 | 180 | 248 | 955 |
| 67 | 167 | 47 | 1832 |
| 69 | 33 | 481 | >5 µM |
| 71 | 3 | 5 | 1251 |
| 73 | 5 | 6 | 903 |
| 75 | 454 | 203 | 4259 |
| 77 | 103 | 132 | 1796 |
| 79 | 341 | 620 | 1885 |
| 81 | 1096 | 8546 | 7878 |
| 83 | 1768 | 1480 | 1087 |
| 85 | 332 | 90 | >5 µM |
| 87 | 238 | 77 | 4131 |
| 89 | 415 | 916 | 2919 |
| 91 | 987 | 3368 | 5212 |
| 93 | 2932 | 5448 | 1807 |
| 95 | 1052 | 136 | 9083 |
| 97 | 696 | 2315 | >5 µM |
| 99 | 116 | 375 | 1145 |
| 101 | 2933 | 8663 | >5 µM |
| 103 | 215 | 278 | 4679 |
| 105 | 172 | 323 | 4920 |
| 107 | 144 | 278 | 2020 |
| 109 | 645 | 389 | 1511 |
| 115 | 8 | 175 | 1761 |
| 117 | 9 | 135 | 1784 |
| 121 | 72 | 39 | 2526 |
| 123 | 394 | 664 | >5 µM |
| 125 | 162 | 229 | 2779 |
| 127 | 419 | 586 | >5 µM |
| 129 | 23 | 87 | 4906 |
| 131 | 14 | 67 | 1180 |
| 137 | 1866 | 1519 | >5 µM |
| 141 | 382 | 124 | 1447 |
| 143 | 9 | 15 | 1795 |
| 145 | 8 | 29 | >5 µM |
| 147 | 387 | 81 | 1796 |
| 149 | 7 | 225 | >5 µM |
| 151 | 220 | 914 | >5 µM |
| 153 | 498 | 1675 | >5 µM |
| 155 | 351 | 1367 | >5 µM |
| 157 | 273 | 91 | 1655 |
| 163 | 35 | 6 | 1688 |
| 165 | 7 | 1 | 812 |
| 167 | 39 | 23 | 4332 |
| 169 | 16 | 12 | 2305 |
| 171 | 21 | 2 | 2223 |
| 173 | 56 | 1 | 1363 |
| 175 | 309 | 685 | 932 |
| 179 | 17260 | 1736 | >10 µM |
| 181 | 21360 | 453 | >10 µM |
| 183 | 15620 | 188 | >10 µM |
| 185 | 13 | 24 | 3042 |
| 187 | 10 | 7 | 2015 |
| 189 | 59 | 42 | 2190 |
| 191 | 44 | 42 | 3090 |
| 194 | 80 | 38 | 2024 |
| 196 | 86 | 56 | >5 µM |
| 198 | 289 | 181 | 2846 |
| 200 | 134 | 212 | >5 µM |
| 202 | 81 | 75 | >5 µM |
| 204 | 312 | 219 | >5 µM |
| 206 | 126 | 82 | >5 µM |
| GFT505 | 820 | 843 | 1844 |
| 5c | 65 | 24 | 2753 |

The experimental results (Table 2) showed that the compounds of the present invention had significant agonistic activity for PPARα and PPARδ. For example, EC₅₀ values for agonistic activity of compounds 55, 57, 59, 61, 71, 73, 129, 131, 143, 145, 163, 165, 167, 169, 171, 173, 185, 187, 189, 191, 194, and the like for PPARα and PPARδ were all at nanomolar levels. EC₅₀ values for agonistic activity of compounds 61 (PPARα: EC₅₀ = 7 nM; PPARδ: EC₅₀ = 8 nM), 71 (PPARα: EC₅₀ = 3 nM; PPARδ: EC₅₀ = 5 nM), 73 (PPARα: EC₅₀ = 5 nM; PPARδ: EC₅₀ = 6 nM), and 165 (PPARα: EC₅₀ = 7 nM; PPARδ: EC₅₀ = 1 nM) for PPARα and PPAR6 were all at single-digit nanomolar levels, and under the same test system, these compounds had significantly better activity than the phase III clinical trial drug GFT505 (PPARα: EC₅₀ = 760 nM; PPARδ: EC₅₀ = 730 nM) and compound 5c with optimal activity reported in the literature at present (ACSMed. Chem. Lett., 2019, 10, 1068) (PPARα: EC₅₀ = 65 nM; PPARδ: EC₅₀ = 24 nM). In addition, the compounds of the present invention showed very high selectivity for activating PPARα/PPARδ relative to activating PPARγ. For example, the agonistic activity of compound 71 for PPARα (EC₅₀ = 3 nM) was 417 times higher than that of the compound for PPARy (EC₅₀ = 1251 nM), the agonistic activity of compound 71 for PPAR6 (EC₅₀ = 5 nM) was 250 times higher than that of the compound for PPARγ, and the selectivity of compound 71 was significantly better than that of GFT505 (PPARα/PPARγ selectivity: 3.67 times, PPAR6/PPARy selectivity: 3.82 times) and compound 5c (PPARα/PPARγ selectivity: 42.35 times; PPAPδ/PPAPγ selectivity: 114.7 times). The above results suggest that the compounds of the present invention are potent and highly selective PPARα/PPARδ dual agonists.

### Example 210

### Evaluation of Metabolic Stability of Compounds in Human Liver Microsomes

A 500 µM solution of the compound in acetonitrile was prepared and diluted to obtain a 1.5 µM drug working solution with a 0.1 M potassium phosphate solution. The drug working solution was co-incubated with a human liver microsome working solution at a final concentration of 0.75 mg/mL and an NADPH solution (final concentration: 550 µM), and the incubation was terminated by adding an acetonitrile solution at 0 min, 15 min, 30 min, 45 min, and 60 min. The residual amount of the compound that remained in the system at each time point was detected by using LC/MS. The absolute value k of the slope was determined by plotting the natural logarithm of the percentage of the residual amount of the compound with the time, and the calculation was performed according to the formula: T_{1/2} (half-life) = ln2/k = 0.693/k. The experimental results are shown in Table 3.

**Table 3. Results for metabolic stability of the compounds in human liver microsomes**

| Compound No. | T_{1/2} (min) | Compound No. | T_{1/2} (min) |
|---|---|---|---|
| 16 | >120 | 131 | >120 |
| 55 | >120 | 129 | >120 |
| 61 | >120 | 143 | >120 |
| 57 | >120 | 165 | >120 |
| 163 | >120 | GFT505 | 15 |

The experimental results (Table 3) showed that compounds 16, 55, 57, 61, 129, 131, 143, and 165 had very good metabolic stability in human liver microsomes, which was much better than that of GFT505 under the same test conditions. Some other compounds of the present invention also had good metabolic stability in human liver microsomes.

### Example 211

### Pharmacokinetic Evaluation of Compound 57 in Mice

Animals: 6 male C57BL/6J mice, SPF grade, sourced from the animal repository of Shanghai Medicilon research institution.

Grouping: Mice were divided into 2 groups of 3 mice each, one group was an oral administration group and the other group was an intravenous injection administration group. The dose in the oral administration group was 10 mpk, and the dose in the intravenous injection group was 2 mpk.

Experimental method: After the intravenous injection administration group was subjected to administration by injection via the tail vein, approximately 0.03 mL of blood was collected via the orbit at 0.083 h, 0.25 h, 0.5 h, 1 h, 2 h, 4 h, 8 h, and 24 h, and an ethylenediaminetetraacetic acid dipotassium salt was quickly added for anticoagulation and the blood was placed on ice after the blood collection. The mice in the oral administration group were fasted for 12 h before administration and fed 4 h after administration; after oral administration, approximately 0.03 mL of blood was collected via the orbit at 0.25 h, 0.5 h, 1 h, 2 h, 4 h, 6 h, 8 h, and 24 h, and an ethylenediaminetetraacetic acid dipotassium salt was quickly added for anticoagulation and the blood was placed on ice after the blood collection. All samples were centrifuged at 18,000 g for 7 min in a low-temperature centrifuge, and plasma was isolated. The content of the compound in the plasma was determined by LC-MS/MS-18, and relevant pharmacokinetic parameters were calculated from the plasma concentration data at different time points. The experimental results are shown in Table 4.

**Table 4. Pharmacokinetic parameters for intravenous injection and oral administration of compound 57 in mice**

| Route of administration | Intravenous injection | Oral administration |
|---|---|---|
| Half-life T_{1/2} (h) | 9.53 ± 3.29 | 7.41 ± 2.05 |
| Time-to-peak Tₘₐₓ (h) | 0.08 ± 0.00 | 0.25 ± 0.00 |
| Peak concentration Cmax (ng/mL) | 7235.29 ± 268.62 | 14031.80 ± 2050.90 |
| Area under the curve AUC_{(0-∞)} (h*ng/mL) | 4363.20 ± 521.90 | 18607.73 ± 2757.53 |
| Bioavailability F (%) | | 87.30 ± 17.34 |

The experimental results (Table 4) showed that the oral half-life of compound 57 was 7.41 ± 2.05 h, and the bioavailability of the compound was 87.30% ± 17.34%. This indicates that compound 57 has good pharmacokinetic properties.

### Example 212

### Pharmacokinetic Evaluation of Compound 61 in Rats

Animals: 6 male SD rats, SPF grade, sourced from the animal repository of Shanghai Medicilon research institution.

Grouping: Rats were divided into 2 groups of 3 mice each, one group was an oral administration group and the other group was an intravenous injection administration group. The dose in the oral administration group was 10 mpk, and the dose in the intravenous injection group was 2 mpk.

Experimental method: After the intravenous injection administration group was subjected to administration by injection via the tail vein, approximately 0.2 mL of blood was collected via the orbit at 0.083 h, 0.25 h, 0.5 h, 1 h, 2 h, 4 h, 8 h, and 24 h, and an ethylenediaminetetraacetic acid dipotassium salt was quickly added for anticoagulation and the blood was placed on ice after the blood collection. The rats in the oral administration group were fasted for 12 h before administration and fed 4 h after administration; after oral administration, approximately 0.2 mL of blood was collected via the orbit at 0.25 h, 0.5 h, 1 h, 2 h, 4 h, 6 h, 8 h, and 24 h, and an ethylenediaminetetraacetic acid dipotassium salt was quickly added for anticoagulation and the blood was placed on ice after the blood collection. All samples were centrifuged at 18,000 g for 7 min in a low-temperature centrifuge, and plasma was isolated. The content of the compound in the plasma was determined by LC-MS/MS-18, and relevant pharmacokinetic parameters were calculated from the plasma concentration data at different time points. The experimental results are shown in Table 5.

**Table 5. Pharmacokinetic parameters for intravenous injection and oral administration of compound 61 in rats**

| Route of administration | Intravenous injection | Oral administration |
|---|---|---|
| Half-life T_{1/2} (h) | 6.78 ± 0.60 | 5.11 ± 0.31 |
| Time-to-peak Tₘₐₓ (h) | 0.08 ± 0.00 | 1.42 ± 1.01 |
| Peak concentration Cmax (ng/mL) | 10786.91 ± 1390.41 | 5094.15 ± 1667.95 |
| Area under the curve AUC_{(0-∞)} (h*ng/mL) | 9126.43 ± 975.60 | 39148.16 ± 7788.06 |
| Bioavailability F (%) | | 86.35 ± 17.83 |

The experimental results (Table 5) showed that the oral half-life of compound 61 was 5.11 ± 0.31 h, and the bioavailability of the compound was 86.35% ± 17.83%. This indicates that compound 61 has good pharmacokinetic properties.

### Example 213

### Pharmacokinetic Evaluation of Compound 163 in Rats

Reference was made to Example 212 for the experimental procedures. The experimental results are shown in Table 6.

**Table 6. Pharmacokinetic parameters for intravenous injection and oral administration of compound 163 in rats**

| Route of administration | Intravenous injection | Oral administration |
|---|---|---|
| Half-life T_{1/2} (h) | 12.71 ± 8.67 | 6.86 ± 0.34 |
| Time-to-peak Tₘₐₓ (h) | 0.08 ± 0.00 | 0.25 ± 0.00 |
| Peak concentration Cmax (ng/mL) | 10864.30 ± 719.85 | 5474.28 ± 1232.94 |
| Area under the curve AUC_{(0-∞)} (h*ng/mL) | 15851.97 ± 3557.93 | 39483.83 ± 5363.60 |
| Bioavailability F (%) | | 53.24 ± 6.87 |

The experimental results (Table 6) showed that the oral half-life of compound 163 was 6.86 ± 0.34 h, and the bioavailability of the compound was 53.24% ± 6.87%. This indicates that compound 163 has relatively good pharmacokinetic properties.

The above results indicate that compounds 57, 61, and 163 of the present invention have good pharmacokinetic properties. Some other compounds of the present invention also have similarly good pharmacokinetic properties.

### Example 214

### Effect of Compounds on Lipid Metabolism-Associated Gene Expression of HepG2 Cells

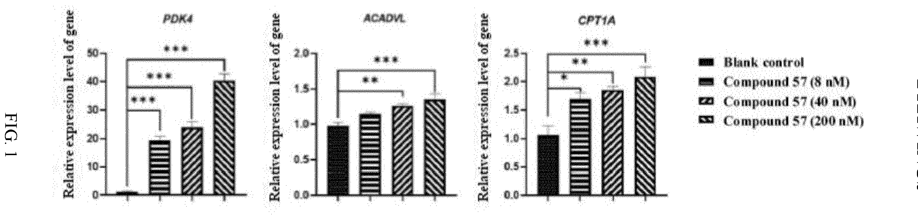

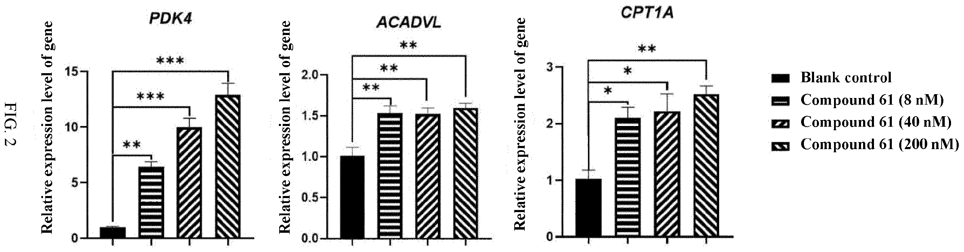

HepG2 cells (human hepatoma cells) were cultured in a 6-well cell culture plate and subjected to adherent culture overnight. Compound 57 or compound 61 was prepared into 8 nM, 40 nM, and 200 nM drug-containing media with a complete medium. The old medium was discarded and replaced with drug-containing medium of different concentrations (complete medium without the compound for the blank control group). After dosing, the cells were incubated for 12 h. The medium was discarded and total RNA was extracted by the phenol-chloroform method. After reverse transcription, the real-time quantitative fluorescent PCR experiment (RNA extraction, reverse transcription, quantitative PCR reagents were all purchased from Nanjing Vazyme Biotech Co., Ltd.) was performed using *ACTB* as an internal reference, and the expression of *PDK4, ACADVL,* and *CPT1A*, which are important genes in oxidative metabolism of lipids, was detected according to the steps in the product instructions (the primer sequences are shown in Table 7). The original data were processed by the ΔΔCt method and analyzed by plotting using the relevant software, and it was found that compound 57 and compound 61 could improve the expression of *PDK4, ACADVL,* and *CPT1A* in a dose-dependent manner with significance (shown in FIGs. 1 and 2). This indicates that compounds 57 and 61 have the ability to increase the oxidative metabolism of liver lipids, which can reduce liver fat accumulation. Some other compounds of the present invention also have similar activity.

**Table 7. Primer sequences for genes**

| Primer name | Primer sequence |
|---|---|
| Homo_*PDK4_*Forward Primer | GGAAGCATTGATCCTAACTGTGA |
| Homo_*PDK4_*Reverse Primer | GGTGAGAAGGAACATACACGATG |
| Homo_*ACADVL_*Forward Primer | ACAGATCAGGTGTTCCCATACC |
| Homo_*ACADVL*_Reverse Primer | CTTGGCGGGATCGTTCACTT |
| Homo_*CPT1A_*Forward Primer | ATCAATCGGACTCTGGAAACGG |
| Homo_*CPT1A*_Reverse Primer | TCAGGGAGTAGCGCATGGT |
| Homo_*ACTB_*Forward Primer | CATGTACGTTGCTATCCAGGC |
| Homo_*ACTB_*Reverse Primer | CTCCTTAATGTCACGCACGAT |

### Example 215

### Effect of Compound 57 on Lipopolysaccharide (LPS)-Induced Inflammation-Associated Gene Expression of THP1 Cells

THP1 cells (human monocytes) were cultured in a 6-well cell culture plate in a medium containing 100 ng/mL phorbol ester (purchased from MCE) and stimulated for differentiation for 48 h. The medium was replaced with a fresh complete medium for further culture for 24 h, and the adherent monocyte-derived macrophages were obtained, which were used for an anti-inflammatory assay on compound 57.

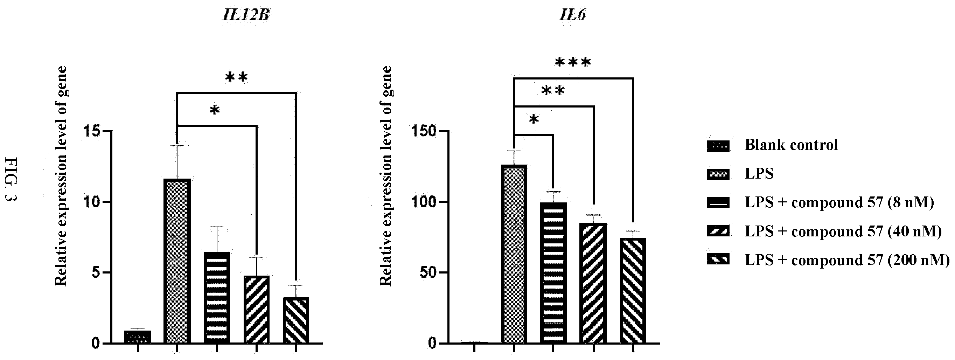

The medium was replaced with a fresh complete medium for the blank control group, the medium was replaced with a complete medium containing 1 mg/mL LPS for the LPS group, and the medium was replaced with 8, 40, and 200 nM drug-containing media prepared with a complete medium containing 1 mg/mL LPS for the administration group. After 6 h of treatment, the medium was discarded. Quantitative PCR was performed according to the procedures in Example 207 to detect the expression of *IL6* and *IL12B,* which are inflammatory factors playing an important role in inflammatory response (the primer sequences are shown in Table 8). The experimental results showed that the compound 57 could resist LPS-induced up-regulation *of IL6* and *IL12B* of macrophages. This indicates that compound 57 has a certain anti-inflammatory effect (as shown in FIG. 3). Some other compounds of the present invention also have similar anti-inflammatory activity.

**Table 8. Primer sequences for genes**

| Primer name | Primer sequence |
|---|---|
| Homo_*IL-6_*Forward Primer | ACTCACCTCTTCAGAACGAATTG |
| Homo_*IL-6_*Reverse Primer | CCATCTTTGGAAGGTTCAGGTTG |
| Homo_*IL-12B_*Forward Primer | TGCCCATTGAGGTCATGGTG |
| Homo_*IL-12B_*Reverse Primer | CTTGGGTGGGTCAGGTTTGA |

### Example 216

### Protective Effect of Compound 61 on Choline-Deficient, L-Amino Acid-Defined Diet (CDAA)-Induced NASH Mouse Model

Animals: 70 male C57 mice, SPF grade, 8 weeks old, weighing about 20 g, purchased from Beijing Vital River Laboratory Animal Technology Co., Ltd. All animals were kept on a 12-h alternating circadian rhythm and were fed *ad libitum.*
Instrument: weighing scale for animals; microtome; automatic biochemical analyzer; inverted microscope
Reagent: compound 61, positive drug GFT505 (PPARα/δ dual agonist, currently in an anti-NASH phase III clinical trial), and positive drug IVA337 (Pan-PPAR agonist, currently in an anti-NASH phase II clinical trial), which were prepared according to the method in the literature (CN100548960C and J.Med. Chem., 2018, 61, 2246); the control feed was purchased from Nantong Trophic (TP36225 MCS); the modeling feed was purchased from Nantong Trophic (TP36225 MCD).

### Experimental procedures:

### 1. Animal grouping and modeling

After 1 week of adaptive feeding of mice, the mice were randomly divided into 7 groups by weight: a control group (MCS), a model group (CDAA), a positive drug GFT505 (10 mg/kg) group (CDAA+ GFT505), a positive drug IVA337 (10 mg/kg) group (CDAA+ IVA337), a compound 61 low dose (3 mg/kg) group (CDAA + 61 low dose), a compound 61 medium dose (10 mg/kg) group (CDAA + 61 medium dose), and a compound 61 high dose (30 mg/kg) group (CDAA + 61 high dose). The control group was fed the control feed (TP36225 MCS); the other groups were given the modeling feed (TP36225 MCD). The mice were all fed water normally and subjected to modeling for 3 weeks.

### 2. Administration

After 3 weeks of modeling, the MCS and CDAA groups were given a 0.5% CMC-Na solution by intragastric administration every day (the administration volume was 10 mL/kg), the CDAA+ GFT505 group was given a 0.5% CMC-Na solution of GFT505 by intragastric administration every day (GFT505 was administered at 10 mg/kg, and the administration volume was 10 mL/kg), the CDAA + IVA337 group was given a 0.5% CMC-Na solution of IVA337 by intragastric administration every day (IVA337 was administered at 10 mg/kg, and the administration volume was 10 mL/kg), the CDAA + 61 low dose group was given a 0.5% CMC-Na solution of compound 61 by intragastric administration every day (compound 61 was administered at 3 mg/kg, and the administration volume was 10 mL/kg), the CDAA + 61 medium dose group was given a 0.5% CMC-Na solution of compound 61 by intragastric administration every day (compound 61 was administered at 10 mg/kg, and the administration volume was 10 mL/kg), and the CDAA + 61 high dose group was given a 0.5% CMC-Na solution of compound 61 by intragastric administration every day (compound 61 was administered at 30 mg/kg, and the administration volume was 10 mL/kg). The administration was performed for 6 weeks, during which time the MCS group was given the control feed and the other groups were given the modeling feed, and the mice were all fed water normally. The mice in each group were weighed daily, and their body weight, hair, feces, and activity were carefully observed and recorded.

### 3. Sampling

Mice were fasted but given free access to water for 12 h in advance, blood was taken from the orbit in the morning the next day, and then the liver was taken after the mice were sacrificed. The right lobular tissue of liver was fixed with 4% paraformaldehyde and used for HE staining of sections. Part of liver tissues were divided into 3 parts and were quickly frozen in liquid nitrogen for subsequent detection of other indexes.

### 4. Determination of biochemical indexes

The whole blood was left to stand at room temperature for 2 h and centrifuged at 3000 rpm for 15 min, and serum was collected. The levels of aspartate transaminase (AST) and alanine aminotransferase (ALT) in serum were determined by an automatic biochemical analyzer of ServiceBio Biotechnology Co., Ltd.

### 5. Liver tissue section

The pretreated tissues were sent to ServiceBio Biotechnology Co., Ltd. to make HE stained sections, Sirius red stained sections, and oil red stained sections.

### 6. Extraction and Western Blot (WB) detection of liver tissue protein

The liver tissue stored at -80 °C was taken out and placed in liquid nitrogen, about 10 mg of liver tissue was rapidly cut off, homogenized with a homogenizer after about 500 µL of precooled tissue lysis buffer was added, and then lysed on ice for 30 min. The lysate was centrifuged at 12,000 rpm for 15 min at 4 °C, and 170 µL of supernatant was added to a clean EP tube, from which 10 µL of supernatant was taken for BCA protein quantification. 40 µL of 5*loading buffer was added to the rest of protein supernatant for boiling for denaturation, followed by SDS-PAGE electrophoresis, and the protein was transferred onto a PVDF membrane for incubation and elution of related antibodies. Finally, imaging analysis was performed by using a chemiluminescence apparatus.

### 7. Extraction and q-PCR detection of liver tissue RNA

The liver tissue stored at -80 °C was taken out and placed in liquid nitrogen, about 10 mg of liver tissue was rapidly cut off, homogenized with a homogenizer after about 500 µL of precooled RNA extraction reagent was added, and then lysed on ice for 15 min. The lysate was shaken vigorously for 15 s after 100 µL of chloroform was added, left to stand on ice for 10 min, and centrifuged at 12,000 rpm for 15 min at 4 °C. The upper aqueous phase was transferred to a clean 1.5 mL EP tube, and 200 µL of isopropanol was added to precipitate RNA. After being left to stand on ice for 10 min, the mixture was centrifuged at 12,000 rpm for 10 min at 4 °C. The supernatant was discarded, the precipitate was washed once with 75% ethanol and centrifuged to remove the supernatant, and the water-soluble RNA precipitate was treated with 40 µL of DEPC. The RNA concentration was quantified using Nano, a reverse transcription reagent from Takara was added according to the instructions, and mRNA was reverse-transcribed into cDNA using a common PCR instrument. Finally, the upstream and downstream primers for the target gene, q-PCR reagent (SYBR Green), and cDNA were added to a 96-well plate dedicated to q-PCR, and amplification and quantification were performed using a q-PCR instrument. The ΔΔCt value was selected to characterize differences in gene expression, and relevant software was adopted to perform data processing and statistical test.

### 8. Experimental results

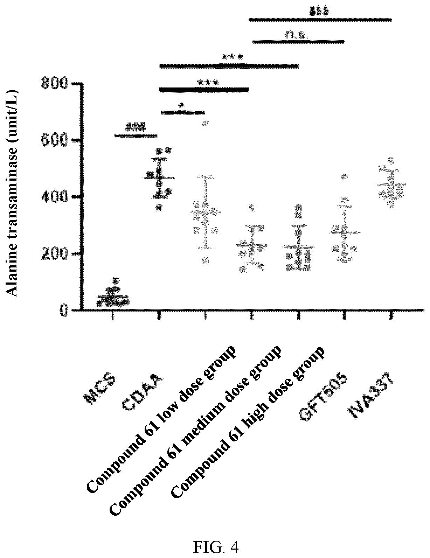

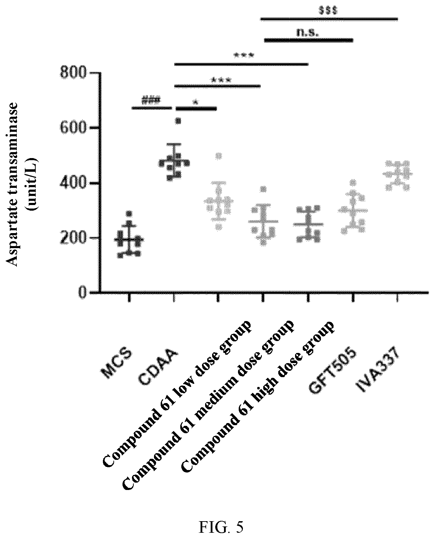

The results in FIGs. 4 and 5 showed that compound 61 could down-regulate the levels of ALT and AST in serum of NASH model mice in a dose-dependent manner. Notably, compound 61 had a stronger liver enzyme-lowering effect than GFT505 and IVA337 at the same dose. This indicates that compound 61 has a stronger protective effect on the liver than GFT505 and IVA337 in the NASH model.

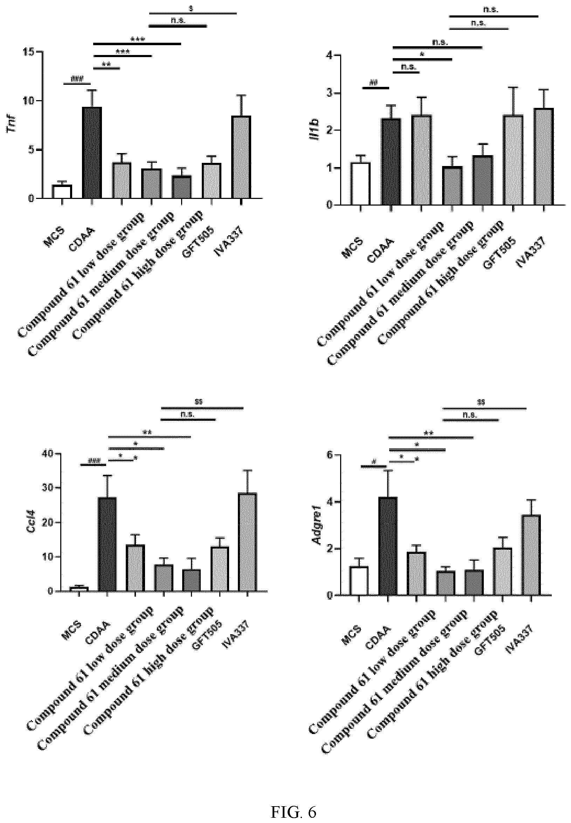

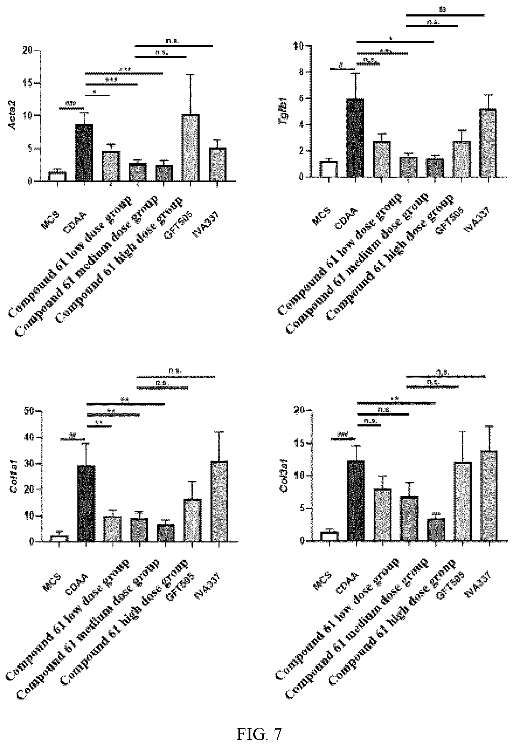

To further detect the effect of compound 61 on reducing liver inflammation and fibrosis in NASH model mice, mRNA expression of the relevant inflammatory factors and fibrosis-associated cytokines in liver tissue was determined (primer sequences for genes are shown in Table 9). The experimental results are shown in FIGs. 6 and 7.

**Table 9. Primer sequences for genes**

| Primer name | Primer sequence |
|---|---|
| Mus_*Tnf_*Forward Primer | CCCTCACACTCAGATCATCTTCT |
| Mus_*Tnf_*Reverse Primer | GCTACGACGTGGGCTACAG |
| Mus_*Il1b_*Forward Primer | TGCCCATTGAGGTCATGGTG |
| Mus_*Il1b_*Reverse Primer | CTTGGGTGGGTCAGGTTTGA |
| Mus_*Ccl4_*Forward Primer | TTCCTGCTGTTTCTCTTACACCT |
| Mus_*Ccl4_*Reverse Primer | CTGTCTGCCTCTTTTGGTCAG |
| Mus_*Adgre1_*Forward Primer | CCCCAGTGTCCTTACAGAGTG |
| Mus*_Adgre1_*Reverse Primer | GTGCCCAGAGTGGATGTCT |
| Mus_*Acta2_*Forward Primer | GTCCCAGACATCAGGGAGTAA |
| Mus_*Acta2*_Reverse Primer | TCGGATACTTCAGCGTCAGGA |
| Mus_*tgfb1_*Forward Primer | CTCCCGTGGCTTCTAGTGC |
| Mus_*tgfb1_*Reverse Primer | GCCTTAGTTTGGACAGGATCTG |
| Mus_*Col1a1_*Forward Primer | GCTCCTCTTAGGGGCCACT |
| Mus_*Col1a1_*Reverse Primer | CCACGTCTCACCATTGGGG |
| Mus_*Col3a1_*Forward Primer | CTGTAACATGGAAACTGGGGAAA |
| Mus_*Col3a1_*Reverse Primer | CCATAGCTGAACTGAAAACCACC |

The results in FIG. 6 showed that compound 61 could inhibit the increase of mRNA expression levels of *Tnf, Illb, Ccl4,* and *Adgrel* due to modeling in a dose-dependent manner, and that compound 61 had a better effect than GFT505 and IVA337 at the same dose. This indicates that compound 61 has a stronger effect in resisting liver inflammation than GFT505 and IVA337 in the NASH model. The results in FIG. 7 showed that compound 61 could significantly inhibit the increase of mRNA expression levels of *Acta2, Tgfbl, Col1a1,* and *Col3a1* due to modeling, and that compound 61 had a better effect than GFT505 and IVA337 at the same dose. This indicates that compound 61 has a stronger effect in resisting liver fibrosis than GFT505 and IVA337 in the NASH model.

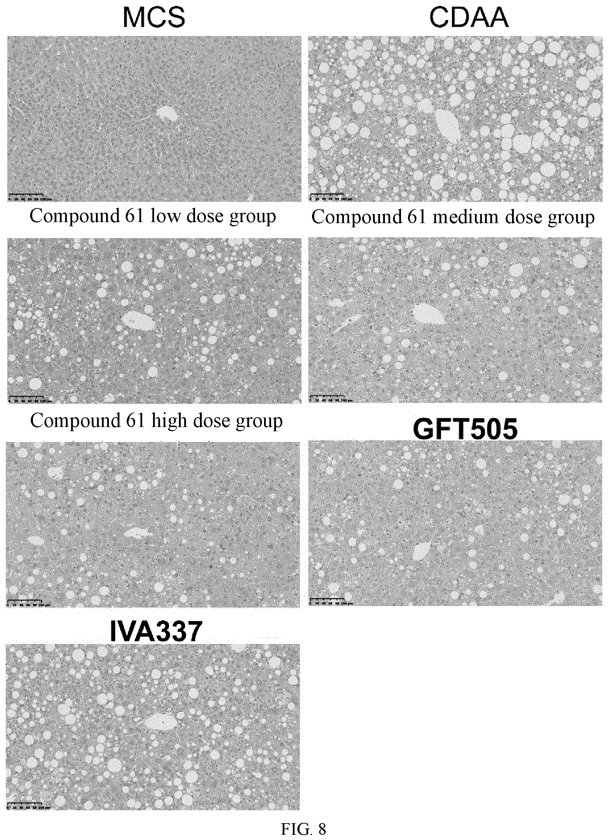

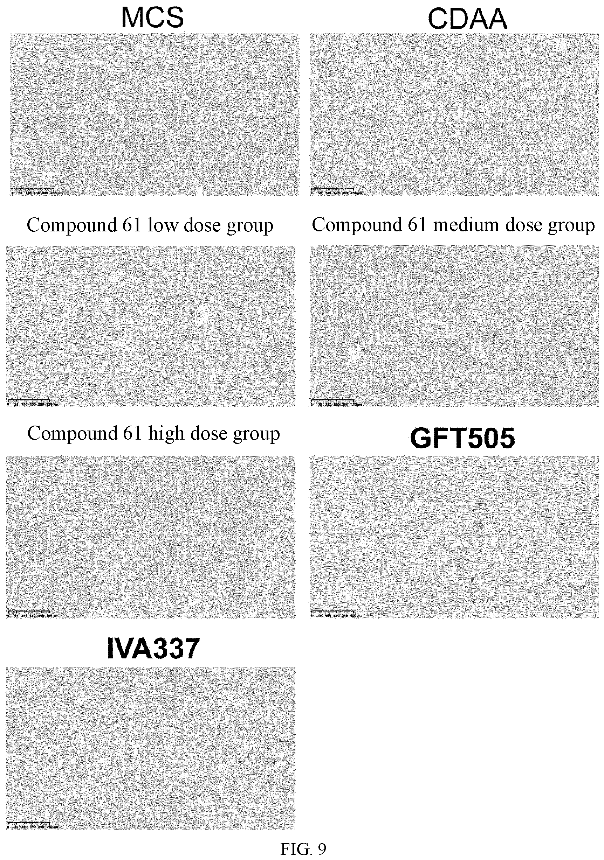

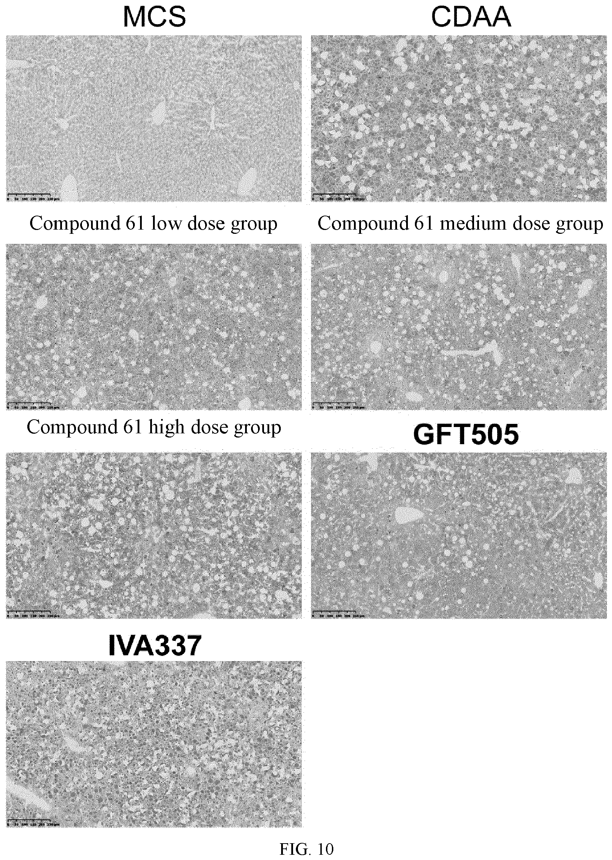

In addition, the anti-NASH effect of compound 61 was evaluated by means of pathological studies. HE staining results (FIG. 8) showed that compound 61 could reduce the infiltration of inflammatory cells in the portal area due to modeling in a dose-dependent manner, and that compound 61 had a better effect than GFT505 and IVA337 at the same dose. Sirius red staining results (FIG. 9) showed that compound 61 could reduce collagen deposition due to modeling in a dose-dependent manner, and resist liver fibrosis during NASH, and that compound 61 had a better effect than GFT505 and IVA337 at the same dose. Oil red staining results (FIG. 10) showed that compound 61 could reduce lipid accumulation due to modeling in a dose-dependent manner.

The above results showed that compound 61 could reduce the aminotransferase level in serum of NASH model mice in a dose-dependent manner, and inhibit the liver inflammatory response and liver fibrosis, and had better efficacy than positive control drugs GFT505 and IVA337 at the same dose. This suggests that compound 61 can be used for the prevention and treatment of chronic liver diseases such as fatty liver disease (particularly, nonalcoholic steatohepatitis), chronic hepatitis, liver fibrosis, and the like. Some other compounds of the present invention also have similar effects.

### Example 217

### Protective Effect of Compound 61 on Carbon Tetrachloride-Induced Liver Fibrosis Mouse Model

Animals: 60 male C57 mice, SPF grade, 8 weeks old, weighing about 20 g, purchased from Beijing Vital River Laboratory Animal Technology Co., Ltd. All animals were kept on a 12-h alternating circadian rhythm and were fed *ad libitum.*
Instrument: weighing scale for animals; microtome; automatic biochemical analyzer; inverted microscope
Reagent: positive drug IVA337 (Pan-PPAR agonist, currently in an anti-NASH phase II clinical trial), which was prepared according to the method in the literature *(*J. Med. Chem., 2018, 61, 2246); carbon tetrachloride (purchased from Shanghai Aladdin Biochemical Technology Co., Ltd.), and sunflower seed oil (purchased from Shanghai Yuanye Biotech Co., Ltd.).

### Experimental procedures:

### 1. Animal grouping and modeling

After 1 week of adaptive feeding of mice, the mice were randomly divided into 6 groups by weight: a control group (Oil), a model group (CCl₄), a positive drug IVA337 (10 mg/kg) group (CCl₄ + IVA337), a compound 61 low dose (3 mg/kg) group (CCl₄ + 61 low dose), a compound 61 medium dose (10 mg/kg) group (CCl₄ + 61 medium dose), and a compound 61 high dose (30 mg/kg) group (CCl₄ + 61 high dose). Mice were given food and water normally and subjected to modeling for 3 weeks. The model group and the administration groups were each injected twice a week with a 25% CCl₄ oil solution at a dose of 2 mL/kg, and the control group was injected with an oil solvent at the same volume.

### 2. Administration

The administration was started at the same time as the modeling, the control group and the model group were given a 0.5% CMC-Na solution by intragastric administration every day (the administration volume was 10 mL/kg), the CCl₄ + IVA337 group was given a 0.5% CMC-Na solution of IVA337 by intragastric administration every day (IVA337 was administered at 10 mg/kg, and the administration volume was 10 mL/kg), the CCl₄ + 61 low dose group was given a 0.5% CMC-Na solution of compound 61 by intragastric administration every day (compound 61 was administered at 3 mg/kg, and the administration volume was 10 mL/kg), the CCl₄ + 61 medium dose group was given a 0.5% CMC-Na solution of compound 61 by intragastric administration every day (compound 61 was administered at 10 mg/kg, and the administration volume was 10 mL/kg), and the CCl₄ + 61 high dose group was given a 0.5% CMC-Na solution of compound 61 by intragastric administration every day (compound 61 was administered at 30 mg/kg, and the administration volume was 10 mL/kg). The administration was performed for 3 weeks, and the mice were given food and water normally. The mice in each group were weighed daily, and their body weight, hair, feces, and activity were carefully observed and recorded.

### 3. Sampling

At 30 h after the sixth injection of CCl₄, the mice were dissected for the samples. Blood was taken from the orbit, and then the liver was taken after the mice were sacrificed. The right lobular tissue of liver was fixed with 4% paraformaldehyde and used for HE staining of sections. Part of liver tissues were divided into 3 parts and were quickly frozen in liquid nitrogen for subsequent detection of other indexes.

### 4. Liver tissue section

The pretreated tissues were sent to ServiceBio Biotechnology Co., Ltd. to make HE stained sections and Sirius red stained sections.

### 5. Extraction and Western Blot (WB) detection of liver tissue protein

The liver tissue stored at -80 °C was taken out and placed in liquid nitrogen, about 10 mg of liver tissue was rapidly cut off, homogenized with a homogenizer after about 500 µL of precooled tissue lysis buffer was added, and then lysed on ice for 30 min. The lysate was centrifuged at 12,000 rpm for 15 min at 4 °C, and 170 µL of supernatant was added to a clean EP tube, from which 10 µL of supernatant was taken for BCA protein quantification. 40 µL of 5*loading buffer was added to the rest of protein supernatant for boiling for denaturation, followed by SDS-PAGE electrophoresis, and the protein was transferred onto a PVDF membrane for incubation and elution of related antibodies. Finally, imaging analysis was performed by using a chemiluminescence apparatus.

### 6. Detection of hydroxyproline in liver tissue.

The liver tissue stored at -80 °C was taken out and placed in liquid nitrogen, about 200 mg of the liver tissue was rapidly cut off, and hydroxyproline in the liver tissue was detected according to the method in the product instructions (Beijing Solarbio Science & Technology Co., Ltd., BC0255).

### 7. Experimental results

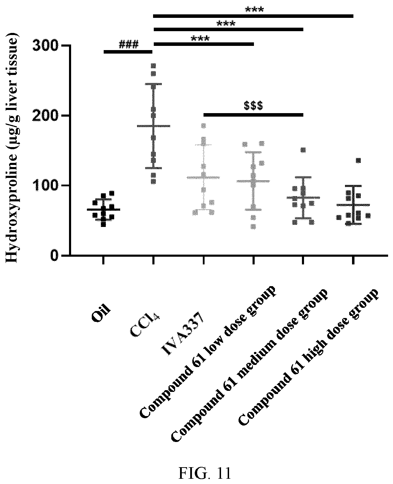

The results in FIG. 11 showed that compound 61 could down-regulate the hepatic hydroxyproline level of liver fibrosis model mice in a dose-dependent manner. Notably, compound 61 had a stronger effect than IVA337 at the same dose. This indicates that compound 61 has a stronger antifibrotic effect than IVA337 in the liver fibrosis model.

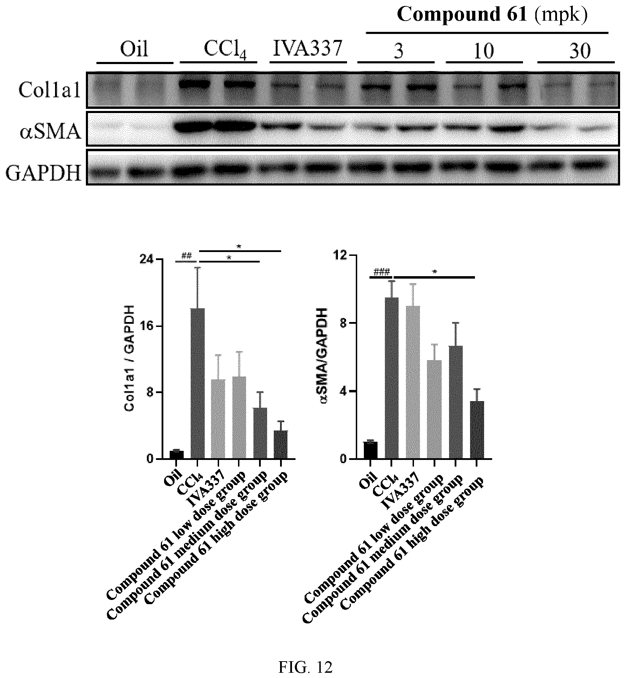

To further detect the effect of compound 61 on reducing liver fibrosis in liver fibrosis model mice, the expression of fibrosis-associated proteins in liver tissue was determined. The results in FIG. 12 showed that compound 61 could inhibit the increase of αSMA and Col1a1 protein levels due to modeling in a dose-dependent manner, and that compound 61 had a better effect than IVA337 at the same dose. This indicates that compound 61 had a stronger effect in resisting liver fibrosis than IVA337 in the NASH model.

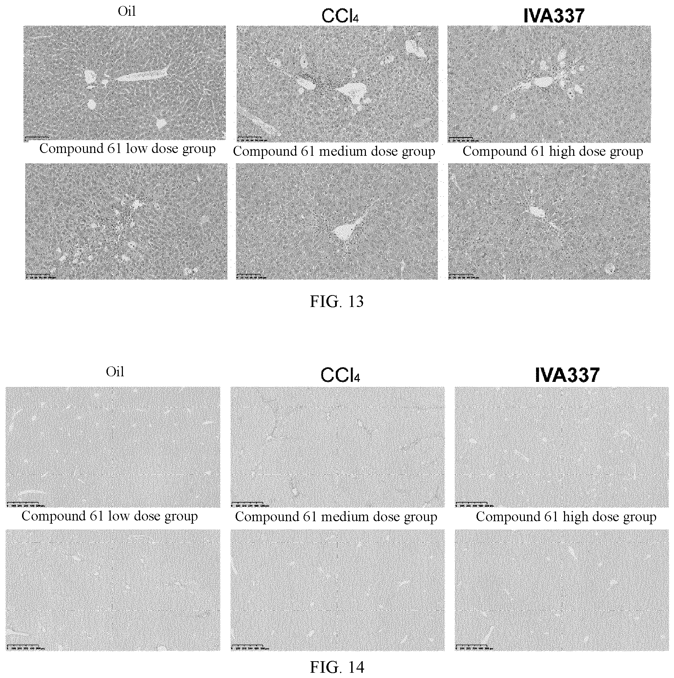

In addition, the anti-liver fibrosis effect of compound 61 was evaluated by pathology. HE staining results (FIG. 13) showed that compound 61 could reduce the infiltration of inflammatory cells in the portal area due to modeling in a dose-dependent manner, and that compound 61 had a better effect than IVA337 at the same dose. Sirius red staining results (FIG. 14) showed that compound 61 could reduce collagen deposition due to modeling in a dose-dependent manner, and resist liver fibrosis, and that compound 61 had a better effect than IVA337 at the same dose.

The above results showed that compound 61 could reduce the hepatic hydroxyproline level of liver fibrosis model mice in a dose-dependent manner, reduce the levels of fibrosis-associated proteins αSMA and Col1a1, and inhibit the liver inflammatory response and liver fibrosis, and had better efficacy than a positive control drug IVA337 at the same dose. This suggests that compound 61 can be used for the prevention and treatment of chronic liver diseases such as liver fibrosis. Some other compounds of the present invention also have similar effects.

### Example 218

### Regulation of PPARα/δ Downstream Target Gene in Mouse Liver and Skeletal Muscles by Compound 61

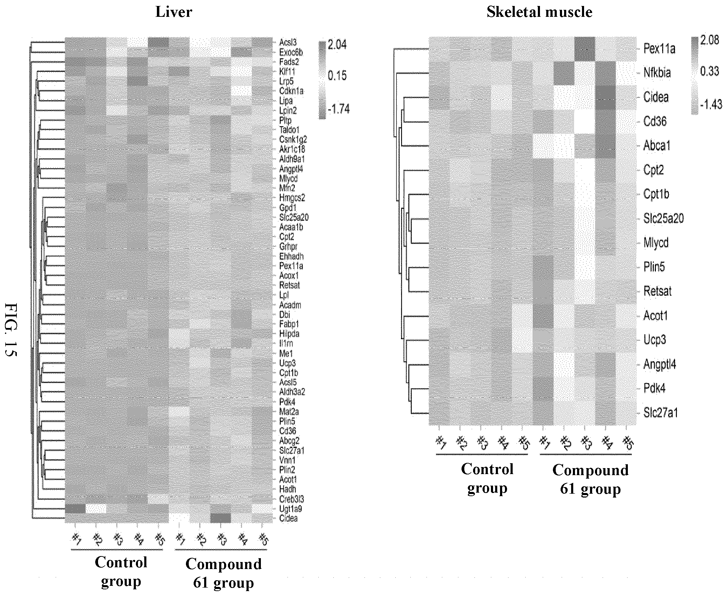

C57 mice were divided into two groups of a control group and an administration group, with 6 mice in each group. The administration group was given compound 61 (10 mg/kg) by intragastric administration for four consecutive days, and the control group was given the same volume of solvent control. After the fourth day of administration, the mice were euthanized and dissected for the samples. The liver and skeletal muscles were quickly frozen in liquid nitrogen for subsequent experiments. RNA from liver and skeletal muscles was extracted and then libraries were constructed for transcriptome sequencing. The results (FIG. 15) showed that 50 target genes were significantly upregulated in the liver and 16 target genes were significantly upregulated in the skeletal muscle. This suggests that compound 61 can have a dual agonistic effect on PPARα/δ *in vivo.*

### Example 219

### Study on Selectivity of Compound 61 for Nuclear Receptors

The ligand binding domains of part of human nuclear receptor proteins were each cloned into a pBIND vector to construct Gal4 hybridized reporter gene plasmids for compound selectivity study.

Plasmid construction: the LBD region sequences (hPPARα (279aa-580aa); hPPARβ (274aa-576aa); hPPARy (281aa-554aa); hRARα (177aa-462aa); hRARy (179aa-454aa); hRARβ (177aa-455aa); hFXR (193aa-486aa); hRXRα (225aa-462aa); hRXRy (229aa-463aa); hRXRβ (294aa-533aa); hVDR (119aa-427aa); hLXRα (187aa-447aa); hLXRβ (199aa-461aa); hTHβ (202aa-461aa); hPXR (138aa-434aa); hCAR (48aa-324aa)) of corresponding nuclear receptors were each cloned into a pBIND vector to construct corresponding expression plasmids (pBIND-Gal4-PPARα(LBD); pBIND-Gal4-PPARδ(LBD); pBIND-Gal4-PPARy(LBD); pBIND-Gal4-RARα(LBD); pBIND-Gal4-RARy(LBD); pBIND-Gal4-RARβ(LBD); pBIND-Gal4-FXR(LBD); pBIND-Gal4-RXRα(LBD); pBIND-Gal4-RXRγ(LBD); pBIND-Gal4-RXRβ(LBD); pBIND-Gal4-VDR(LBD); pBIND-Gal4-LXRα(LBD); pBIND-Gal4-LXRβ(LBD); pBIND-Gal4-THβ(LBD); pBIND-Gal4-PXR(LBD); pBIND-Gal4-CAR(LBD)) for fusion proteins of Gal4 and nuclear receptor LBD regions. pGL4.35-9×Gal4 UAS plasmid (purchased from Promega (Beijing) Biotech Co., Ltd.).

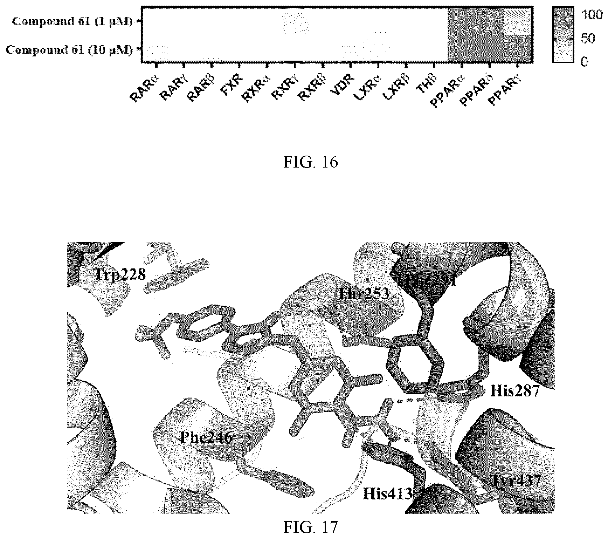

Cos-7 cells (African green monkey kidney fibroblasts, commonly used tool cells) were cultured in a 10 cm cell culture dish in a DMEM complete medium containing 10% fetal bovine serum. When the cells grew to a density of about 70%, the medium was replaced with a fresh medium for later transfection. The procedures for preparing the plasmid working fluid were as follows: 15 µg of pBIND-Gal4-PPARα(LBD) plasmid or pBIND-Gal4-PPARδ(LBD) plasmid or pBIND-Gal4-PPARy(LBD) plasmid or pBIND-Gal4-RARα(LBD) plasmid or pBIND-Gal4-RARy(LBD) plasmid or pBIND-Gal4-RARβ(LBD) plasmid or pBIND-Gal4-FXR(LBD) plasmid or pBIND-Gal4-RXRα(LBD) plasmid or pBIND-Gal4-RXRy(LBD) plasmid or pBIND-Gal4-RXRβ(LBD) plasmid or pBIND-Gal4-VDR(LBD) plasmid or pBIND-Gal4-LXRα(LBD) plasmid or pBIND-Gal4-LXRβ(LBD) plasmid or pBIND-Gal4-THβ(LBD) plasmid or pBIND-Gal4-PXR(LBD) plasmid or pBIND-Gal4-CAR(LBD) plasmid, 15 µg of pGL4.35-9×Gal4 UAS plasmid (purchased from Promega (Beijing) Biotech Co., Ltd.), and 60 µL of transfection reagent (HighGene, purchased from Wuhan ABclonal Technology Co., Ltd.) were added to 2 mL of Opti-MEM, and the mixture was left to stand at room temperature for 15 min to obtain a working solution. The plasmid working solution was then added to the cell culture dish for cell transfection. After 4 h of transfection, the cells were washed with PBS, digested with trypsin, seeded into a 96-well plate with 20,000-30,000 cells in each well, and subjected to adherent culture for 24 h. Compound 61 was prepared into the appropriate test concentration with a complete medium and then added to the 96-well plate. Meanwhile, the PPARα agonistic activity was defined as 100% for GW7647 (purchased from MCE) at a final concentration of 10 nM, the PPARδ agonistic activity was defined as 100% for GW501516 (purchased from MCE) at a final concentration of 10 nM, the PPARγ agonistic activity was defined as 100% for Rosiglitazone (purchased from Adamas) at a final concentration of 1 µM, the RARs agonistic activity was defined as 100% for Tretinoin (purchased from MCE) at a final concentration of 1 µM, the RXRs agonistic activity was defined as 100% for Bexarotene (purchased from MCE) at a final concentration of 1 µM, the FXR agonistic activity was defined as 100% for GW4064 (purchased from Beyotime) at a final concentration of 1 µM, the VDR agonistic activity was defined as 100% for Doxercalciferol (purchased from MCE) at a final concentration of 1 µM, the LXRs agonistic activity was defined as 100% for T0901317 (purchased from MCE) at a final concentration of 1 µM, the Thβ agonistic activity was defined as 100% for T3 (purchased from MCE) at a final concentration of 1 µM, the PXR agonistic activity was defined as 100% for SR12813 (purchased from MCE) at a final concentration of 1 µM, and the CAR agonistic activity was defined as 100% for CITCO (purchased from Glpbio) at a final concentration of 1 µM. After 16 h of drug action, the medium was discarded, and 100 µL of reporter gene lysis buffer (purchased from Shanghai Beyotime Biological Tech. Co., Ltd.) was added to lyse the cells for 15 min. 10 µL of lysate was pipetted into a white opaque 384-well plate, and then 10 µL reporter gene assay buffer (purchased from Shanghai Beyotime Biological Tech. Co., Ltd.) was added. The bioluminescence was detected by using a multimode microplate reader, and the corresponding relative agonistic rate was calculated according to the detection value. The experimental results are shown in FIG. 16. Compound 61 had no significant agonistic effect on nuclear receptors other than PPAR and also had a relatively weak agonistic effect on PPARγ under the condition of 1 µM, so it was considered that compound 61 had high selectivity for the nuclear receptor PPARα/δ. The other compounds of the present invention also have similar effects.

### Example 220

### Eutectic Structure of Complex of Compound 61 with hPPARδ-LBD

The protein required for crystallization was first expressed. The vector containing the human PPARδ ligand binding domain (amino acid residues 173-441) was transformed into BL21 cells to express the protein. After purification using nickel and anion columns, the recombinant protein was dissolved in a solution of 20 mM Tris, pH 8.0, 150 mM NaCl, and 10% glycerol. Subsequently, compound 61 at 2 mM was added to the above purified protein (hPPARδ-LBD) at 7 mg/mL. The eutectic crystal of the complex of compound 61 with hPPARδ-LBD grew at 16 °C, and the crystallization solvent was a mixture of 0.5 M sodium citrate, pH 5.5, 19% PEG3350, and 20% glycerol. The crystal was quickly frozen in liquid nitrogen for data collection. X-ray diffraction data were collected on beam line BL02U at the Shanghai Synchrotron Radiation Facility with the help of the X-ray crystallography facility platform at the National Protein Research Facility Base (Tsinghua University). Data were processed with HKL2000. The structure was solved by molecular replacement using the Phenix program, and the search model was PDB code 3SP950. This model was constructed using coot and refined using the program PHENIX. The experimental results are shown in FIG. 17. The binding pocket was surrounded by some residues including Trp228, Phe246, Thr253, His287, Phe291, His413, and Tyr437. Compound 61 showed the classic binding conformation of the PPARδ agonist in the pocket. By mimicking endogenous fatty acids, key hydrogen bonding interactions between the carboxylic acid group of compound 61 and the three key amino acid residues His287, His413, and Tyr437 were observed. In contrast to the crystal binding patterns of other PPARδ agonists, a "water bridge" was formed between the carbonyl O atoms of triazolone and Thr253. This unique PPARδ-agonist interaction is probably the main reason for the better potency and selectivity of compound 61 for PPARδ.

### Example 221

### Tablet

Compound 61 (50 g) obtained in Example 61, hydroxypropylmethylcellulose E (150 g), starch (200 g), an appropriate amount of povidone K30, and magnesium stearate (1 g) were mixed, granulated, and tableted.

In addition, the compounds prepared in Examples 1-208 can be prepared into capsules, powders, granules, pills, injections, syrups, oral liquids, inhalants, ointments, suppositories, patches, or the like, with various pharmaceutical excipients according to the conventional preparation method of Chinese Pharmacopoeia 2015.

## Claims

1. A triazolone compound of formula (I) or a pharmaceutically acceptable salt or a solvate thereof: 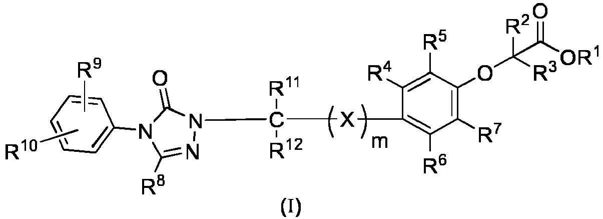
R¹ is selected from: H, linear or branched C1-C6 alkyl, C3-C6 cycloalkyl, (CH₂)ₚOR¹⁴, or (CH₂)_{q}NR¹⁵; wherein p = any integer from 2 to 6; q = any integer from 2 to 6; R¹⁴ and R¹⁵ are each independently selected from H, R¹⁶, or C(O)R¹⁷; wherein R¹⁶ and R¹⁷ are each independently selected from linear or branched C1-C6 alkyl or C3-C6 cycloalkyl;
R² and R³ are each independently selected from: H or linear or branched C1-C4 alkyl; or R² and R³, together with the carbon atoms to which they are bonded, form a 3- to 6-membered cycloalkyl ring;
R⁴, R⁵, R⁶, and R⁷ are each independently selected from: H, halogen, OR¹³, hydroxy, linear or branched C1-C4 alkyl, trifluoromethyl, methylthio, trifluoromethoxy, trifluoromethylthio, C3-C6 cycloalkyl, cycloalkenyl, heterocycloalkyl, heterocycloalkenyl, alkynyl, phenyl, substituted phenyl, heteroaryl, substituted heteroaryl, fused aryl, or substituted fused aryl; or at least two substituents of R⁴, R⁵, R⁶, and R⁷, together with the atoms to which they are attached, may form a substituted or unsubstituted benzene ring, a substituted or unsubstituted heteroaromatic ring, a substituted or unsubstituted cycloalkane ring, a substituted or unsubstituted heterocycloalkane ring, or a substituted or unsubstituted heterocycloalkene ring;
R¹³ is selected from: linear or branched C1-C4 alkyl, C3-C6 cycloalkyl, hydroxyalkyl, alkoxyalkyl, alkoxyalkoxyalkyl, cycloalkyl, or alkynylalkoxyalkyl;
X is selected from CH₂, O, or S;
m is selected from any integer from 0 to 4;
R⁸ is selected from H or C₁-C₄ alkyl;
R⁹ and R¹⁰ are independently selected from: H, hydroxy, halogen, cyano, linear or branched C1-C4 alkyl, trifluoromethyl, methylthio, trifluoromethoxy, trifluoromethylthio, alkylsulfonyl, alkoxy, cycloalkyl, cycloalkenyl, heterocycloalkyl, heterocycloalkenyl, alkynyl, phenyl, substituted phenyl, phenoxy, substituted phenyloxy, heteroaryl, substituted heteroaryl, fused aryl, or substituted fused aryl, wherein the substituted phenyl may independently be substituted with 1 to 2 of the following substituents: halogen, hydroxy, cyano, linear or branched C1-C4 alkyl, trifluoromethyl, methylthio, trifluoromethoxy, trifluoromethylthio, or alkylsulfonyl; or R⁹ and R¹⁰, together with the atoms to which they are attached, may form a substituted or unsubstituted benzene ring, a substituted or unsubstituted heteroaromatic ring, a substituted or unsubstituted cycloalkane ring, a substituted or unsubstituted heterocycloalkane ring, or a substituted or unsubstituted heterocycloalkene ring;
R¹¹ and R¹² are each independently selected from: H, deuterium, linear or branched C1-C4 alkyl, or halogen; or R¹¹ and R¹², together with the carbon atoms to which they are bonded, form a 3- to 6-membered cycloalkyl ring.

2. The triazolone compound or the pharmaceutically acceptable salt or the solvate thereof according to claim 1, wherein,
R¹ is selected from: H, linear or branched C1-C4 alkyl, alkoxyalkyl, or acetamidoethyl;
R² and R³ are each independently selected from: H or linear or branched C1-C4 alkyl; or R² and R³, together with the carbon atoms to which they are bonded, form a 3- to 6-membered cycloalkyl ring;
R⁴, R⁵, R⁶, and R⁷ are each independently selected from: H, halogen, trifluoromethyl, trifluoromethoxy, trifluoromethylthio, OR¹³, or linear or branched C1-C4 alkyl;
R¹³ is selected from: linear or branched C1-C4 alkyl, hydroxyalkyl, alkoxyalkyl, alkoxyalkoxyalkyl, cycloalkyl, or alkynylalkoxyalkyl;
X is selected from CH₂, O, or S;
m is selected from any integer from 0 to 2;
R⁸ is selected from H or linear or branched C1-C4 alkyl;
R⁹ and R¹⁰ are each independently selected from: H, halogen, cyano, linear or branched C1-C4 alkyl, trifluoromethyl, methylthio, trifluoromethoxy, trifluoromethylthio, alkylsulfonyl, alkoxy, cycloalkyl, cycloalkenyl, heterocycloalkyl, heterocycloalkenyl, alkynyl, phenyl, substituted phenyl, phenoxy, substituted phenyloxy, heteroaryl, substituted heteroaryl, fused aryl, or substituted fused aryl, wherein the substituted phenyl may independently be substituted with 1 to 2 of the following substituents: halogen, cyano, linear or branched C1-C4 alkyl, trifluoromethyl, methylthio, trifluoromethoxy, trifluoromethylthio, or alkylsulfonyl;
R¹¹ and R¹² are each independently selected from: H, deuterium, linear or branched C1-C4 alkyl, or halogen; or R¹¹ and R¹², together with the carbon atoms to which they are bonded, form a 3- to 6-membered cycloalkyl ring.

3. The triazolone compound or the pharmaceutically acceptable salt or the solvate thereof according to claim 1, wherein the triazolone compound further comprises a pharmaceutically acceptable salt, a tautomer, a mesomer, a racemate, a stereoisomer, a metabolite, a metabolic precursor, a prodrug or a solvate thereof.

4. The triazolone compound or the pharmaceutically acceptable salt or the solvate thereof according to any one of claims 1-3, wherein the compound or the pharmaceutically acceptable salt, the tautomer, the mesomer, the racemate, the stereoisomer, the metabolite, the metabolic precursor, the prodrug or the solvate thereof is any one of the following compounds:
| Compound No. | Compound structure | Name |
|---|---|---|
| 1 | 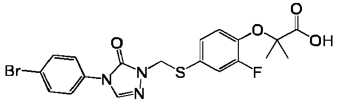 | 2-(4-(((4-(4-Bromophenyl)-5-oxo-4,5-dihydro-1*H*-1,2,4-triazol-1-yl)methyl)thio)-2-fluorophenoxy)-2-methylpropionic acid |
| 2 | 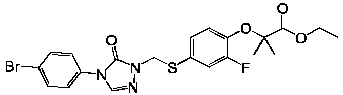 | Ethyl 2-(4-(((4-(4-bromophenyl)-5-oxo-4,5-dihydro-1*H*-1,2,4-triazol-1-yl)methyl)thio)-2-fluorophenoxy)ethyl-2-methylpropionate |
| 3 | 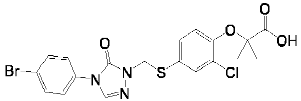 | 2-(4-(((4-(4-Bromophenyl)-5-oxo-4,5-dihydro-1*H*-1,2,4-triazol-1-yl)methyl)thio)-2-chlorophenoxy)-2-methylpropionic acid |
| 4 | 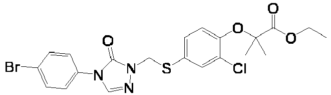 | Ethyl 2-(4-(((4-(4-bromophenyl)-5-oxo-4,5-dihydro-1*H*-1,2,4-triazol-1-yl)methyl)thio)-2-chlorophenoxy)ethyl-2-methylpropionate |
| 5 | 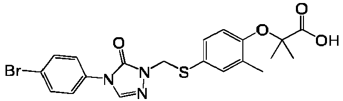 | 2-(4-(((4-(4-Bromophenyl)-5-oxo-4,5-dihydro-1*H*-1,2,4-triazol-1-yl)methyl)thio)-2-methylphenoxy)-2-methylpropionic acid |
| 6 | 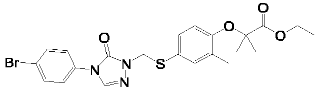 | Ethyl 2-(4-(((4-(4-bromophenyl)-5-oxo-4,5-dihydro-1*H*-1,2,4-triazol-1-yl)methyl)thio)-2-methylphenoxy)ethyl-2-methylpropionate |
| 7 | 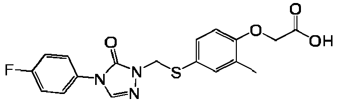 | 2-(4-(((4-(4-Fluorophenyl)-5-oxo-4,5-dihydro-1*H*-1,2,4-triazol-1-yl)methyl)thio)-2-methylphenoxy)acetic acid |
| 8 | 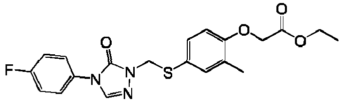 | Ethyl 2-(4-(((4-(4-fluorophenyl)-5-oxo-4, 5-dihydro-1*H*-1,2,4-triazol-1-yl)methyl)thio)-2-methylphenoxy)acetate |
| 9 | 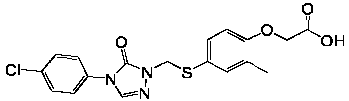 | 2-(4-(((4-(4-Chlorophenyl)-5-oxo-4,5-dihydro-1*H*-1,2,4-triazol-1-yl)methyl)thio)-2-methylphenoxy)acetic acid |
| 10 | 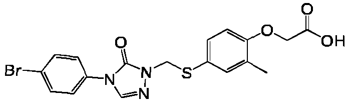 | 2-(4-(((4-(4-Bromophenyl)-5-oxo-4,5-dihydro-1*H*-1,2,4-triazol-1-yl)methyl)thio)-2-methylphenoxy)acetic acid |
| 11 | 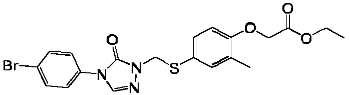 | Ethyl 2-(4-(((4-(4-bromophenyl)-5-oxo-4,5-dihydro-1*H*-1,2,4-triazol-1-yl)methyl)thio)-2-methylphenoxy)acetate |
| 12 | 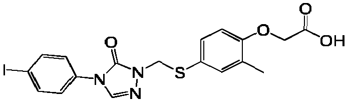 | 2-(4-(((4-(4-Iodophenyl)-5-oxo-4,5-dihydro-1*H*-1,2,4-triazol-1-yl)methyl)thio)-2-methylphenoxy)acetic acid |
| 13 | 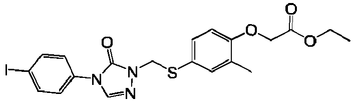 | Ethyl 2-(4-(((4-(4-iodophenyl)-5-oxo-4,5-dihydro-1*H*-1,2,4-triazol-1-yl)methyl)thio)-2-methylphenoxy)acetate |
| 14 | 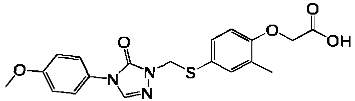 | 2-(4-(((4-(4-Methoxyphenyl)-5-oxo-4,5-dihydro-1*H*-1,2,4-triazol-1-yl)methyl)thio)-2-methylphenoxy)acetic acid |
| 15 | 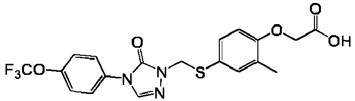 | 2-(2-Methyl-4-(((5-oxo-4-(4-(trifluoromethoxy)phenyl)-4,5-dihydro-1*H*-1,2,4-triazol-1-yl)methyl)thio)phenoxy)acetic acid |
| 16 | 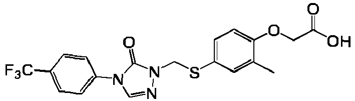 | 2-(2-Methyl-4-(((5-oxo-4-(4-(trifluoromethyl)phenyl)-4,5-dihydro-1*H*-1,2,4-triazol-1-yl)methyl)thio)phenoxy)acetic acid |
| 17 | 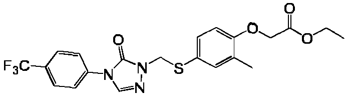 | Ethyl 2-(2-methyl-4-(((5-oxo-4-(4-(trifluoromethyl)phenyl)-4, 5-dihydro-1*H*-1,2,4-triazol-1-yl)methyl)thio)phenoxy)acetate |
| 18 | 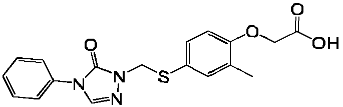 | 2-(2-Methyl-4-(((5-oxo-4-phenyl-4,5-dihydro-1*H*-1,2,4-triazol-1-yl)methyl)thio)phenoxy)acetic acid |
| 19 | 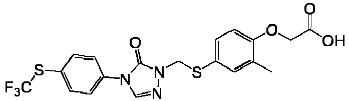 | 2-(2-Methyl-4-(((5-oxo-4-(4-((trifluoromethyl)thio)phenyl)-4,5-dihydro-1*H*-1,2,4-triazol-1-yl)4-methyl)thio)phenoxy)acetic acid |
| 20 | 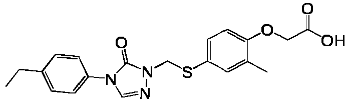 | 2-(4-(((4-(4-Ethylphenyl)-5-oxo-4,5-dihydro-1*H*-1,2,4-triazol-1-yl)methyl)thio)-2-methylphenoxy)acetic acid |
| 21 | 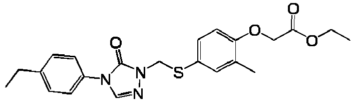 | Ethyl 2-(4-(((4-(4-ethylphenyl)-5-oxo-4,5-dihydro-1*H*-1,2,4-triazol-1-yl)methyl)thio)-2-methylphenoxy)acetate |
| 22 | 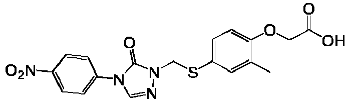 | 2-(2-Methyl-4-(((4-(4-nitrophenyl)-5-oxo-4,5-dihydro-1*H*-1,2,4-triazol-1-yl)methyl)thio]phenoxy)acetic acid |
| 23 | 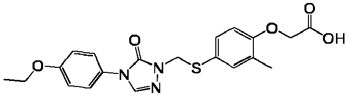 | 2-(4-(((4-(4-Ethoxyphenyl)-5-oxo-4,5-dihydro-1*H*-1,2,4-triazol-1- yl)methyl)thio)-2-methylphenoxy)acetic acid |
| 24 | 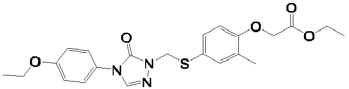 | Ethyl 2-(4-(((4-(4-ethoxyphenyl)-5-oxo-4,5-dihydro-1*H*-1,2,4-triazol-1-yl)methyl)thio)-2-methylphenoxy)acetate |
| 25 | 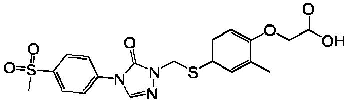 | 2-(2-Methyl-4-(((4-(4-(methylsulfonyl)phenyl)-5-oxo-4,5-dihydro-1*H*-1,2,4-triazol-1-yl)methyl]thio)phenoxy)acetate |
| 26 | 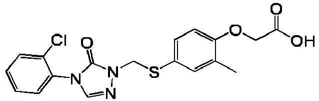 | 2-(4-(((4-(2-Chlorophenyl)-5-oxo-4,5-dihydro-1*H*-1,2,4-triazol-1-yl)methyl)thio)-2-methylphenoxy)acetic acid |
| 27 | 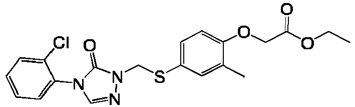 | Ethyl 2-(4-(((4-(2-chlorophenyl)-5-oxo-4,5-dihydro-1*H*-1,2,4-triazol-1-yl)methyl)thio)-2-methylphenoxy)acetate |
| 28 | 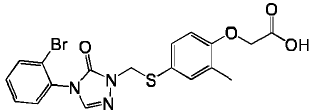 | 2-(4-(((4-(2-Bromophenyl)-5-oxo-4,5-dihydro-1*H*-1,2,4-triazol-1-yl)methyl)thio)-2-methylphenoxy)acetic acid |
| 29 | 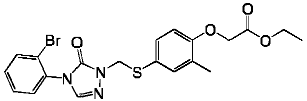 | Ethyl 2-(4-(((4-(2-bromophenyl)-5-oxo-4,5-dihydro-1*H*-1,2,4-triazol-1-yl)methyl)thio)-2-methylphenoxy)acetate |
| 30 | 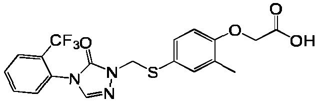 | 2-(2-Methyl-4-(((5-oxo-4-(2-(trifluoromethyl)phenyl)-4,5-dihydro-1*H*-1,2,4-triazol-1-yl)methyl)thio)phenoxy)acetic acid |
| 31 | 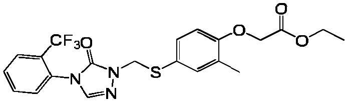 | Ethyl 2-(2-methyl-4-(((5-oxo-4-(2-(trifluoromethyl)phenyl)-4,5-dihydro-1*H*-1,2,4-triazol-1-yl)methyl)thio)phenoxy)acetate |
| 32 | 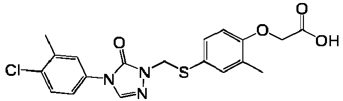 | 2-(4-(((4-(4-Chloro-3-methylphenyl)-5-oxo-4,5-dihydro-1*H*-1,2,4-triazol-1-yl)methyl)thio)-2-methylphenoxy)acetic acid |
| 33 | 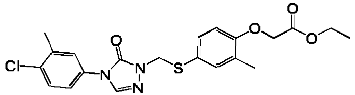 | Ethyl 2-(4-(((4-(4-chloro-3-methylphenyl)-5-oxo-4,5-dihydro-1*H*-1,2,4-triazol-1-yl)methyl)thio)-2-methylphenoxy)acetate |
| 34 | 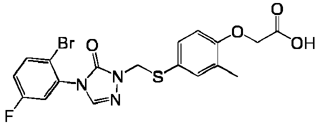 | 2-(4-(((4-(2-Bromo-5-fluorophenyl)-5-oxo-4,5-dihydro-1*H*-1,2,4-triazol-1-yl)methyl)thio)-2-methylphenoxy)acetic acid |
| 35 | 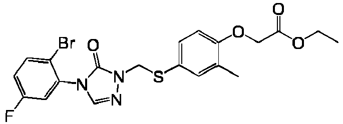 | Ethyl 2-(4-(((4-(2-bromo-5-fluorophenyl)-5-oxo-4,5-dihydro-1*H-*1,2,4-triazol-1-yl)methyl)thio)-2-methylphenoxy)acetate |
| 36 | 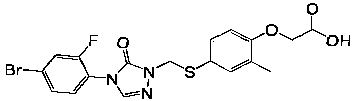 | 2-(4-(((4-(4-Bromo-2-fluorophenyl)-5-oxo-4,5-dihydro-1*H*-1,2,4-triazol-1-yl)methyl)thio)-2-methylphenoxy)acetic acid |
| 37 | 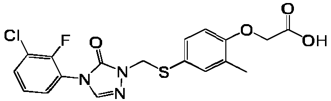 | 2-(4-(((4-(3 -Chl oro-2-fluorophenyl)-5-oxo-4,5-dihydro-1*H*-1,2,4-triazol-1-yl)methyl)thio)-2-methylphenoxy)acetic acid |
| 38 | 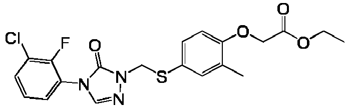 | Ethyl 2-(4-(((4-(3-chloro-2-fluorophenyl)-5-oxo-4,5-dihydro-1*H-*1,2,4-triazol-1-yl)methyl)thio)-2-methylphenoxy)acetate |
| 39 | 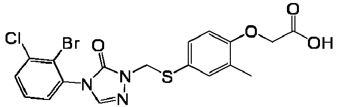 | 2-(4-(((4-(2-Bromo-3-chlorophenyl)-5-oxo-4,5-dihydro-1*H*-1,2,4-triazol-1-yl)methyl)thio)-2-methylphenoxy)acetic acid |
| 40 | 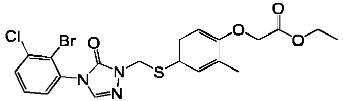 | Ethyl 2-(4-(((4-(2-bromo-3-chlorophenyl)-5 -oxo-4,5 -dihydro-1*H*-1,2,4-triazol-1-yl)methyl)thio)-2-methylphenoxy)acetate |
| 41 | 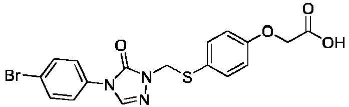 | 2-(4-(((4-(4-Bromophenyl)-5-oxo-4,5-dihydro-1*H*-1,2,4-triazol-1-yl)methyl)thio)phenoxy)acetic acid |
| 42 | 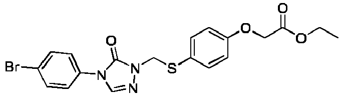 | Ethyl 2-(4-(((4-(4-bromophenyl)-5-oxo-4,5-dihydro-1*H*-1,2,4-triazol-1-yl)methyl)thio)phenoxy)acetate |
| 43 | 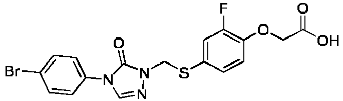 | 2-(4-(((4-(4-Bromophenyl)-5-oxo-4,5-dihydro-1*H*-1,2,4-triazol-1-yl)methyl)thio)-2-fluorophenoxy)acetic acid |
| 44 | 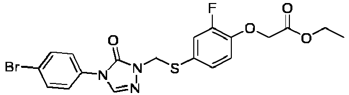 | Ethyl 2-(4-(((4-(4-bromophenyl)-5-oxo-4,5-dihydro-1*H*-1,2,4-triazol-1-yl)methyl)thio)-2-fluorophenoxy)acetate |
| 45 | 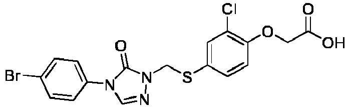 | 2-(4-(((4-(4-Bromophenyl)-5-oxo-4,5-dihydro-1*H*-1,2,4-triazol-1-yl)methyl)thio)-2-chlorophenoxy)acetic acid |
| 46 | 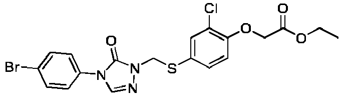 | Ethyl 2-(4-(((4-(4-bromophenyl)-5-oxo-4,5-dihydro-1*H*-1,2,4-triazol-1-yl)methyl)thio)-2-chlorophenoxy)acetate |
| 47 | 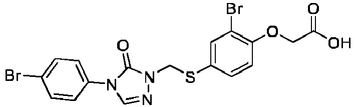 | 2-(2-Bromo-4-(((4-(4-bromophenyl)-5-oxo-4,5-dihydro-1*H*-1,2,4-triazol-1-yl)methyl)thio)phenoxy)acetic acid |
| 48 | 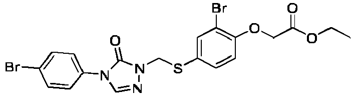 | Ethyl 2-(2-bromo-4-(((4-(4-bromophenyl)-5-oxo-4,5-dihydro-1*H*-1,2,4-triazol-1-yl)methyl)thio)phenoxy)acetate |
| 49 | 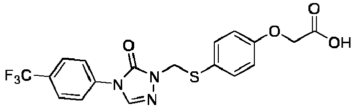 | 2-(4-(((5-Oxo-4-(4-(trifluoromethyl)phenyl)-4,5-dihydro-1*H*-1,2,4-triazol-1-yl)methyl)thio)phenoxy)acetic acid |
| 50 | 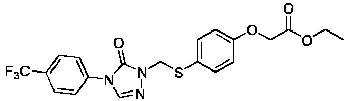 | Ethyl 2-(4-(((5-oxo-4-(4-(trifluoromethyl)phenyl)-4,5-dihydro-1*H*-1,2,4-triazol-1-yl)methyl)thio)phenoxy)acetate |
| 51 | 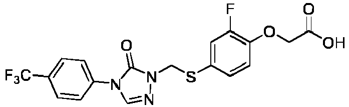 | 2-(2-Fluoro-4-(((5-oxo-4-(4-(trifluoromethyl)phenyl)-4,5-dihydro-1*H*-1,2,4-triazol-1-yl)methyl)thio)phenoxy)acetic acid |
| 52 | 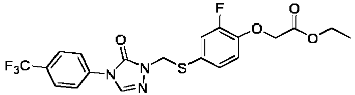 | Ethyl 2-(2-fluoro-4-(((5-oxo-4-(4-(trifluoromethyl)phenyl)-4,5-dihydro-1*H*-1,2,4-triazol-1-yl)methyl)thio)phenoxy)acetate |
| 53 | 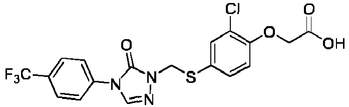 | 2-(2-Chloro-4-(((5-oxo-4-(4-(trifluoromethyl)phenyl)-4,5-dihydro-1*H*-1,2,4-triazol-1-yl)methyl)thio)phenoxy)acetic acid |
| 54 | 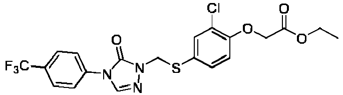 | Ethyl 2-(2-chloro-4-(((5-oxo-4-(4-(trifluoromethyl)phenyl)-4,5-dihydro-1*H*-1,2,4-triazol-1- yl)methyl)thio)phenoxy)acetate |
| 55 | 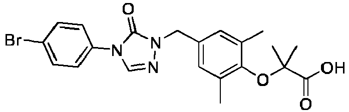 | 2-(4-((4-(4-Bromophenyl)-5-oxo-4,5-dihydro-1*H*-1,2,4-triazol-1-yl)methyl)-2,6-dimethylphenoxy)-2-methylpropionic acid |
| 56 | 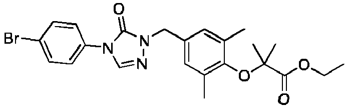 | Ethyl 2-(4-((4-(4-bromophenyl)-5-oxo-4, 5-dihydro-1*H*-1,2,4-triazol-1-yl)methyl)-2,6-dimethylphenoxy)-2-methylpropionate |
| 57 | 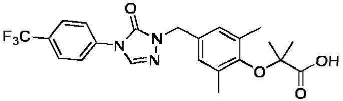 | 2-(2,6-Dimethyl-4-((5-oxo-4-(4-(trifluoromethyl)phenyl)-4,5-dihydro-1*H*-1,2,4-triazol-1-yl)methyl)phenoxy)-2-methylpropionic acid |
| 58 | 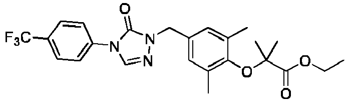 | Ethyl 2-(2,6-dimethyl-4-((5-oxo-4-(4-(trifluoromethyl)phenyl)-4,5-dihydro-1*H*-1,2,4-triazol-1-yl)methyl)phenoxy)-2-methylpropionate |
| 59 | 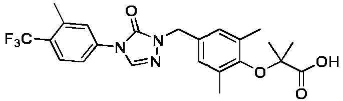 | 2-(2,6-Dimethyl-4-((4-(3-methyl-4-(trifluoromethyl)phenyl)-5-oxo-4,5-dihydro-1*H*-1,2,4-triazol-1-yl)methyl)phenoxy)-2-methylpropionic acid |
| 60 | 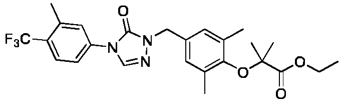 | Ethyl 2-(2,6-dimethyl-4-((4-(3-methyl-4-(trifluoromethyl)phenyl)-5-oxo-4,5-dihydro-1*H*-1,2,4-triazol-1-yl)methyl)phenoxy)-2-methylpropionate |
| 61 | 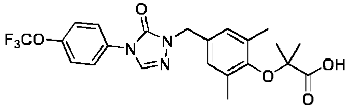 | 2-(2,6-Dimethyl-4-((5 -oxo-4-(4-(trifluoromethoxy)phenyl)-4,5-dihydro-1*H*-1,2,4-triazol-1-yl)methyl)phenoxy)-2-methylpropionic acid |
| 62 | 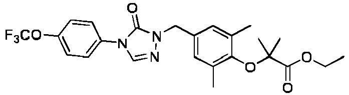 | Ethyl 2-(2,6-dimethyl-4-((5-oxo-4-(4-(trifluoromethoxy)phenyl)-4,5-dihydro-1*H*-1,2,4-triazol-1-yl)methyl)phenoxy)-2-methylpropionate |
| 63 | 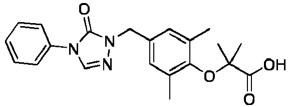 | 2-(2,6-Dimethyl-4-((5-oxo-4-phenyl-4,5-dihydro-1*H*-1,2,4-triazol-1-yl)methyl)phenoxy)-2-methylpropionic acid |
| 64 | 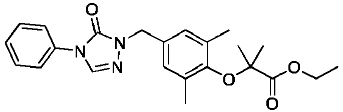 | Ethyl 2-(2,6-dimethyl-4-((5-oxo-4-phenyl-4,5-dihydro-1*H*-1,2,4-triazol-1-yl)methyl)phenoxy)-2-methylpropionate |
| 65 | 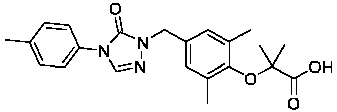 | 2-(2,6-Dimethyl-4-((5-oxo-4-(*p-*tolyl)-4,5-dihydro-1*H*-1,2,4-triazol-1-yl)methyl)phenoxy)-2-methylpropionic acid |
| 66 | 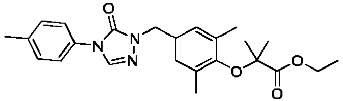 | Ethyl 2-(2,6-dimethyl-4-((5-oxo-4-(*p*-tolyl)-4,5-dihydro-1*H*-1,2,4-triazol-1-yl)methyl)phenoxy)-2-methylpropionate |
| 67 | 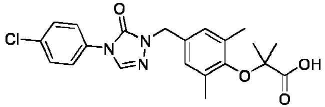 | 2-(4-((4-(4-Chlorophenyl)-5-oxo-4,5-dihydro-1*H*-1,2,4-triazol-1-yl)methyl)-2,6-dimethylphenoxy)-2-methylpropionic acid |
| 68 | 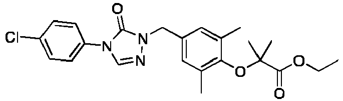 | Ethyl 2-(4-((4-(4-chlorophenyl)-5-oxo-4,5-dihydro-1*H*-1,2,4-triazol-1-yl)methyl)-2,6-dimethylphenoxy)-2-methylpropionate |
| 69 | 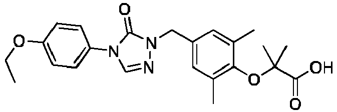 | 2-(4-((4-(4-Ethoxyphenyl)-5-oxo-4,5-dihydro-1*H*-1,2,4-triazol-1-yl)methyl)-2,6-dimethylphenoxy)-2-methylpropionic acid |
| 70 | 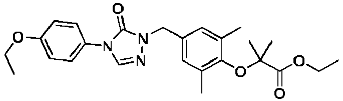 | Ethyl 2-(4-((4-(4-ethoxyphenyl)-5-oxo-4,5-dihydro-1*H*-1,2,4-triazol-1-yl)methyl)-2,6-dimethylphenoxy)-2-methylpropionate |
| 71 | 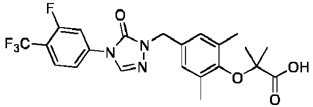 | 2-(4-((4-(3-Fluoro-4-(trifluoromethyl)phenyl)-5-oxo-4,5-dihydro-1*H*-1,2,4-triazol-1-yl)methyl)-2,6-dimethylphenoxy)-2-methylpropionic acid |
| 72 | 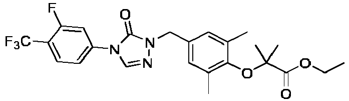 | Ethyl 2-(4-((4-(3-fluoro-4-(trifluoromethyl)phenyl)-5-oxo-4,5-dihydro-1*H*-1,2,4-triazol-1-yl)methyl)-2,6-dimethylphenoxy)-2-methylpropionate |
| 73 | 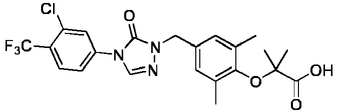 | 2-(4-((4-(3-Chloro-4-(trifluoromethyl)phenyl)-5-oxo-4,5-dihydro-1*H*-1,2,4-triazol-1-yl)methyl)-2,6-dimethylphenoxy)-2-methylpropionic acid |
| 74 | 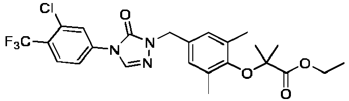 | Ethyl 2-(4-((4-(3-chloro-4-(trifluoromethyl)phenyl)-5-oxo-4,5-dihydro-1*H*-1,2,4-triazol-1-yl)methyl)-2,6-dimethylphenoxy)-2-methylpropionate |
| 75 | 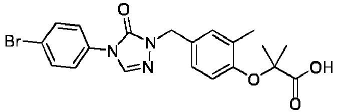 | 2-(4-((4-(4-Bromophenyl)-5-oxo-4,5-dihydro-1*H*-1,2,4-triazol-1-yl)methyl)-2-methylphenoxy)-2-methylpropionic acid |
| 76 | 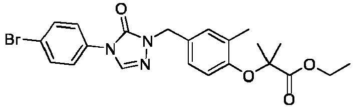 | Ethyl 2-(4-((4-(4-bromophenyl)-5-oxo-4,5-dihydro-1*H*-1,2,4-triazol-1-yl)methyl)-2-methylphenoxy)-2-methylpropionate |
| 77 | 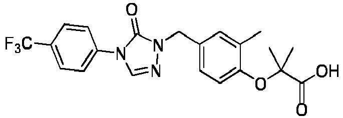 | 2-Methyl-2-(2-methyl-4-((5-oxo-4-(4-(trifluoromethyl)phenyl)-4,5-dihydro-1*H*-1,2,4-triazol-1-yl)methyl)phenoxy)propionic acid |
| 78 | 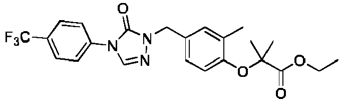 | Ethyl 2-methyl-2-(2-methyl-4-((5-oxo-4-(4-(trifluoromethyl)phenyl)-4,5-dihydro-1*H*-1,2,4-triazol-1-yl)methyl)phenoxy)propionate |
| 79 | 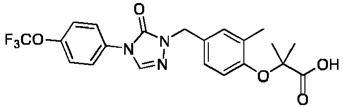 | 2-Methyl-2-(2-methyl-4-((5-oxo-4-(4-(trifluoromethoxy)phenyl)-4,5-dihydro-1*H*-1,2,4-triazol-1-yl)methyl)phenoxy)propionic acid |
| 80 | 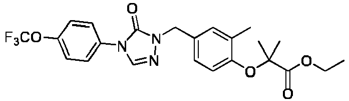 | Ethyl 2-methyl-2-(2-methyl-4-((5-oxo-4-(4-(trifluoromethoxy)phenyl)-4,5-dihydro-1*H*-1,2,4-triazol-1-yl)methyl)phenoxy)propionate |
| 81 | 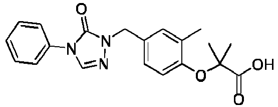 | 2-Methyl-2-(2-methyl-4-((5-oxo-4-phenyl-4,5-dihydro-1*H*-1,2,4-triazol-1-yl)methyl)phenoxy)propionic acid |
| 82 | 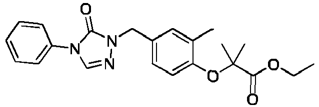 | Ethyl 2-methyl-2-(2-methyl-4-((5-oxo-4-phenyl-4,5-dihydro-1*H*-1,2,4-triazol-1-yl)methyl)phenoxy)propionate |
| 83 | 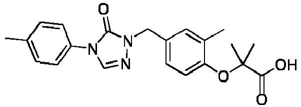 | 2-Methyl-2-(2-methyl-4-((5-oxo-4-(*p*-tolyl)-4,5-dihydro-1*H*-1,2,4-triazol-1-yl)methyl)phenoxy)propionate |
| 84 | 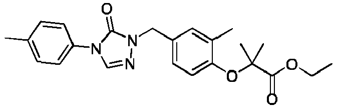 | Ethyl 2-methyl-2-(2-methyl-4-((5-oxo-4-(*p*-tolyl)-4,5-dihydro-1*H-*1,2,4-triazol-1-yl)methyl)phenoxy)propionate |
| 85 | 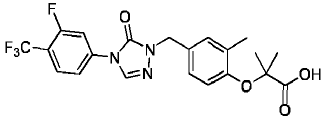 | 2-(4-((4-(3 -Fluoro-4-(trifluoromethyl)phenyl)-5-oxo-4,5-dihydro-1*H*-1,2,4-triazol-1-yl)methyl)-2-methylphenoxy)-2-methylpropionic acid |
| 86 | 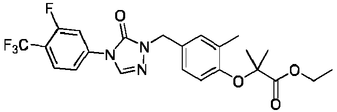 | Ethyl 2-(4-((4-(3-fluoro-4-(trifluoromethyl)phenyl)-5-oxo-4,5-dihydro-1*H*-1,2,4-triazol-1-yl)methyl)-2-methylphenoxy)-2-methylpropionate |
| 87 | 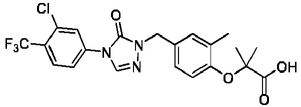 | 2-(4-((4-(3-Chloro-4-(trifluoromethyl)phenyl)-5-oxo-4,5-dihydro-1*H*-1,2,4-triazol-1-yl)methyl)-2-methylphenoxy)-2-methylpropionic acid |
| 88 | 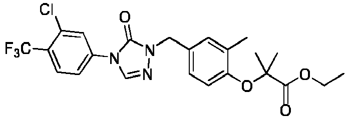 | Ethyl 2-(4-((4-(3-chloro-4-(trifluoromethyl)phenyl)-5-oxo-4,5-dihydro-1*H*-1,2,4-triazol-1-yl)methyl)-2-methylphenoxy)-2-methylpropionate |
| 89 | 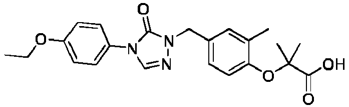 | 2-(4-((4-(4-Ethoxyphenyl)-5-oxo-4,5-dihydro-1*H*-1,2,4-triazol-1-yl)methyl)-2-methylphenoxy)-2-methylpropionic acid |
| 90 | 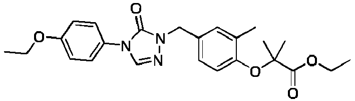 | Ethyl 2-(4-((4-(4-ethoxyphenyl)-5-oxo-4,5-dihydro-1*H*-1,2,4-triazol-1-yl)methyl)-2-methylphenoxy)-2-methylpropionate |
| 91 | 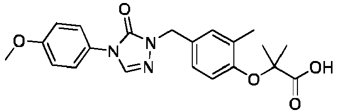 | 2-(4-((4-(4-Methoxyphenyl)-5-oxo-4,5-dihydro-1*H*-1,2,4-triazol-1-yl)methyl)-2-methylphenoxy)-2-methylpropionic acid |
| 92 | 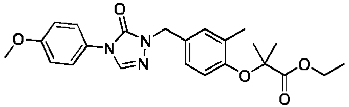 | Ethyl 2-(4-((4-(4-methoxyphenyl)-5-oxo-4,5-dihydro-1*H*-1,2,4-triazol-1-yl)methyl)-2-methylphenoxy)-2-methylpropionate |
| 93 | 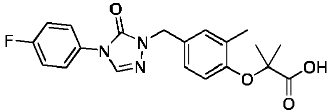 | 2-(4-((4-(4-Flourophenyl)-5-oxo-4,5-dihydro-1*H*-1,2,4-triazol-1-yl)methyl)-2-methylphenoxy)-2-methylpropionic acid |
| 94 | 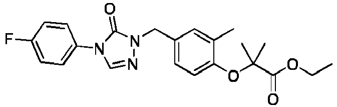 | Ethyl 2-(4-((4-(4-flourophenyl)-5-oxo-4,5-dihydro-1*H*-1,2,4-triazol-1-yl)methyl)-2-methylphenoxy)-2-methylpropionate |
| 95 | 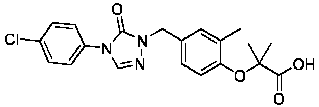 | 2-(4-((4-(4-Chlorophenyl)-5-oxo-4,5-dihydro-1*H*-1,2,4-triazol-1-yl)methyl)-2-methylphenoxy)-2-methylpropionic acid |
| 96 | 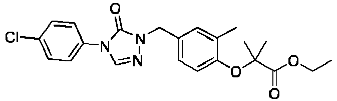 | Ethyl 2-(4-((4-(4-chlorophenyl)-5-oxo-4,5-dihydro-1*H*-1,2,4-triazol-1-yl)methyl)-2-methylphenoxy)-2-methylpropionate |
| 97 | 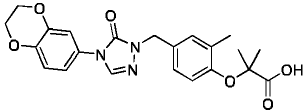 | 2-(4-((4-(2,3-Dihydrobenzo[*b*][1,4]dioxin-6-yl)-5-oxo-4,5-dihydro-1*H*-1,2,4-triazol-1-yl)methyl)-2-methylphenoxy)-2-methylpropionic acid |
| 98 | 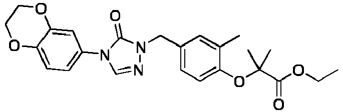 | Ethyl 2-(4-((4-(2,3-dihydrobenzo[*b*][1,4]dioxin-6-yl)-5-oxo-4,5-dihydro-1*H*-1,2,4-triazol-1-yl)methyl)-2-methylphenoxy)-2-methylpropionate |
| 99 | 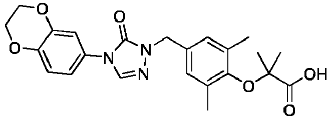 | 2-(4-((4-(2,3-Dihydrobenzo[*b*][1,4]dioxin-6-yl)-5-oxo-4, 5-dihydro-1*H*-1,2,4-triazol-1-yl)methyl)-2,6-dimethylphenoxy)-2-methylpropionic acid |
| 100 | 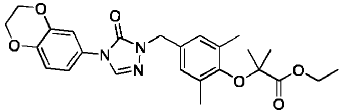 | Ethyl 2-(4-((4-(2,3-dihydrobenzo[*b*][1,4]dioxin-6-yl)-5-oxo-4,5-dihydro-1*H*-1,2,4-triazol-1-yl)methyl)-2,6-dimethylphenoxy)-2-methylpropionate |
| 101 | 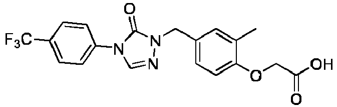 | 2-(2-Methyl-4-((5-oxo-4-(4-(trifluoromethyl)phenyl)-4,5-dihydro-1*H*-1,2,4-triazol-1-yl)methyl)phenoxyacetic acid |
| 102 | 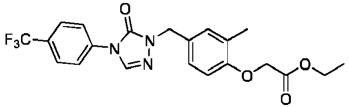 | Ethyl 2-(2-methyl-4-((5-oxo-4-(4-(trifluoromethyl)phenyl)-4,5-dihydro-1*H*-1,2,4-triazol-1-yl)methyl)phenoxy acetate |
| 103 | 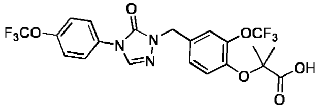 | 2-Methyl-2-(4-((5-oxo-4-(4-(trifluoromethoxy)phenyl)-4,5-dihydro-1*H*-1,2,4-triazol-1-yl)methyl)-2-(trifluoromethoxy)phenoxy)propionic acid |
| 104 | 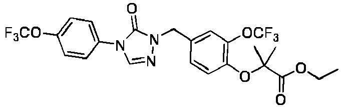 | Ethyl 2-methyl-2-(4-((5-oxo-4-(4-(trifluoromethoxy)phenyl)-4,5-dihydro-1*H*-1,2,4-triazol-1-yl)methyl)-2-(trifluoromethoxy)phenoxy)propionat e |
| 105 | 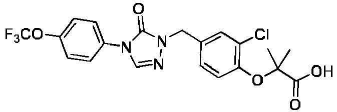 | 2-(2-Chloro-4-((5-oxo-4-(4-(trifluoromethoxy)phenyl)-4,5-dihydro-1*H*-1,2,4-triazol-1-yl)methyl)phenoxy)-2-methylpropionic acid |
| 106 | 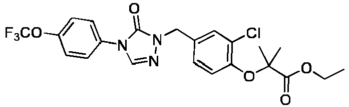 | Ethyl 2-(2-chloro-4-((5-oxo-4-(4-(trifluoromethoxy)phenyl)-4,5-dihydro-1*H*-1,2,4-triazol-1-yl)methyl)phenoxy)-2-methylpropionate |
| 107 | 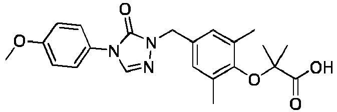 | 2-(4-((4-(4-Methoxyphenyl)-5-oxo-4,5-dihydro-1*H*-1,2,4-triazol-1-yl)methyl)-2,6-dimethylphenoxy)-2-methylpropionic acid |
| 108 | 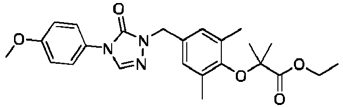 | Ethyl 2-(4-((4-(4-methoxyphenyl)-5-oxo-4,5-dihydro-1*H*-1,2,4-triazol-1-yl)methyl)-2,6-dimethylphenoxy)-2-methylpropionate |
| 109 | 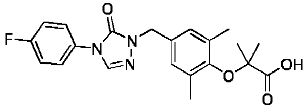 | 2-(4-((4-(4-Flourophenyl)-5-oxo-4,5-dihydro-1*H*-1,2,4-triazol-1-yl)methyl)-2,6-dimethylphenoxy)-2-methylpropionic acid |
| 110 | 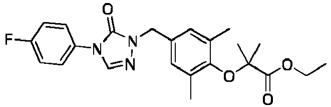 | Ethyl 2-(4-((4-(4-flourophenyl)-5-oxo-4,5-dihydro-1*H*-1,2,4-triazol-1-yl)methyl)-2,6-dimethylphenoxy)-2-methylpropionate |
| 111 | 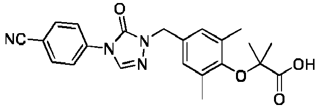 | 2-(4-((4-(4-Cyanophenyl)-5-oxo-4,5-dihydro-1*H*-1,2,4-triazol-1-yl)methyl)-2,6-dimethylphenoxy)-2-methylpropionic acid |
| 112 | 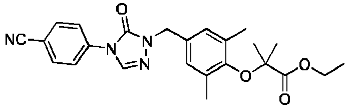 | Ethyl 2-(4-((4-(4-cyanophenyl)-5-oxo-4, 5-dihydro-1*H*-1,2,4-triazol-1-yl)methyl)-2,6-dimethylphenoxy)-2-methylpropionate |
| 113 | 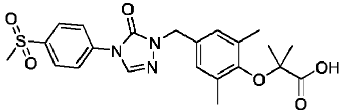 | 2-(2,6-Dimethyl-4-((4-(4-(methylsulfonyl)phenyl)-5-oxo-4,5-dihydro-1*H*-1,2,4-triazol-1-yl)methyl)phenoxy)-2-methylpropionic acid |
| 114 | 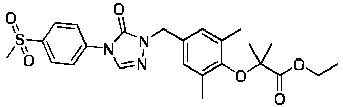 | Ethyl 2-(2,6-dimethyl-4-((4-(methylsulfonyl)phenyl)-5-oxo-4,5-dihydro-1*H*-1,2,4-triazol-1-yl)methyl)phenoxy)-2-methylpropionate |
| 115 | 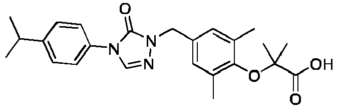 | 2-(4-((4-(4-Isopropylphenyl)-5-oxo-4,5-dihydro-1*H*-1,2,4-triazol-1-yl)methyl)-2,6-dimethylphenoxy)-2-methylpropionic acid |
| 116 | 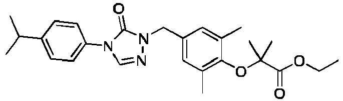 | Ethyl 2-(4-((4-(4-isopropylphenyl)-5-oxo-4,5-dihydro-1*H*-1,2,4-triazol-1-yl)methyl)-2,6-dimethylphenoxy)-2-methylpropionate |
| 117 | 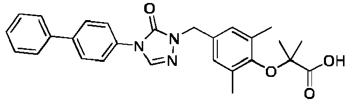 | 2-(4-((4-([1,1'-Biphenyl]-4-yl)-5-oxo-4,5-dihydro-1*H*-1,2,4-triazol-1-yl)methyl)-2,6-dimethylphenoxy)-2-methylpropionic acid |
| 118 | 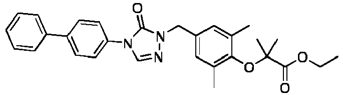 | Ethyl 2-(4-((4-([1,1'-biphenyl]-4-yl)-5-oxo-4,5-dihydro-1*H*-1,2,4-triazol-1-yl)methyl)-2,6-dimethylphenoxy)-2-methylpropionate |
| 119 | 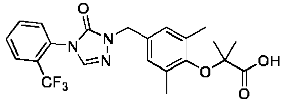 | 2-(2,6-Dimethyl-4-((5-oxo-4-(2-(trifluoromethyl)phenyl)-4,5-dihydro-1*H*-1,2,4-triazol-1-yl)methyl)phenoxy)-2-methylpropionic acid |
| 120 | 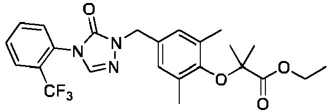 | Ethyl 2-(2,6-dimethyl-4-((5-oxo-4-(2-(trifluoromethyl)phenyl)-4,5-dihydro-1*H*-1,2,4-triazol-1-yl)methyl)phenoxy)-2-methylpropionate |
| 121 | 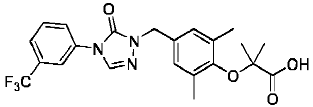 | 2-(2,6-Dimethyl-4-((5-oxo-4-(3-(trifluoromethyl)phenyl)-4,5-dihydro-1*H*-1,2,4-triazol-1-yl)methyl)phenoxy)-2-methylpropionic acid |
| 122 | 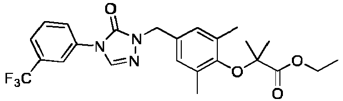 | Ethyl 2-(2,6-dimethyl-4-((5-oxo-4-(3-(trifluoromethyl)phenyl)-4, 5-dihydro-1*H*-1,2,4-triazol-1-yl)methyl)phenoxy)-2-methylpropionate |
| 123 | 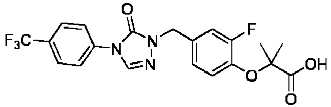 | 2-(2-Fluoro-4-((5-oxo-4-(4-(trifluoromethyl)phenyl)-4,5-dihydro-1*H*-1,2,4-triazol-1-yl)methyl)phenoxy)-2-methylpropionic acid |
| 124 | 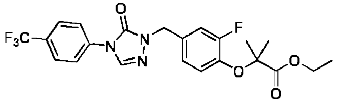 | Ethyl 2-(2-fluoro-4-((5-oxo-4-(4-(trifluoromethyl)phenyl)-4,5-dihydro-1*H*-1,2,4-triazol-1-yl)methyl)phenoxy)-2-methylpropionate |
| 125 | 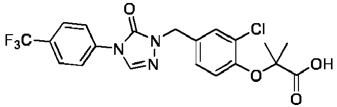 | 2-(2-Chloro-4-((5-oxo-4-(4-(trifluoromethyl)phenyl)-4,5-dihydro-1*H*-1,2,4-triazol-1-yl)methyl)phenoxy)-2-methylpropionic acid |
| 126 | 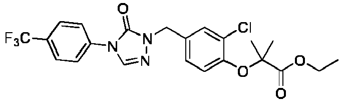 | Ethyl 2-(2-chloro-4-((5-oxo-4-(4-(trifluoromethyl)phenyl)-4,5-dihydro-1*H*-1,2,4-triazol-1-yl)methyl)phenoxy)-2-methylpropionate |
| 127 | 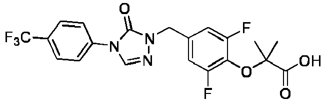 | 2-(2,6-Difluoro-4-((5-oxo-4-(4-(trifluoromethyl)phenyl)-4,5-dihydro-1*H*-1,2,4-triazol-1-yl)methyl)phenoxy)-2-methylpropionic acid |
| 128 | 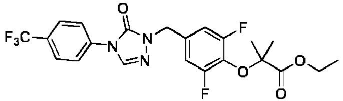 | Ethyl 2-(2,6-difluoro-4-((5-oxo-4-(4-(trifluoromethyl)phenyl)-4,5-dihydro-1*H*-1,2,4-triazol-1-yl)methyl)phenoxy)-2-methylpropionate |
| 129 | 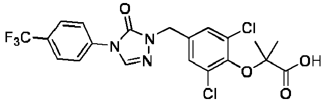 | 2-(2,6-Dichloro-4-((5-oxo-4-(4-(trifluoromethyl)phenyl)-4,5-dihydro-1*H*-1,2,4-triazol-1-yl)methyl)phenoxy)-2-methylpropionic acid |
| 130 | 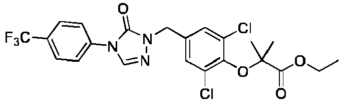 | Ethyl 2-(2,6-dichloro-4-((5-oxo-4-(4-(trifluoromethyl)phenyl)-4,5-dihydro-1*H*-1,2,4-triazol-1-yl)methyl)phenoxy)-2-methylpropionate |
| 131 | 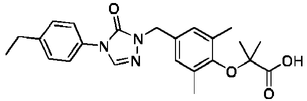 | 2-(4-((4-(4-Ethylphenyl)-5-oxo-4,5-dihydro-1*H*-1,2,4-triazol-1-yl)methyl)-2,6-dimethylphenoxy)-2-methylpropionic acid |
| 132 | 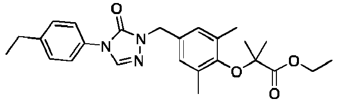 | Ethyl 2-(4-((4-(4-ethylphenyl)-5-oxo-4,5-dihydro-1*H*-1,2,4-triazol-1-yl)methyl)-2,6-dimethylphenoxy)-2-methylpropionate |
| 133 | 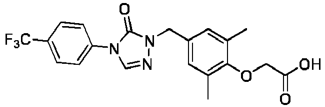 | 2-(2,6-Dimethyl-4-((5-oxo-4-(4-(trifluoromethyl)phenyl)-4,5-dihydro-1*H*-1,2,4-triazol-1-yl)methyl)phenoxy)acetic acid |
| 134 | 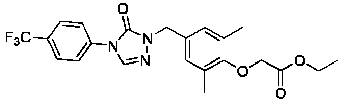 | Ethyl 2-(2,6-dimethyl-4-((5-oxo-4-(4-(trifluoromethyl)phenyl)-4,5-dihydro-1*H*-1,2,4-triazol-1-yl)methyl)phenoxy)acetate |
| 135 | 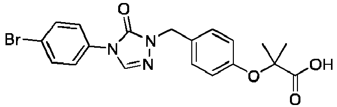 | 2-(4-((4-(4-Bromophenyl)-5-oxo-4,5-dihydro-1*H*-1,2,4-triazol-1-yl)methyl)phenoxy)-2-methylpropionic acid |
| 136 | 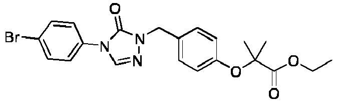 | Ethyl 2-(4-((4-(4-Bromophenyl)-5-oxo-4,5-dihydro-1*H*-1,2,4-triazol-1-yl)methyl)phenoxy)-2-methylpropionate |
| 137 | 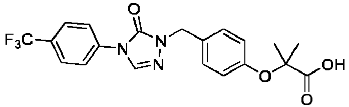 | 2-(4-((4-(4-Trifluoromethylphenyl)-5-oxo-4,5-dihydro-1*H*-1,2,4-triazol-1-yl)methyl)phenoxy)-2-methylpropionic acid |
| 138 | 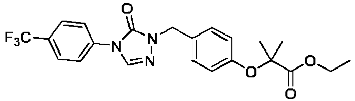 | Ethyl 2-(4-((4-(4-trifluoromethylphenyl)-5-oxo-4,5-dihydro-1*H*-1,2,4-triazol-1-yl)methyl)phenoxy)-2-methylpropionate |
| 139 | 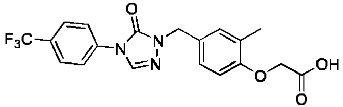 | 2-(2-Methyl-4-((5-oxo-4-(4-(trifluoromethyl)phenyl)-4,5-dihydro-1*H*-1,2,4-triazol-1-yl)methyl)phenoxy)acetic acid |
| 140 | 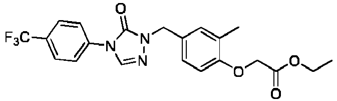 | Ethyl 2-(2-methyl-4-((5-oxo-4-(4-(trifluoromethyl)phenyl)-4,5-dihydro-1*H*-1,2,4-triazol-1-yl)methyl)phenoxy)acetate |
| 141 | 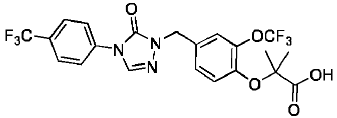 | 2-(2-Trifluoromethoxy-4-((5-oxo-4-(4-(trifluoromethyl)phenyl)-4,5-dihydro-1*H*-1,2,4-triazol-1-yl)methyl)phenoxy)acetic acid |
| 142 | 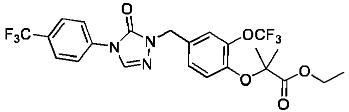 | Ethyl 2-(2-trifluoromethoxy-4-((5-oxo-4-(4-(trifluoromethyl)phenyl)-4,5-dihydro-1*H*-1,2,4-triazol-1-yl)methyl)phenoxy)acetate |
| 143 | 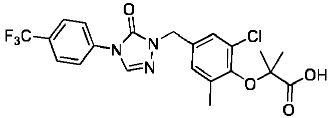 | 2-(2-Chloro-6-methyl-4-((5-oxo-4-(4-(trifluoromethyl)phenyl)-4,5-dihydro-1*H*-1,2,4-triazol-1-yl)methyl)phenoxy)-2-methylpropionic acid |
| 144 | 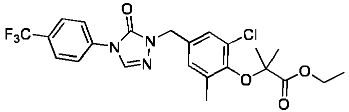 | Ethyl 2-(2-chloro-6-methyl-4-((5-oxo-4-(4-(trifluoromethyl)phenyl)-4,5-dihydro-1*H*-1,2,4-triazol-1-yl)methyl)phenoxy)-2-methylpropionate |
| 145 | 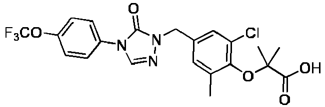 | 2-(2-Chloro-6-methyl-4-((5-oxo-4-(4-(trifluoromethoxy)phenyl)-4,5-dihydro-1*H*-1,2,4-triazol-1-yl)methyl)phenoxy)-2-methylpropionic acid |
| 146 | 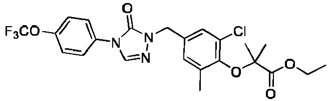 | Ethyl 2-(2-chloro-6-methyl-4-((5-oxo-4-(4-(trifluoromethoxy)phenyl)-4,5-dihydro-1*H*-1,2,4-triazol-1-yl)methyl)phenoxy)-2-methylpropionate |
| 147 | 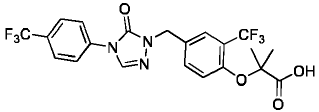 | 2-Methyl-2-(4-((5-oxo-4-(4-(trifluoromethyl)phenyl)-4,5-dihydro-1*H*-1,2,4-triazol-1-yl)methyl)-2-(trifluoromethyl)phenoxy)propionic acid |
| 148 | 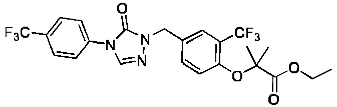 | Ethyl 2-methyl-2-(4-((5-oxo-4-(4-(trifluoromethyl)phenyl)-4,5-dihydro-1*H*-1,2,4-triazol-1-yl)methyl)-2-(trifluoromethyl)phenoxy)propionate |
| 149 | 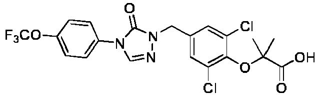 | 2-(2,6-Dichloro-4-((5-oxo-4-(4-(trifluoromethoxy)phenyl)-4,5-dihydro-1*H*-1,2,4-triazol-1-yl)methyl)phenoxy)-2-methylpropionic acid |
| 150 | 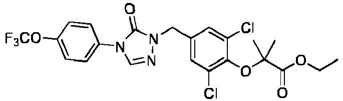 | Ethyl 2-(2,6-dichloro-4-((5-oxo-4-(4-(trifluoromethoxy)phenyl)-4,5-dihydro-1*H*-1,2,4-triazol-1-yl)methyl)phenoxy)-2-methylpropionate |
| 151 | 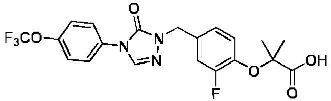 | 2-(2-Fluoro-4-((5-oxo-4-(4-(trifluoromethoxy)phenyl)-4,5-dihydro-1*H*-1,2,4-triazol-1-yl)methyl)phenoxy)-2-methylpropionic acid |
| 152 | 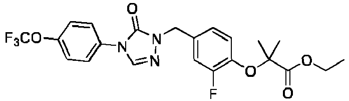 | Ethyl 2-(2-fluoro-4-((5-oxo-4-(4-(trifluoromethoxy)phenyl)-4,5-dihydro-1*H*-1,2,4-triazol-1-yl)methyl)phenoxy)-2-methylpropropioniate |
| 153 | 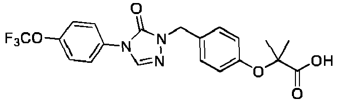 | 2-Methyl-2-(4-((5-oxo-4-(4-(trifluoromethoxy)phenyl)-4,5-dihydro-1*H*-1,2,4-triazol-1-yl)methyl)phenoxy)propionic acid |
| 154 | 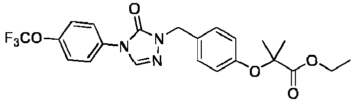 | Ethyl 2-methyl-2-(4-((5-oxo-4-(4-(trifluoromethoxy)phenyl)-4,5-dihydro-1*H*-1,2,4-triazol-1-yl)methyl)phenoxy)propionate |
| 155 | 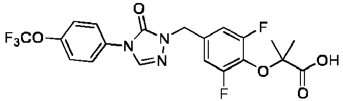 | 2-(2,6-Difluoro-4-((5-oxo-4-(4-(trifluoromethoxy)phenyl)-4,5-dihydro-1*H*-1,2,4-triazol-1-yl)methyl)phenoxy)-2-methylpropionic acid |
| 156 | 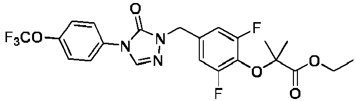 | Ethyl 2-(2,6-difluoro-4-((5-oxo-4-(4-(trifluoromethoxy)phenyl)-4,5-dihydro-1*H*-1,2,4-triazol-1-yl)methyl)phenoxy)-2-methylpropionate |
| 157 | 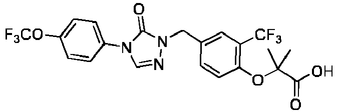 | 2-Methyl-2-(4-((5-oxo-4-(4-(trifluoromethoxy)phenyl)-4,5-dihydro-1*H*-1,2,4-triazol-1-yl)methyl)-2-(trifluoromethyl)phenoxy)propionic acid |
| 158 | 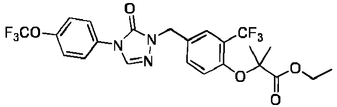 | Ethyl 2-methyl-2-(4-((5-oxo-4-(4-(trifluoromethoxy)phenyl)-4,5-dihydro-1*H*-1,2,4-triazol-1-yl)methyl)-2-(trifluoromethyl)phenoxy)propionate |
| 159 | 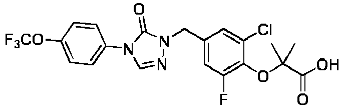 | 2-(2-Chloro-6-fluoro-4-((5-oxo-4-(4-(trifluoromethoxy)phenyl)-4,5-dihydro-1*H*-1,2,4-triazol-1-yl)methyl)phenoxy)-2-methylpropionic acid |
| 160 | 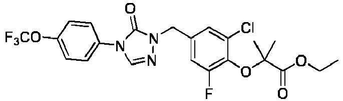 | Ethyl 2-(2-chloro-6-fluoro-4-((5-oxo-4-(4-(trifluoromethoxy)phenyl)-4,5-dihydro-1*H*-1,2,4-triazol-1-yl)methyl)phenoxy)-2-methylpropionate |
| 161 | 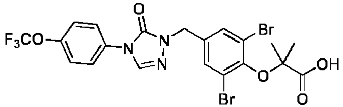 | 2-(2,6-Dibromo-4-((5-oxo-4-(4-(trifluoromethoxy)phenyl)-4,5-dihydro-1*H*-1,2,4-triazol-1-yl)methyl)phenoxy)-2-methylpropionic acid |
| 162 | 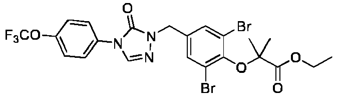 | Ethyl 2-(2,6-dibromo-4-((5-oxo-4-(4-(trifluoromethoxy)phenyl)-4,5-dihydro-1*H*-1,2,4-triazol-1-yl)methyl)phenoxy)-2-methylpropionate |
| 163 | 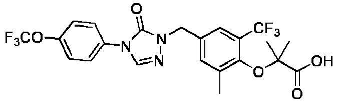 | 2-Methyl-2-(2-methyl-4-((5-oxo-4-(4-(trifluoromethoxy)phenyl)-4, 5-dihydro-1*H*-1,2,4-triazol-1-yl)methyl)-6-(trifluoromethyl)phenoxy)propionic acid |
| 164 | 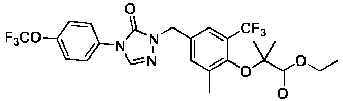 | Ethyl 2-methyl-2-(2-methyl-4-((5-oxo-4-(4-(trifluoromethoxy)phenyl)-4,5-dihydro-1*H*-1,2,4-triazol-1-yl)methyl)-6-(trifluoromethyl)phenoxy)propionate |
| 165 | 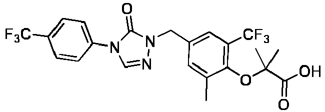 | 2-Methyl-2-(2-methyl-4-((5-oxo-4-(4-(trifluoromethyl)phenyl)-4,5-dihydro-1*H*-1,2,4-triazol-1-yl)methyl)-6-(trifluoromethyl)phenoxy)propionic acid |
| 166 | 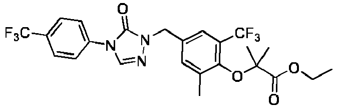 | Ethyl 2-methyl-2-(2-methyl-4-((5-oxo-4-(4-(trifluoromethyl)phenyl)-4,5-dihydro-1*H*-1,2,4-triazol-1-yl)methyl)-6-(trifluoromethyl)phenoxy)propionate |
| 167 | 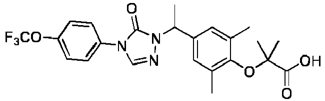 | 2-(2,6-Dimethyl-4-(1-(5-oxo-4-(4-(trifluoromethoxy)phenyl)-4,5-dihydro-1*H*-1,2,4-triazol-1-yl)ethyl)phenoxy)-2-methylpropionic acid |
| 168 | 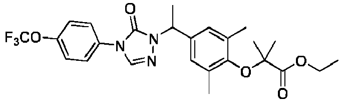 | Ethyl 2-(2,6-dimethyl-4-(1-(5-oxo-4-(4-(trifluoromethoxy)phenyl)-4,5-dihydro-1*H*-1,2,4-triazol-1-yl)ethyl)phenoxy)-2-methylpropionate |
| 169 | 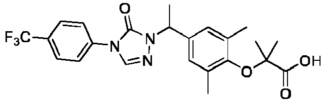 | 2-(2,6-Dimethyl-4-(1-(5-oxo-4-(4-(trifluoromethyl)phenyl)-4,5-dihydro-1*H*-1,2,4-triazol-1-yl)ethyl)phenoxy)-2-methylpropionic acid |
| 170 | 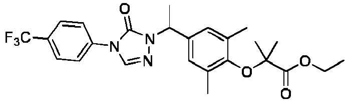 | Ethyl 2-(2,6-dimethyl-4-(1-(5-oxo-4-(4-(trifluoromethyl)phenyl)-4, 5-dihydro-1*H*-1,2,4-triazol-1-yl)ethyl)phenoxy)-2-methylpropionate |
| 171 | 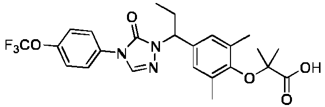 | 2-(2,6-Dimethyl-4-(1-(5-oxo-4-(4-(trifluoromethoxy)phenyl)-4,5-dihydro-1*H*-1,2,4-triazol-1-yl)propyl)phenoxy)-2-methylpropionic acid |
| 172 | 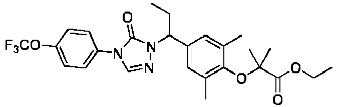 | Ethyl 2-(2,6-dimethyl-4-(1-(5-oxo-4-(4-(trifluoromethoxy)phenyl)-4,5-dihydro-1*H*-1,2,4-triazol-1-yl)propyl)phenoxy)-2-methylpropionate |
| 173 | 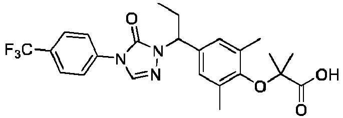 | 2-(2,6-Dimethyl-4-(1-(5-oxo-4-(4-(trifluoromethyl)phenyl)-4,5-dihydro-1*H*-1,2,4-triazol-1-yl)propyl)phenoxy)-2-methylpropionic acid |
| 174 | 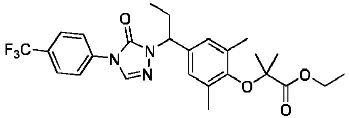 | Ethyl 2-(2,6-dimethyl-4-(1-(5-oxo-4-(4-(trifluoromethyl)phenyl)-4, 5-dihydro-1*H*-1,2,4-triazol-1-yl)propyl)phenoxy)-2-methylpropionate |
| 175 | 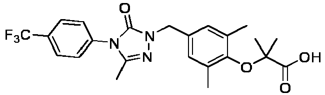 | 2-(2,6-Dimethyl-4-((3-methyl-5-oxo-4-(4-(trifluoromethyl)phenyl)-4,5-dihydro-1*H*-1,2,4-triazol-1-yl)methyl)phenoxy)-2-methylpropionic acid |
| 176 | 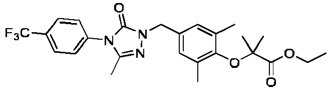 | Ethyl 2-(2,6-dimethyl-4-((3-methyl-5-oxo-4-(4-(trifluoromethyl)phenyl)-4,5-dihydro-1*H*-1,2,4-triazol-1-yl)methyl)phenoxy)-2-methylpropionate |
| 177 | 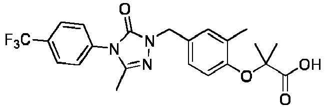 | 2-Methyl-2-(2-methyl-4-((3-methyl-5-oxo-4-(4-(trifluoromethyl)phenyl)-4,5-dihydro-1*H*-1,2,4-triazol-1-yl)methyl)phenoxy)propionic acid |
| 178 | 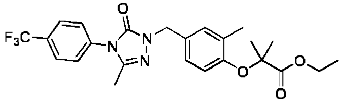 | Ethyl 2-methyl-2-(2-dimethyl-4-((3-methyl-5-oxo-4-(4-(trifluoromethyl)phenyl)-4,5-dihydro-1*H*-1,2,4-triazol-1-yl)methyl)phenoxy)propionate |
| 179 | 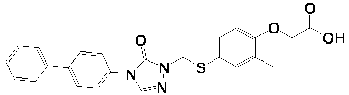 | 2-(4-(((4-([1,1'-Biphenyl]-4-yl)-5-oxo-4, 5-dihydro-1*H*-1,2,4-triazol-1-yl)methyl)thio)-2-methylphenoxy)acetic acid |
| 180 | 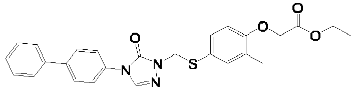 | Ethyl 2-(4-(((4-([1,1'-biphenyl]-4-yl)-5-oxo-4,5-dihydro-1*H*-1,2,4-triazol-1-yl)methyl)thio)-2-methylphenoxy)acetate |
| 181 | 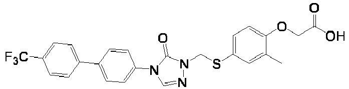 | 2-(2-Methyl-4-((((5-oxo-4-(4'-(trifluoromethyl)-[1,1'-biphenyl]-4-yl)-4,5-dihydro-1*H*-1,2,4-triazol-1-yl)methyl)thio)phenoxy)acetic acid |
| 182 | 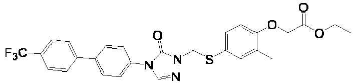 | Ethyl 2-(2-methyl-4-(((5-oxo-4-(4'-(trifluoromethyl)-[1,1'-biphenyl]-4-yl)-4,5-dihydro-1*H*-1,2,4-triazol-1-yl)methyl)thio)phenoxy)acetate |
| 183 | 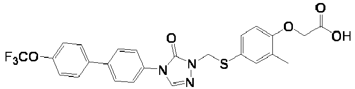 | 2-(2-Methyl-4-((((5-oxo-4-(4'-(trifluoromethoxy)-[1,1'-biphenyl]-4-yl)-4,5-dihydro-1*H*-1,2,4-triazol-1-yl)methyl)thio)phenoxy)acetic acid |
| 184 | 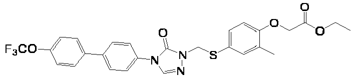 | Ethyl 2-(2-methyl-4-((((5-oxo-4-(4'-(trifluoromethoxy)-[1,1'-biphenyl]-4-yl)-4,5-dihydro-1*H*-1,2,4-triazol-1-yl)methyl)thio)phenoxy)acetate |
| 185 | 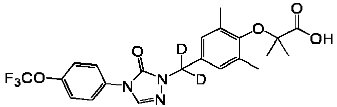 | 2-(2,6-Dimethyl-4-((5 -oxo-4-(4-(trifluoromethoxy)phenyl)-4,5-dihydro-1*H*-1,2,4-triazol-1-yl)methyl-*d*₂)phenoxy)-2-methylpropionic acid |
| 186 | 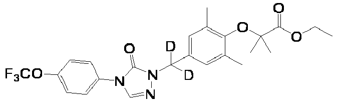 | Ethyl 2-(2,6-dimethyl-4-((5-oxo-4-(4-(trifluoromethoxy)phenyl)-4, 5-dihydro-1*H*-1,2,4-triazol-1-yl)methyl-*d*₂)phenoxy)-2-methylpropionate |
| 187 | 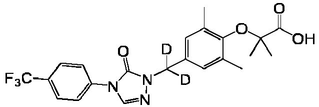 | 2-(2,6-Dimethyl-4-((5-oxo-4-(4-(trifluoromethyl)phenyl)-4,5-dihydro-1*H*-1,2,4-triazol-1-yl)methyl-*d*₂)phenoxy)-2-methylpropionic acid |
| 188 | 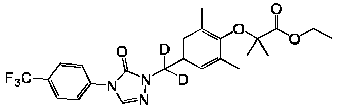 | Ethyl 2-(2,6-dimethyl-4-((5-oxo-4-(4-(trifluoromethyl)phenyl)-4, 5-dihydro-1*H*-1,2,4-triazol-1-yl)methyl-*d*₂)phenoxy)-2-methylpropionate |
| 189 | 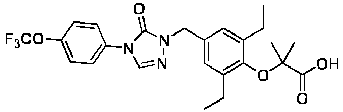 | 2-(2,6-Diethyl-4-((5-oxo-4-(4-(trifluoromethoxy)phenyl)-4,5-dihydro-1*H*-1,2,4-triazol-1-yl)methyl)phenoxy)-2-methylpropionic acid |
| 190 | 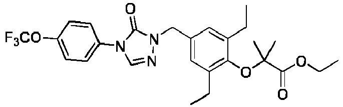 | Ethyl 2-(2,6-diethyl-4-((5-oxo-4-(4-(trifluoromethoxy)phenyl)-4,5-dihydro-1*H*-1,2,4-triazol-1-yl)methyl)phenoxy)-2-methylpropionate |
| 191 | 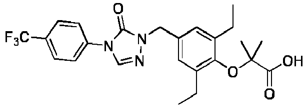 | 2-(2,6-Diethyl-4-((5-oxo-4-(4-(trifluoromethyl)phenyl)-4,5-dihydro-1*H*-1,2,4-triazol-1-yl)methyl)phenoxy)-2-methylpropionic acid |
| 192 | 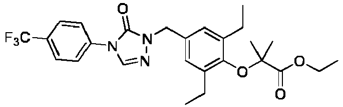 | Ethyl 2-(2,6-diethyl-4-((5-oxo-4-(4-(trifluoromethyl)phenyl)-4,5-dihydro-1*H*-1,2,4-triazol-1-yl)methyl)phenoxy)-2-methylpropionate |
| 193 | 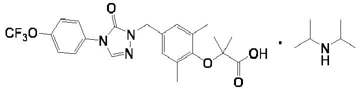 | 2-(2,6-Dimethyl-4-((5 -oxo-4-(4-(trifluoromethoxy)phenyl)-4,5-dihydro-1*H*-1,2,4-triazol-1-yl)methyl)phenoxy)-2-methylpropionic acid diisopropylamine salt |
| 194 | 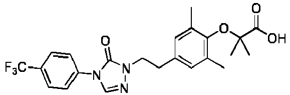 | 2-(2,6-Dimethyl-4-(2-(5-oxo-4-(4-(trifluoromethyl)phenyl)-4,5-dihydro-1*H*-1,2,4-triazol-1-yl)ethyl)phenoxy)-2-methylpropionic acid |
| 195 | 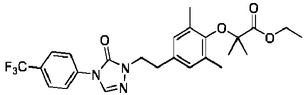 | Ethyl 2-(2,6-dimethyl-4-(2-(5-oxo-4-(4-(trifluoromethyl)phenyl)-4, 5-dihydro-1*H*-1,2,4-triazol-1-yl)ethyl)phenoxy)-2-methylpropionate |
| 196 | 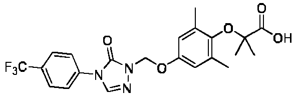 | 2-(2,6-Dimethyl-4-((5 -oxo-4-(4-(trifluoromethyl)phenyl)-4,5-dihydro-1*H*-1,2,4-triazol-1-yl)methoxy)phenoxy)-2-methylpropionic acid |
| 197 | 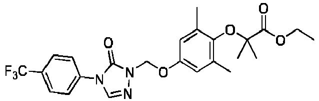 | Ethyl 2-(2,6-dimethyl-4-((5-oxo-4-(4-(trifluoromethyl)phenyl)-4, 5-dihydro-1*H*-1,2,4-triazol-1-yl)methoxy)phenoxy)-2-methylpropionate |
| 198 | 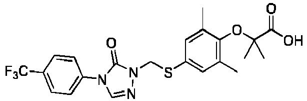 | 2-(2,6-Dimethyl-4-(((5-oxo-4-(4-(trifluoromethyl)phenyl)-4,5-dihydro-1*H*-1,2,4-triazol-1-yl)methyl)thio)phenoxy)-2-methylpropionic acid |
| 199 | 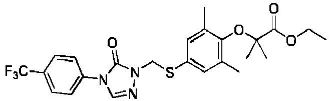 | Ethyl 2-(2,6-dimethyl-4-(((5-oxo-4-(4-(trifluoromethyl)phenyl)-4,5-dihydro-1*H*-1,2,4-triazol-1-yl)methyl)thio)phenoxy)-2-methylpropionate |
| 200 | 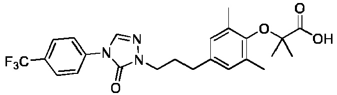 | 2-(2,6-Dimethyl-4-(3-(5-oxo-4-(4-(trifluoromethyl)phenyl)-4,5-dihydro-1*H*-1,2,4-triazol-1-yl)propyl)phenoxy)-2-methylpropionic acid |
| 201 | 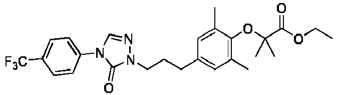 | Ethyl 2-(2,6-dimethyl-4-(3-(5-oxo-4-(4-(trifluoromethyl)phenyl)-4,5-dihydro-1*H*-1,2,4-triazol-1-yl)propyl)phenoxy)-2-methylpropionate |
| 202 | 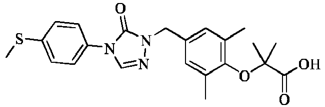 | 2-(2,6-Dimethyl-4-((4-(4-(methylthio)phenyl)-5-oxo-4,5-dihydro-1*H*-1,2,4-triazol-1-yl)methyl)phenoxy)-2-methylpropionic acid |
| 203 | 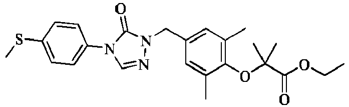 | Ethyl 2-(2,6-dimethyl-4-((4-(4-(methylthio)phenyl)-5-oxo-4,5-dihydro-1*H*-1,2,4-triazol-1-yl)methyl)phenoxy)-2-methylpropionate |
| 204 | 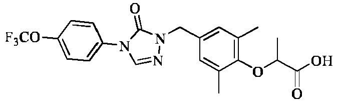 | 2-(2,6-Dimethyl-4-((5-oxo-4-(4-(trifluoromethoxy)phenyl)-4,5-dihydro-1*H*-1,2,4-triazol-1-yl)methyl)phenoxy)propionic acid |
| 205 | 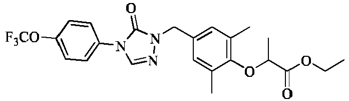 | Ethyl 2-(2,6-dimethyl-4-((5-oxo-4-(4-(trifluoromethoxy)phenyl)-4, 5-dihydro-1*H*-1,2,4-triazol-1-yl)methyl)phenoxy)propionate |
| 206 | 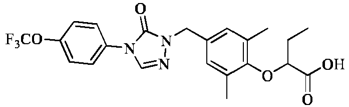 | 2-(2,6-Dimethyl-4-((5-oxo-4-(4-(trifluoromethoxy)phenyl)-4,5-dihydro-1*H*-1,2,4-triazol-1-yl)methyl)phenoxy)butyric acid |
| 207 | 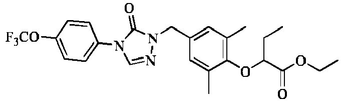 | Ethyl 2-(2,6-dimethyl-4-((5-oxo-4-(4-(trifluoromethoxy)phenyl)-4, 5-dihydro-1*H*-1,2,4-triazol-1-yl)methyl)phenoxy)butyrate |
| 208 | 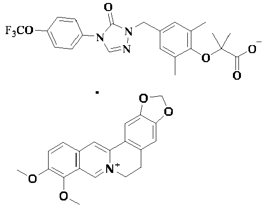 | 2-(2,6-Dimethyl-4-((5 -oxo-4-(4-(trifluoromethoxy)phenyl)-4,5-dihydro-1*H*-1,2,4-triazol-1-yl)methyl)phenoxy)-2-methylpropionic acid berberine salt |

5. The triazolone compound or the pharmaceutically acceptable salt or the solvate thereof according to any one of claims 1-3, wherein the pharmaceutically acceptable salt of the triazolone compound comprises a salt formed by the triazolone compound and metal ions or pharmaceutically acceptable amine or ammonium ions.

6. Use of the triazolone compound or the pharmaceutically acceptable salt, the tautomer, the mesomer, the racemate, the stereoisomer, the metabolite, the metabolic precursor, the prodrug or the solvate thereof according to any one of claims 1-3 in the preparation of a PPARα/δ dual agonist.

7. Use of the triazolone compound or the pharmaceutically acceptable salt, the tautomer, the mesomer, the racemate, the stereoisomer, the metabolite, the metabolic precursor, the prodrug or the solvate thereof according to any one of claims 1-3 as a PPARα/δ dual agonist in the preparation of a medicament for preventing or treating diseases mediated by PPARα and/or PPARδ.

8. The use according to claim 7, wherein the diseases mediated by PPARα and/or PPARδ comprise metabolic diseases, cardiovascular and cerebrovascular diseases, inflammatory diseases, autoimmune diseases, organ fibrosis diseases, neurological injury diseases, secondary diseases caused by pathogen infections, mitochondrial dysfunction and disorder diseases, or tumors.

9. A pharmaceutical composition for preventing or treating diseases mediated by PPARα and/or PPARδ, comprising the triazolone compound or the pharmaceutically acceptable salt, the tautomer, the mesomer, the racemate, the stereoisomer, the metabolite, the metabolic precursor, the prodrug or the solvate thereof according to any one of claims 1-3 as an active ingredient and a pharmaceutically acceptable carrier.

10. The pharmaceutical composition according to claim 9, wherein the pharmaceutical composition is a capsule, a powder, a tablet, a granule, a pill, an injection, a syrup, an oral liquid, an inhalant, an ointment, a suppository, or a patch.
